# EUROPÄISCHE PATENTSCHRIFT

(11) **EP 0 648 749 B1**
(45) Veröffentlichungstag und Bekanntmachung des Hinweises auf die Patenterteilung: **10.12.1997**
(21) Anmeldenummer: 94112295.4
(22) Anmeldetag: 05.08.1994
(51) Int. Cl.: C07D 239/54, C07D 239/52, C07C 265/12, C07C 311/53, A01N 43/54

(54) **N-Cyanoaryl-Stickstoffheterocyclen**
N-cyanoaryl nitrogencontaining heterocycles
N-cyanoaryle hétérocycles azotés

(30) Priorität: 18.08.1993 DE 4327743; 08.04.1994 DE 4412079
(43) Veröffentlichungstag der Anmeldung: 19.04.1995
(73) Patentinhaber: BAYER AG, 51368 Leverkusen (DE)
(72) Erfinder: Andree, Roland, Dr., D-40764 Langenfeld (DE); Drewes, Mark Wilhelm, Dr., D-40764 Langenfeld (DE); Marhold, Albrecht, Dr., D-51373 Leverkusen (DE); Santel, Hans-Joachim, Dr., D-51371 Leverkusen (DE); Dollinger, Markus, Dr., D-51381 Leverkusen (DE)

(56) Entgegenhaltungen:
- EP-A- 0 255 047
- EP-A- 0 438 209
- EP-A- 0 563 384
- DE-A- 3 641 825
- GB-A- 1 336 871
- US-A- 3 539 616
- US-A- 5 084 084

## Beschreibung

Die Erfindung betrifft neue N-Cyanoaryl-Stickstoffheterocyclen, mehrere Verfahren zu ihrer Herstellung, ihre Verwendung als Herbizide sowie neue Zwischenprodukte.

Es ist bereits bekannt, daß bestimmte N-Cyanoaryl-Stickstoffheterocyclen herbizide Eigenschaften aufweisen (vgl. WO 91/00278, WO 92/11244, DE 4237920, EP 408382/US 5084084, EP 438209, EP 473551).

Die herbizide Wirkung bzw. die Verträglichkeit der vorbekannten N-Cyanoaryl-Stickstoffheterocyclen gegenüber Kulturpflanzen sind jedoch nicht ganz zufriedenstellend.

Es wurden nun die neuen N-Cyanoaryl-Stickstoffheterocyclen der allgemeinen Formel (I) gefunden, 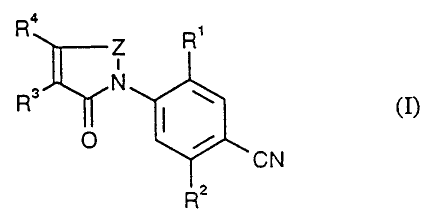 in welcher
- R¹: für Fluor steht,
- R²: für Fluor, Chlor, Brom, Cyano oder die Gruppierung -N(A¹)SO₂A steht, worin
- A: für einen jeweils gegebenenfalls durch Fluor, Chlor, Brom, Cyano oder C₁-C₄-Alkoxy substituierten Rest der Reihe Alkyl, Alkenyl oder Alkinyl mit jeweils bis zu 10 Kohlenstoffatomen steht,
- A: weiterhin für jeweils gegebenenfalls durch Fluor, Chlor, Brom, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht,
- A: weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl, Phenyloxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und 1 bis 4 Kohlenstoffatomen im Alkylteil steht,
- A: weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl, C₁-C₄-Alkylsulfonyl oder C₁-C₄-Alkoxycarbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Heterocyclyl oder Heterocyclylalkyl mit 2 bis 6 Kohlenstoffatomen und 1 bis 4 Stickstoffatomen und/oder 1 bis 2 Sauerstoff- oder Schwefelatomen im gesättigten oder ungesättigten Heterocyclylteil und gegebenenfalls 1 bis 4 Kohlenstoffatomen im Alkylteil steht, und
- A¹: für Wasserstoff oder für jeweils gegebenenfalls durch Halogen oder C₁-C₄-Alkoxy substituiertes Alkyl, Alkenyl, Alkinyl, Alkoxy, Alkylcarbonyl oder Alkoxycarbonyl mit jeweils bis zu 6 Kohlenstoffatomen, oder für Phenylcarbonyl, Naphthylcarbonyl, Phenylmethylcarbonyl, Naphthylmethylcarbonyl, Phenoxycarbonyl oder Naphthyloxycarbonyl (welche jeweils gegebenenfalls durch Fluor, Chlor, Brom, Cyano, Methyl, Methoxy, Trifluormethyl und/oder Trifluormethoxy substituiert sind) steht,
- R³: für Wasserstoff, Fluor, Chlor, Brom, Cyano oder für gegebenenfalls durch Fluor und/oder Chlor substituiertes Alkyl mit 1 bis 6 Kohlenstoffatomen steht,
- R⁴: für gegebenenfalls durch Fluor, Chlor, Brom, Methoxy oder Ethoxy substituiertes Alkyl mit 1 bis 6 Kohlenstoffatomen steht,
oder zusammen mit R³ für Alkandiyl mit 2 bis 8 Kohlenstoffatomen steht, und
- Z: für eine der nachstehenden Gruppierungen steht 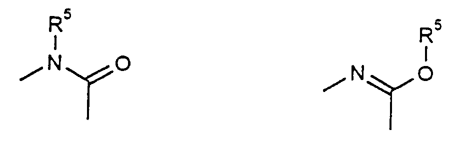 worin
- R⁵: für Amino oder durch Cyano, C₁-C₄-Alkoxy, C₁-C₄-Alkylcarbonyl oder C₁-C₄-Alkoxy-carbonyl substituiertes Alkyl mit bis zu 6 Kohlenstoffatomen steht,
oder
- R⁵: für jeweils gegebenenfalls durch Fluor, Chlor, Brom, Cyano, C₁-C₄-Alkoxy, C₁-C₄-Alkyl-carbonyl oder C₁-C₄-Alkoxycarbonyl substituiertes Alkenyl, Alkinyl, Alkylcarbonyl oder Alkoxycarbonyl mit jeweils bis zu 6 Kohlenstoffatomen steht, sowie deren Salze mit Basen, wie z.B. Natrium-, Kalium- oder Calcium-hydroxid, -hydrid, -amid oder -carbonat, Natrium- oder Kalium-C₁-C₄-alkanolaten, Ammoniak, C₁-C₄-Alkylamino, Di-(C₁-C₄-alkyl)-aminen, Tri-(C₁-C₄-alkyl)-aminen oder auch mit Tris-(2-hydroxyethyl)amin.

Die allgemeine Formel (I) steht also für die isomeren Verbindungen der nachstehenden allgemeinen Formeln (IA) und (IB)

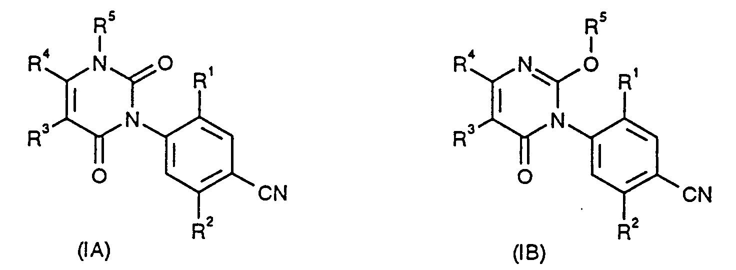

Man erhält die neuen N-Cyanoaryl-Stickstoffheterocyclen der allgemeinen Formel (I) sowie deren Salze, wenn man
(a) zur Herstellung von Verbindungen der Formeln (IA) und/oder (IB), in welchen R⁵ für Cyano, C₁-C₄-Alkoxy, C₁-C₄-Alkylcarbonyl oder C₁-C₄-Alkoxycarbonyl substituiertes Alkyl mit bis zu 6 Kohlenstoffatomen oder für jeweils gegebenenfalls durch Fluor, Chlor, Brom, Cyano, C₁-C₄-Alkoxy, C₁-C₄-Alkylcarbonyl oder C₁-C₄-Alkoxycarbonyl substituiertes Alkenyl, Alkinyl, Alkylcarbonyl oder Alkoxycarbonyl mit jeweils bis zu 6 Kohlenstoffatomen steht sowie R¹, R², R³ und R⁴ die oben angegebenen Bedeutungen haben,
   N-Cyanoaryl-Stickstoffheterocyclen der allgemeinen Formeln (IA) oder (IB)
   in welchen R⁵ für Wasserstoff steht sowie R¹, R², R³ und R⁴ die oben angegebenen Bedeutungen haben,
   mit Alkylierungsmitteln bzw. Acylierungsmitteln der allgemeinen Formeln (V) oder (VI)

   X-R⁵ (V)

   R⁵-O-SO₂-O-R⁵ (VI)

   in welchen R⁵ die oben angegebene Bedeutung hat und X in der Formel (V) für Halogen steht,
   gegebenenfalls in Gegenwart eines Säureakzeptors und gegebenenfalls in Gegenwart eines Verdünnungsmittels umsetzt,
   oder daß man
(b) zur Herstellung von Verbindungen der Formel (I), in welcher R² für die Gruppierung -N(A¹)SO₂A steht sowie A, A¹, R¹, R³ R⁴ und Z die oben angegebenen Bedeutungen haben,
   N-Cyanoaryl-Stickstoffheterocyclen der allgemeinen Formel (I), in welcher R² für Fluor, Chlor, Brom steht sowie R¹, R³, R⁴ und Z die oben angegebenen Bedeutungen haben,
   mit Ammoniak oder mit Amiden der allgemeinen Formel (VII)

   HN(A¹)SO₂A (VII)

   in welcher
   - A und A¹: die oben angegebenen Bedeutungen haben,
   gegebenenfalls in Gegenwart eines Säureakzeptors und gegebenenfalls in Gegenwart eines Verdünnungsmittels umsetzt.

Die Verbindungen der Formel (IA), in welcher R⁵ für Amino steht, können auf bekannte Weise durch Umsetzung entsprechender Verbindungen der Formel (IA), in welcher R⁵ für Wasserstoff steht, mit Aminierungsmitteln, wie z.B. 1-Aminooxy-2,4-dinitro-benzol (ADNB), gemäß nachstehendem Formelschema hergestellt werden (vgl. EP 476697, Example 4):

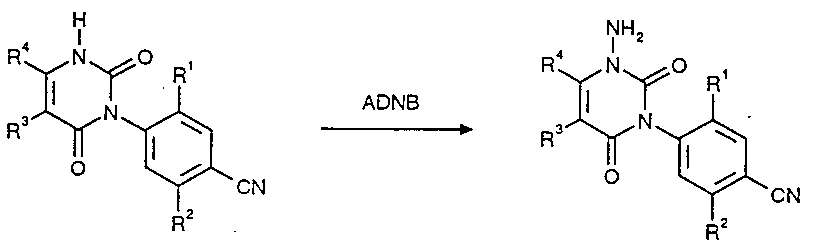

Die neuen N-Cyanoaryl-Stickstoffheterocyclen der allgemeinen Formel (I) sowie deren Salze zeichnen sich durch starke herbizide Wirksamkeit aus.

In den Definitionen sind die gesättigten oder ungesättigten Kohlenwasserstoffketten, wie Alkyl, Alkandiyl, Alkenyl oder Alkinyl, jeweils geradkettig oder verzweigt.

Die Erfindung betrifft insbesondere Verbindungen der Formel (I), in welcher
- R¹: für Fluor steht,
- R²: für Fluor, Chlor, Brom, Cyano oder die Gruppierung -N(A¹)SO₂A steht, worin
- A: für jeweils gegebenenfalls durch Fluor oder Chlor substituiertes Methyl, Ethyl, n- oder i-Propyl, n-, i-, s- oder t-Butyl, n-, i-, s- oder t-Pentyl steht,
- A: weiterhin für jeweils gegebenenfalls durch Fluor, Chlor, Brom, Methyl und/oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
- A: weiterhin für jeweils gegebenenfalls durch Fluor, Chlor, Brom, Cyano, Nitro, Carboxy, Methyl, Trifluormethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl, Dimethylaminosulfonyl, Methoxycarbonyl, Ethoxycarbonyl, Propoxycarbonyl oder Phenyl substituiertes Phenyl, Naphthyl, Phenylmethyl oder Phenylethyl steht,
- A: weiterhin für jeweils gegebenenfalls durch Fluor, Chlor, Brom, Cyano, Methyl, Ethyl, Trifluormethyl, Methoxy, Ethoxy, Methylthio, Ethylthio, Methylsulfinyl, Ethylsulfinyl, Methylsulfonyl, Ethylsulfonyl, Methoxycarbonyl oder Ethoxycarbonyl substituiertes Thienyl, Pyrazolyl oder Pyridyl steht, und
- A¹: für Wasserstoff, für jeweils gegebenenfalls durch Fluor, Chlor, Methoxy oder Ethoxy substituiertes Methyl, Ethyl, n- oder i-Propyl, n-, i- oder s-Butyl, Propenyl, Butenyl, Propinyl, Butinyl, Methoxy, Ethoxy, n- oder i-Propoxy, n-, i-, s- oder t-Butoxy, Acetyl, Propionyl, Methoxycarbonyl oder Ethoxycarbonyl, für Phenylcarbonyl oder Phenoxycarbonyl steht,
- R³: für Wasserstoff, Fluor, Chlor, Brom, Methyl, Trifluormethyl, Ethyl, n- oder i-Propyl steht,
- R⁴: für jeweils gegebenenfalls durch Fluor oder Chlor substituiertes Methyl, Ethyl, n- oder i-Propyl steht,
oder zusammen mit R³ für Trimethylen oder Tetramethylen steht, und
- Z: für eine der nachstehenden Gruppierungen steht 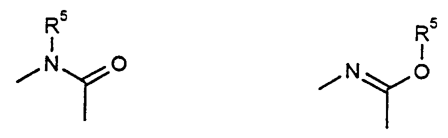 worin
- R⁵: für Amino oder für Cyano substituiertes Methyl, Ethyl, n- oder i-Propyl, n-, i- oder s-Butyl steht,
oder
- R⁵: für jeweils gegebenenfalls durch Fluor, Chlor oder Cyano substituiertes Propenyl, Butenyl, Propinyl, Butinyl, Acetyl, Propionyl, Methoxycarbonyl oder Ethoxycarbonyl steht,
sowie deren Salze mit Basen, wie z.B. Natrium-, Kalium- oder Calcium-hydroxid, -hydrid, -amid oder -carbonat, Natrium- oder Kalium-C₁-C₄-alkanolaten, Ammoniak, C₁-C₄-Alkylaminen, Di-(C₁-C₄-alkyl)-aminen, Tri-(C₁-C₄-alkyl)-aminen oder auch mit Tris-(2-hydroxyethyl)amin.

Eine ganz besonders bevorzugte Gruppe von Verbindungen der Formel (I) sind die Verbindungen der Formel (IA), in welcher R¹, R², R³, R⁴ und R⁵ die oben als insbesondere bevorzugt angegebene Bedeutung haben.

Die oben aufgeführten allgemeinen oder in Vorzugsbereichen angegebenen Restedefinitionen gelten sowohl für die Endprodukte der Formel (I) als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Beispiele für die erfindungsgemäßen Verbindungen der Formel (I) sind in der nachstehenden Tabelle 1 aufgeführt.

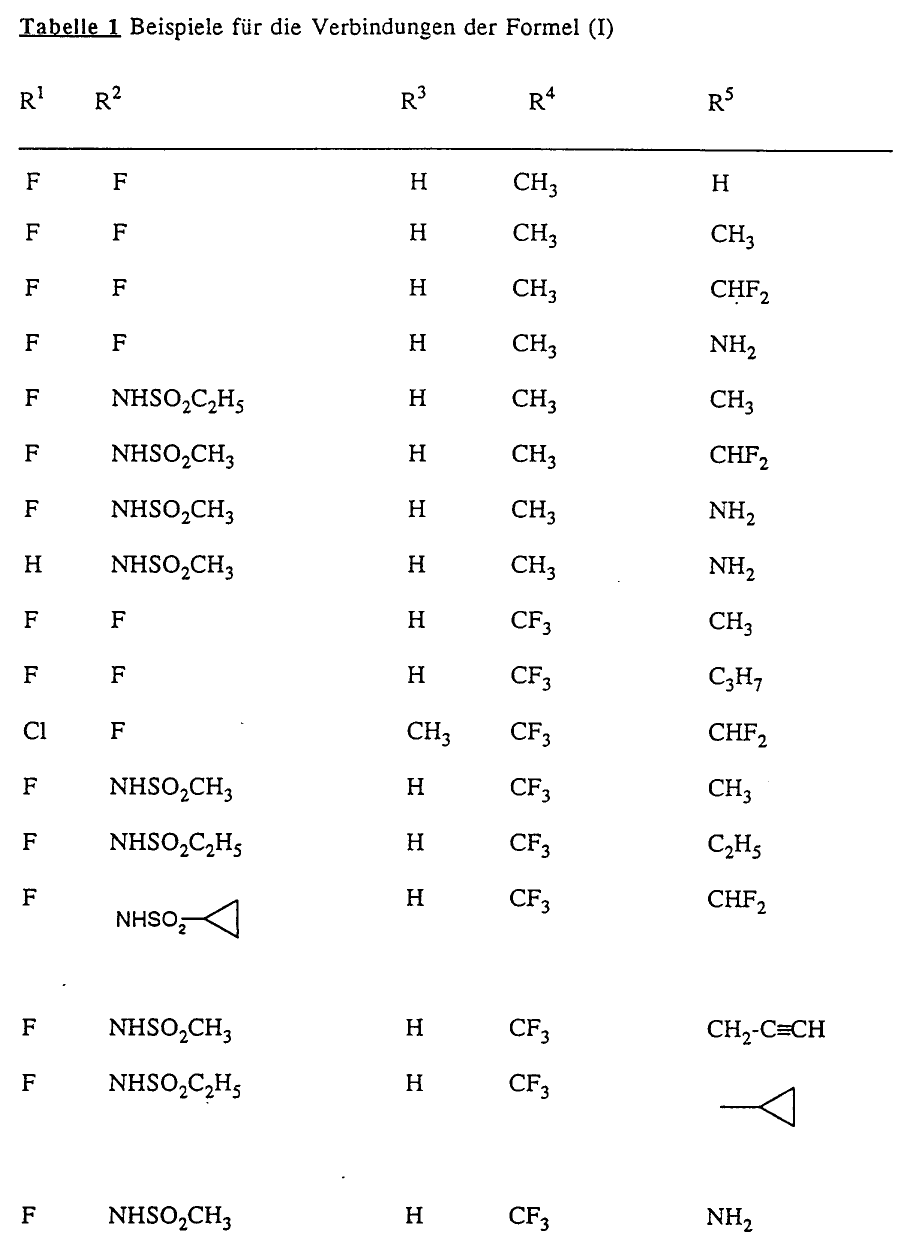

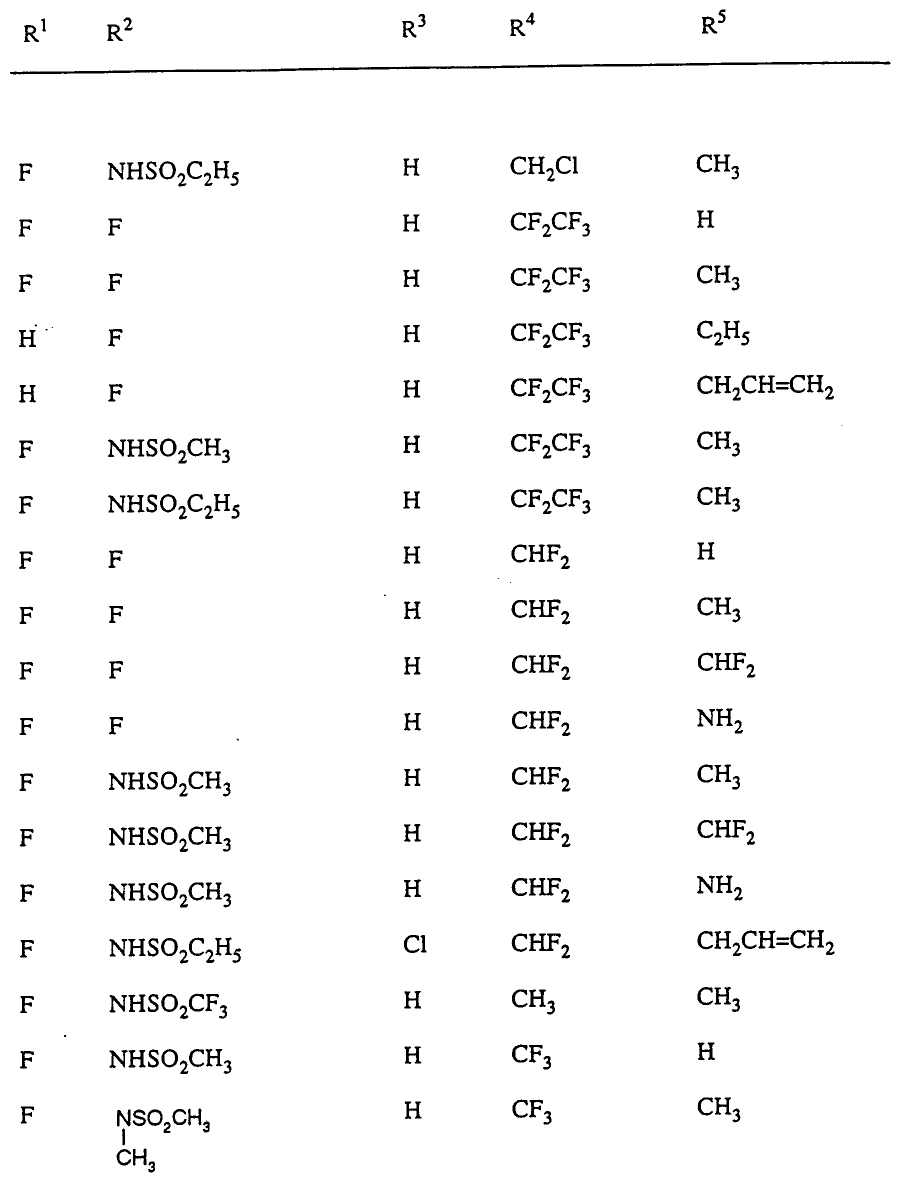

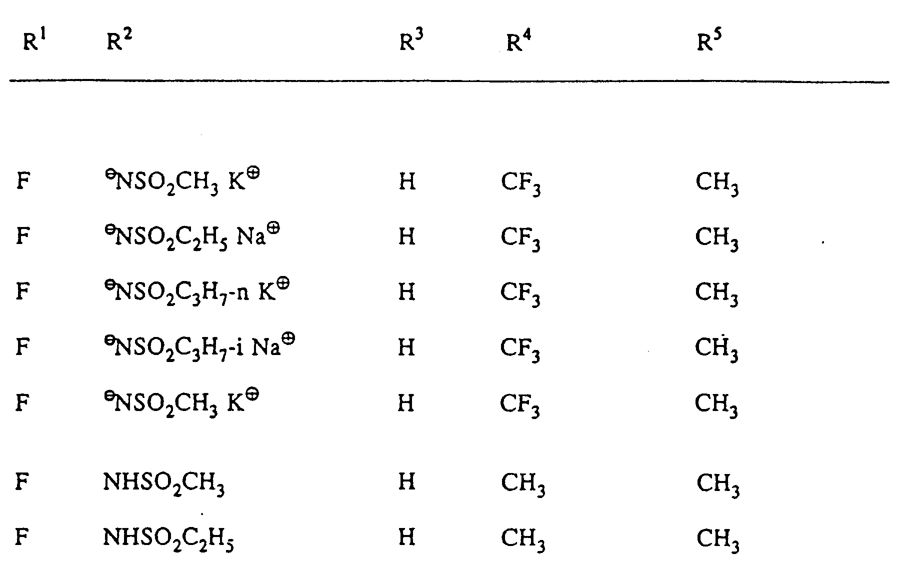

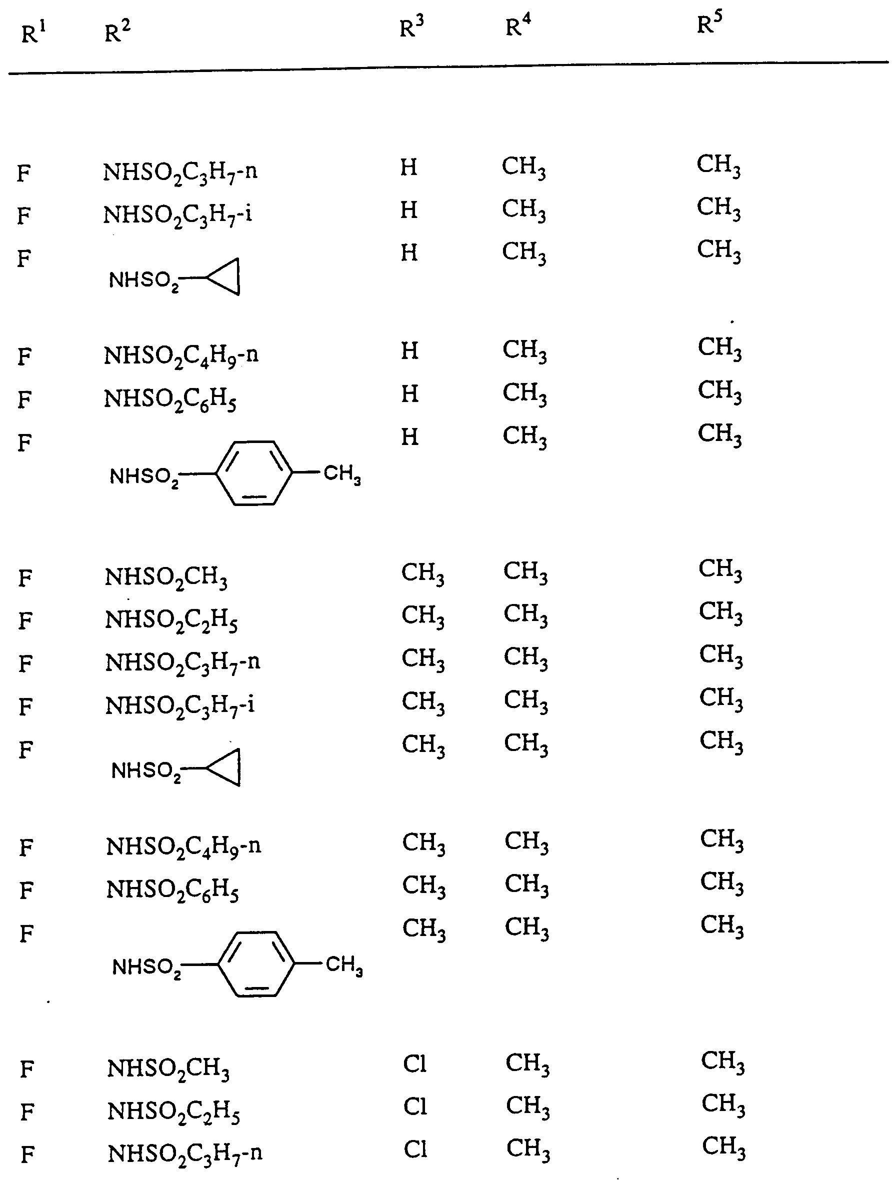

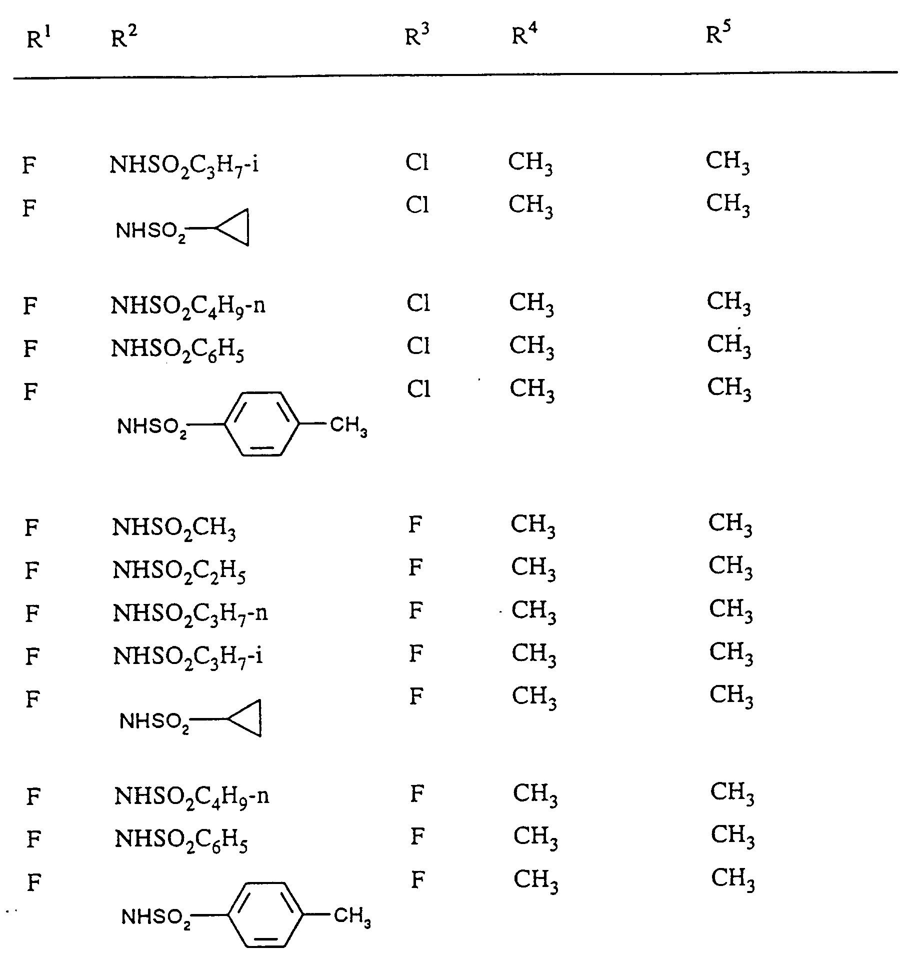

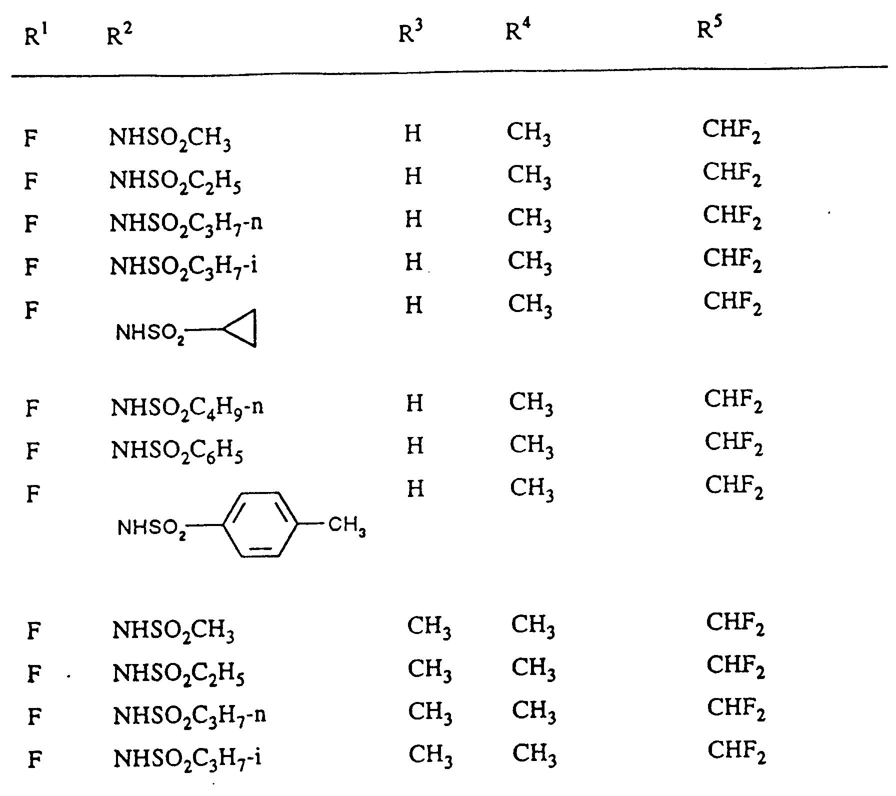

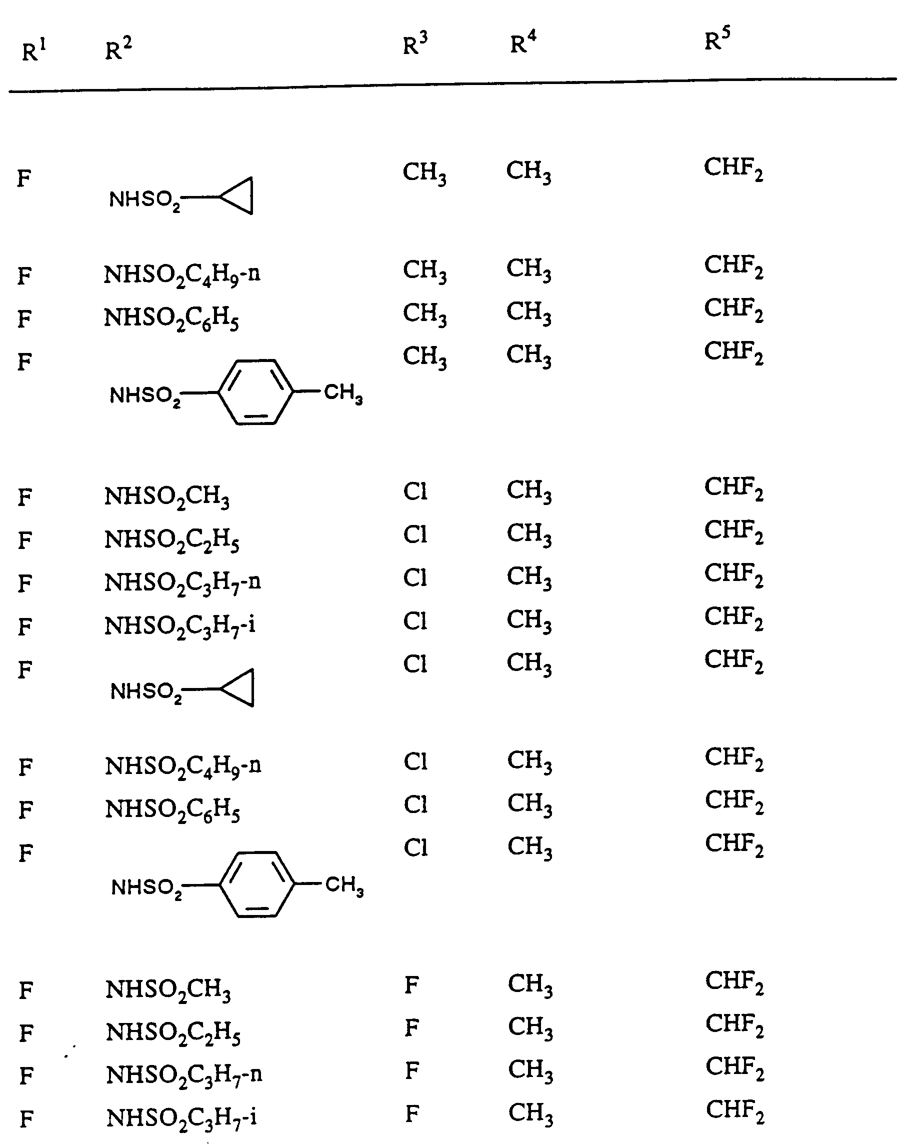

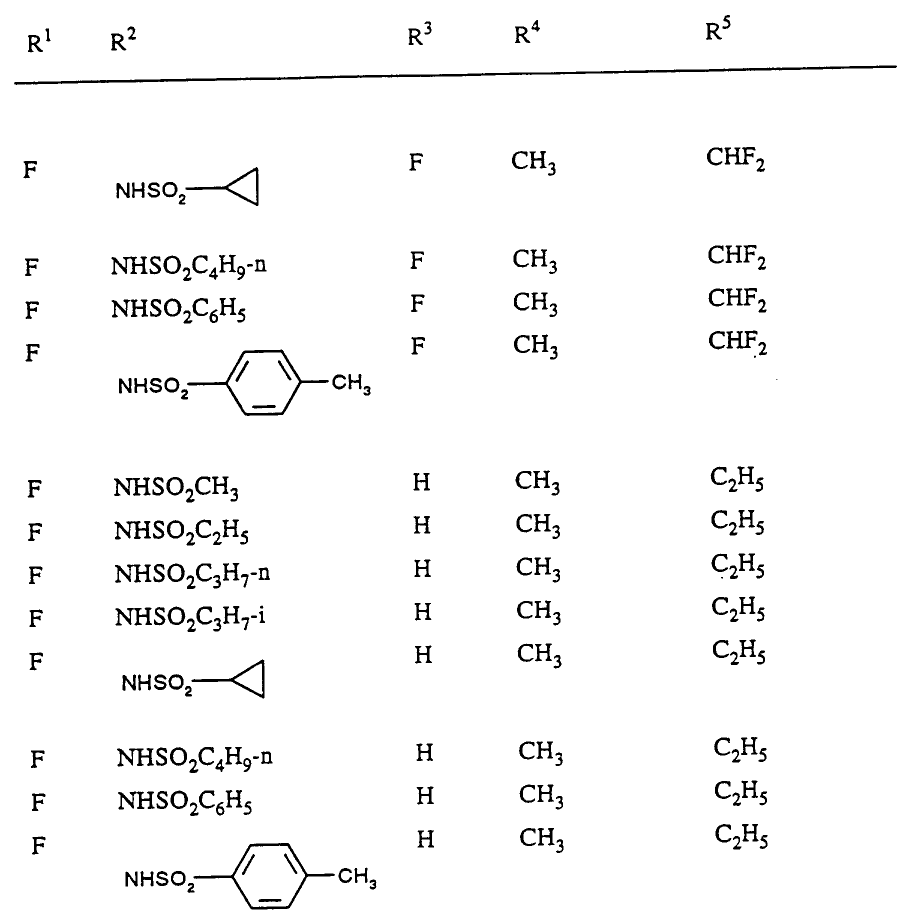

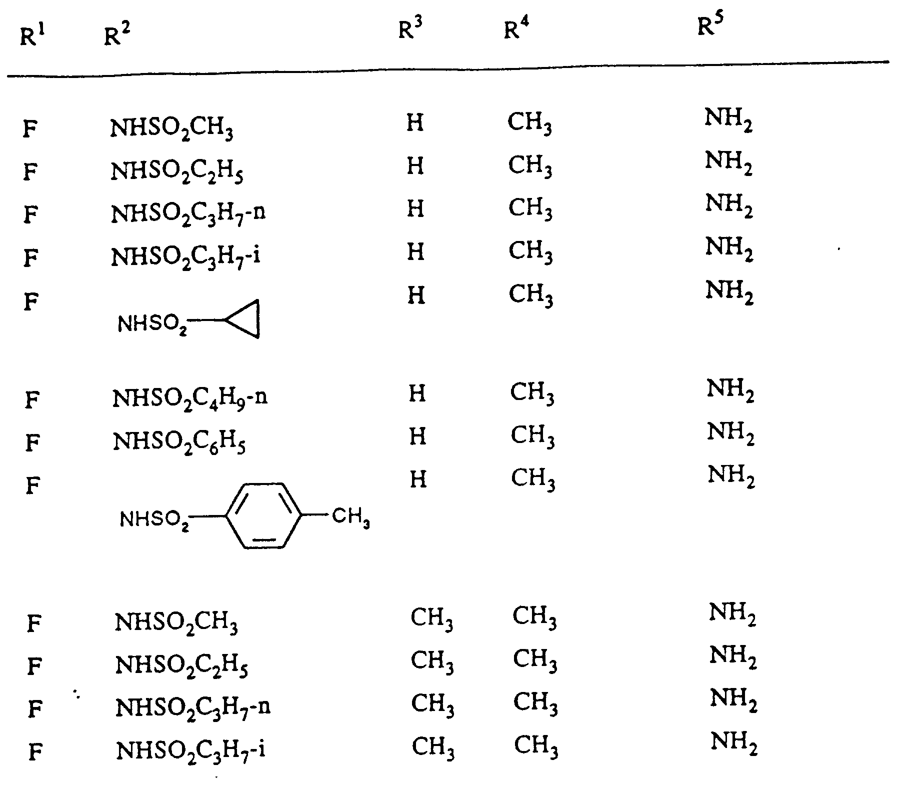

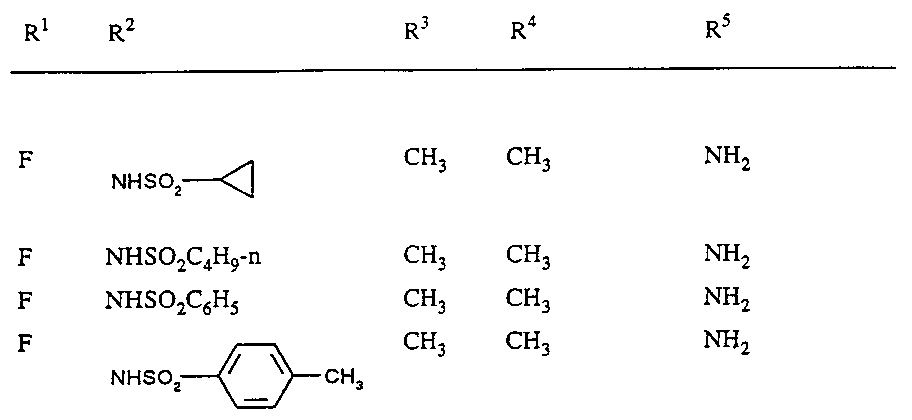

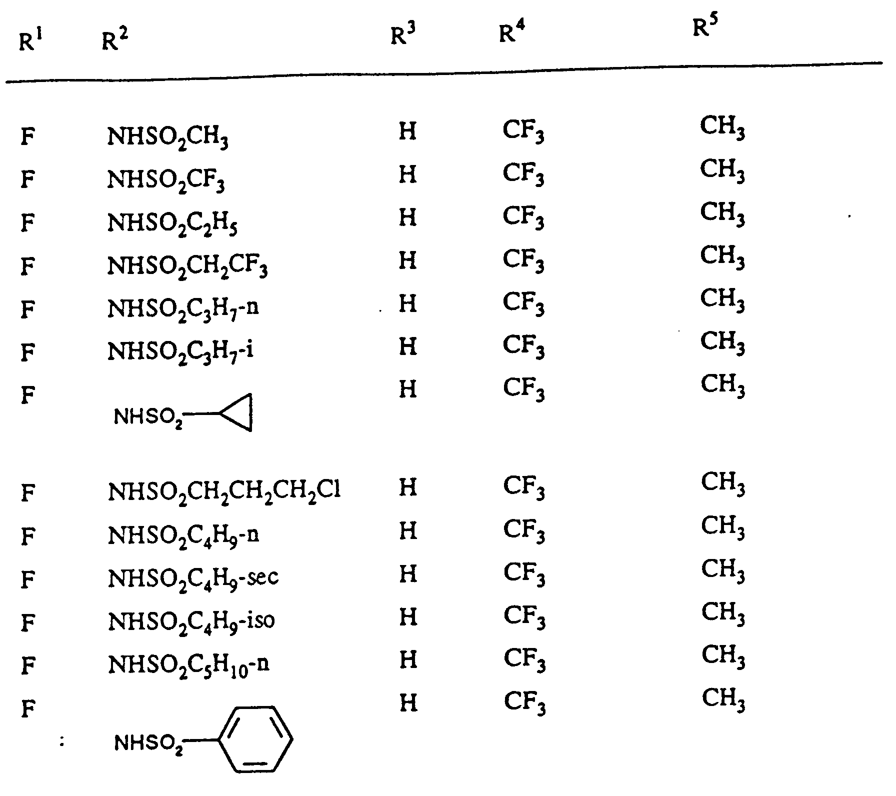

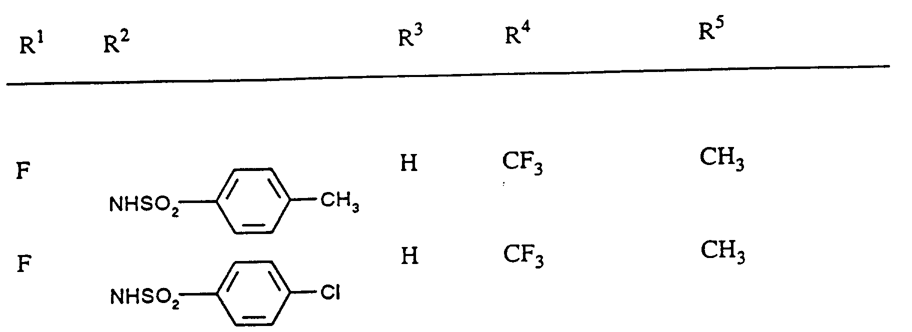

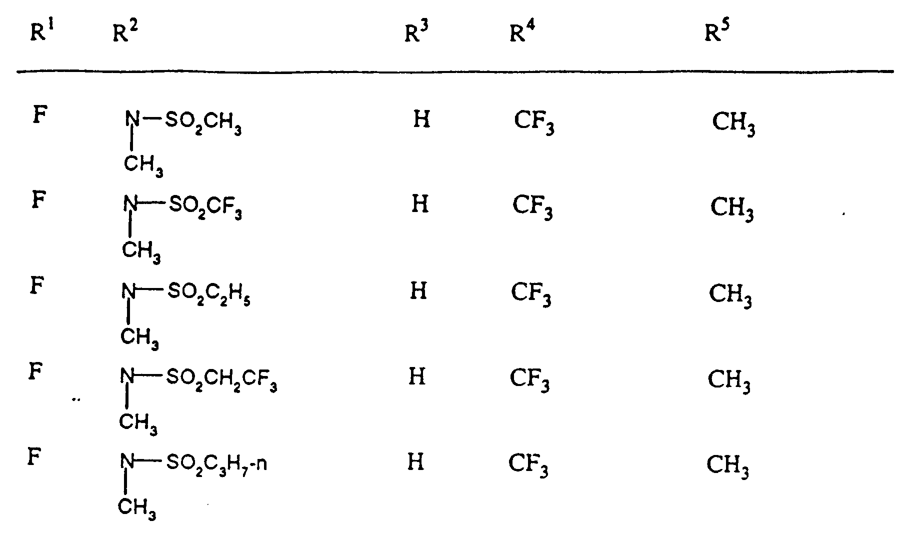

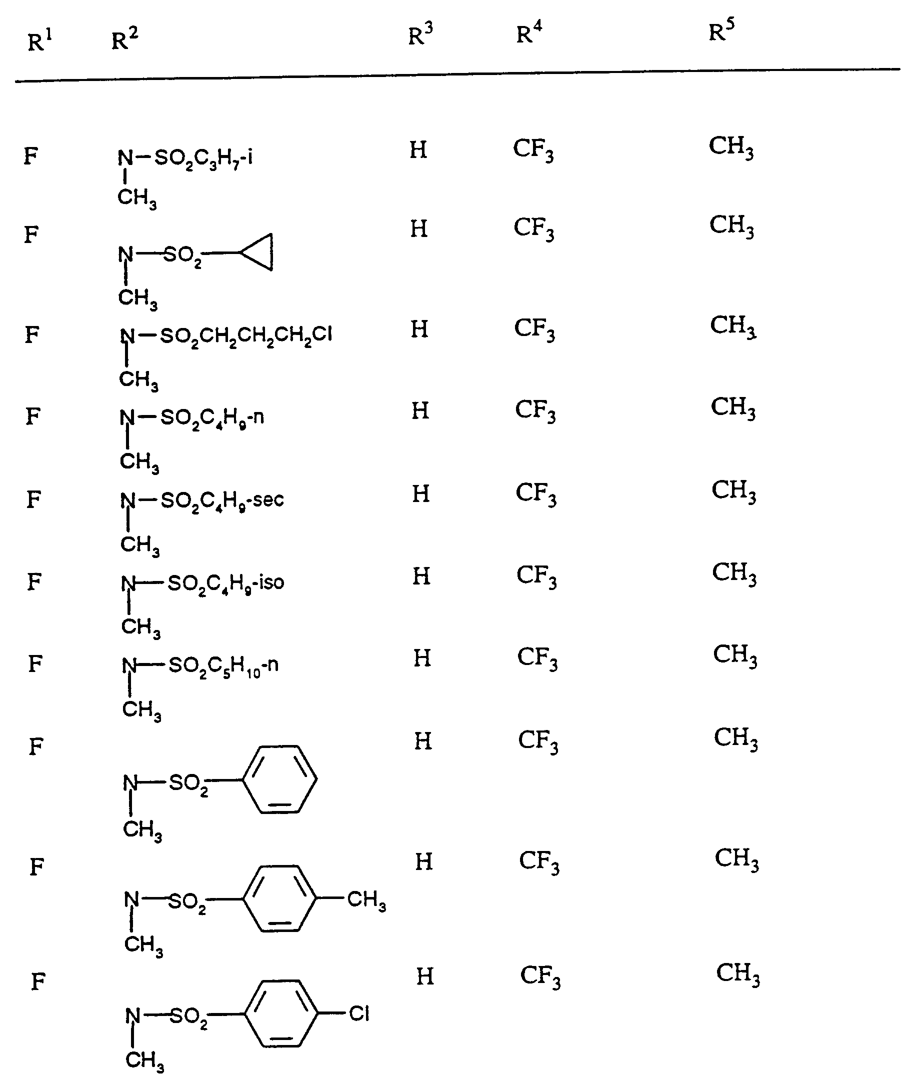

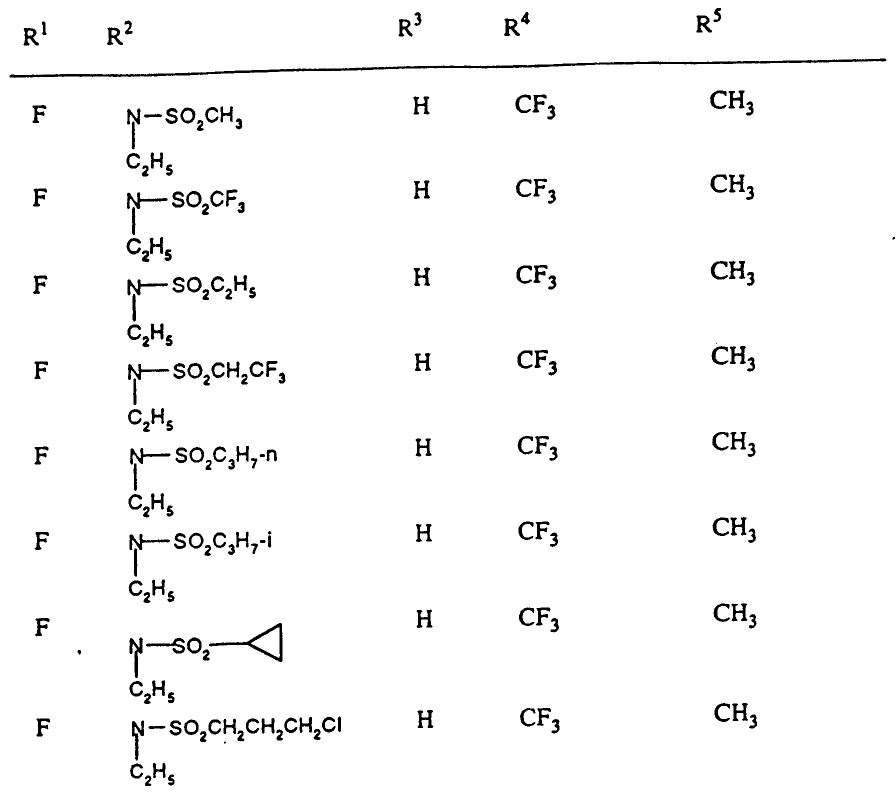

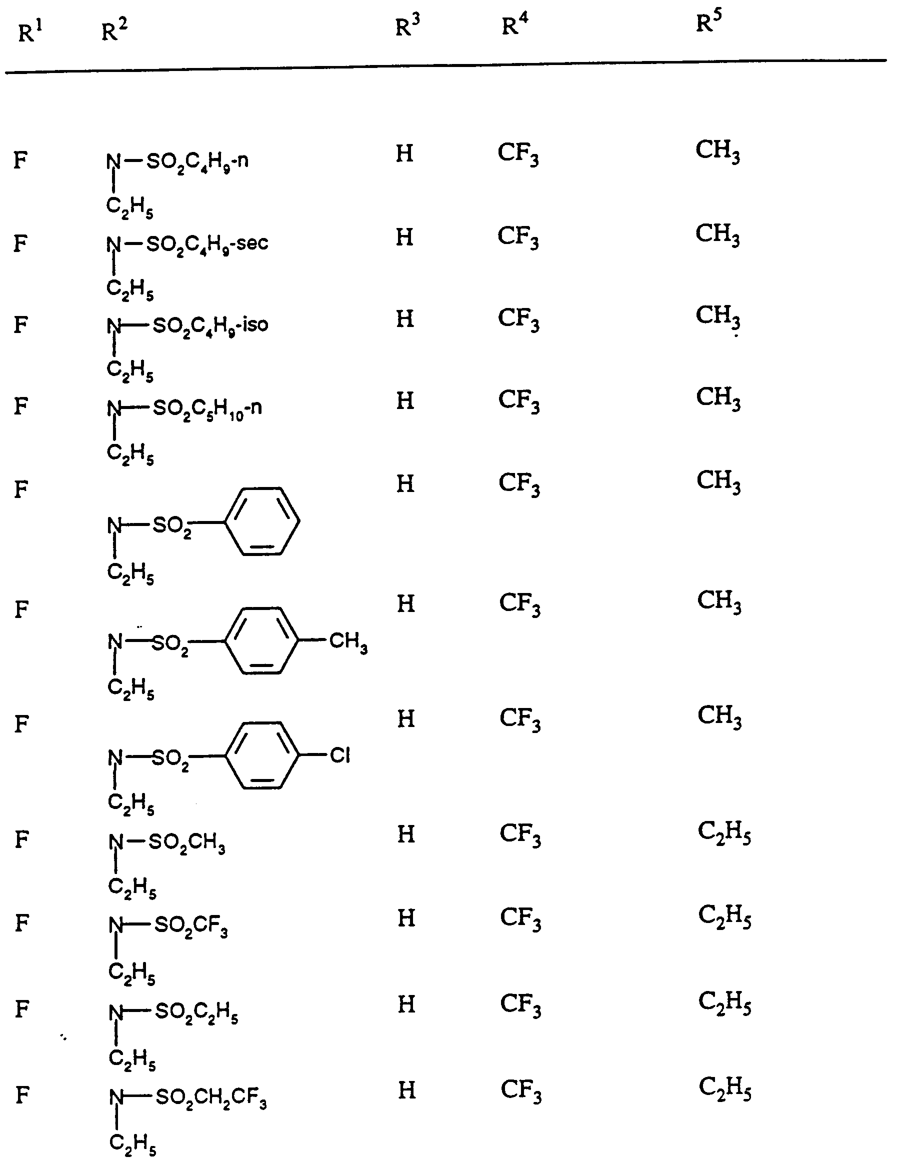

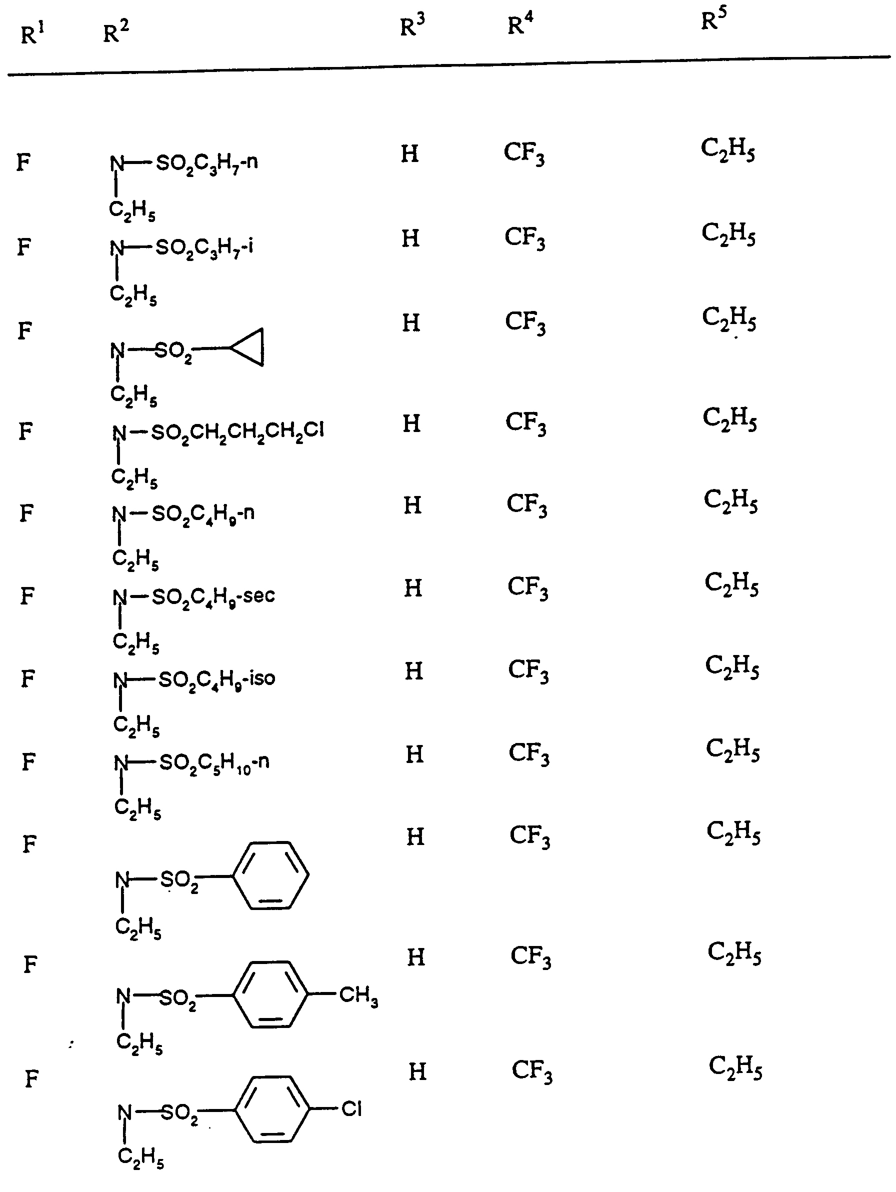

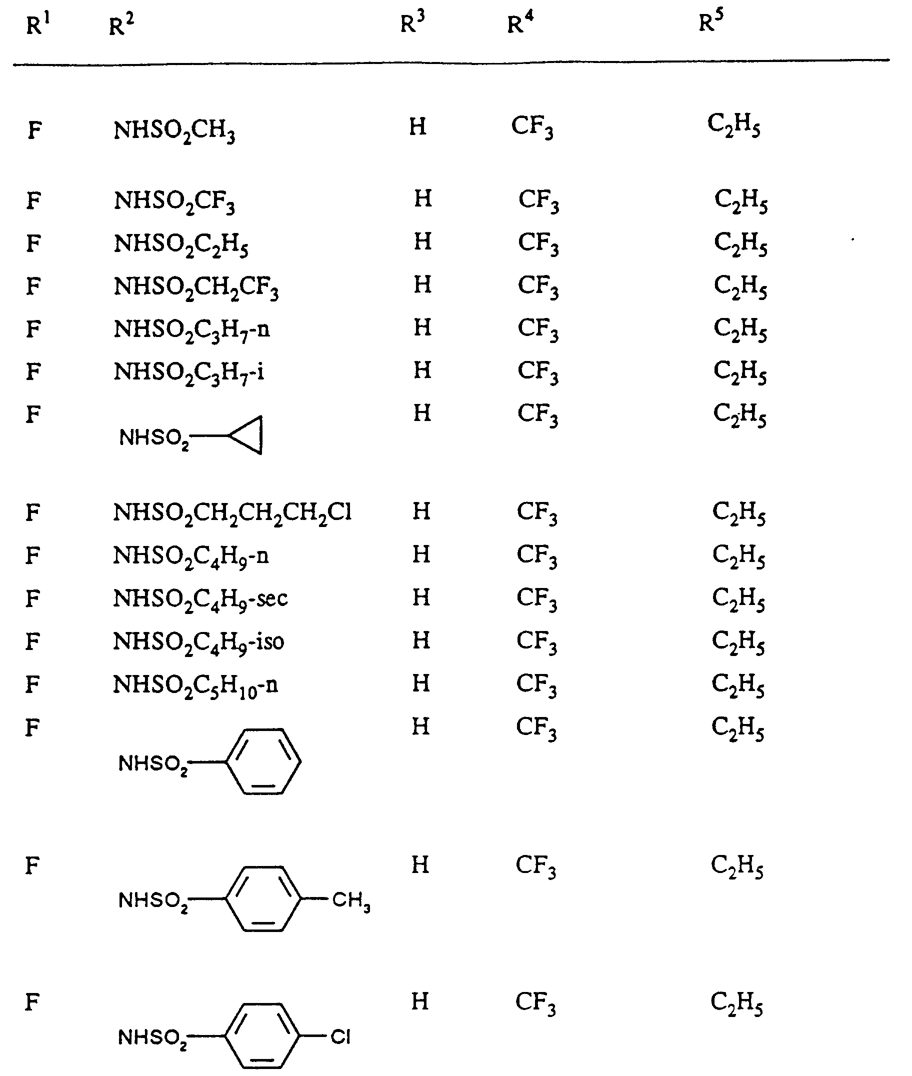

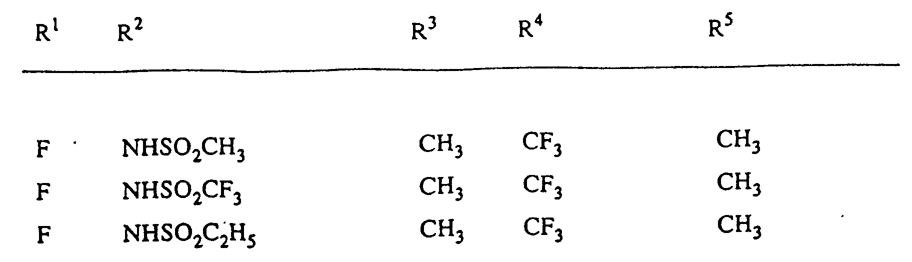

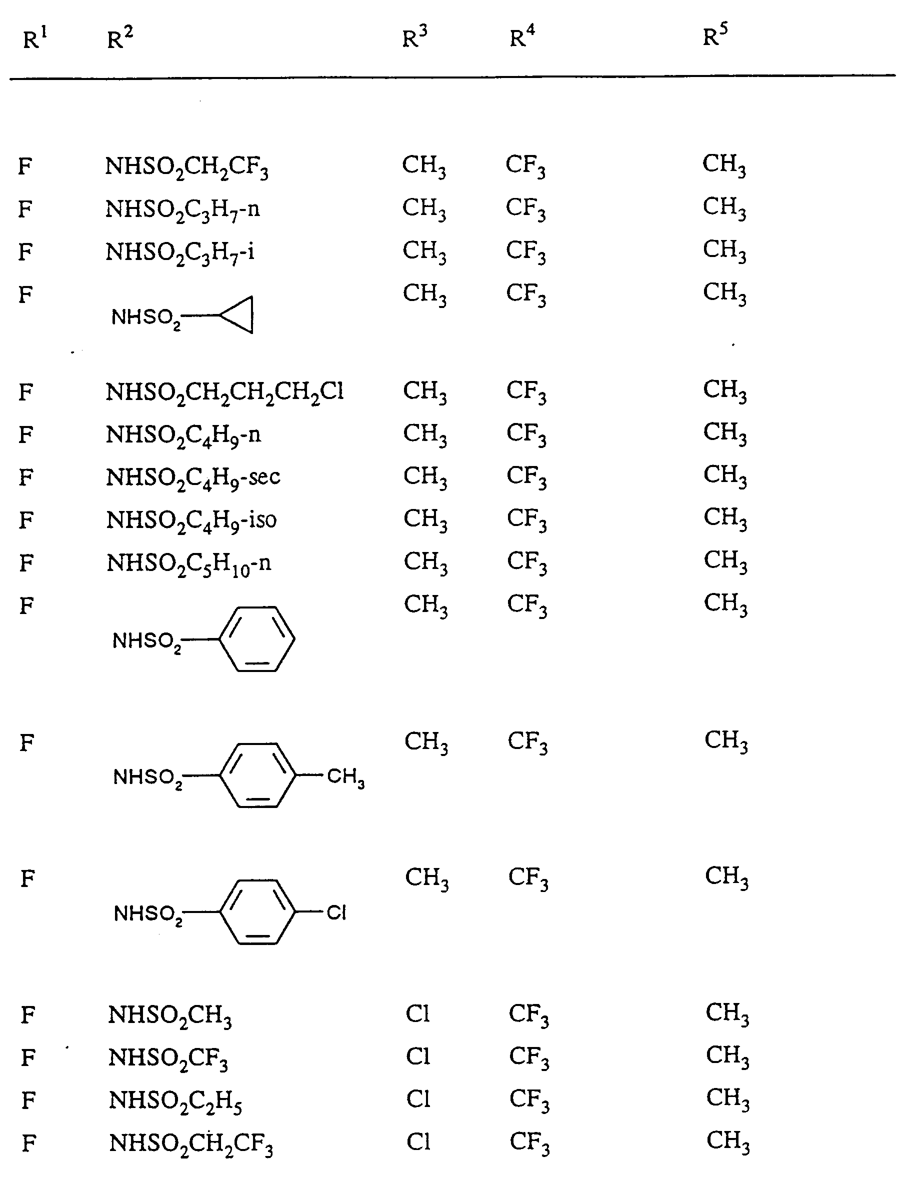

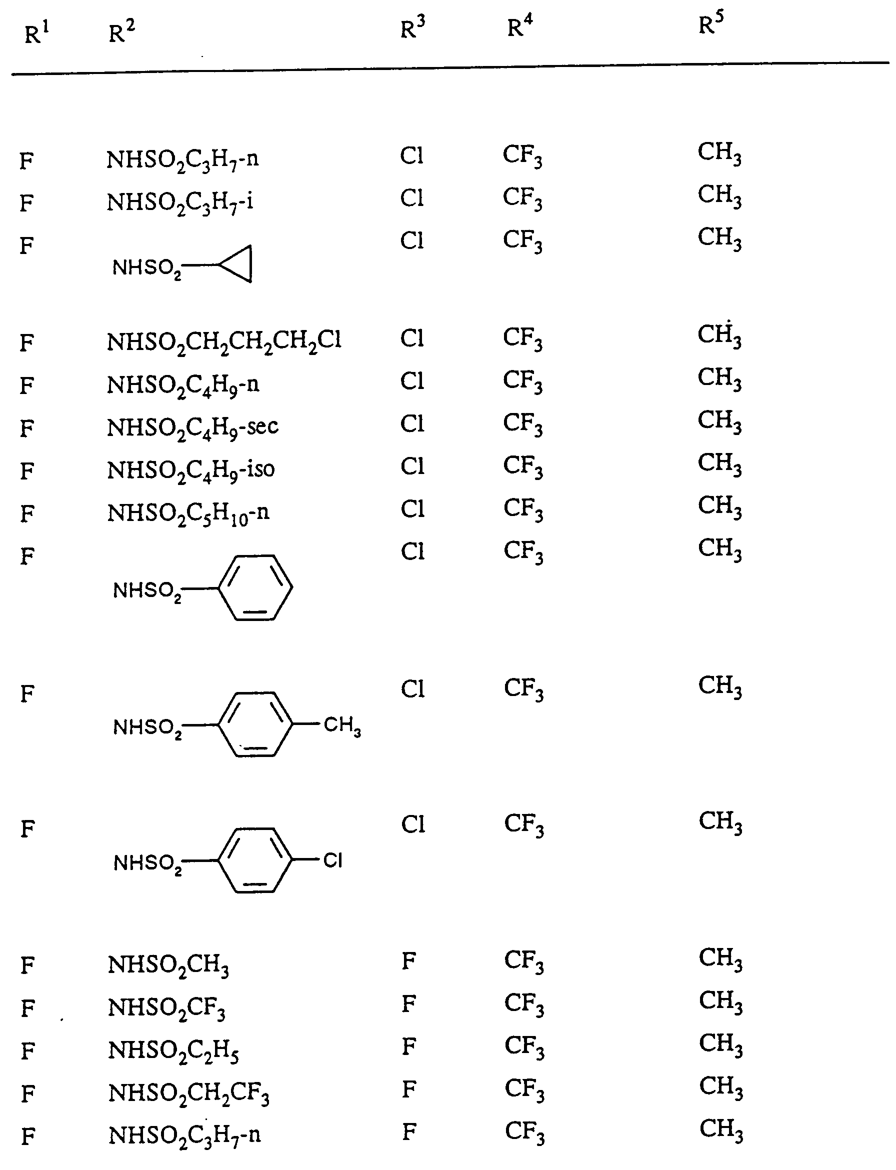

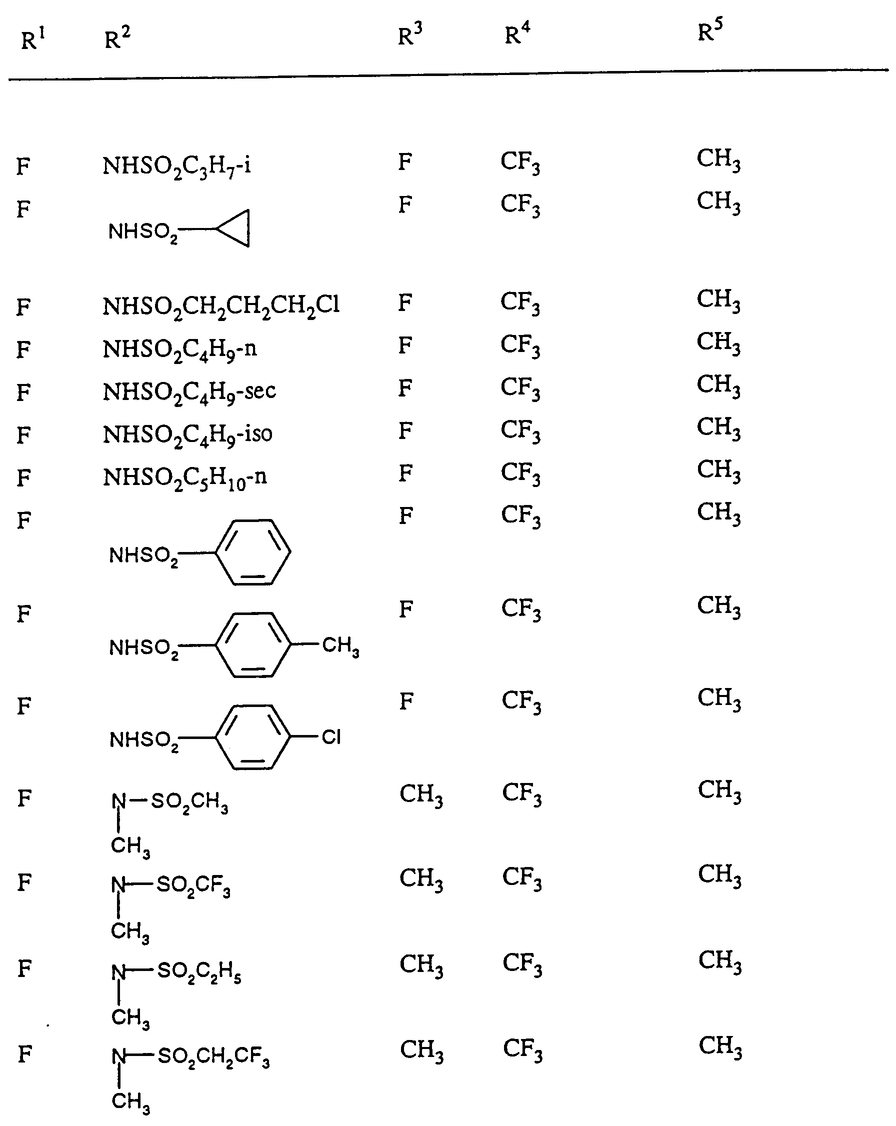

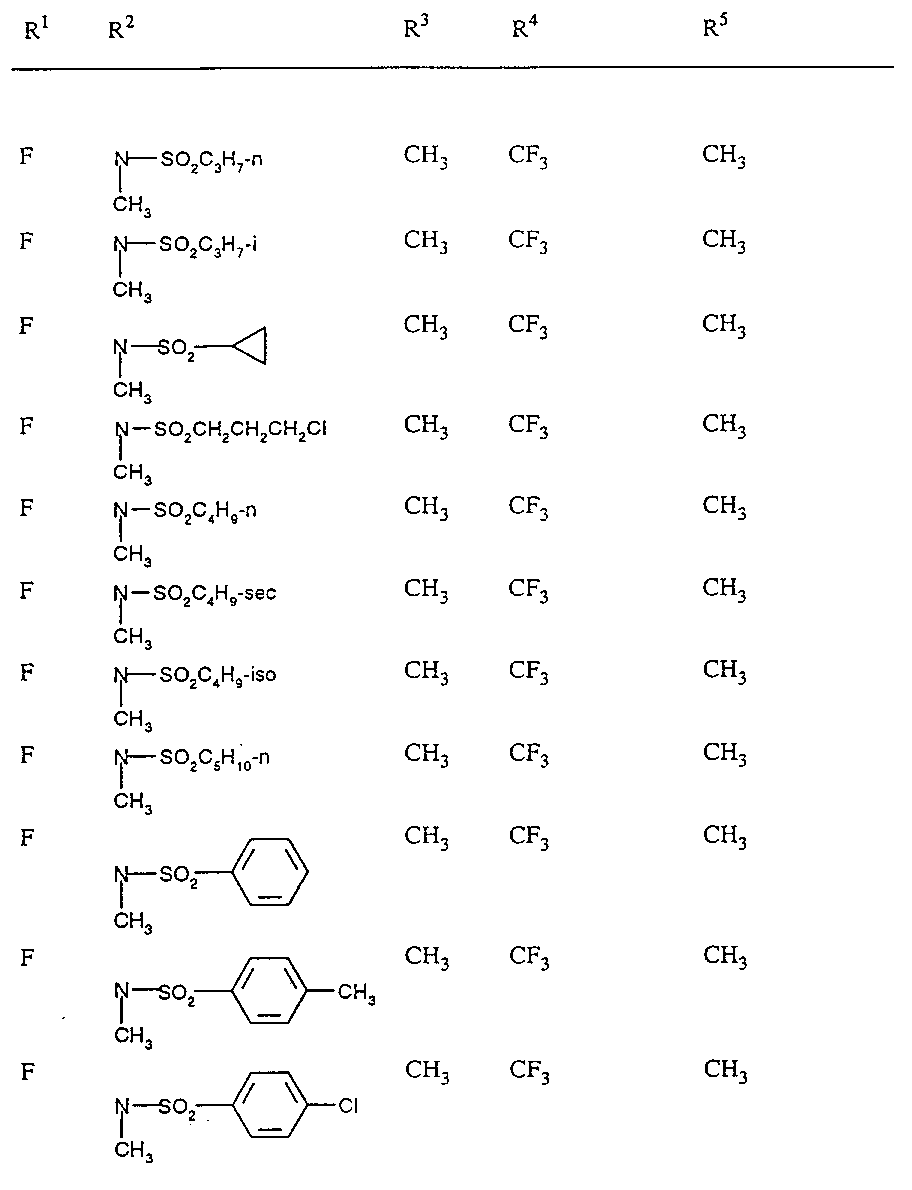

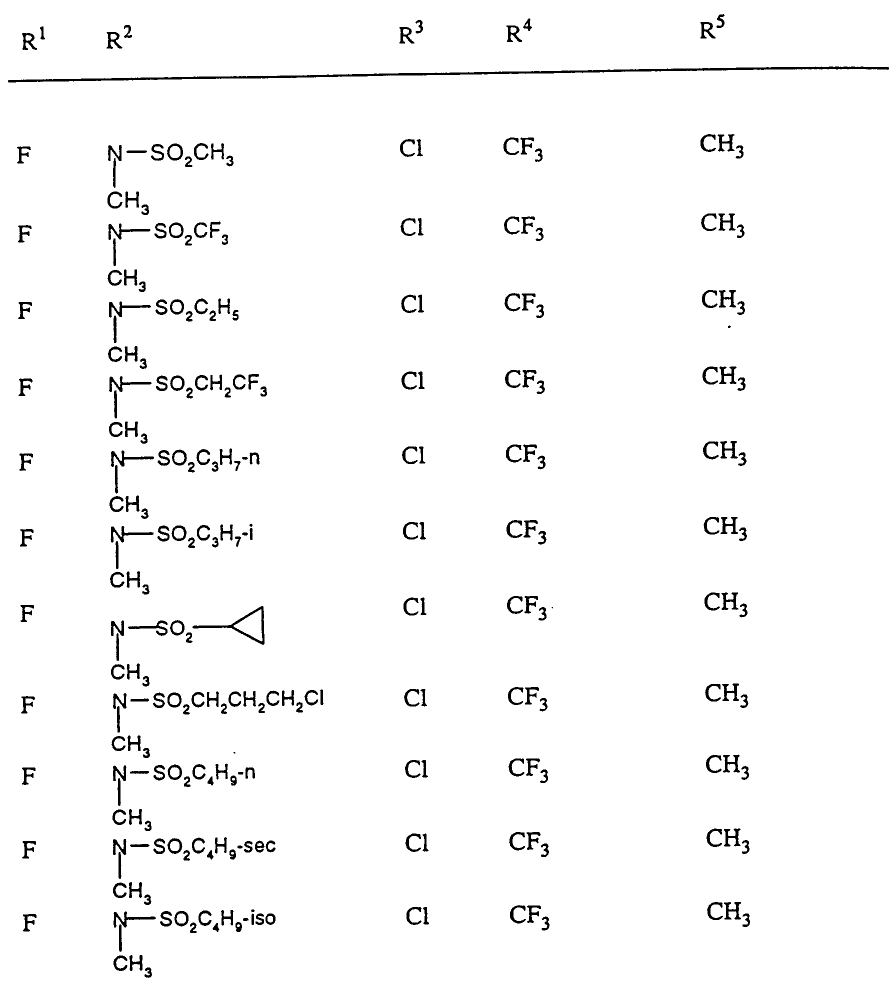

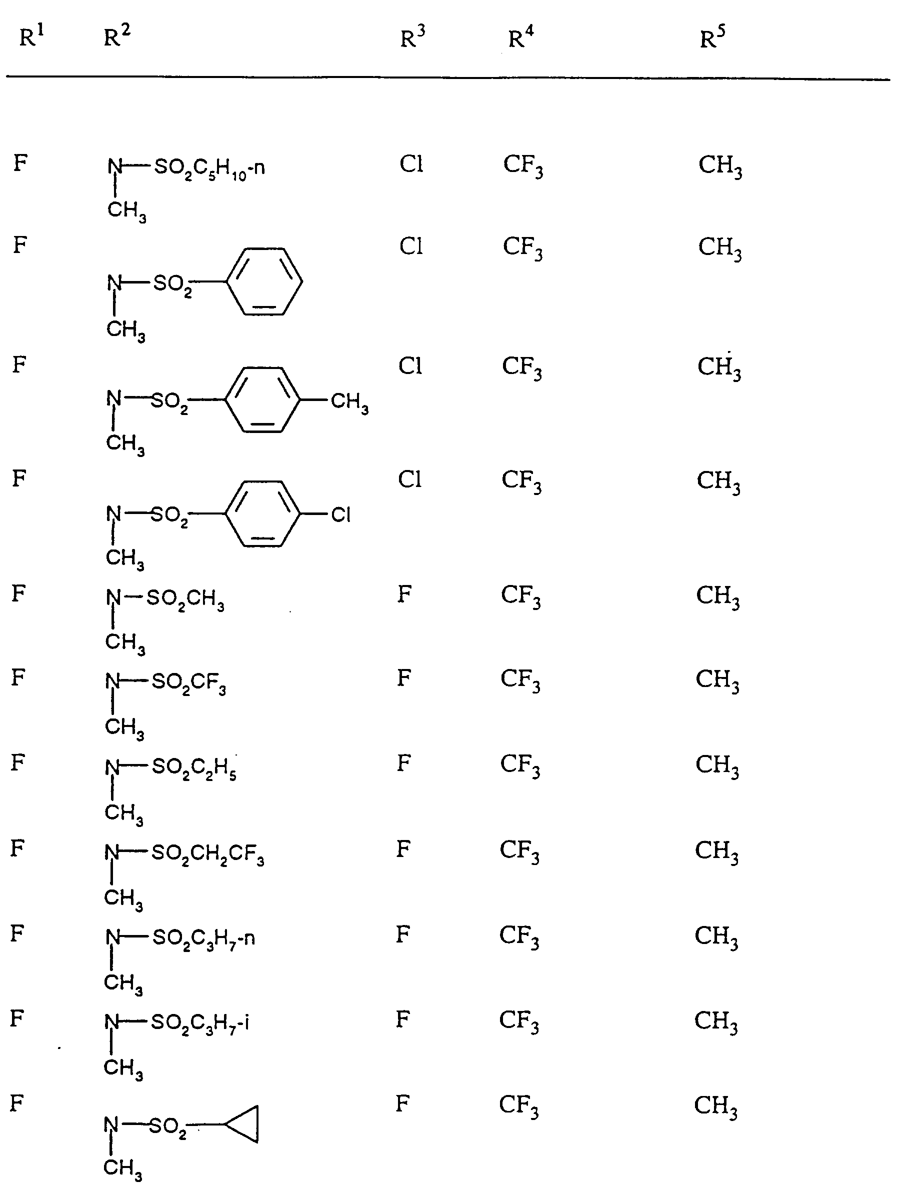

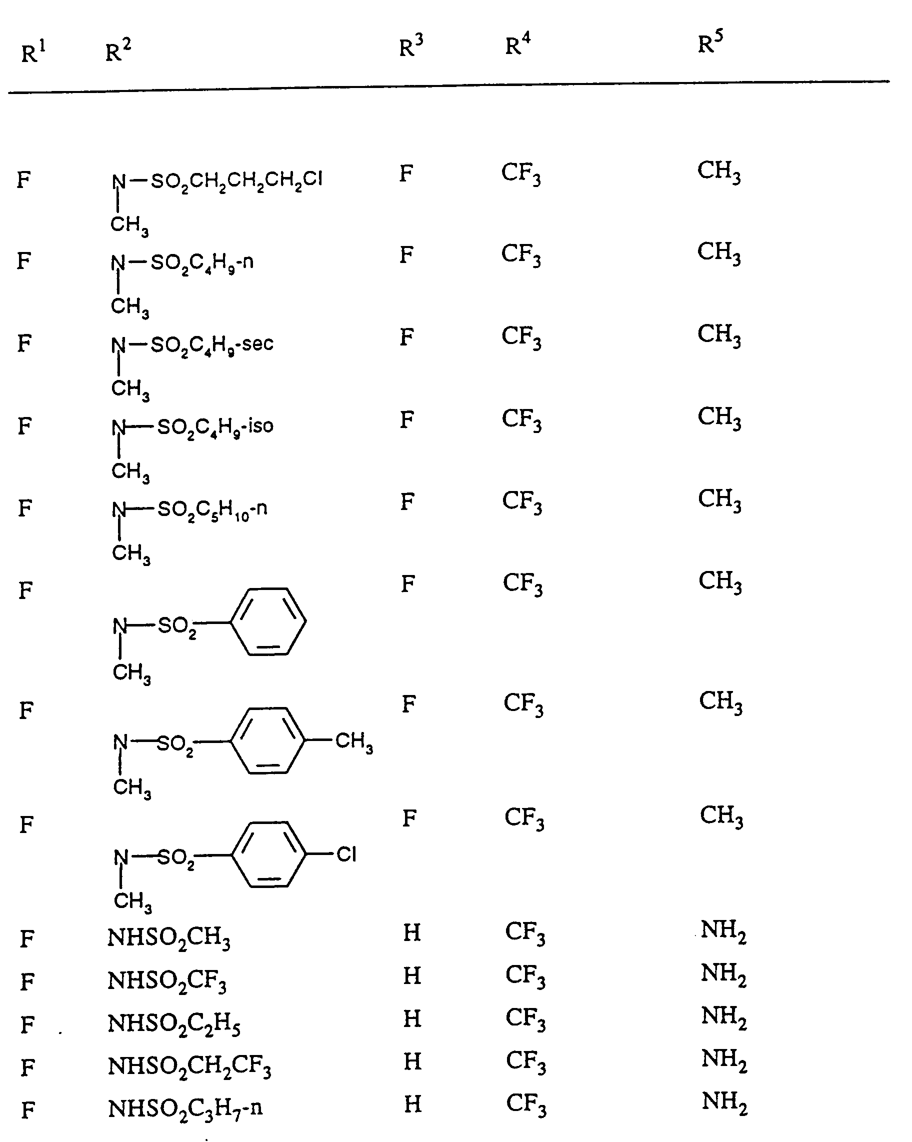

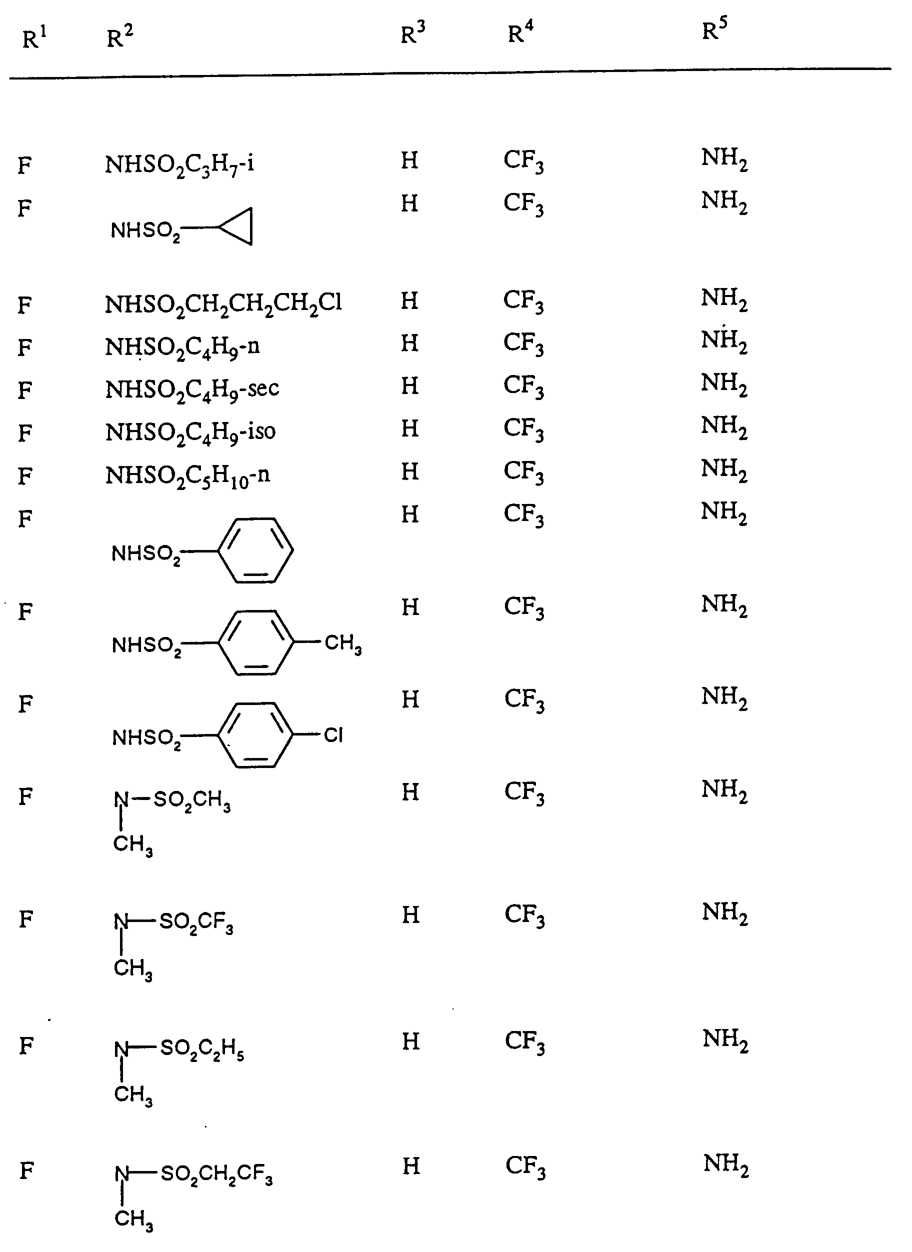

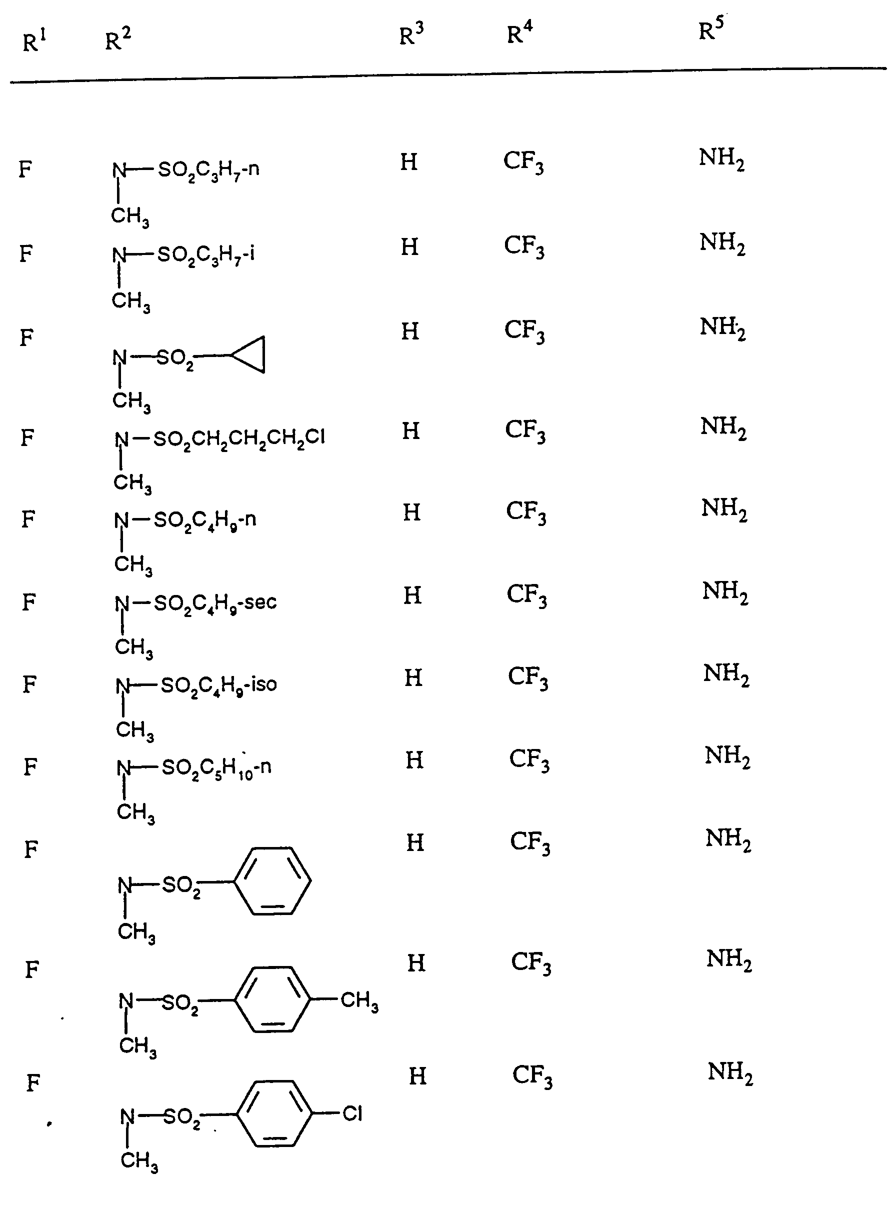

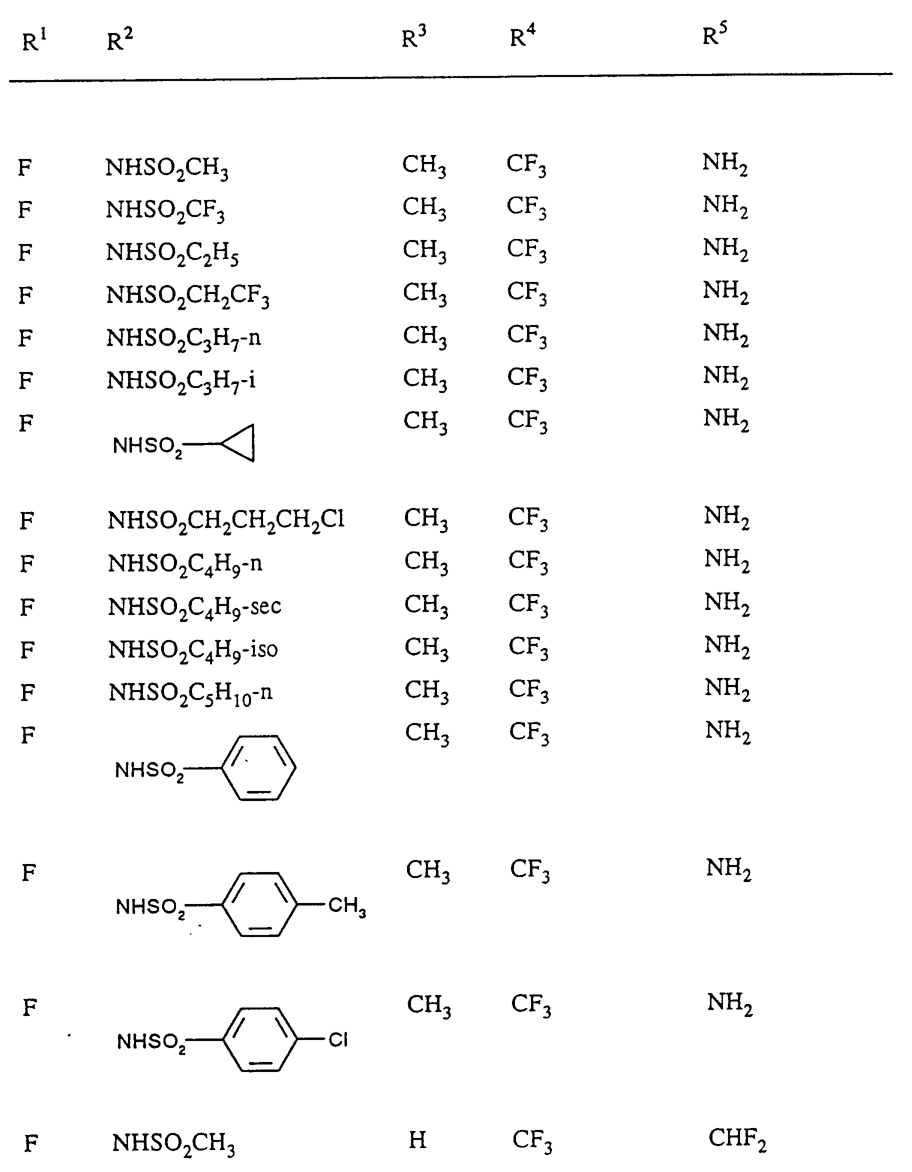

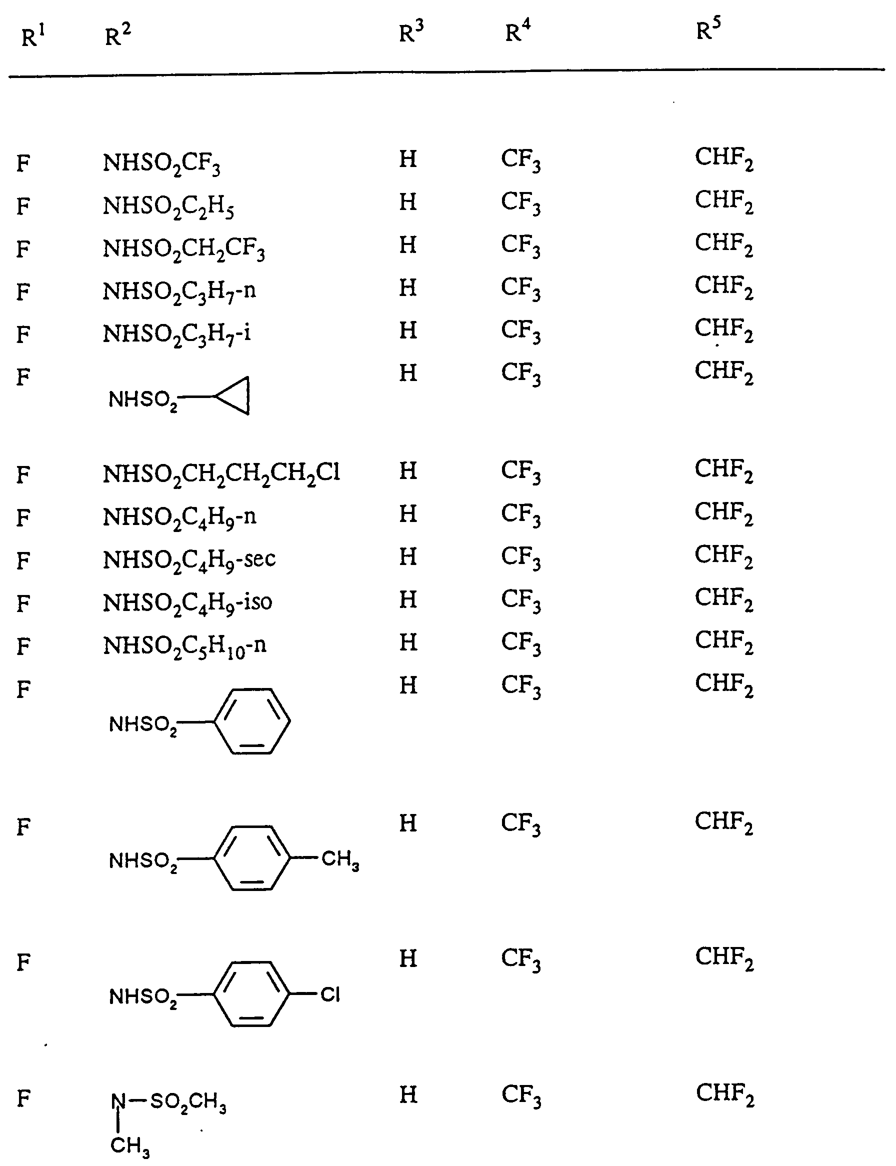

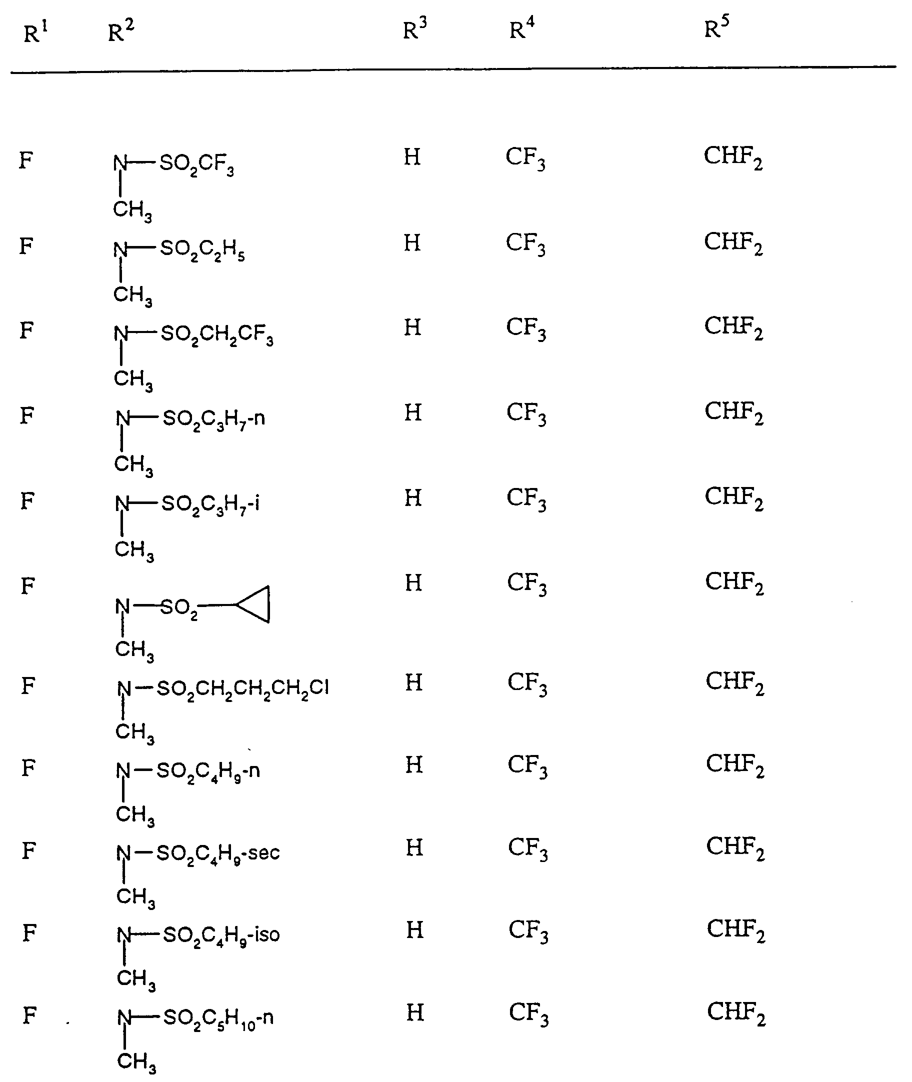

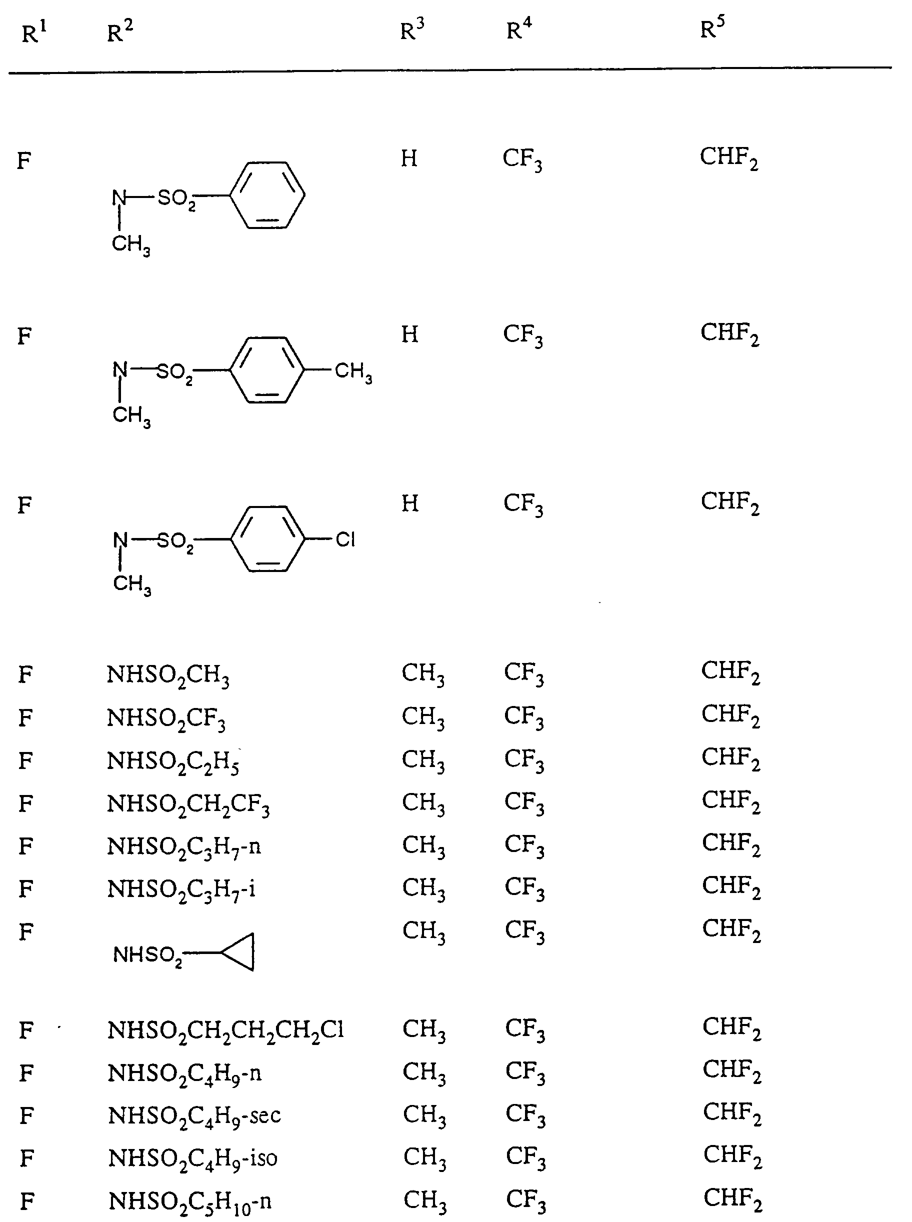

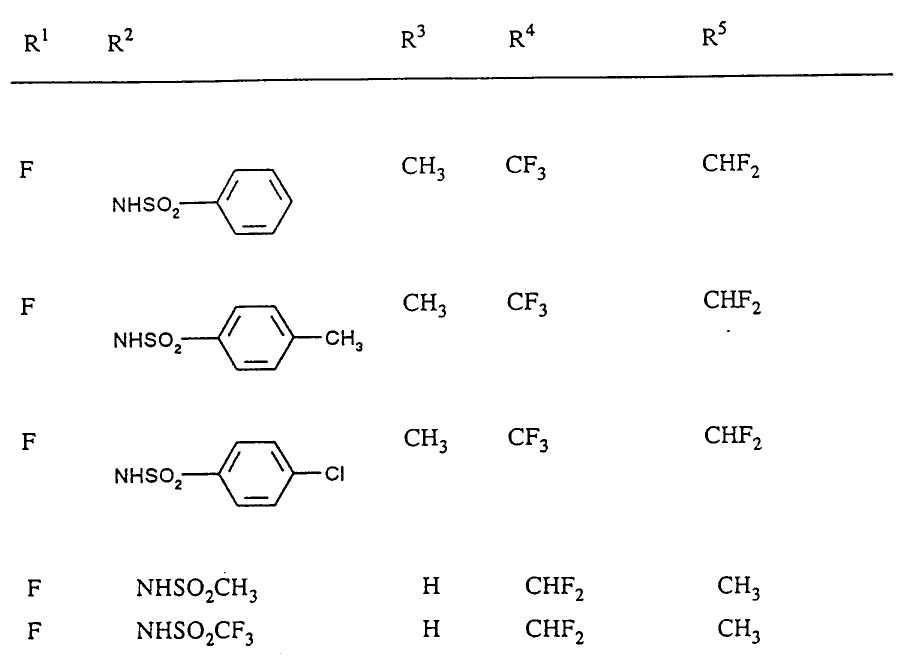

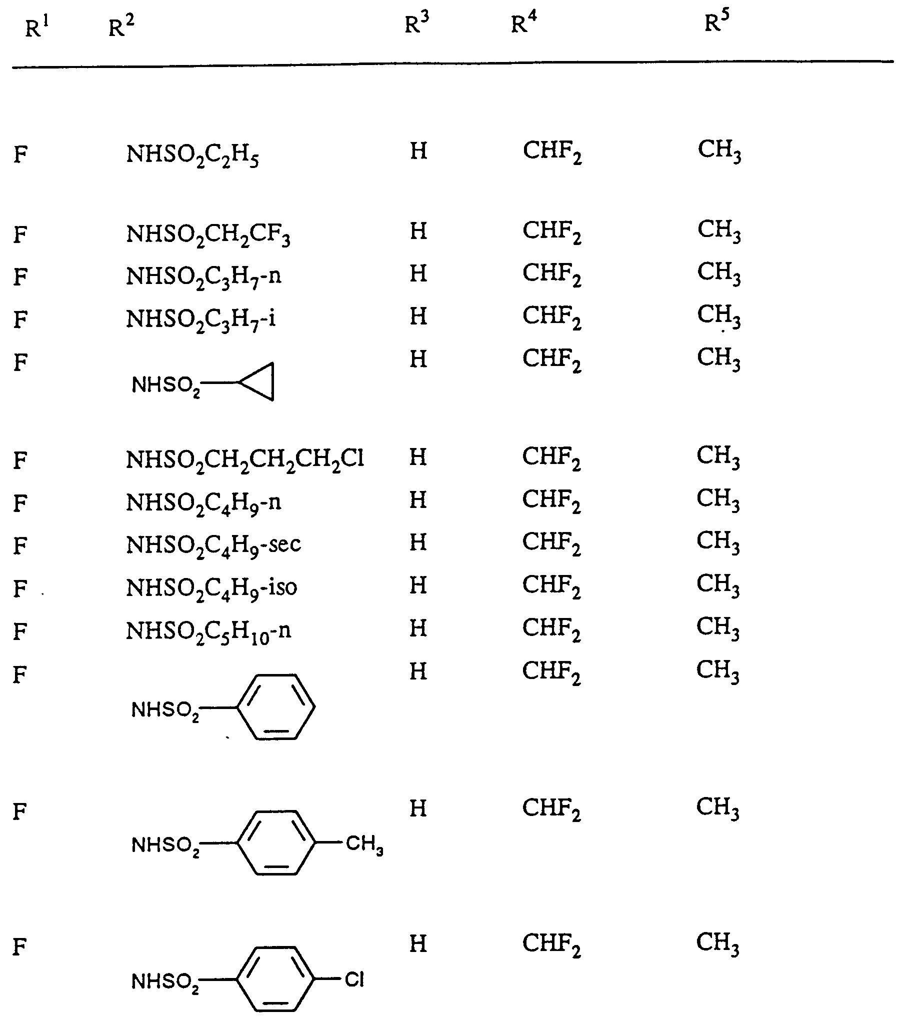

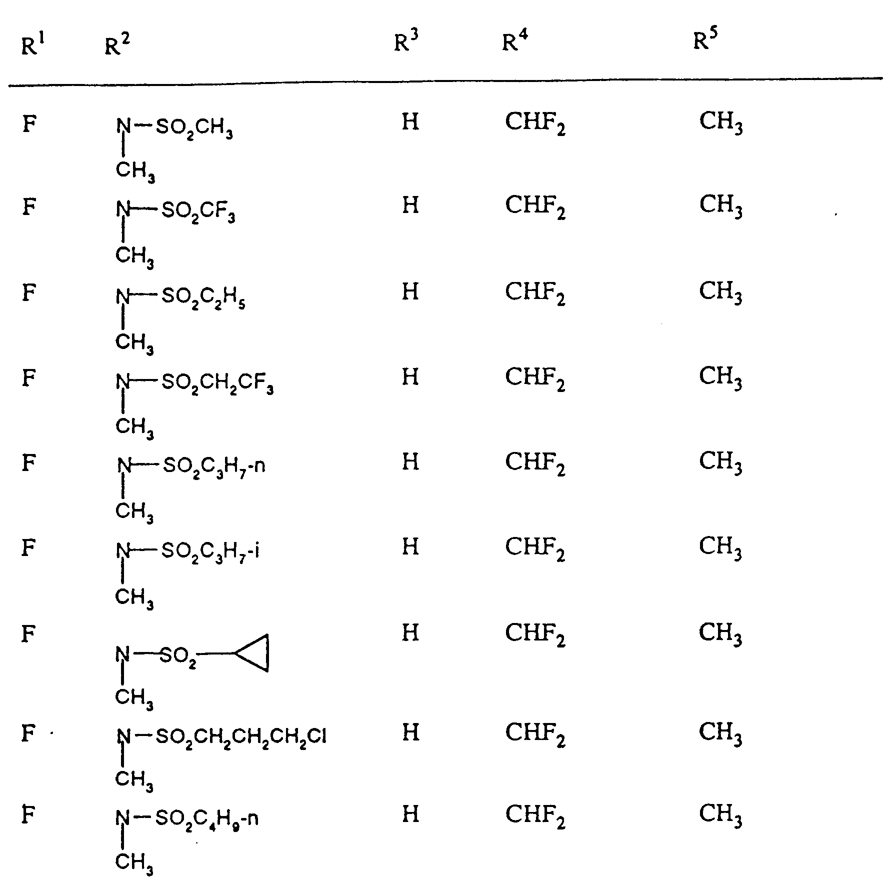

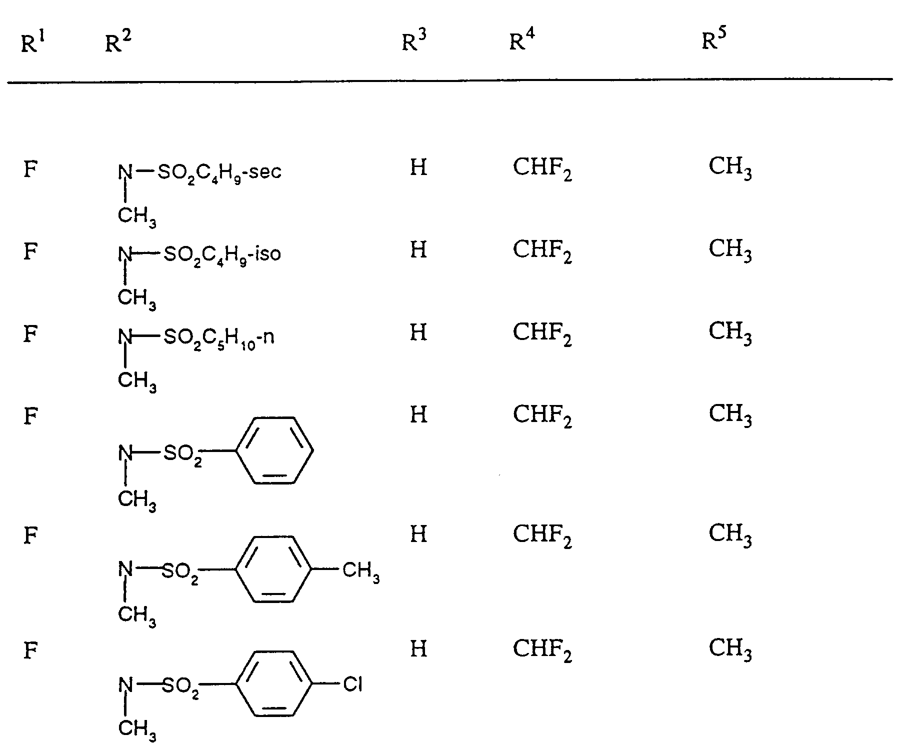

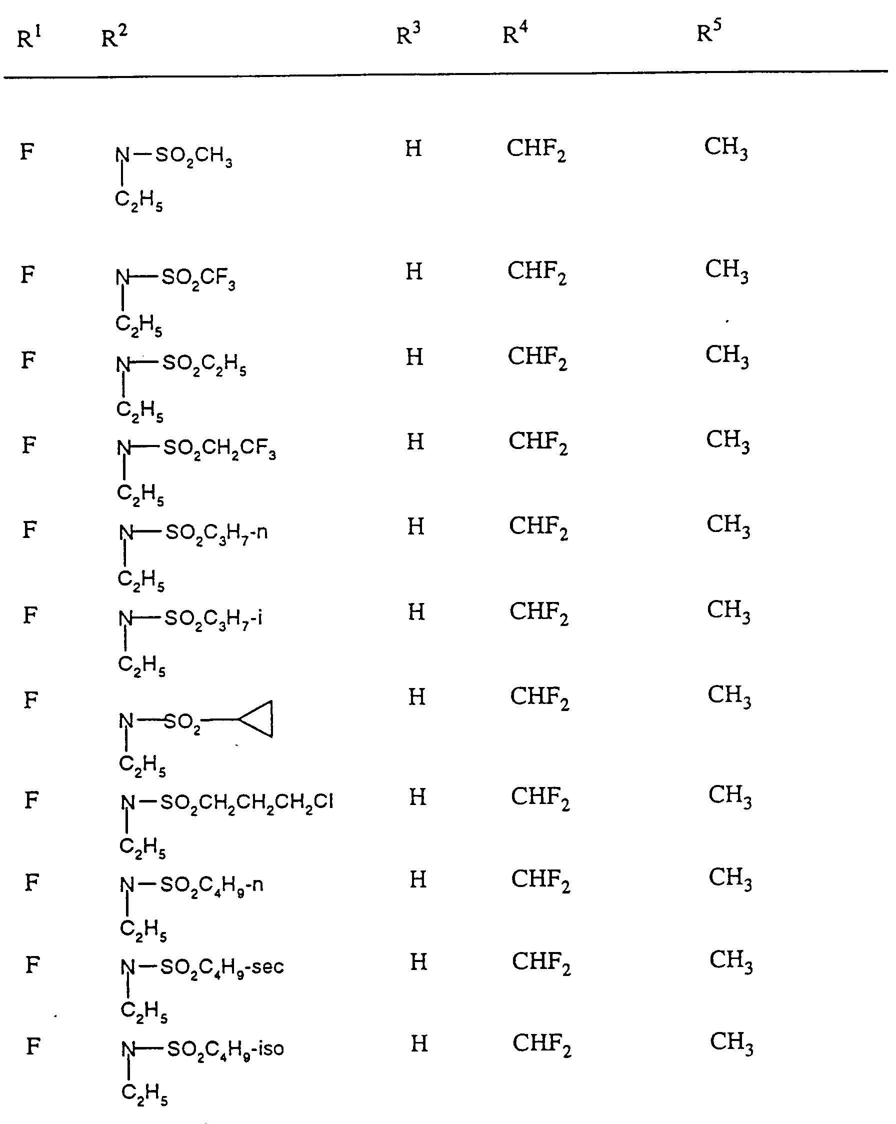

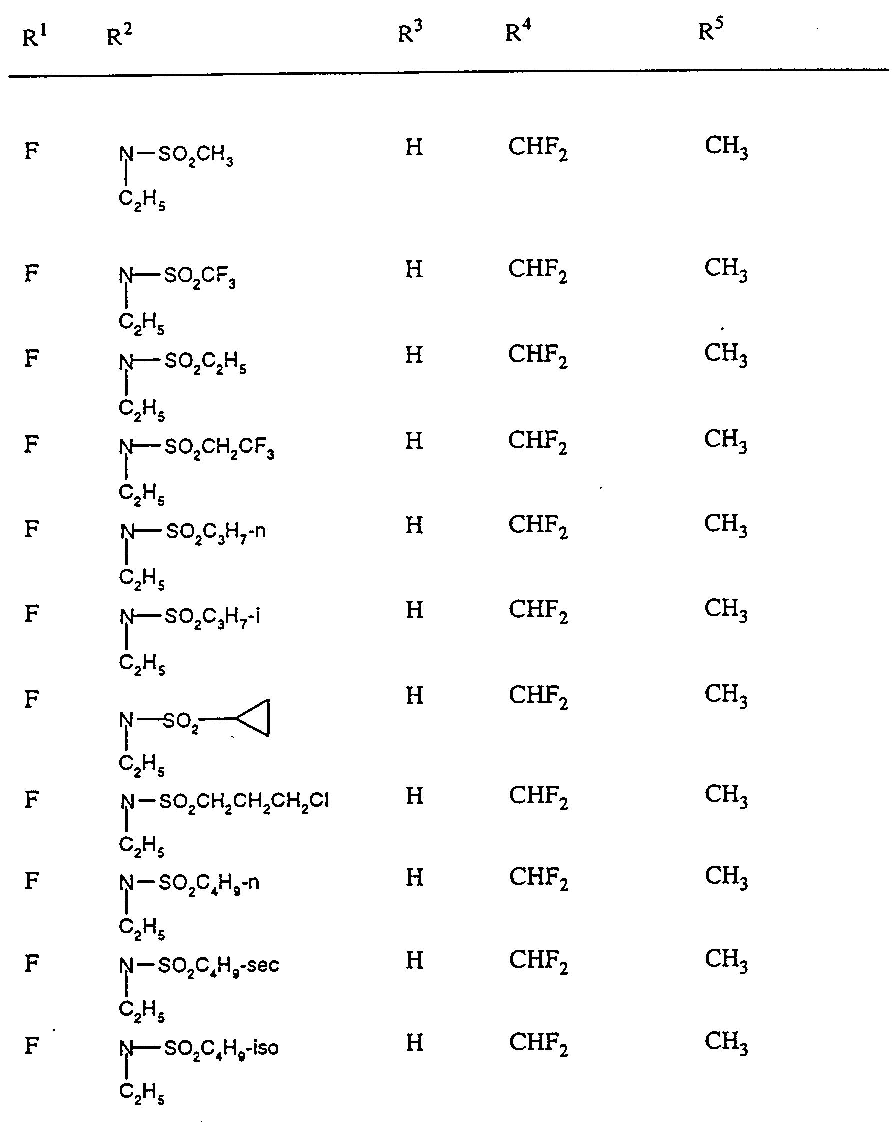

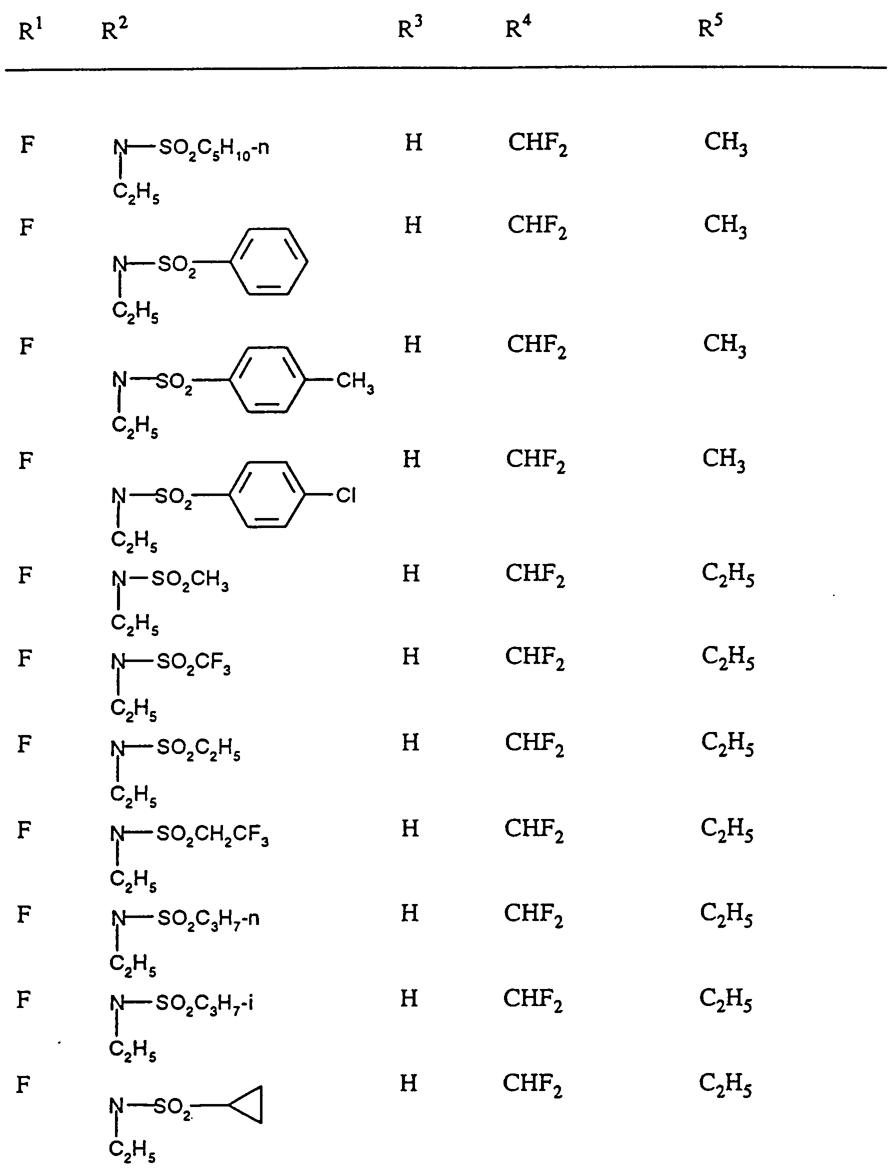

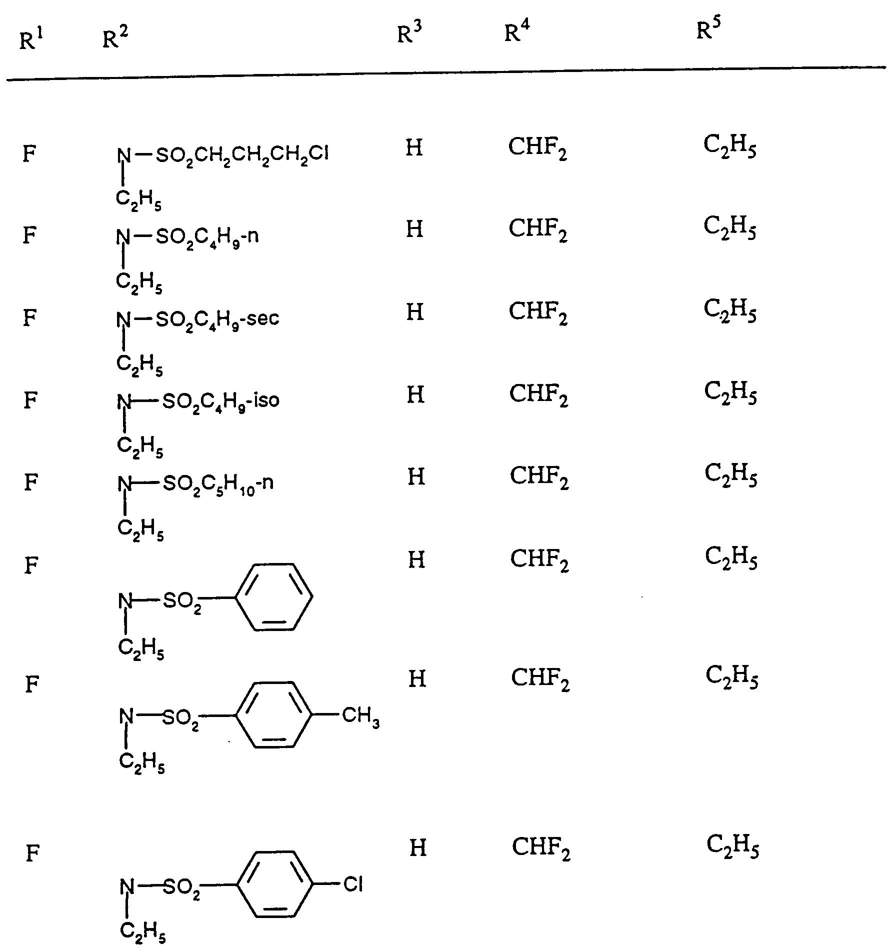

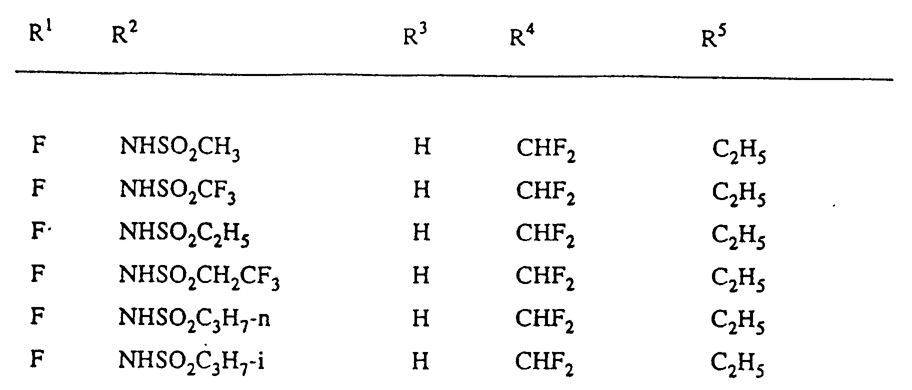

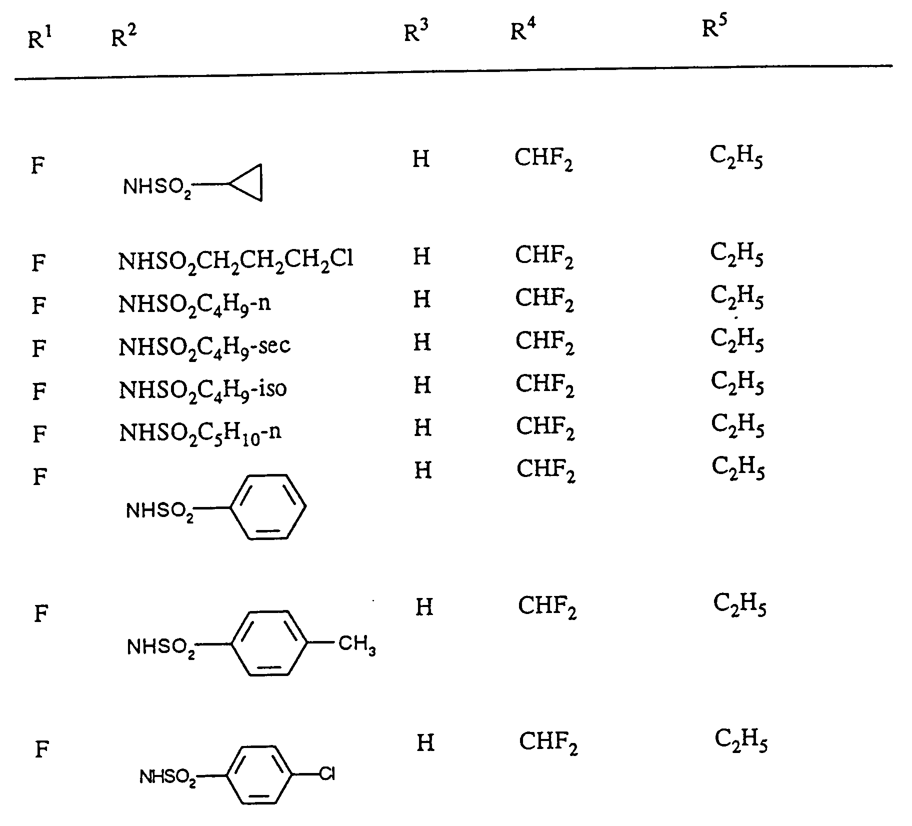

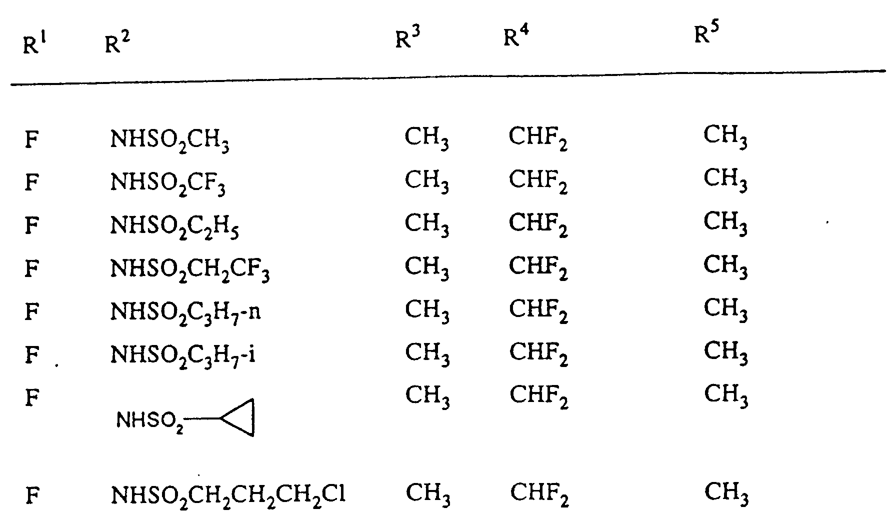

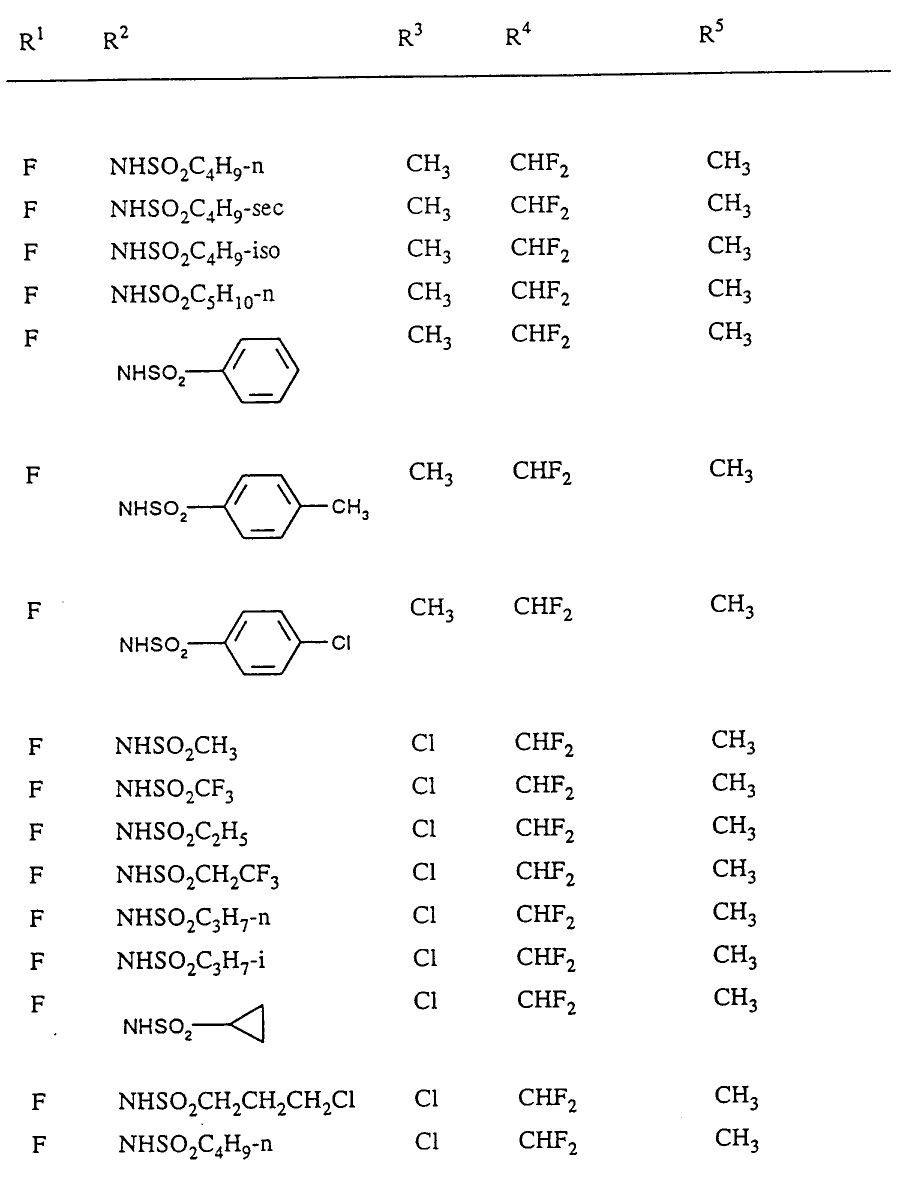

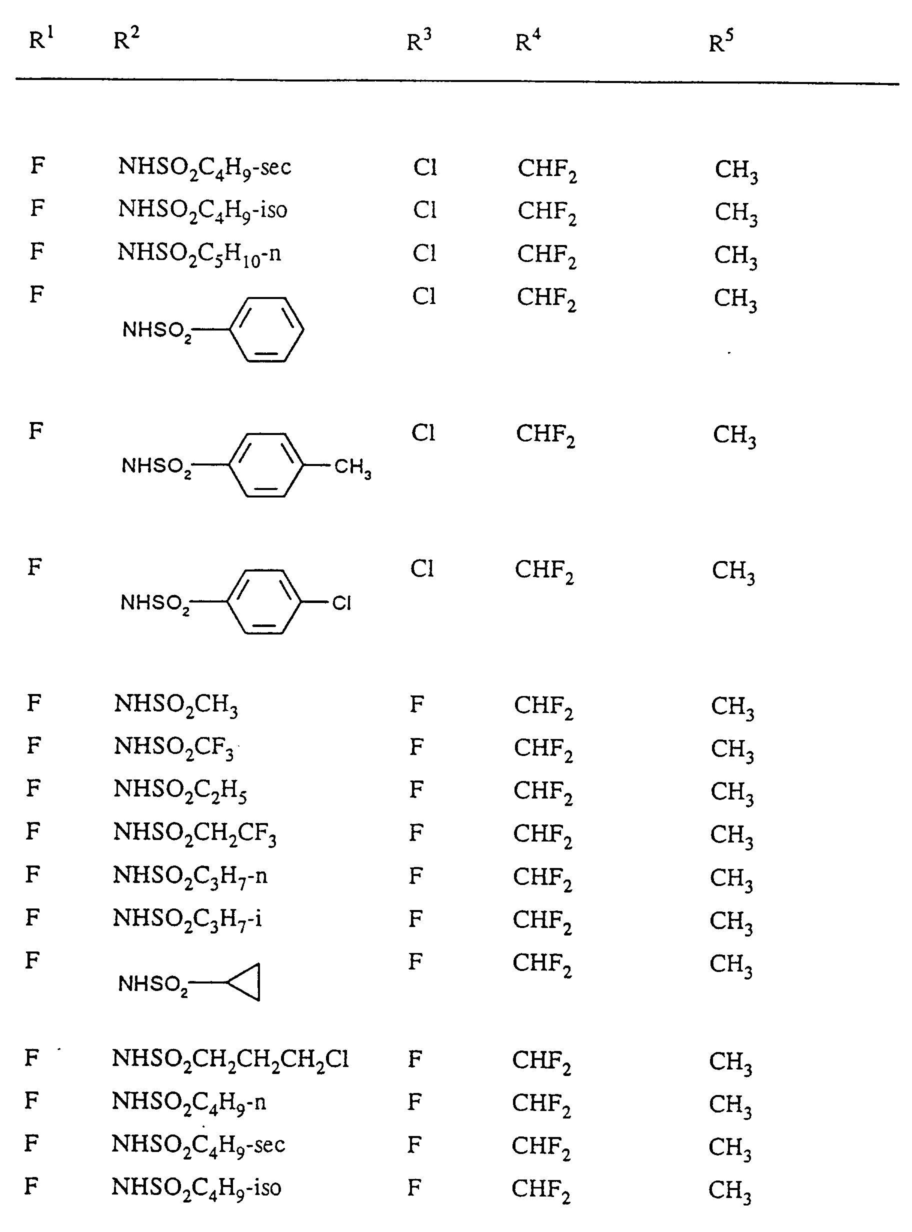

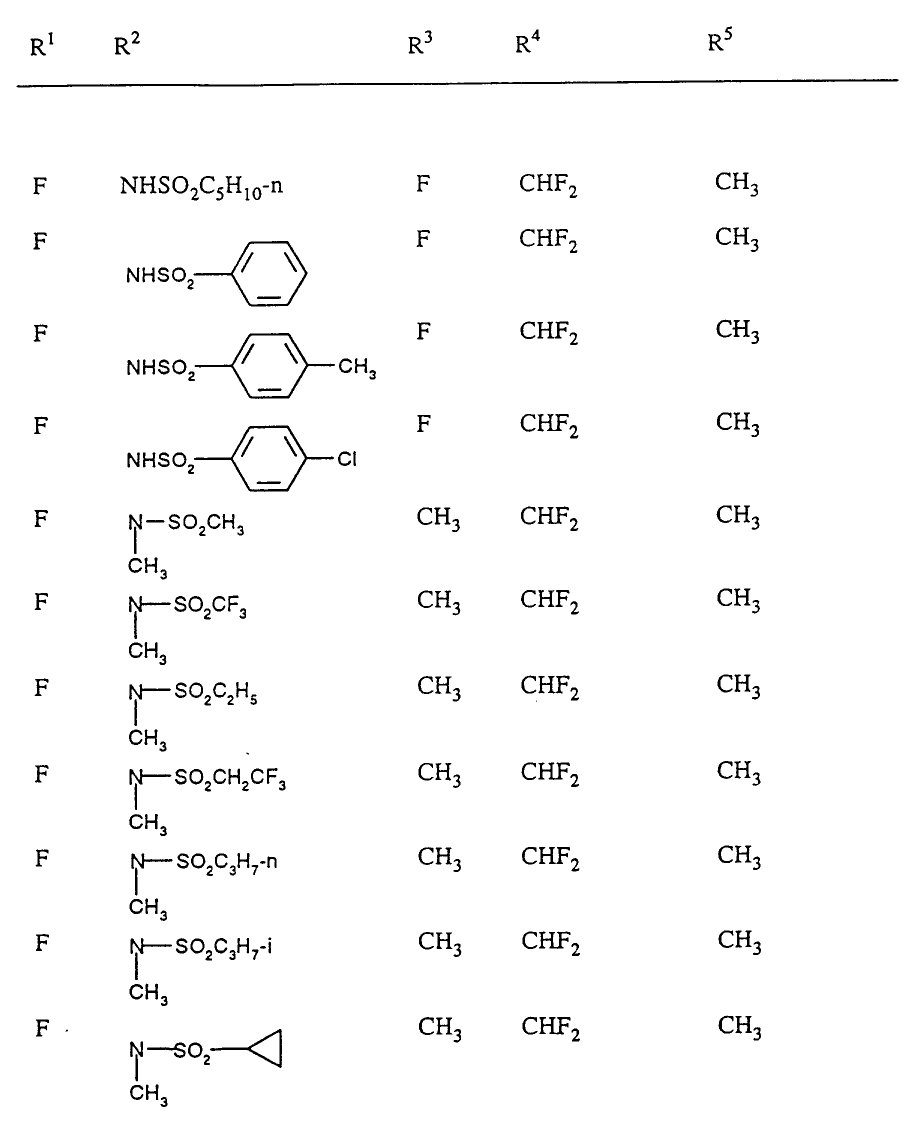

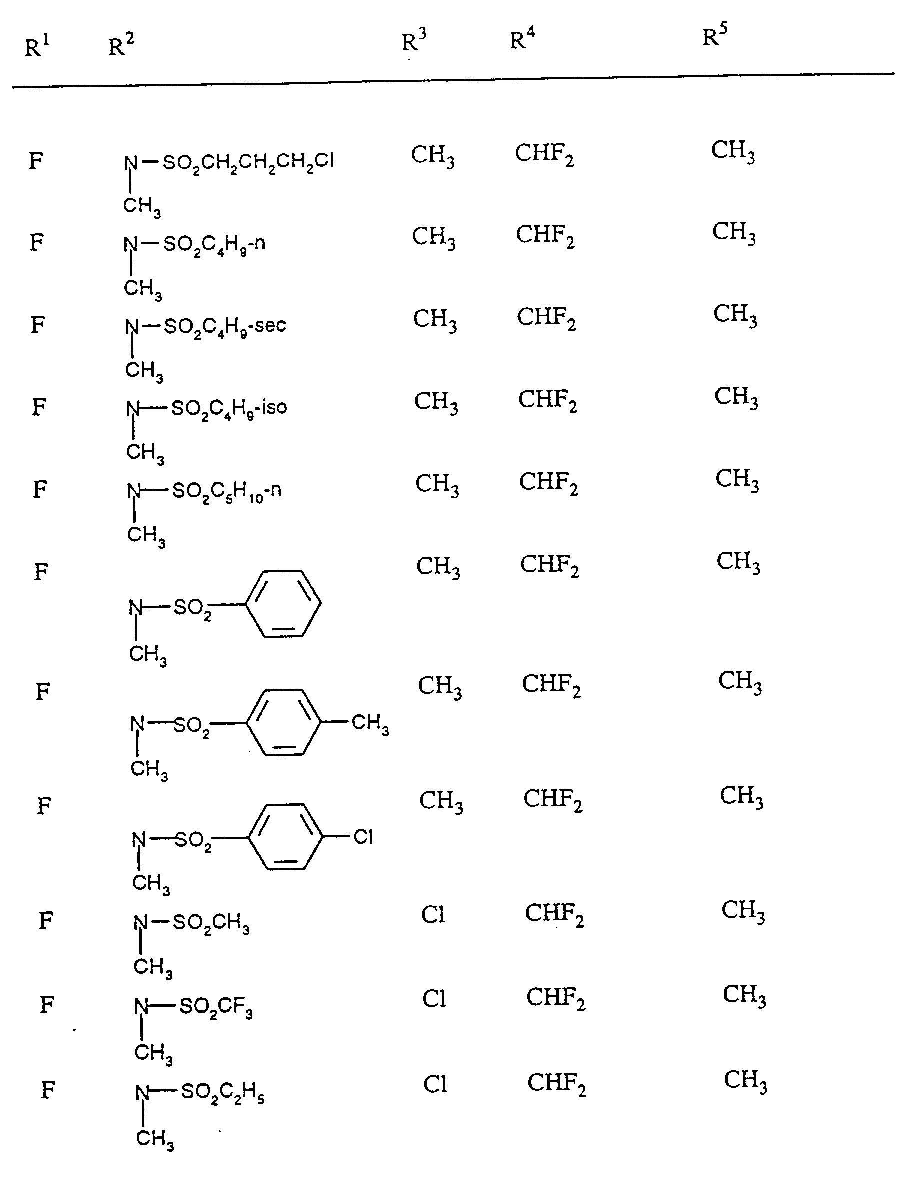

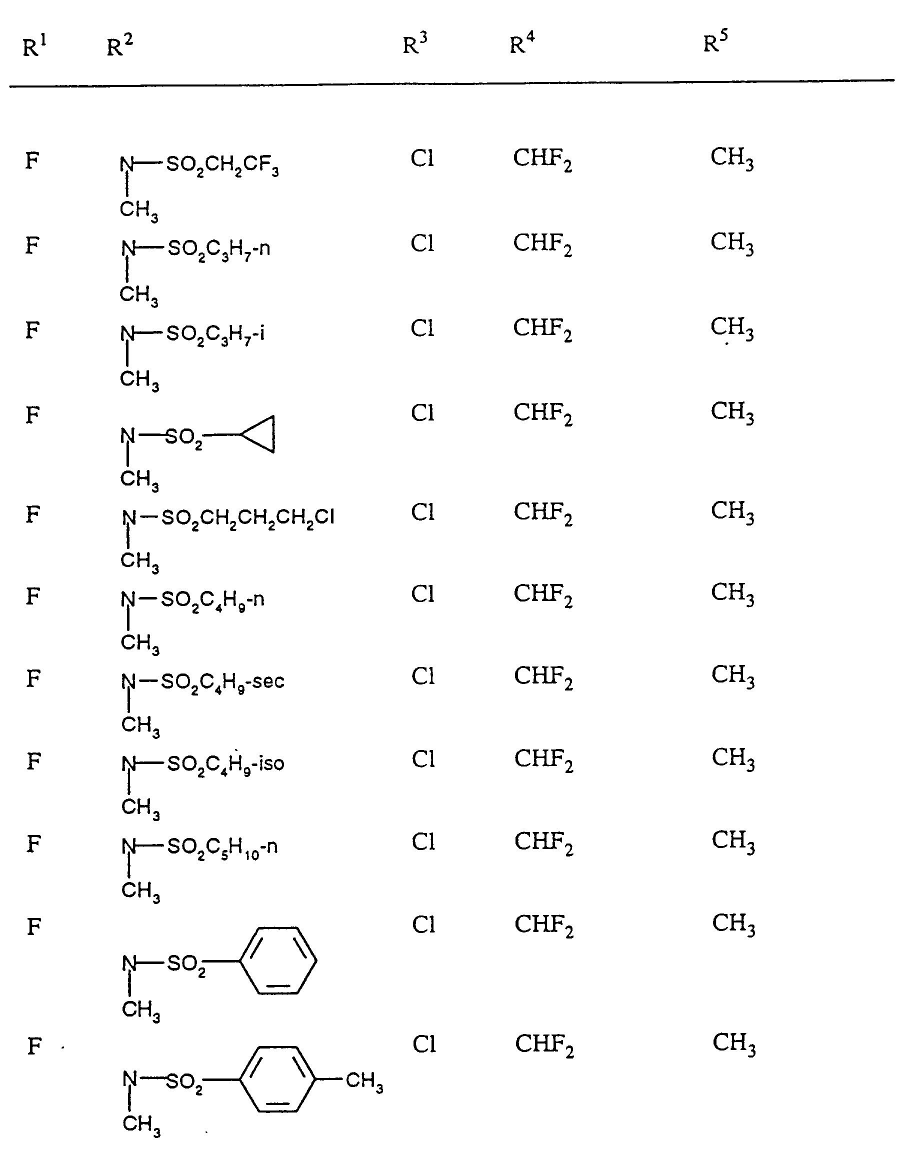

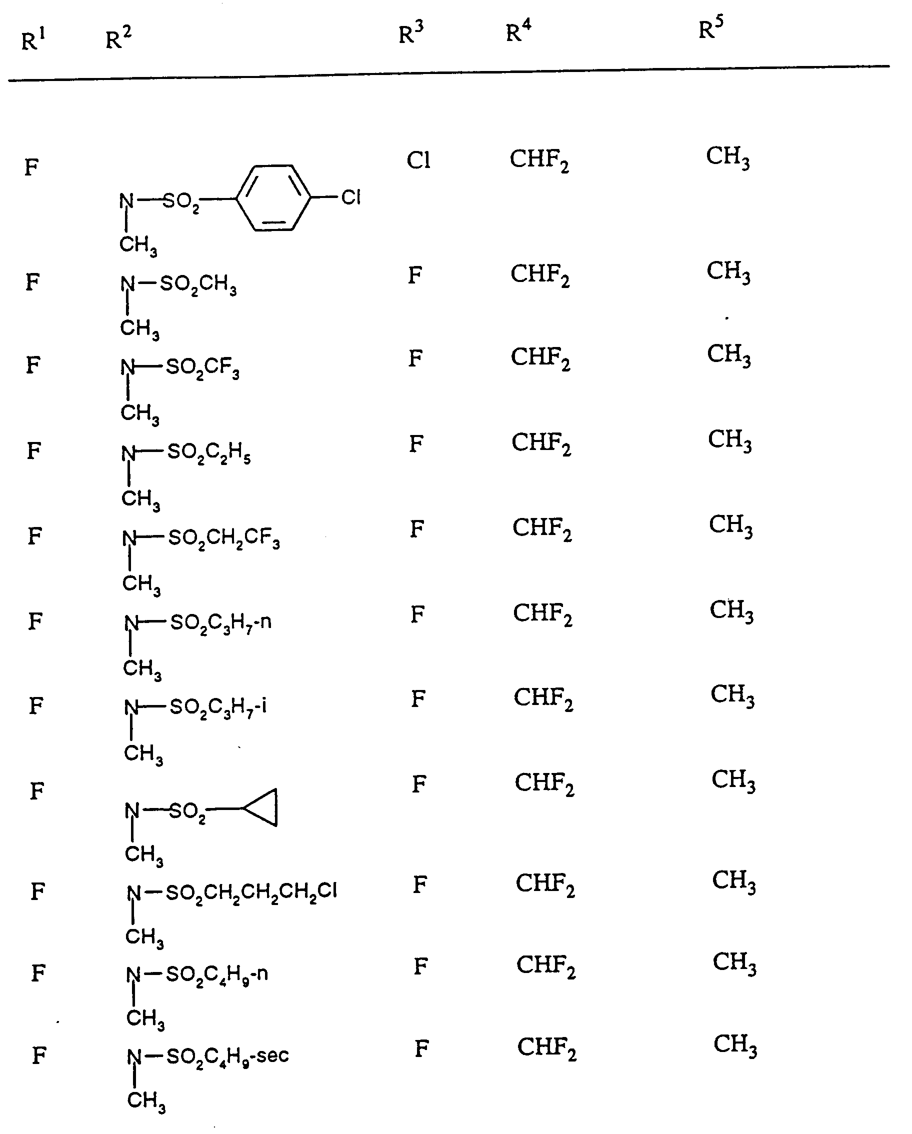

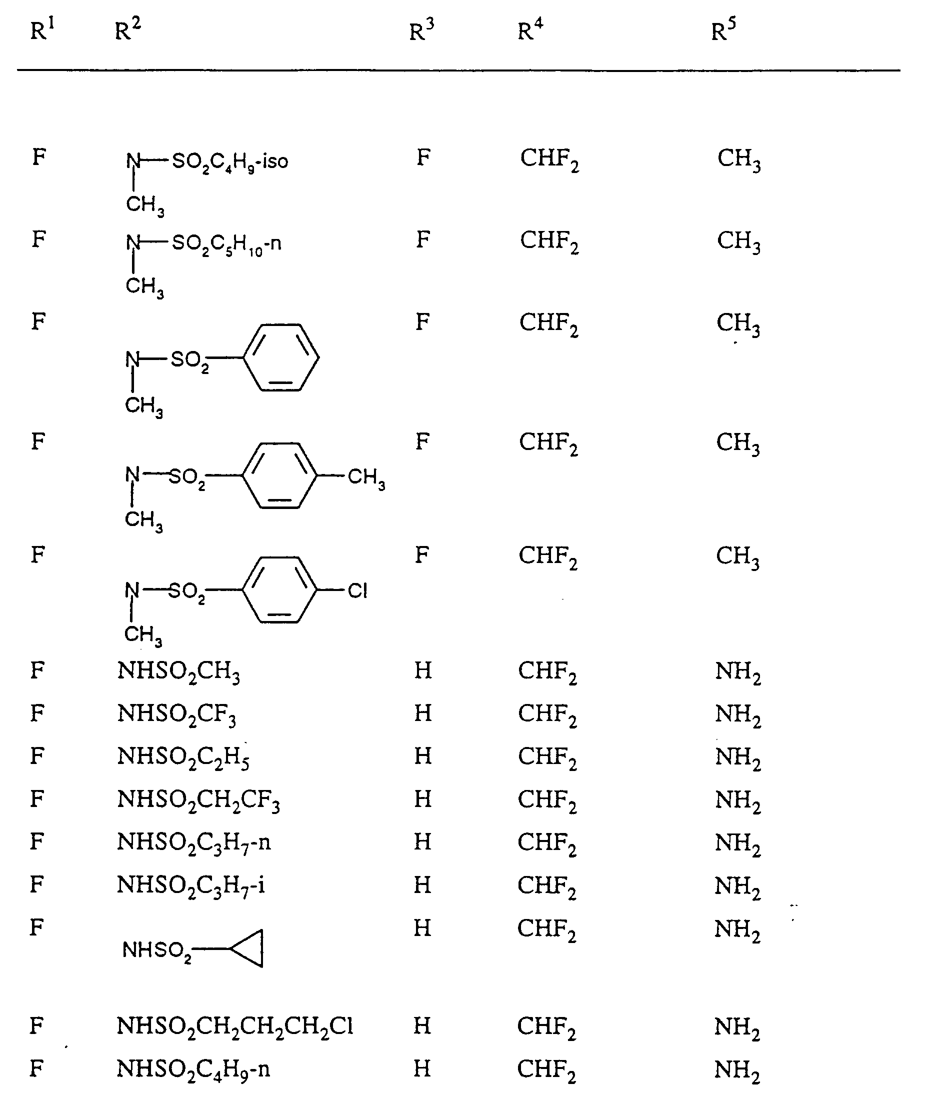

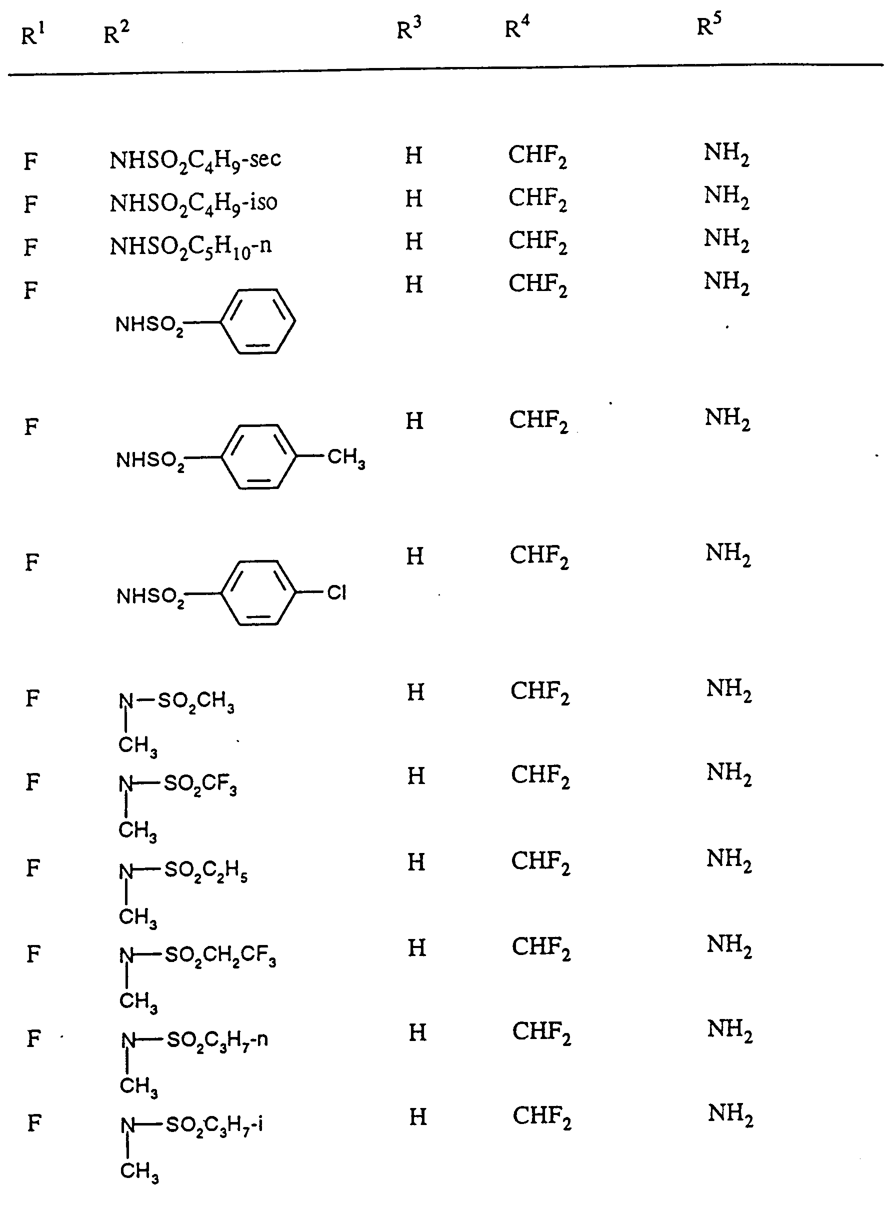

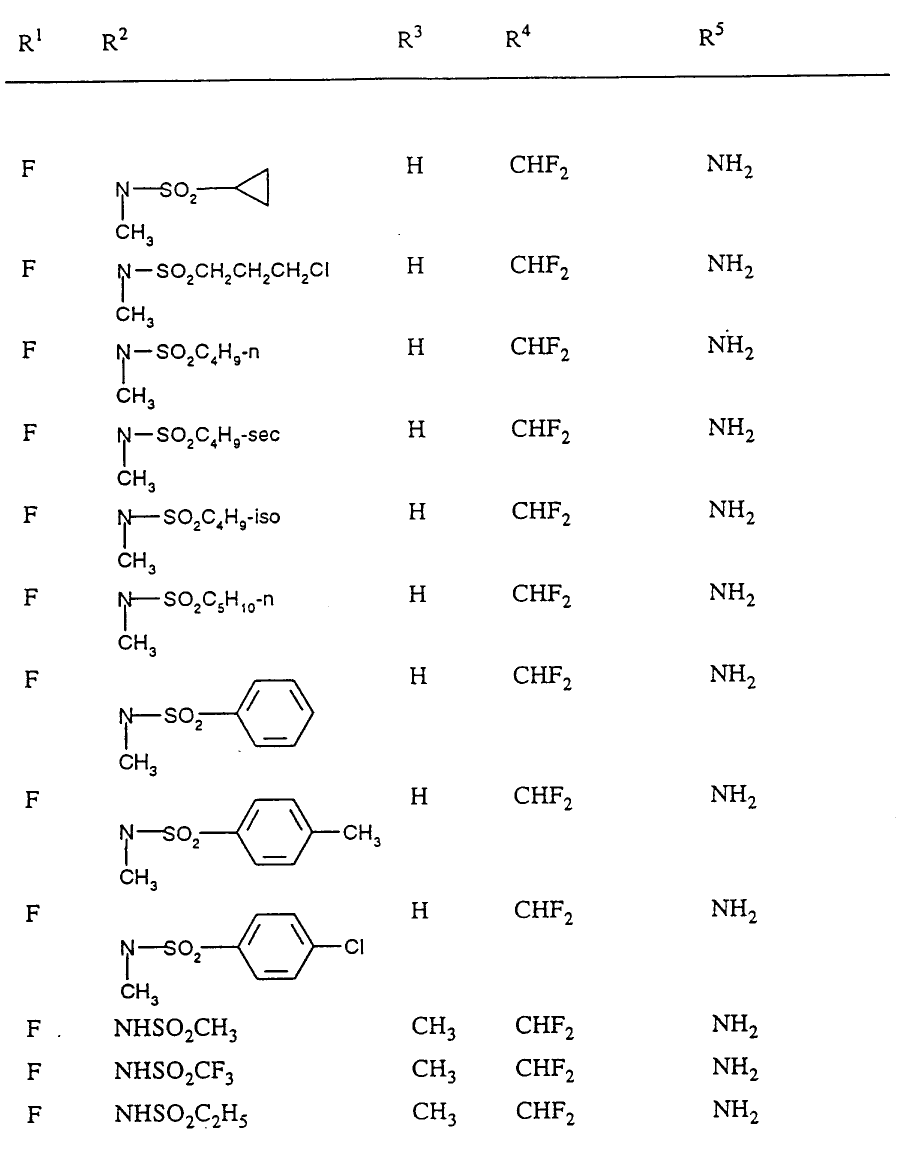

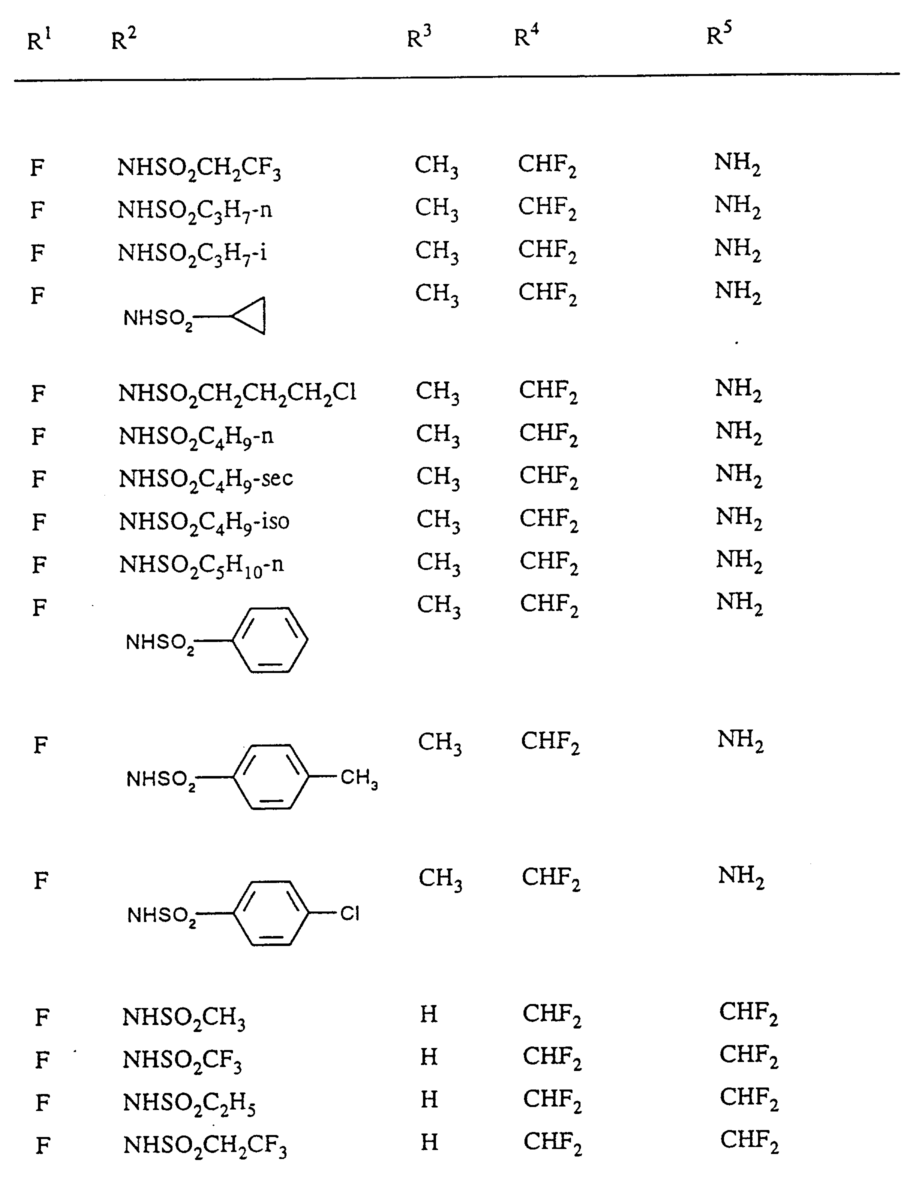

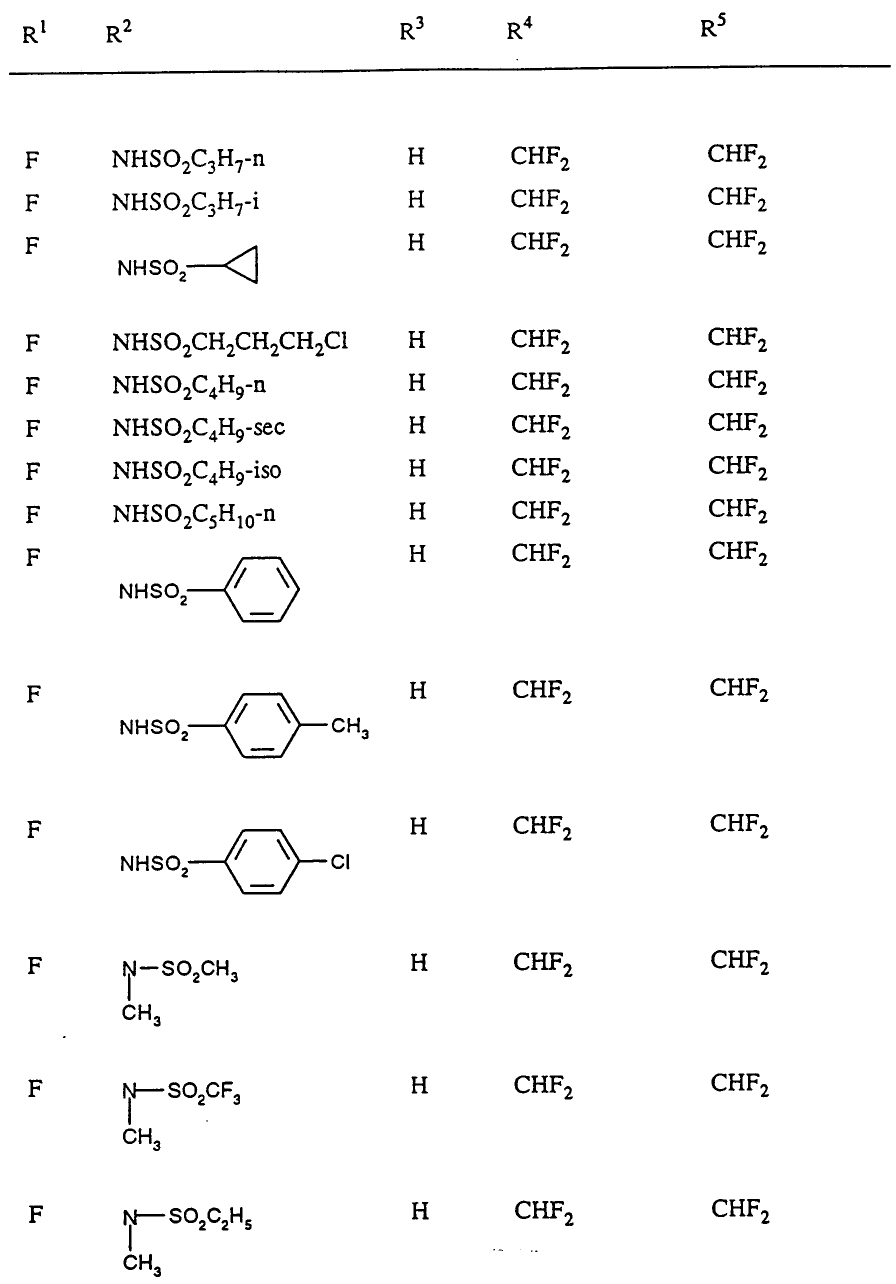

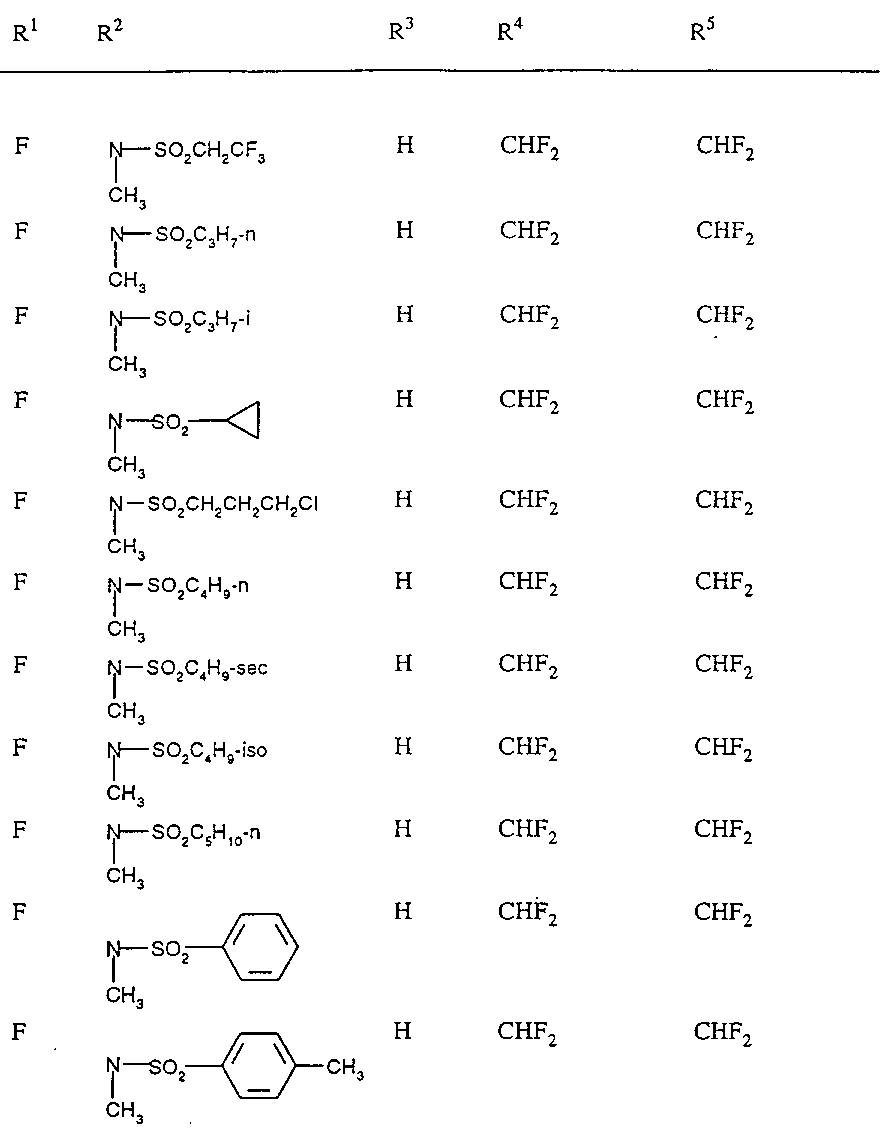

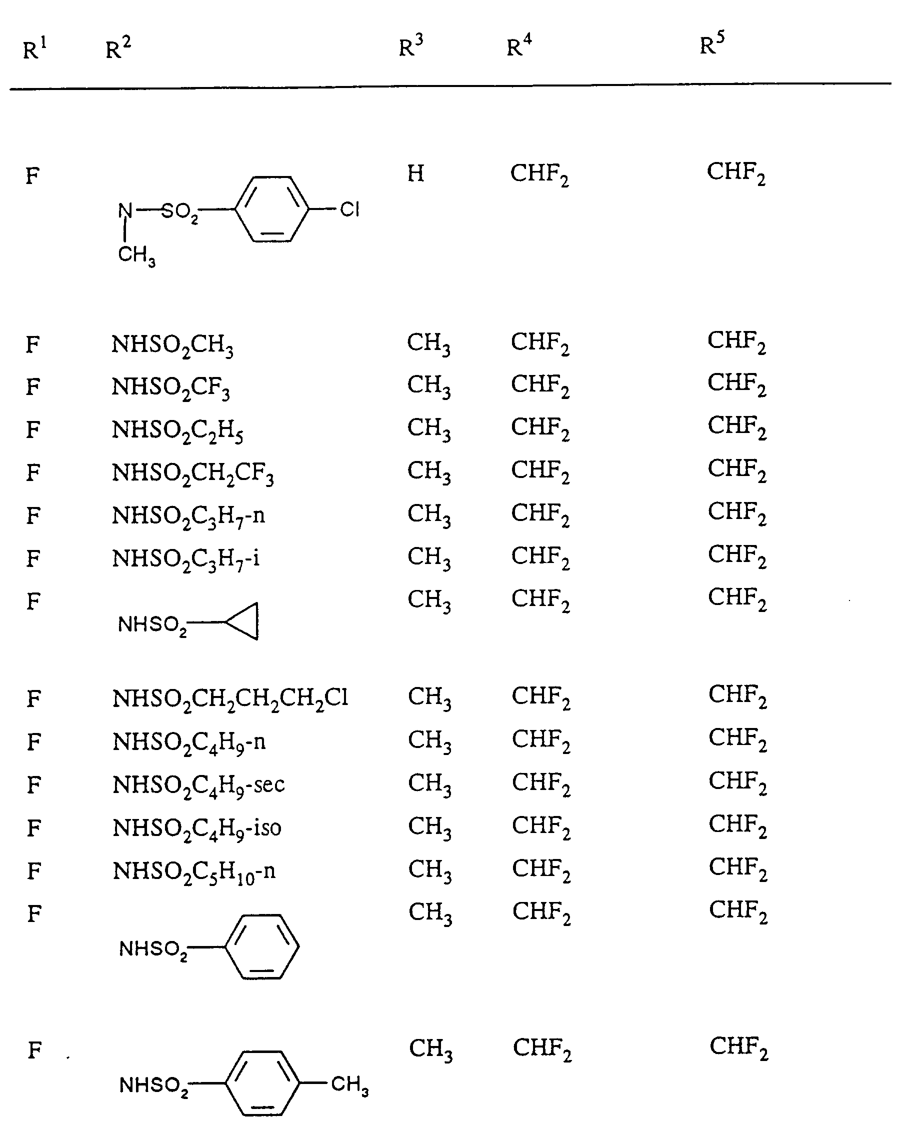

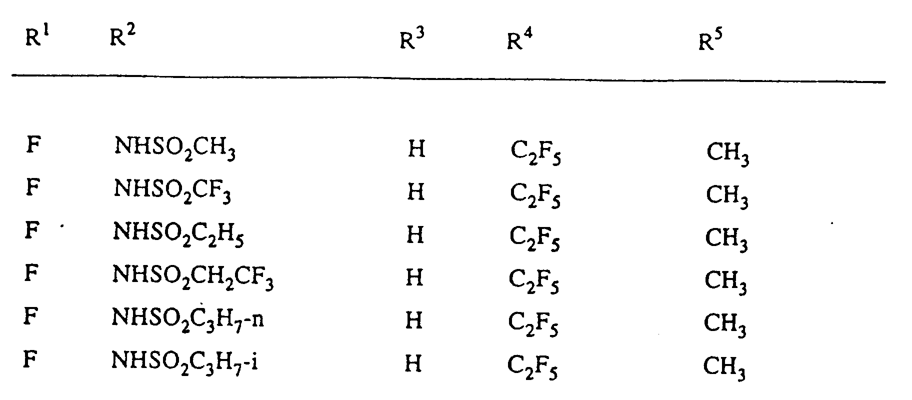

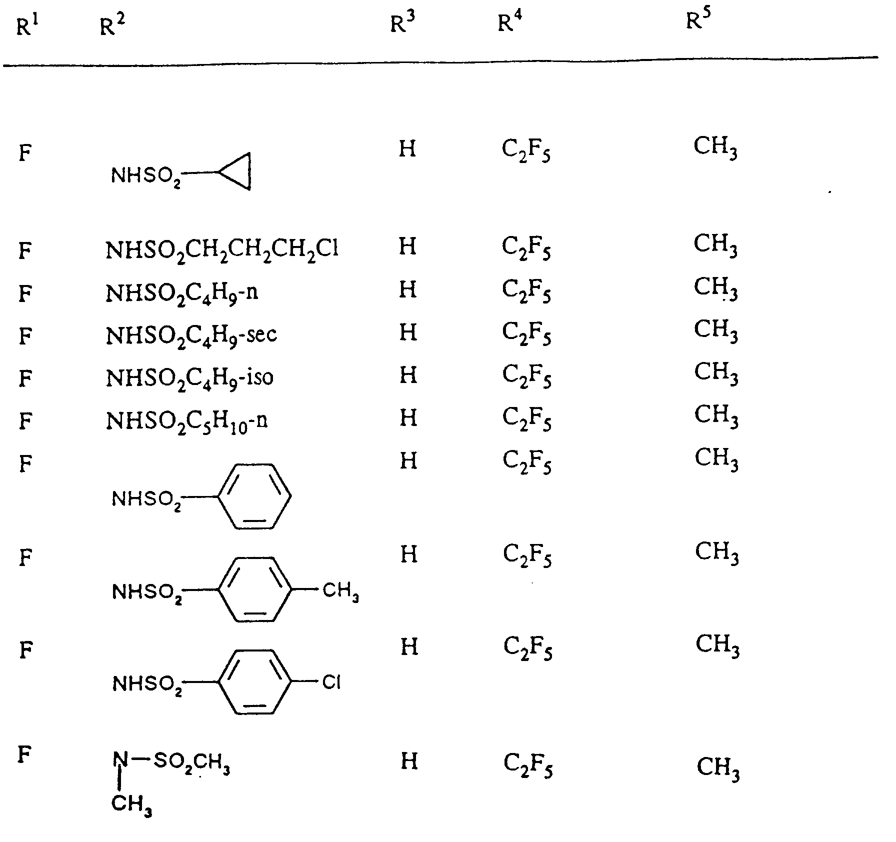

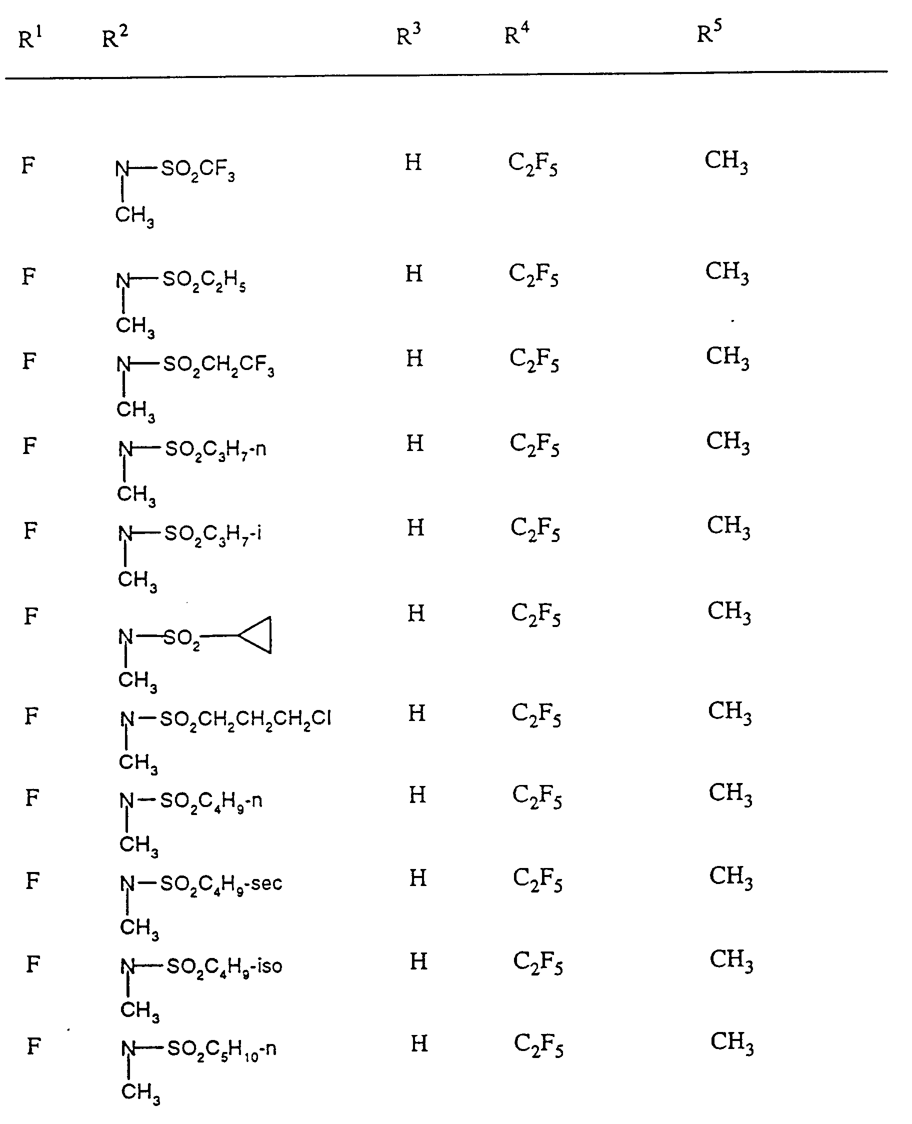

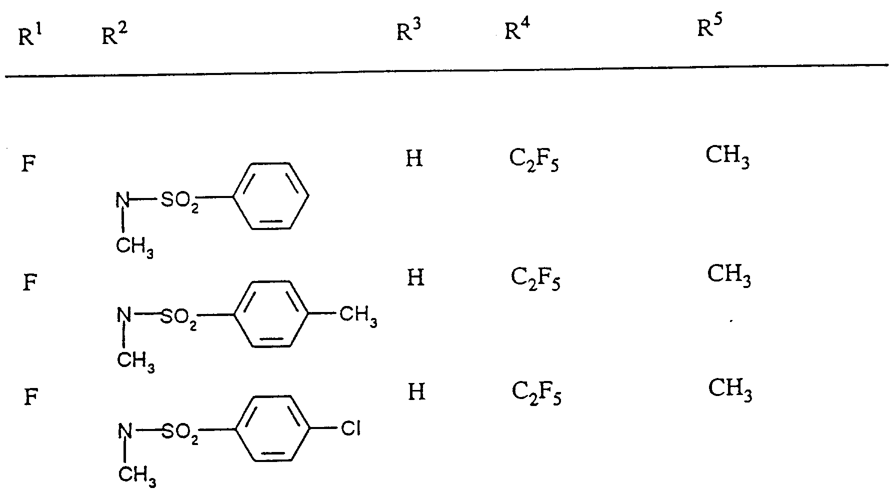

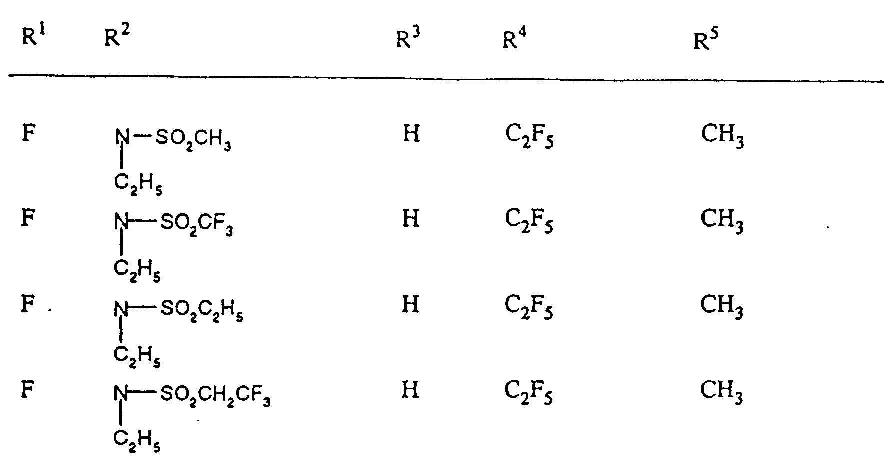

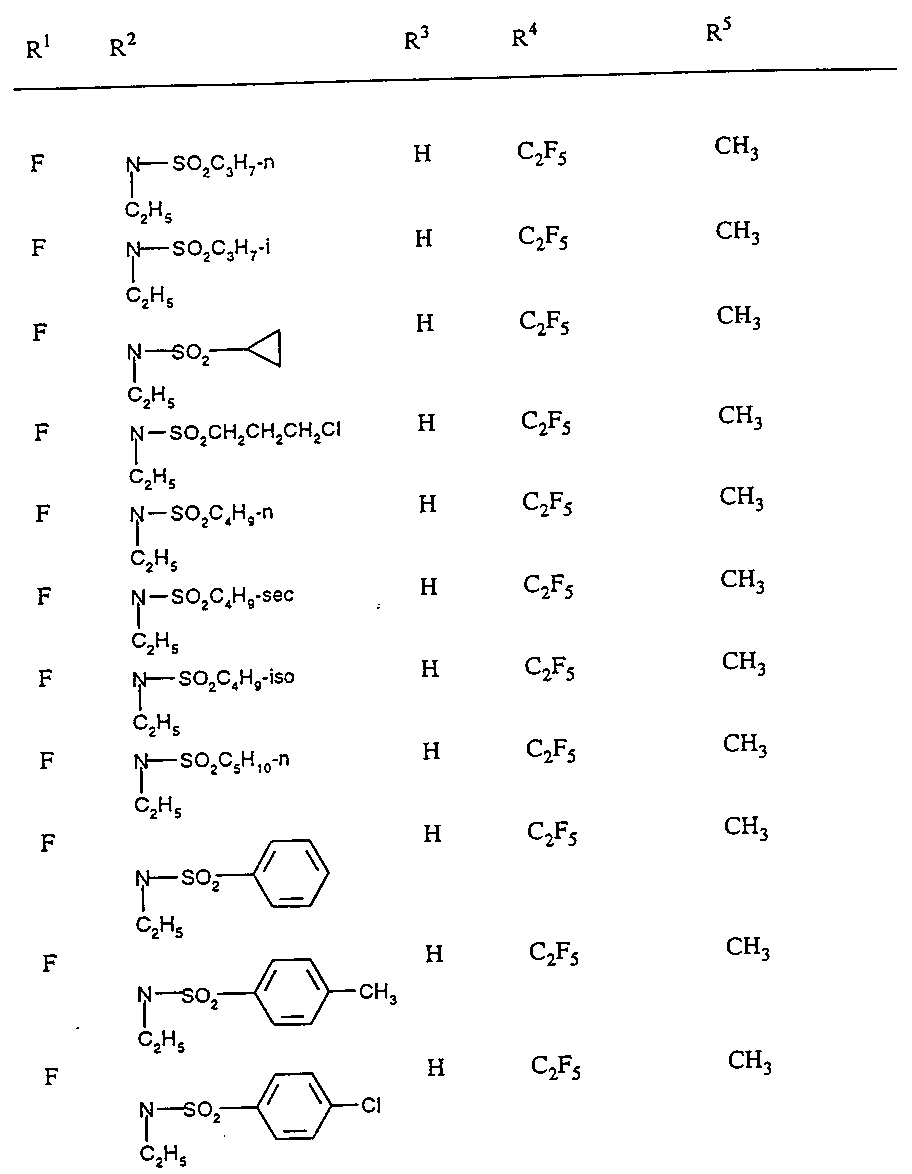

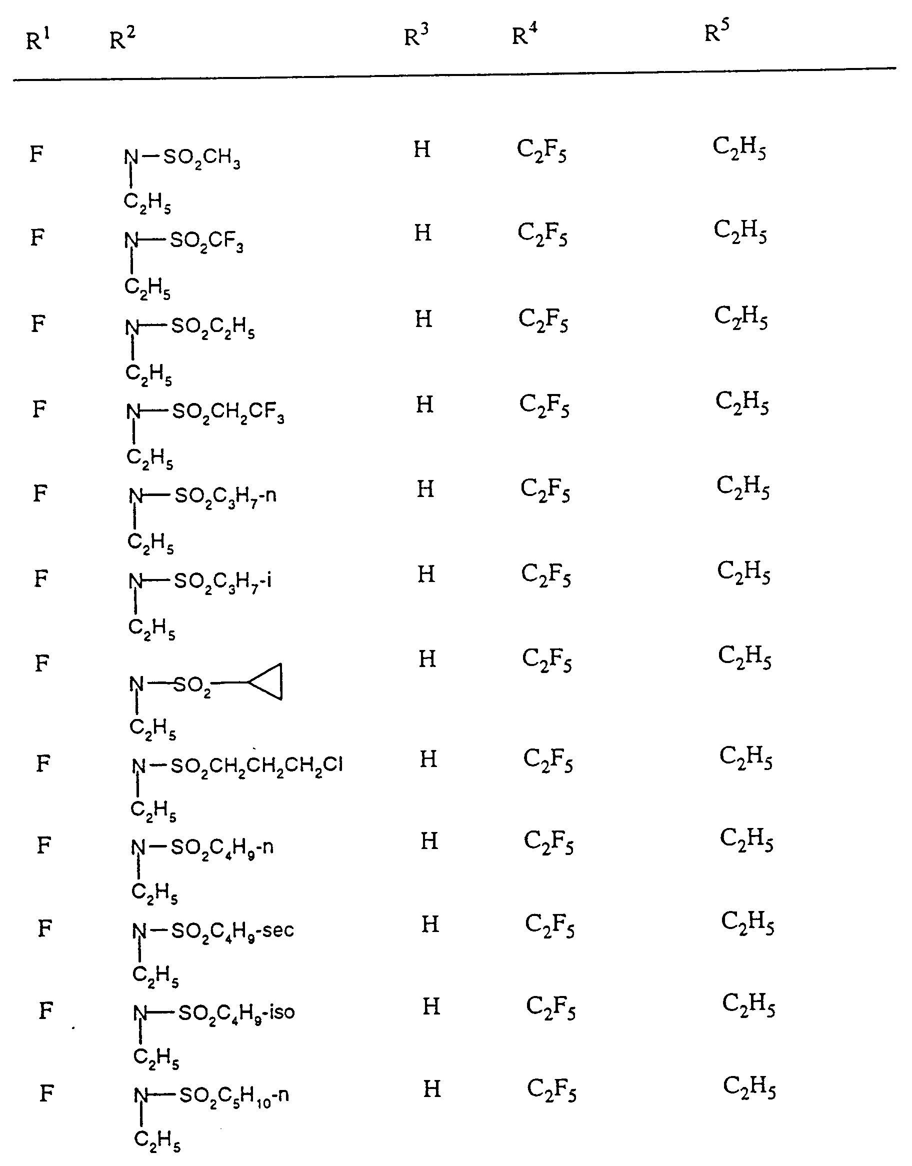

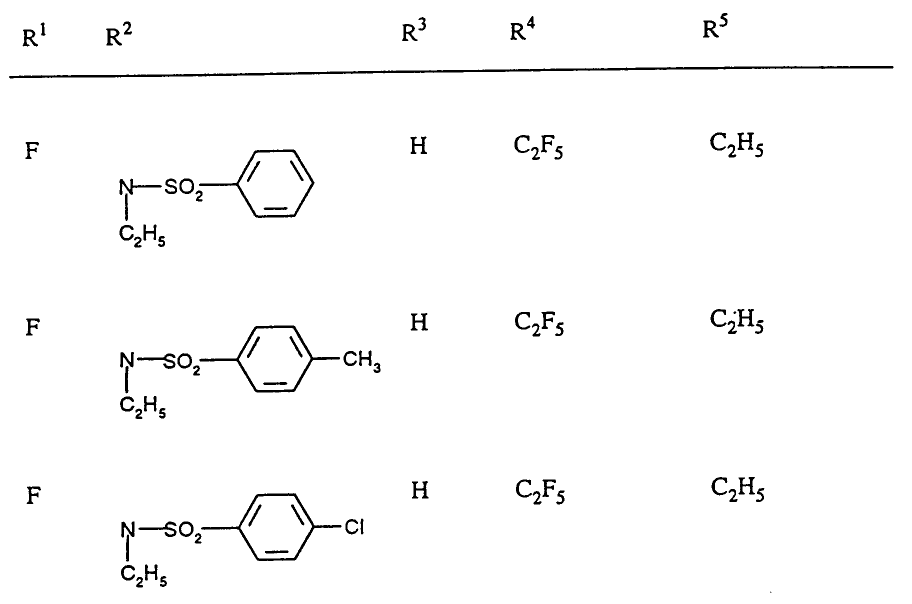

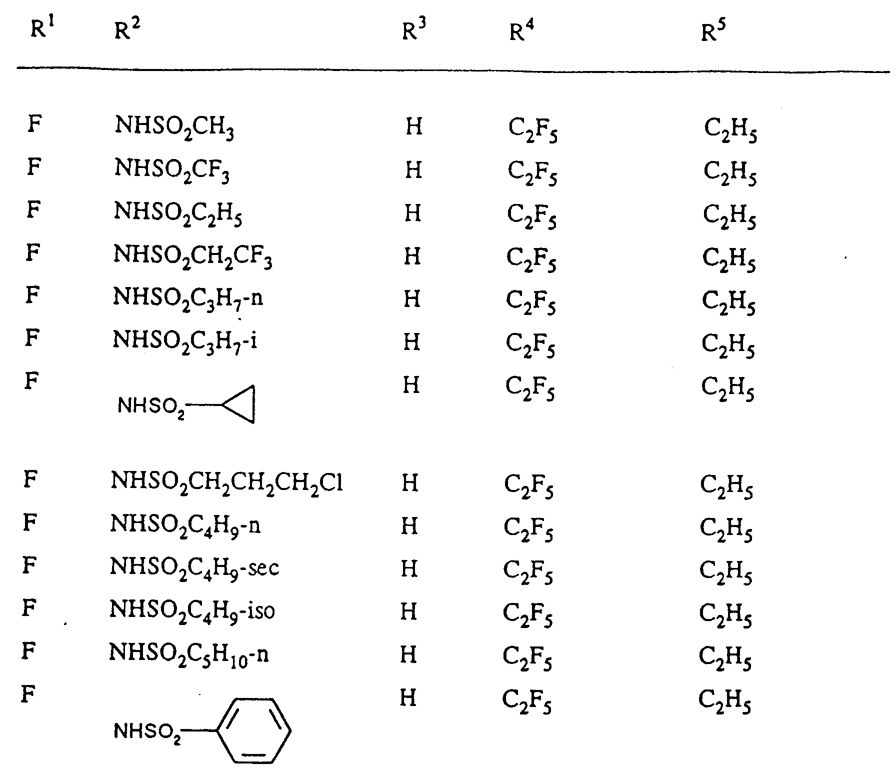

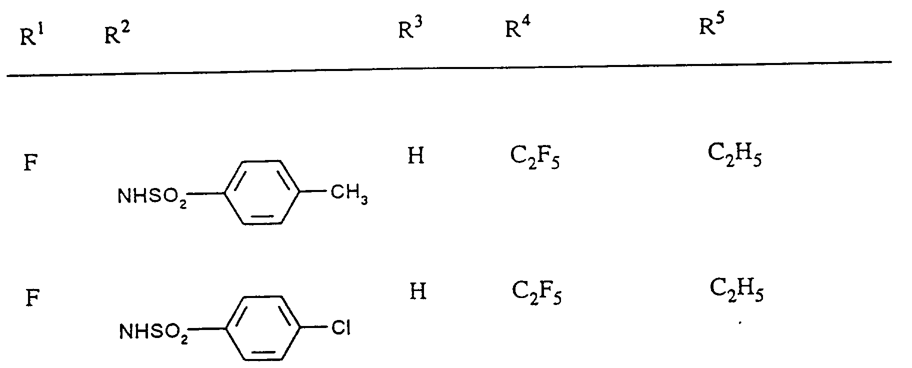

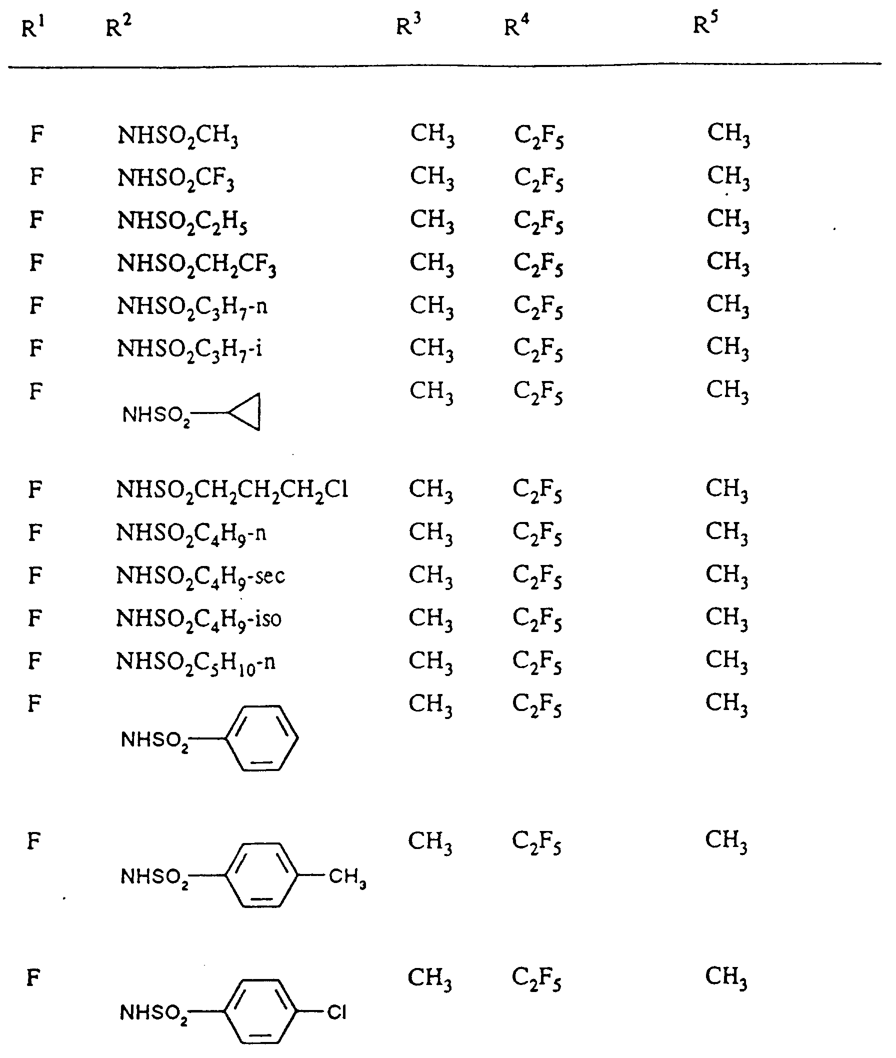

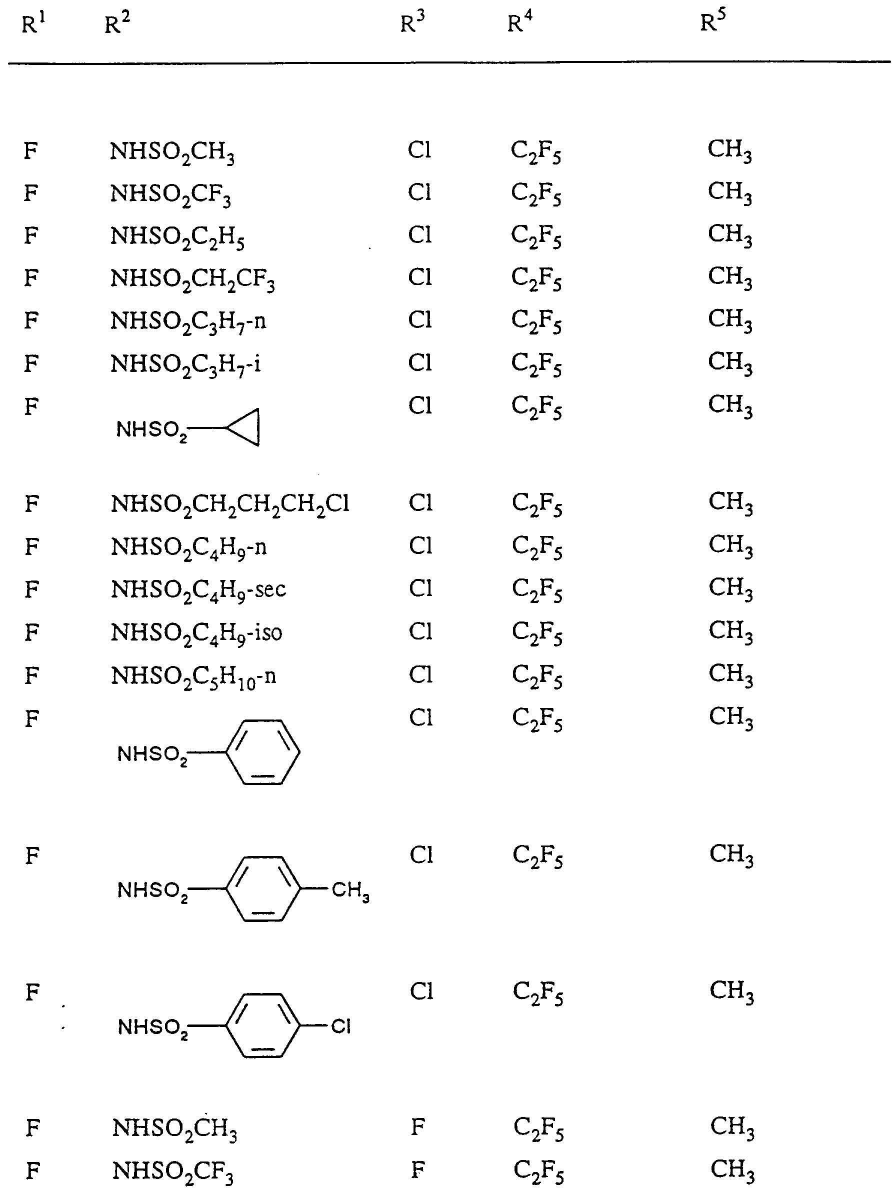

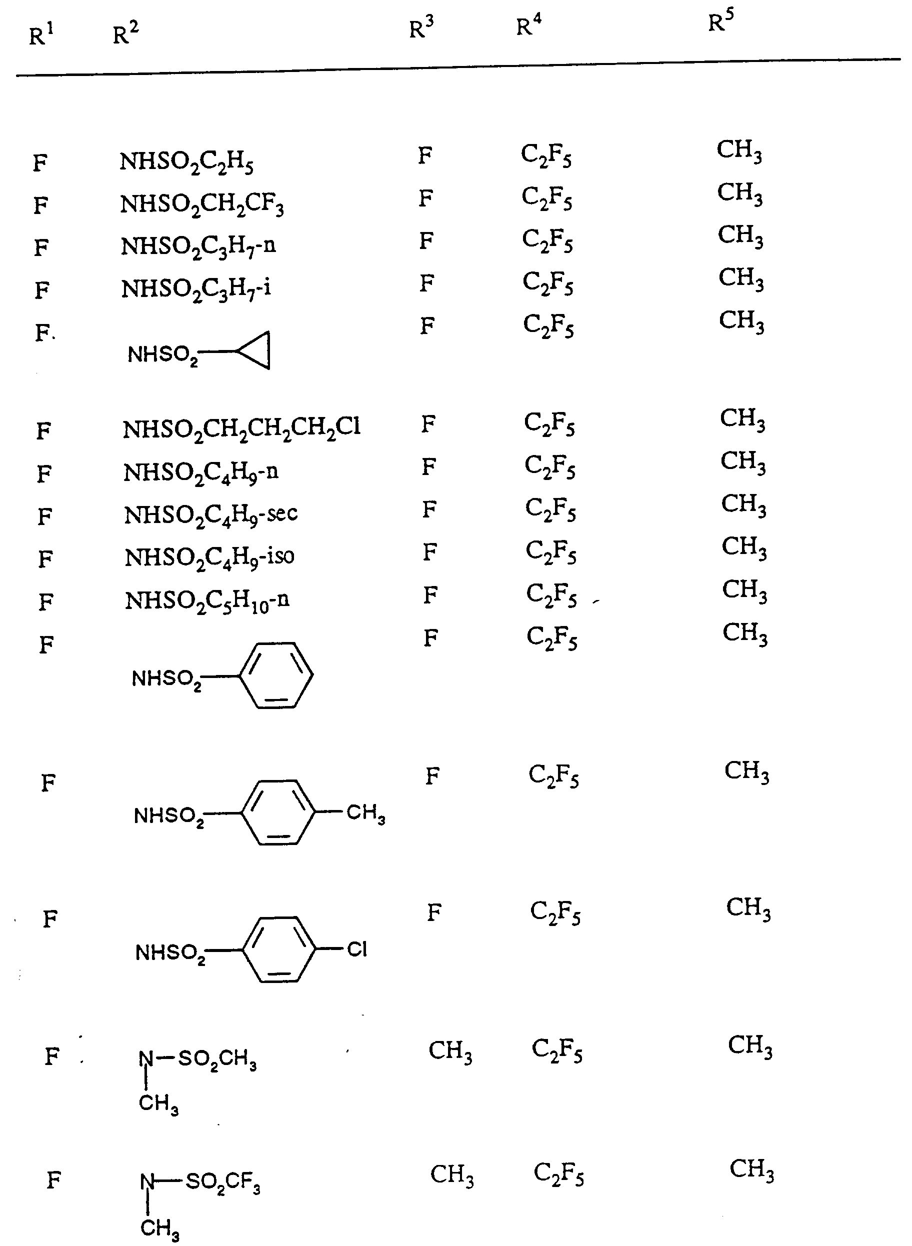

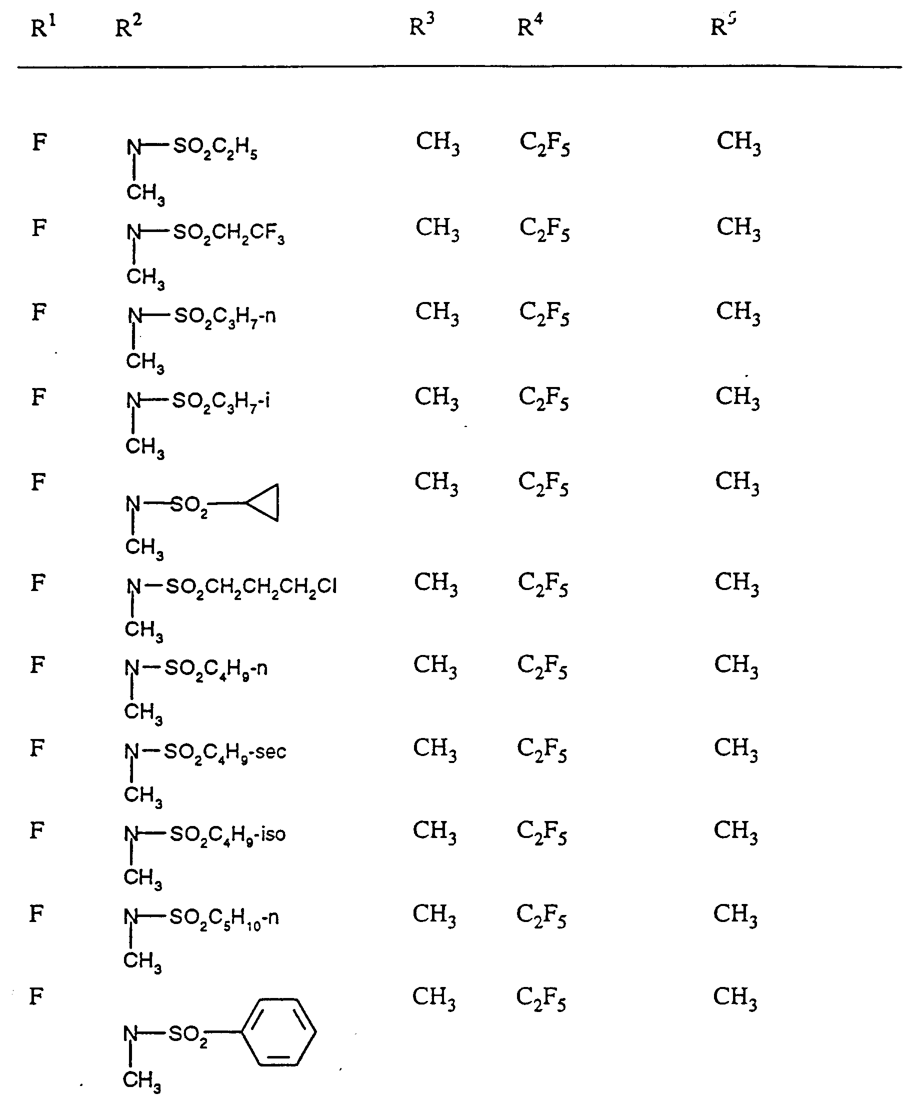

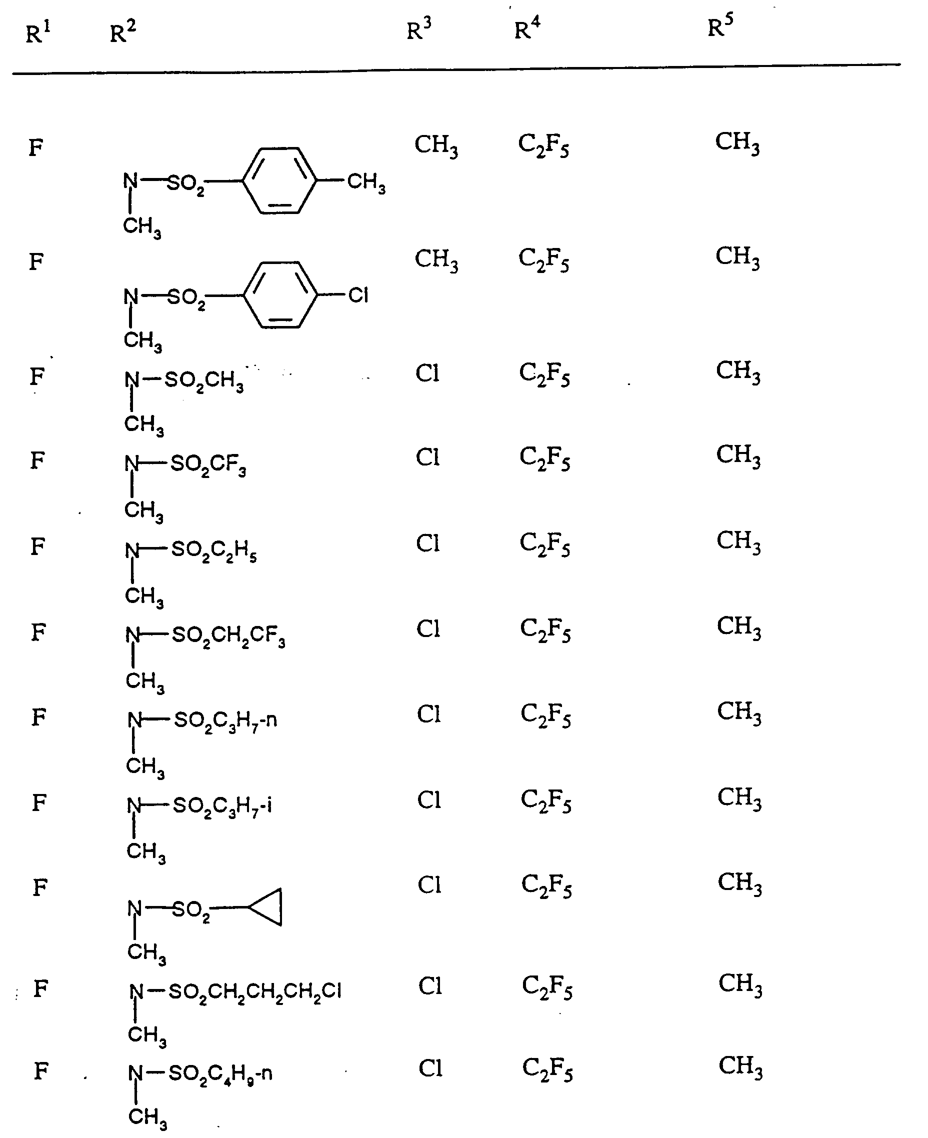

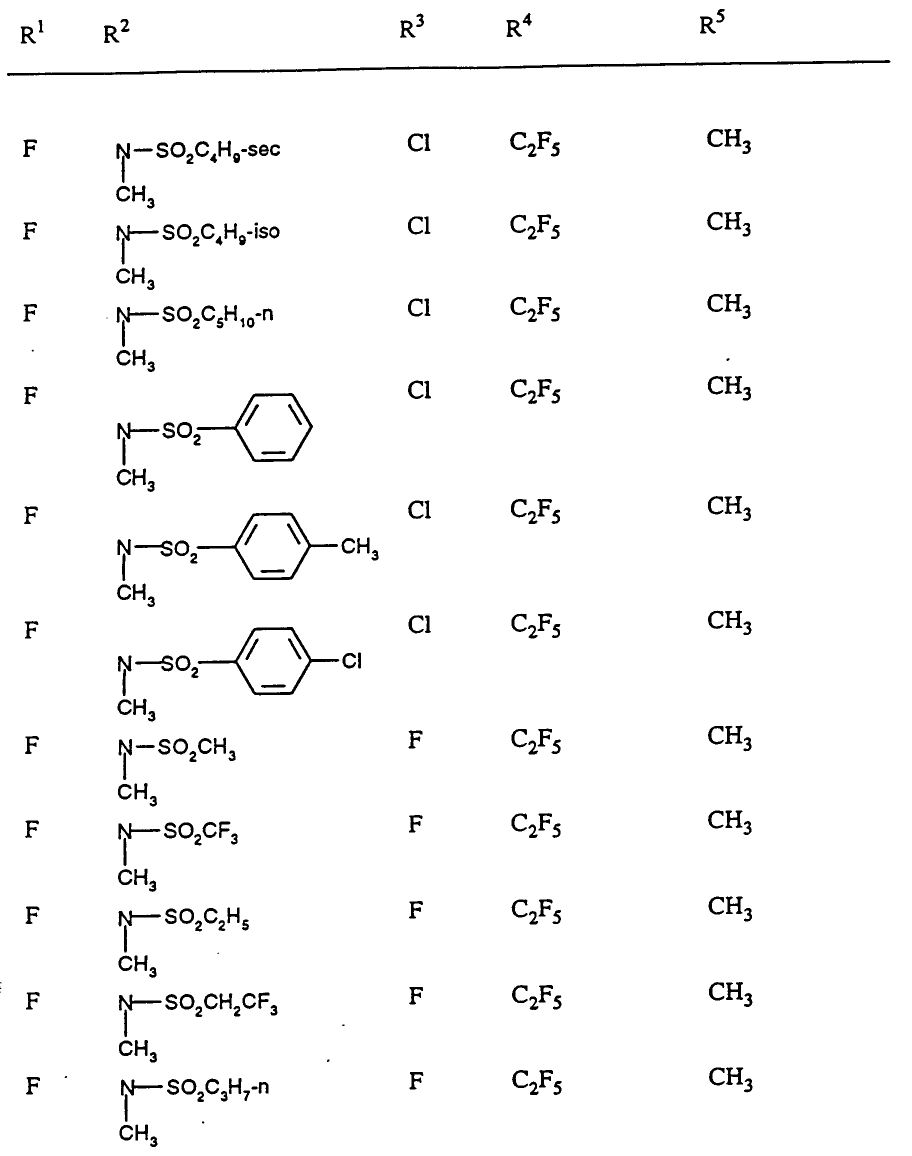

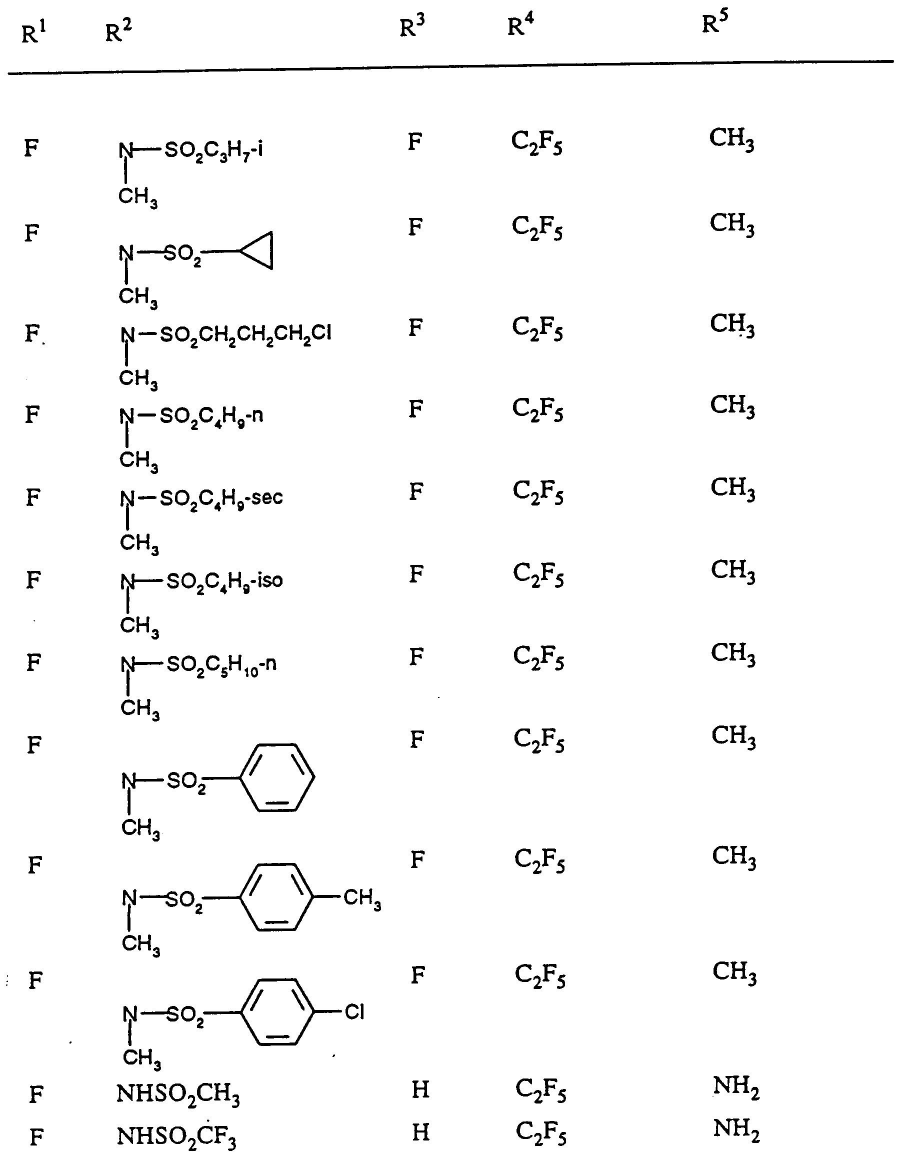

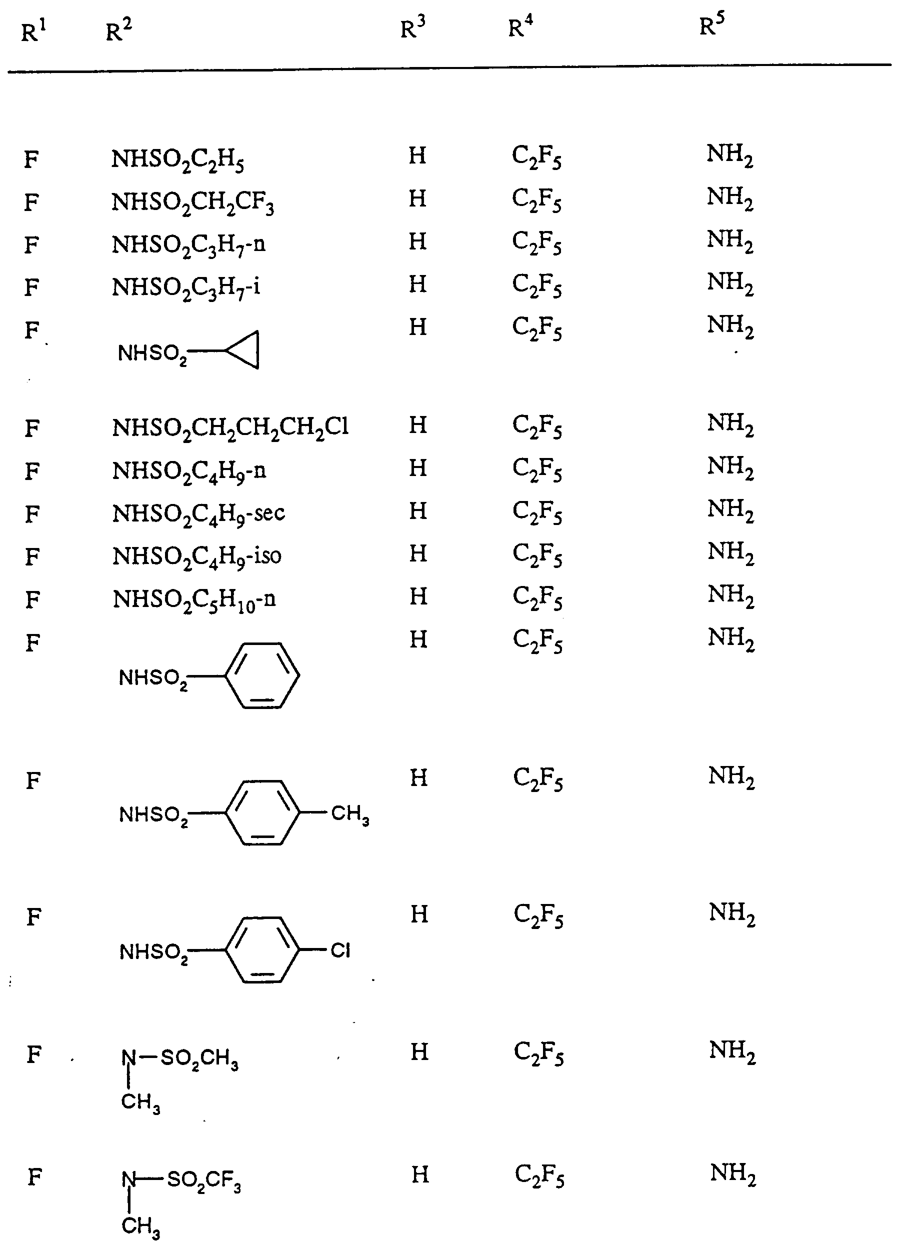

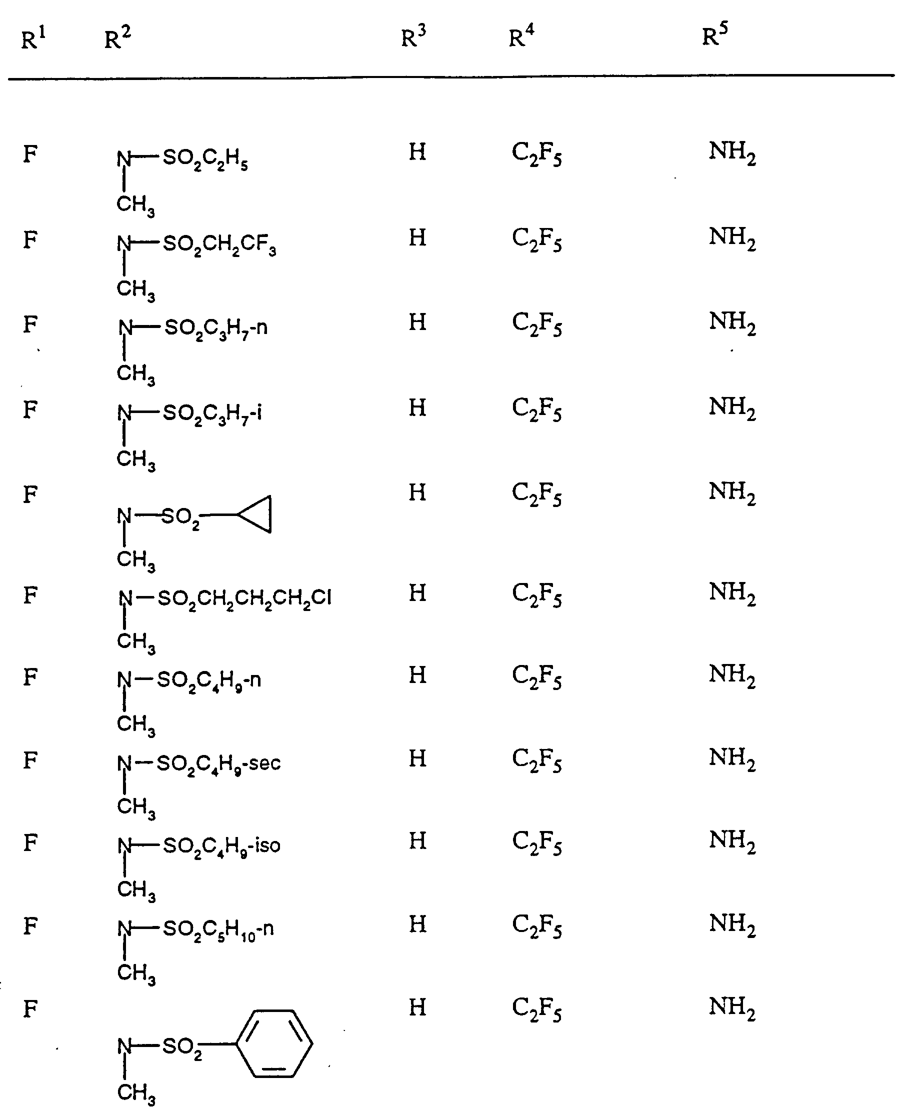

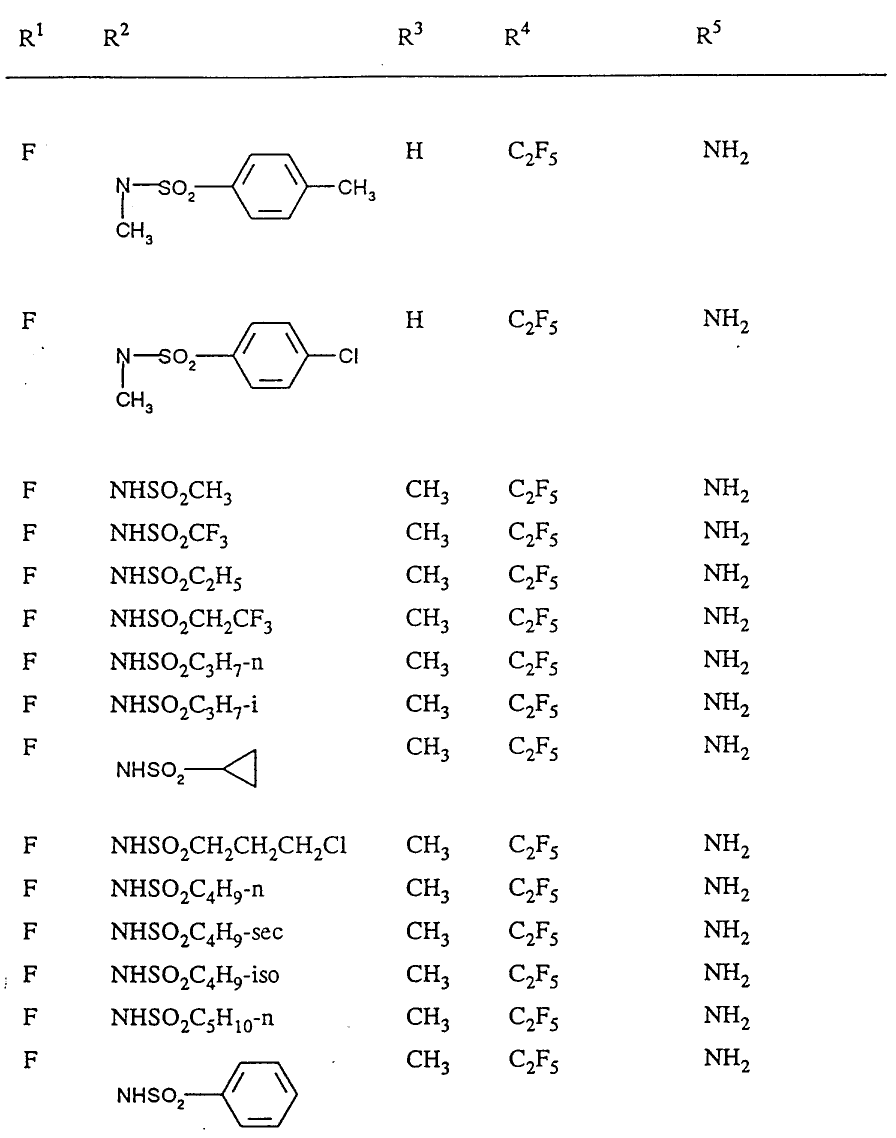

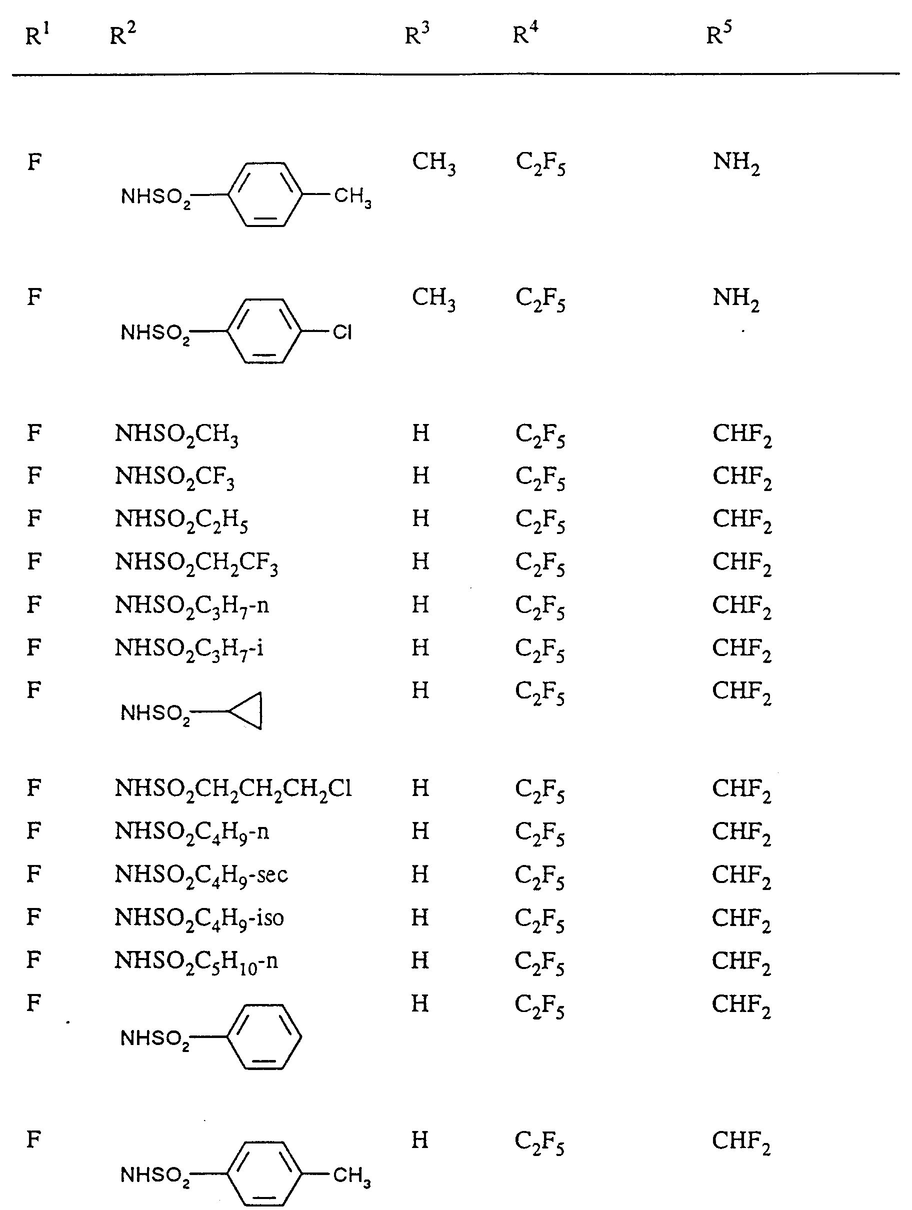

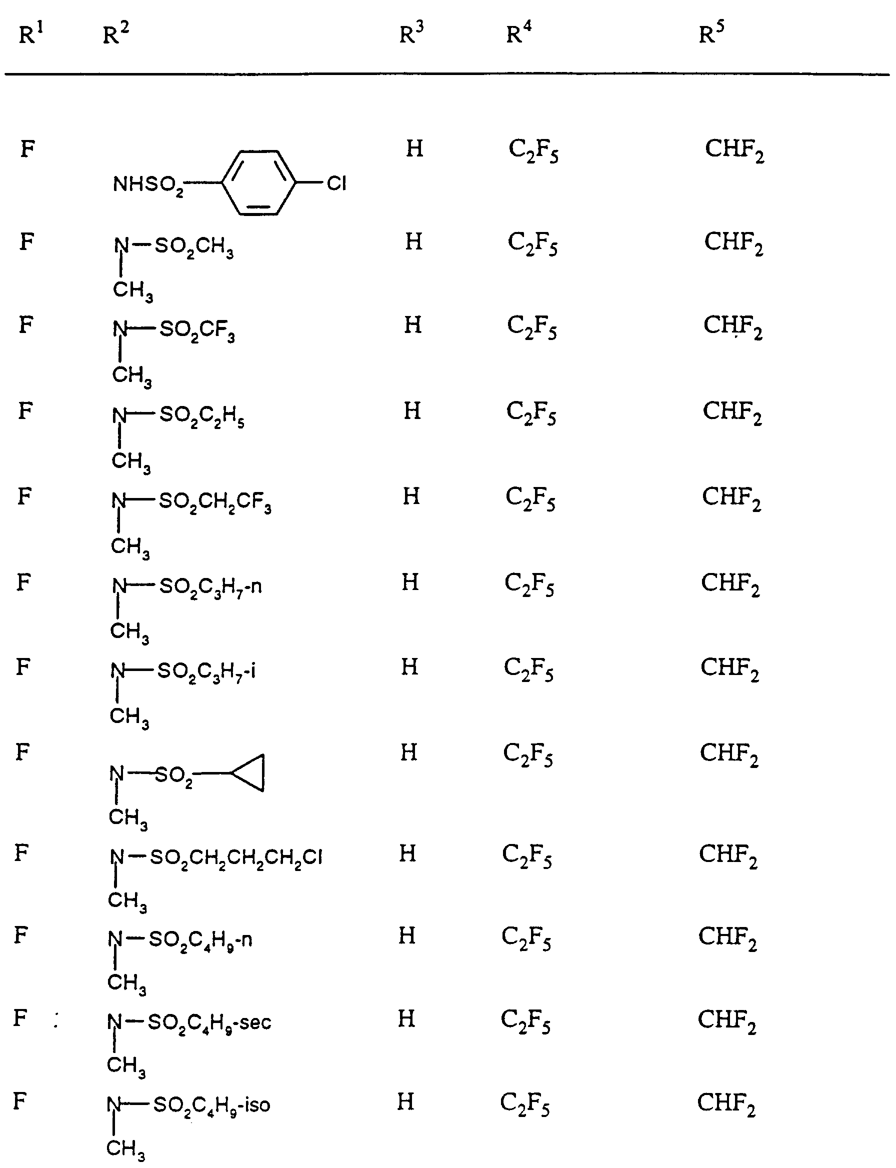

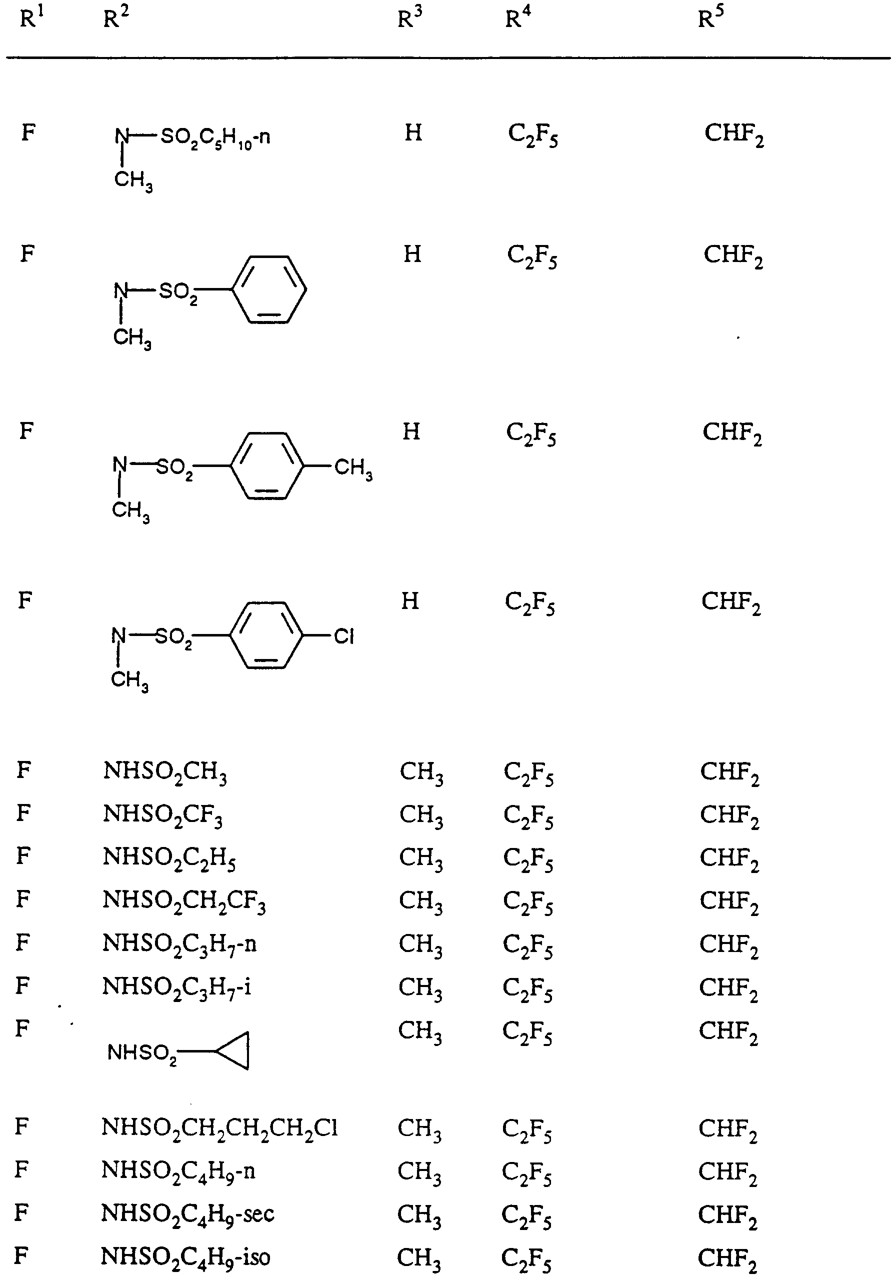

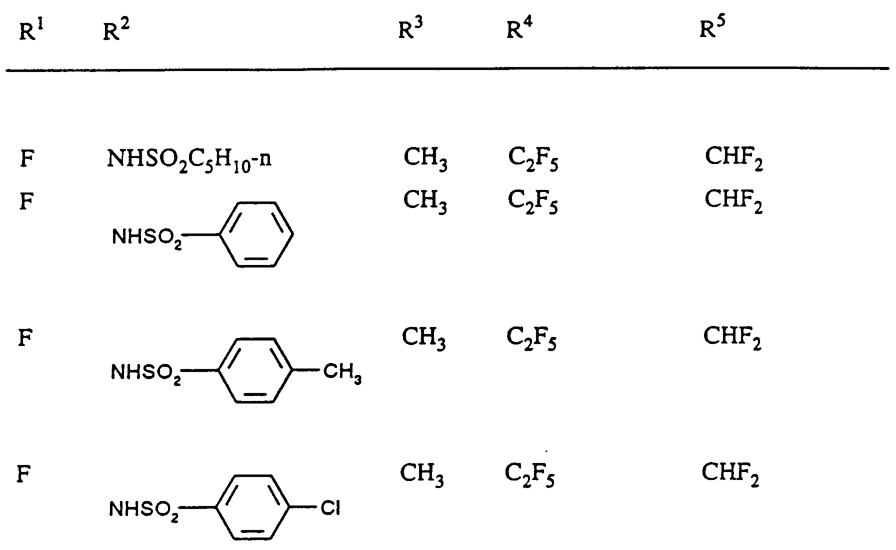

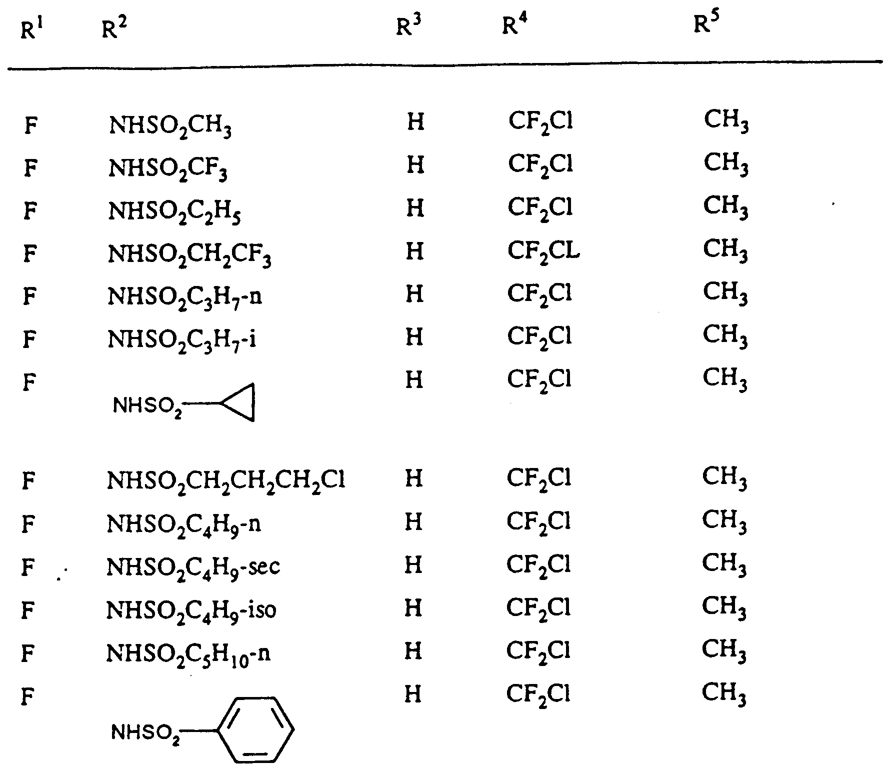

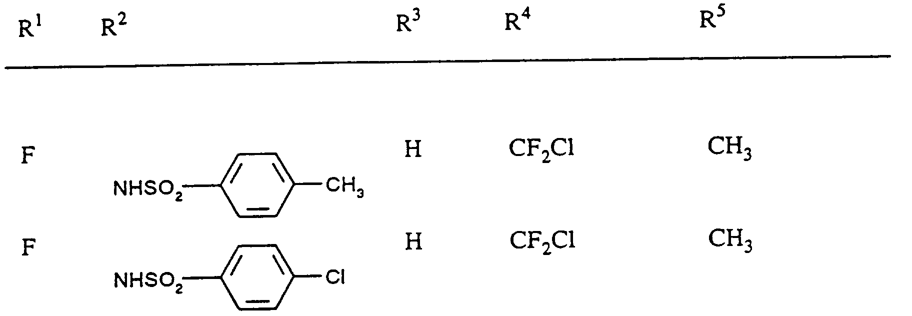

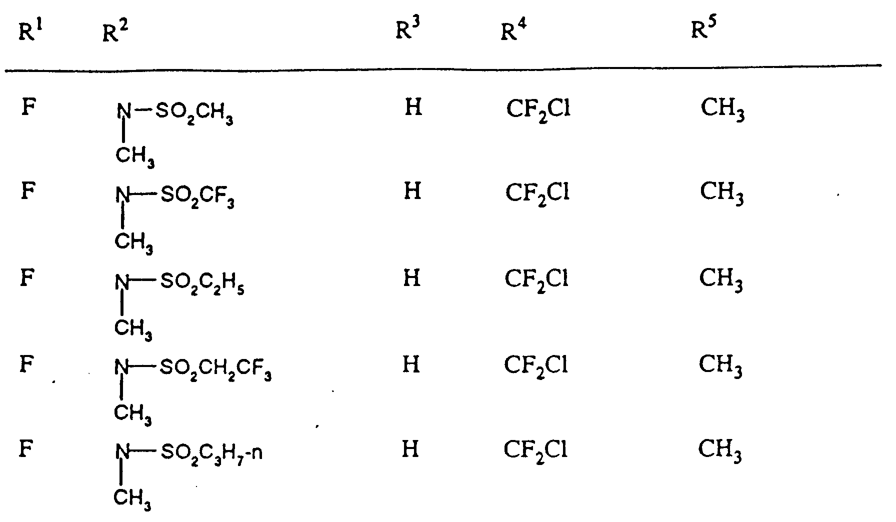

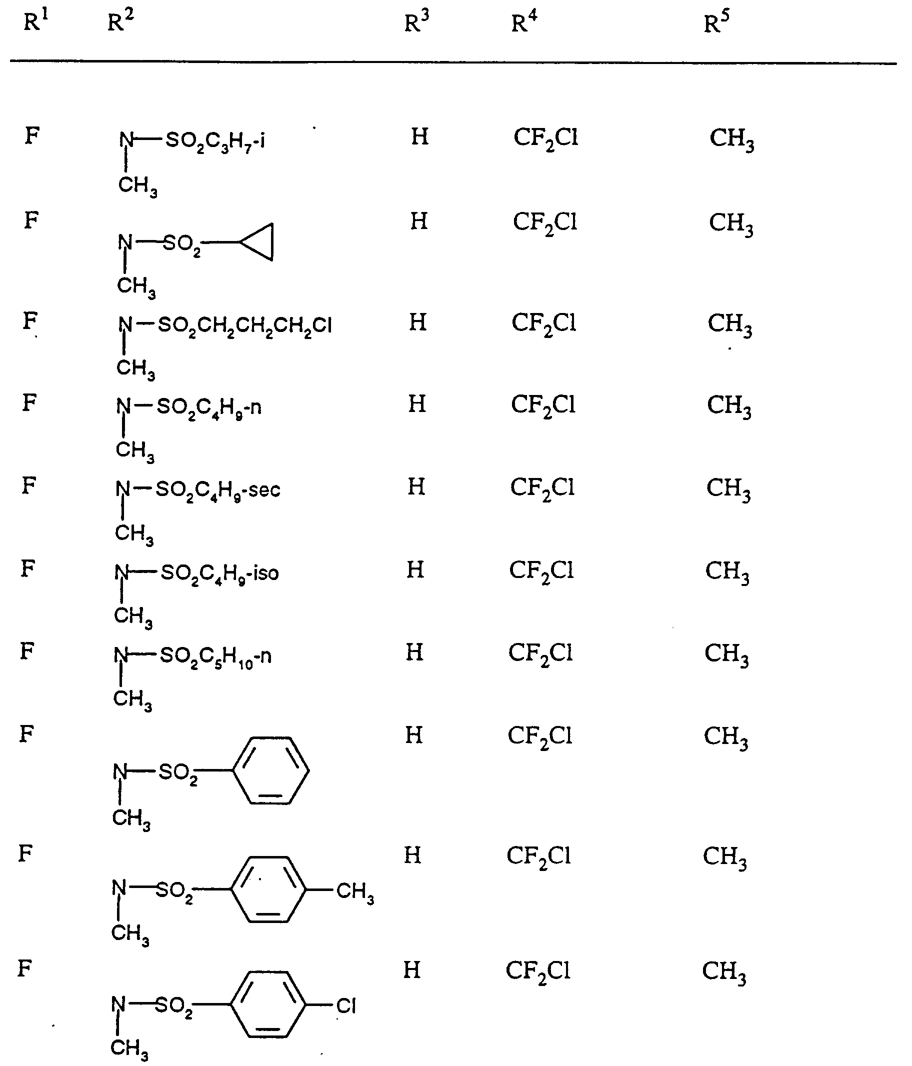

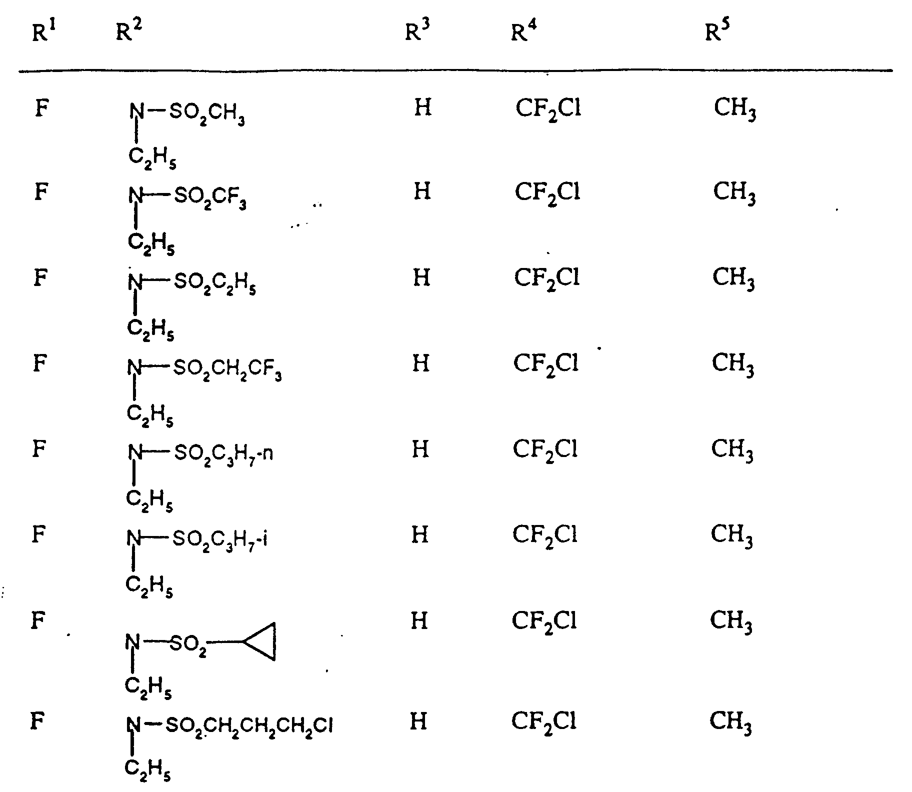

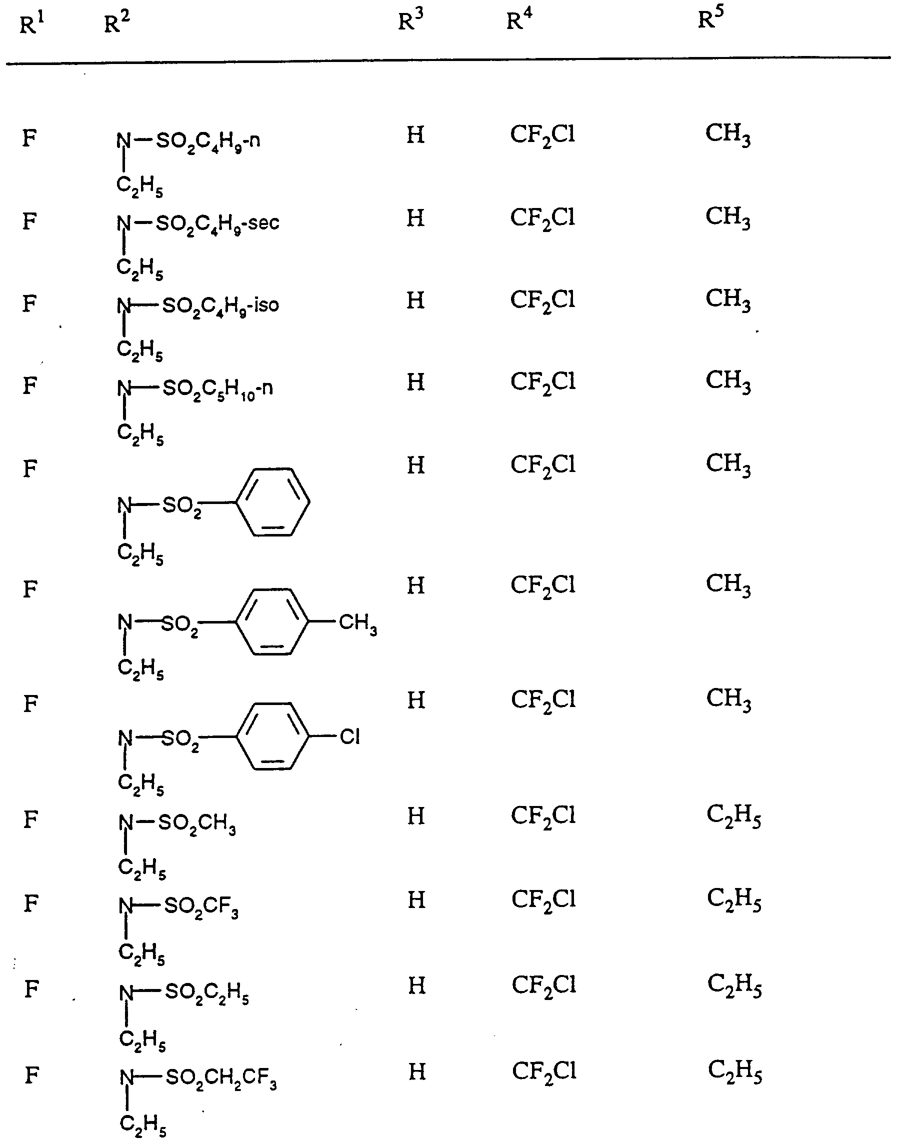

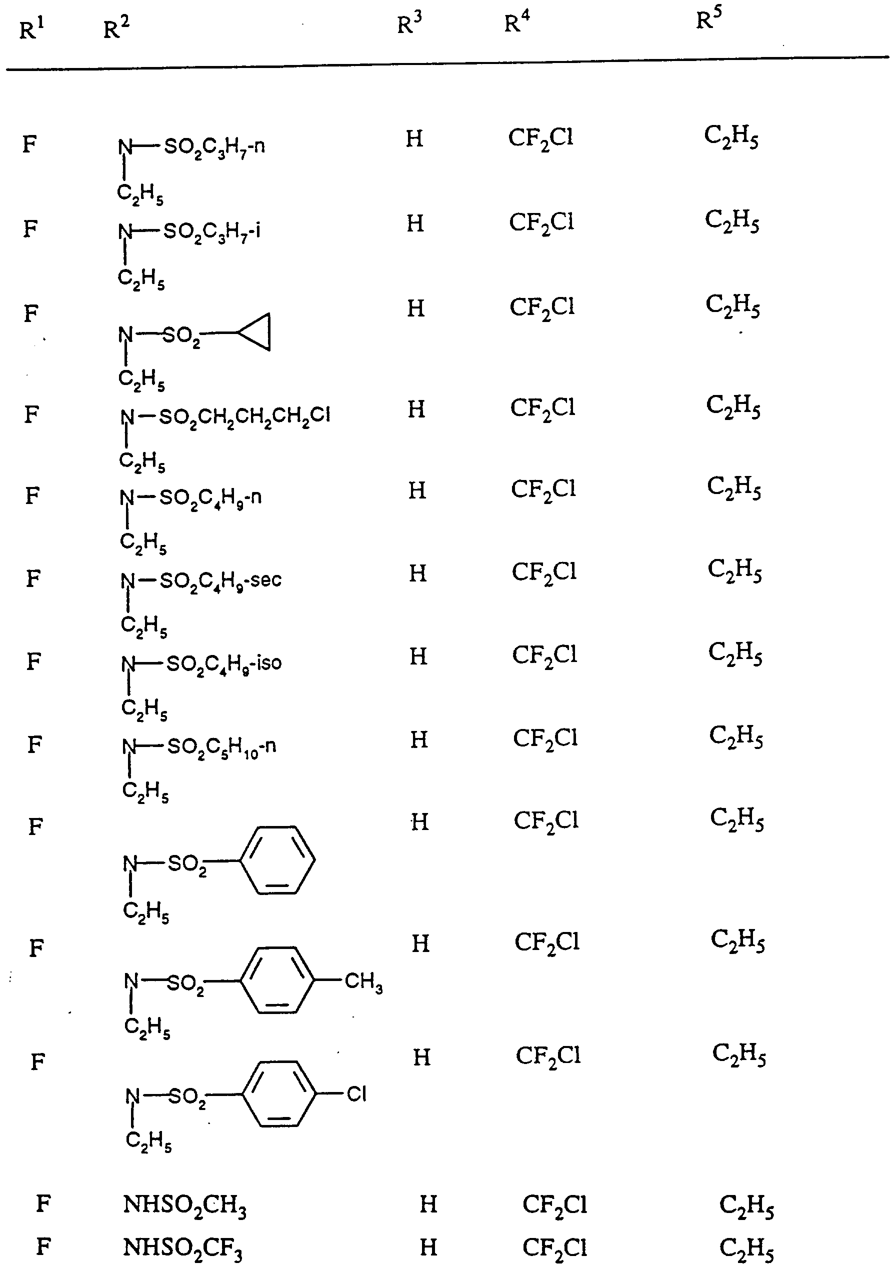

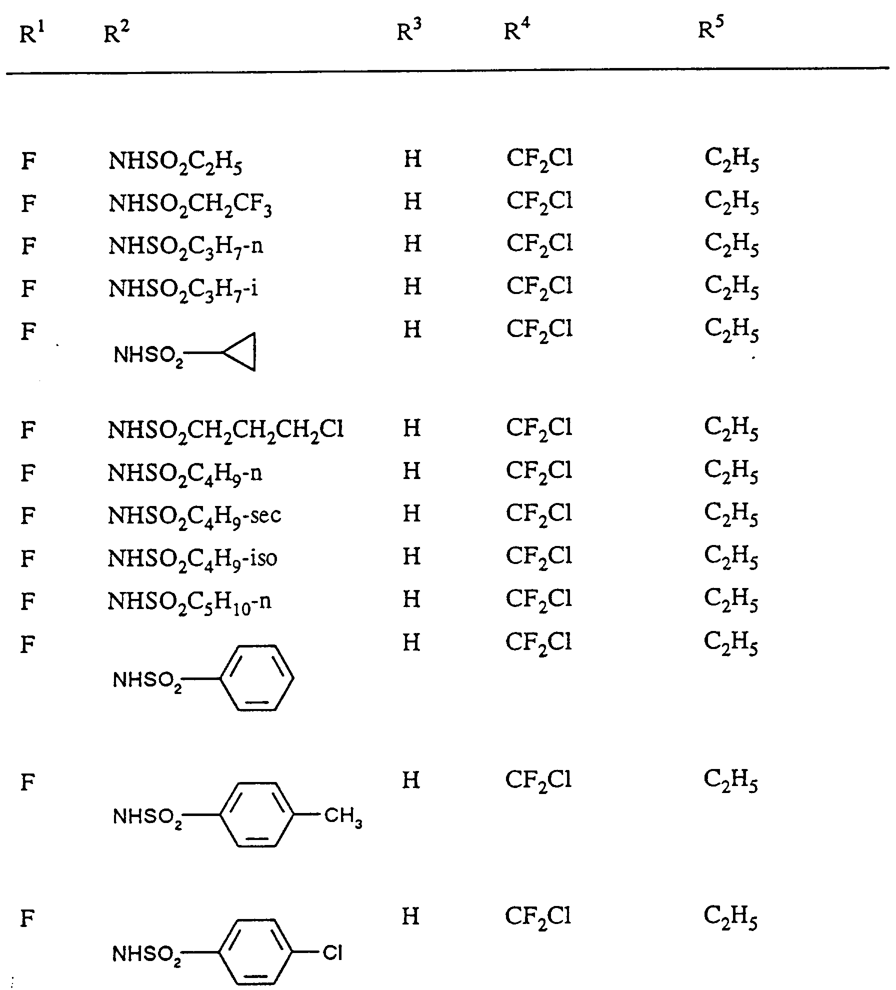

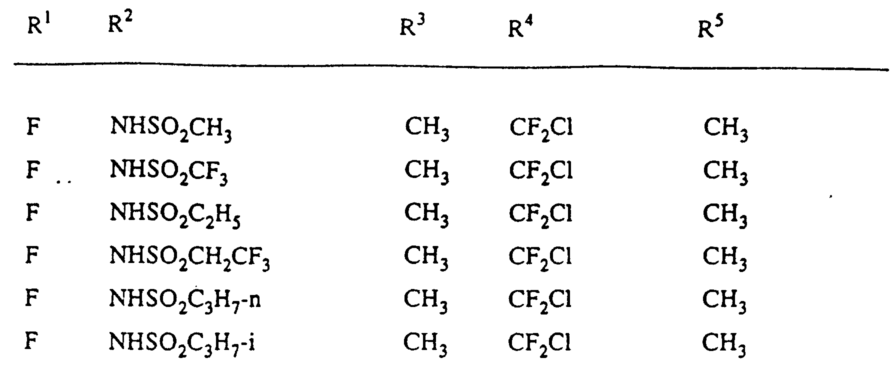

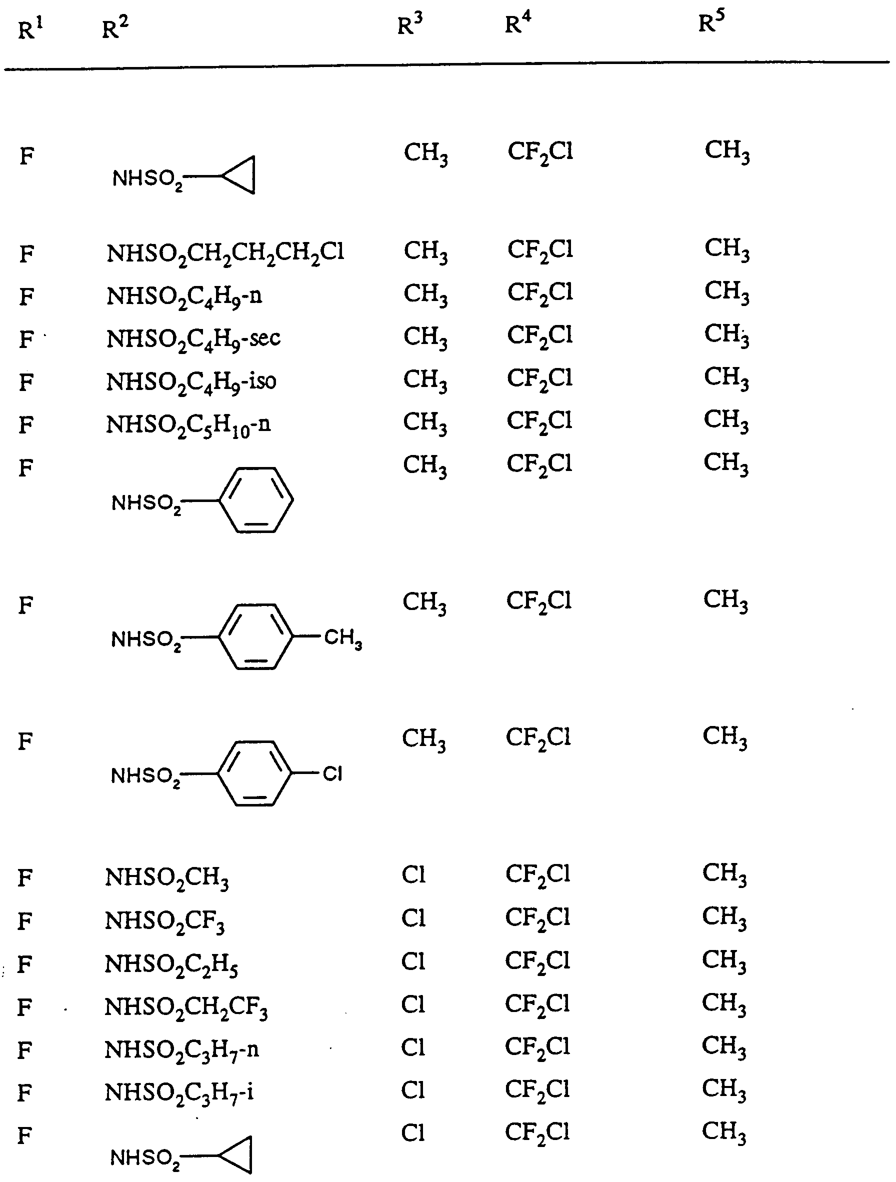

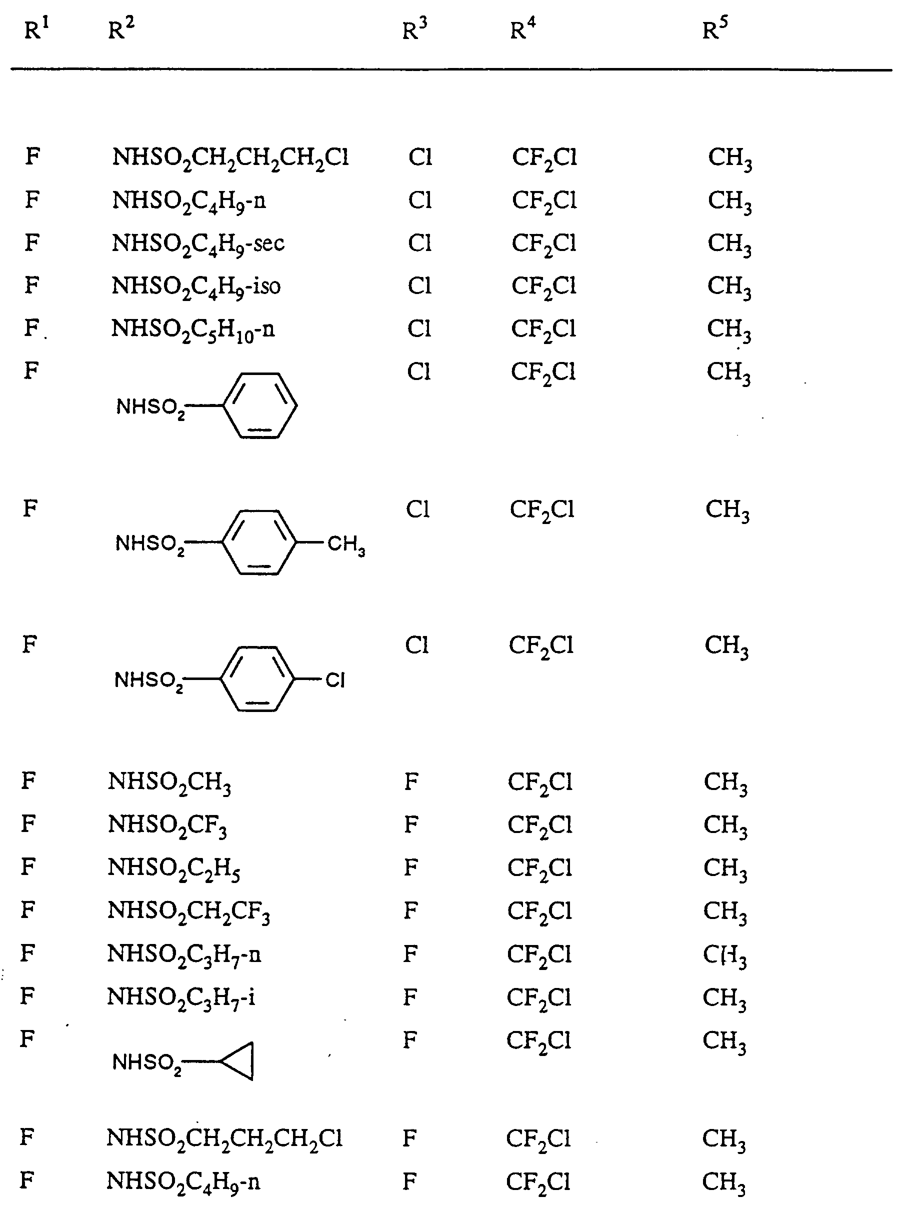

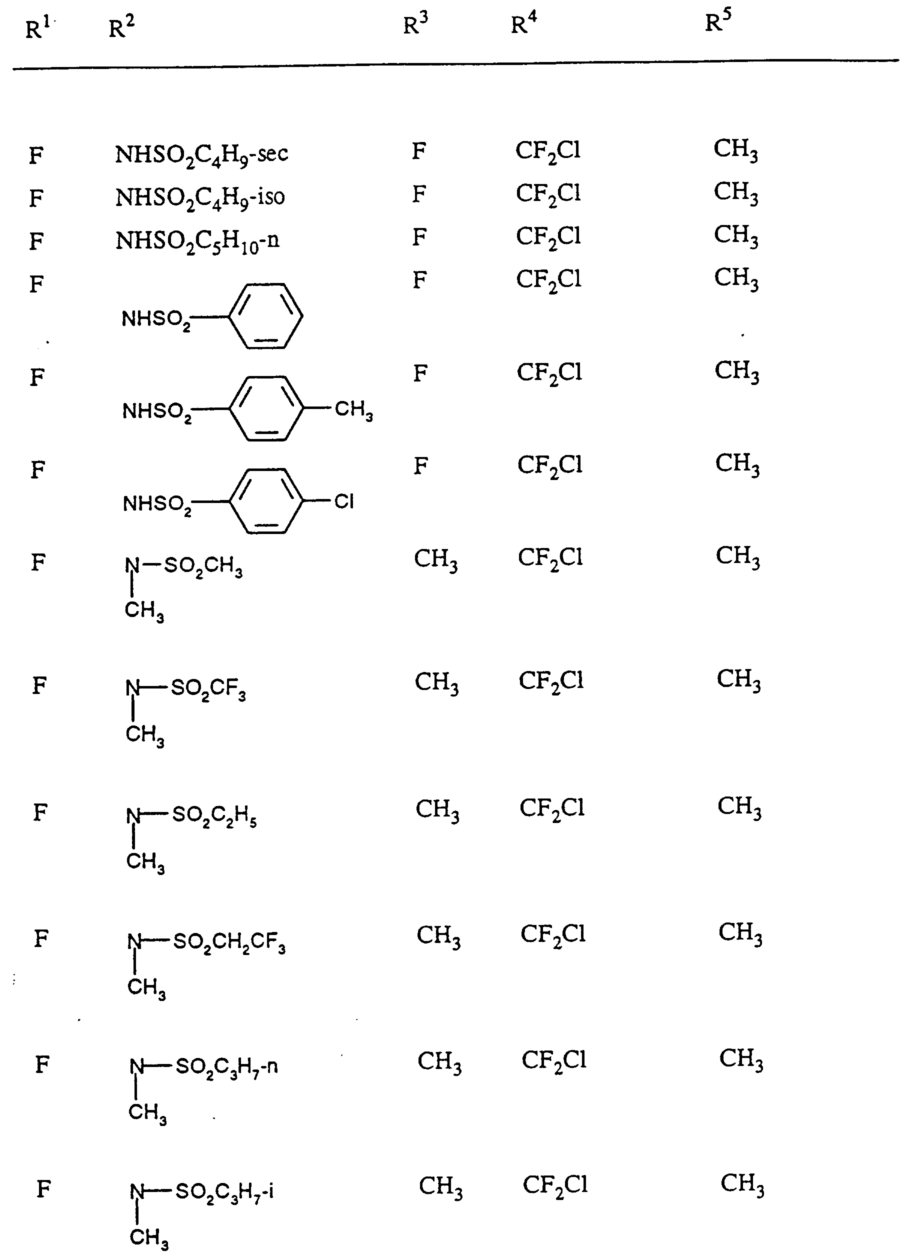

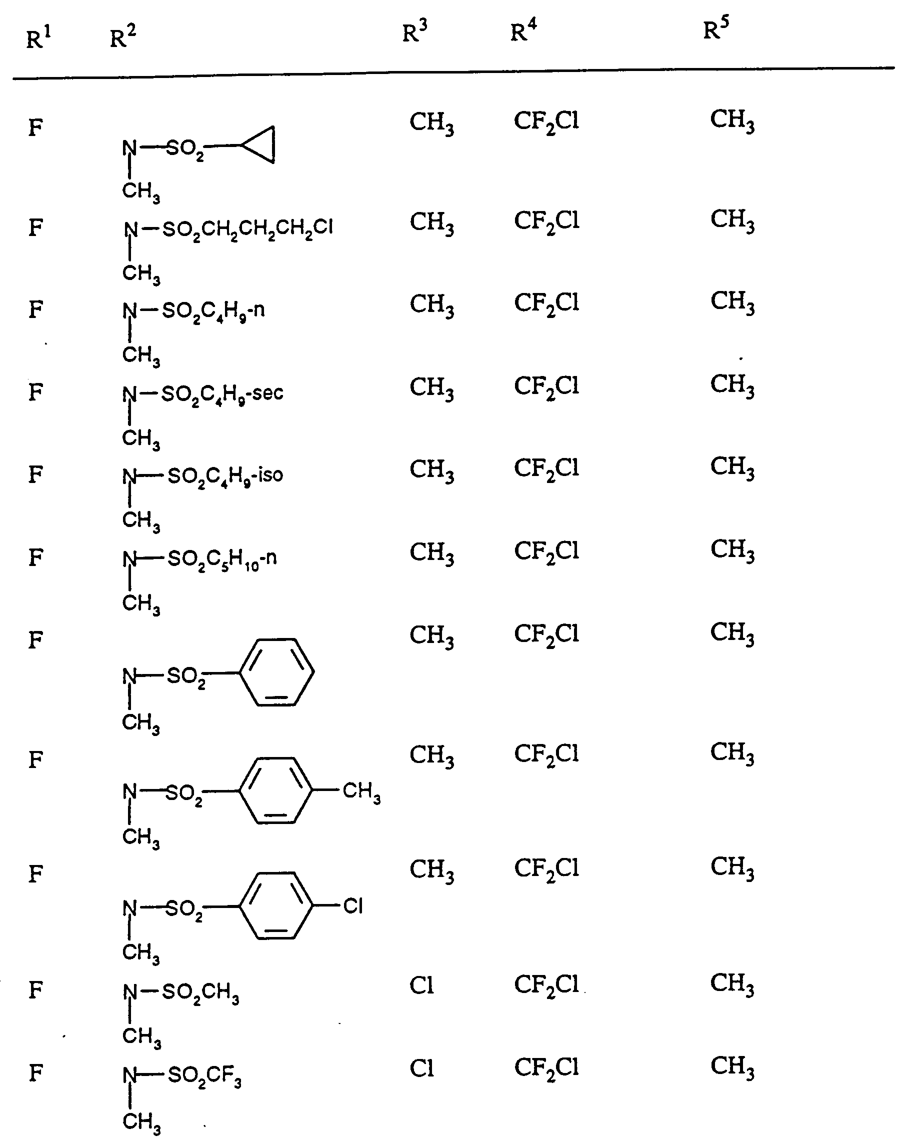

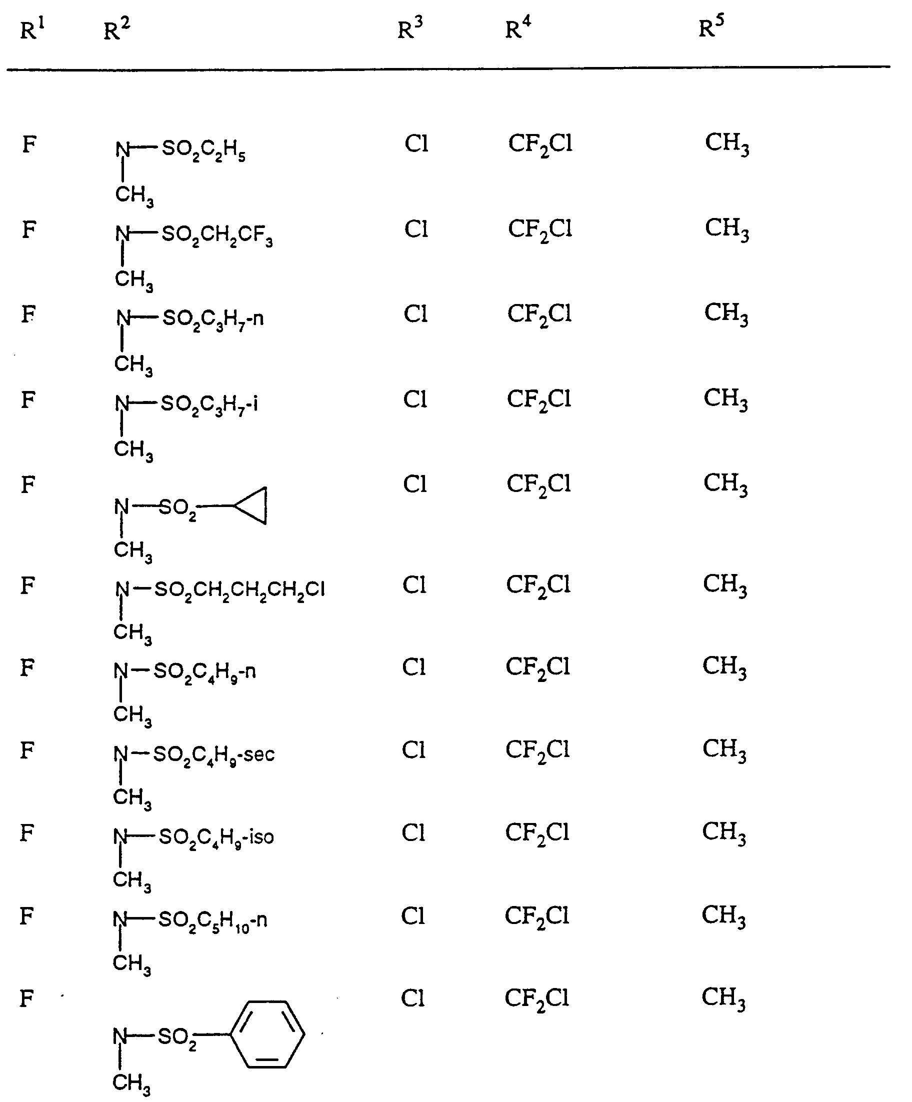

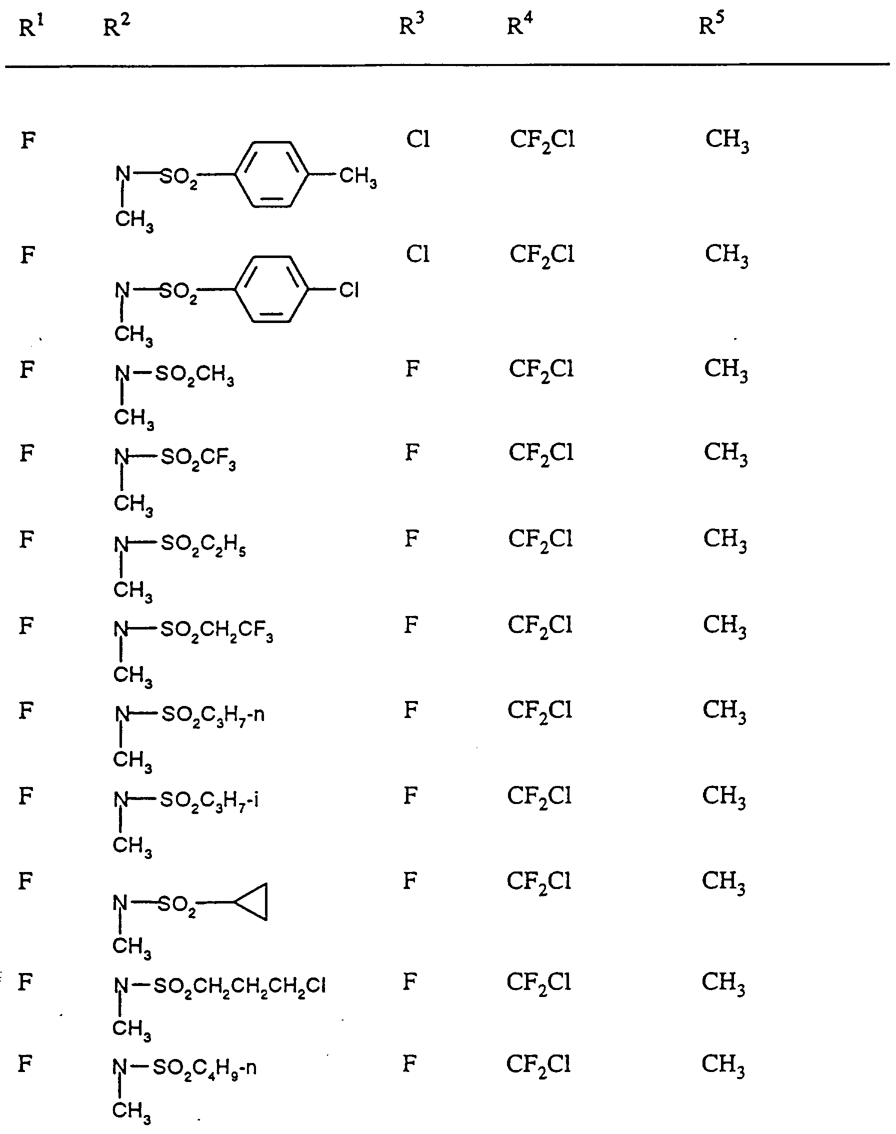

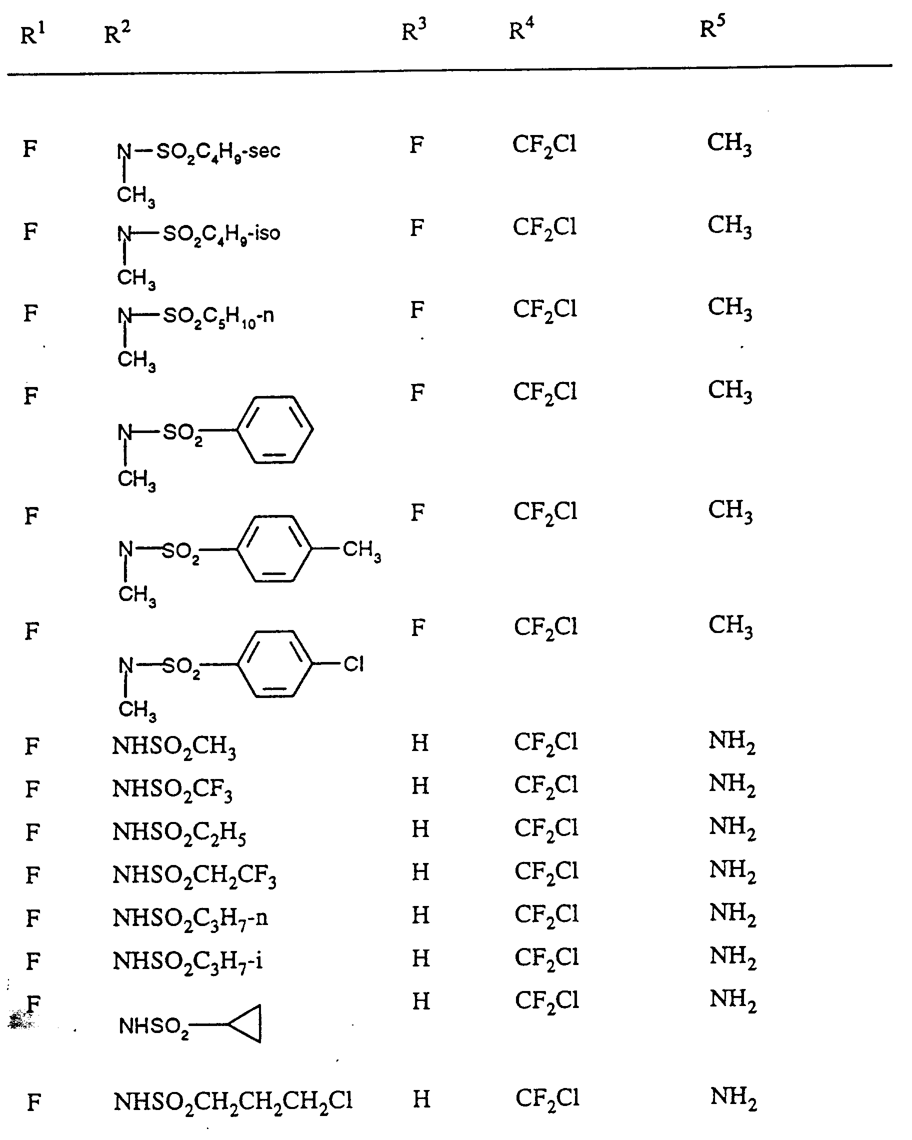

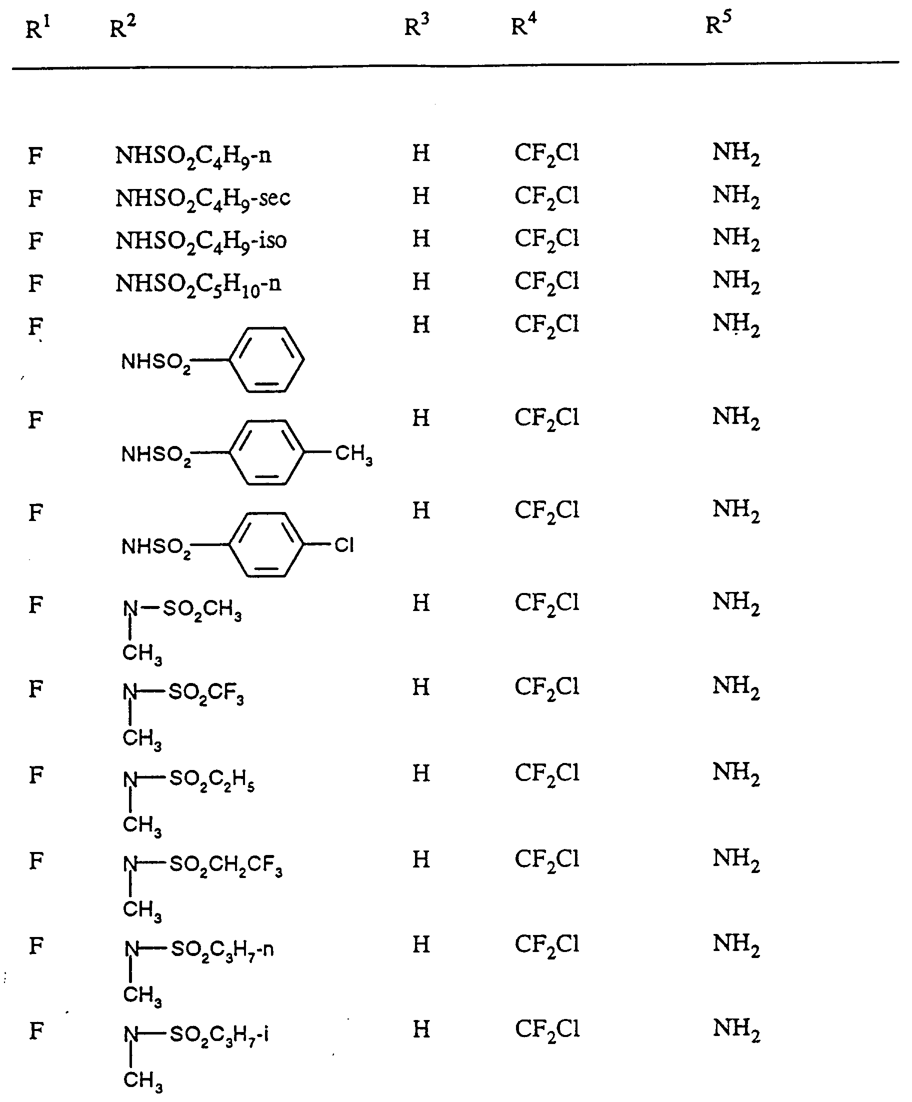

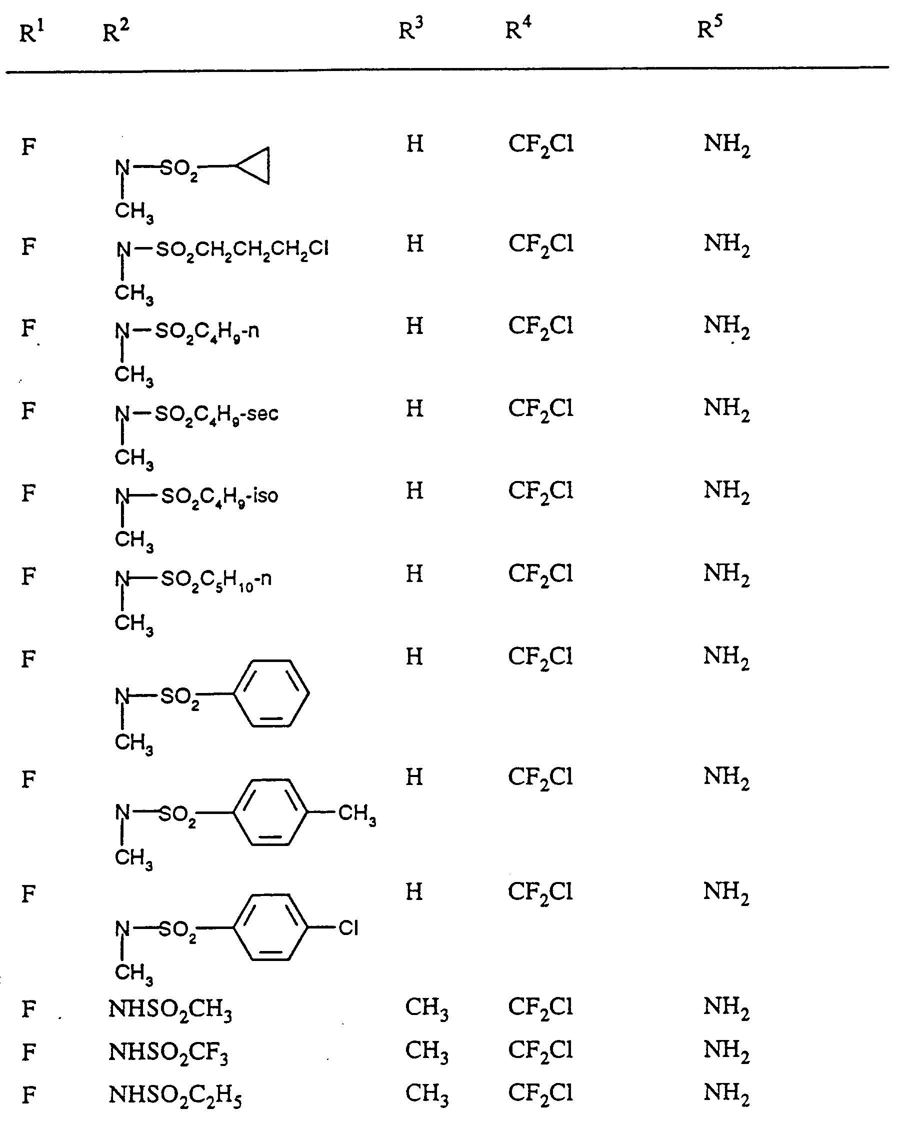

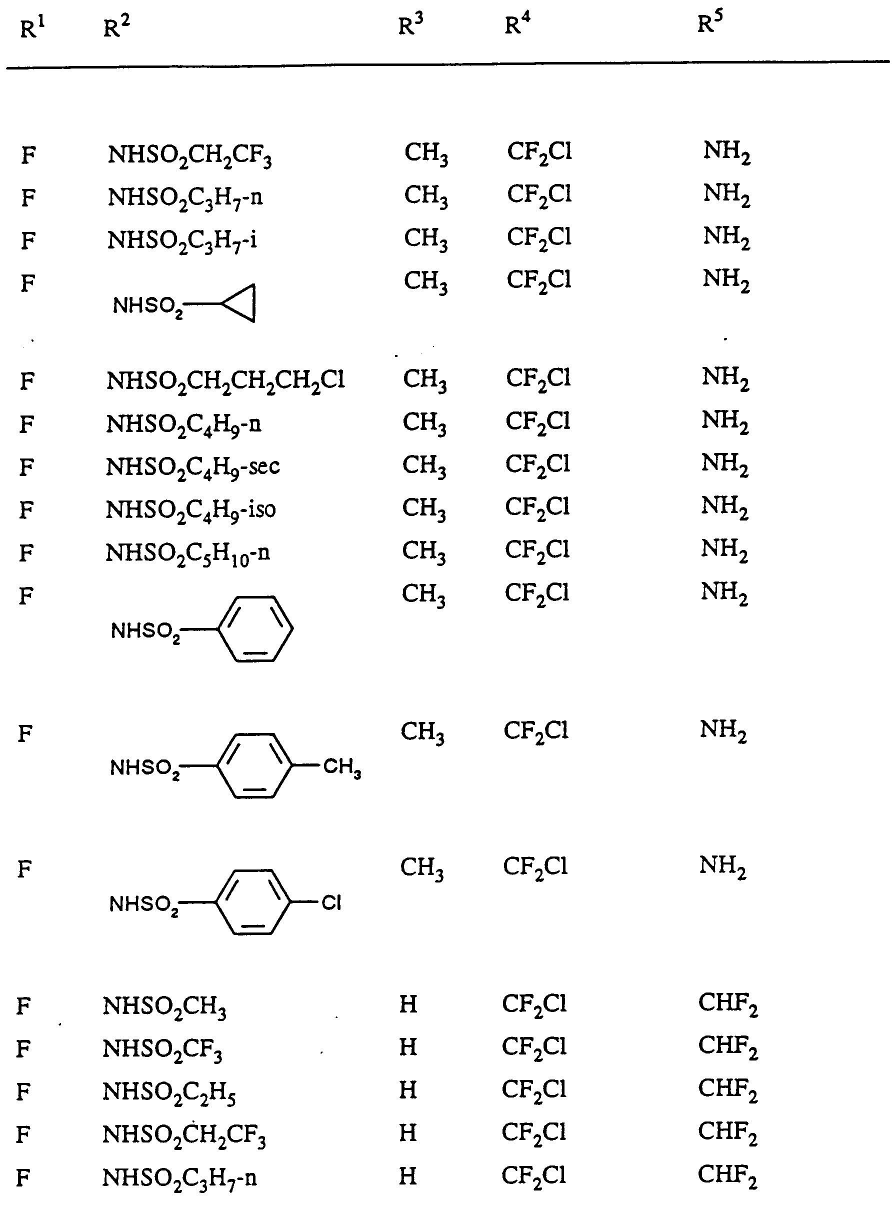

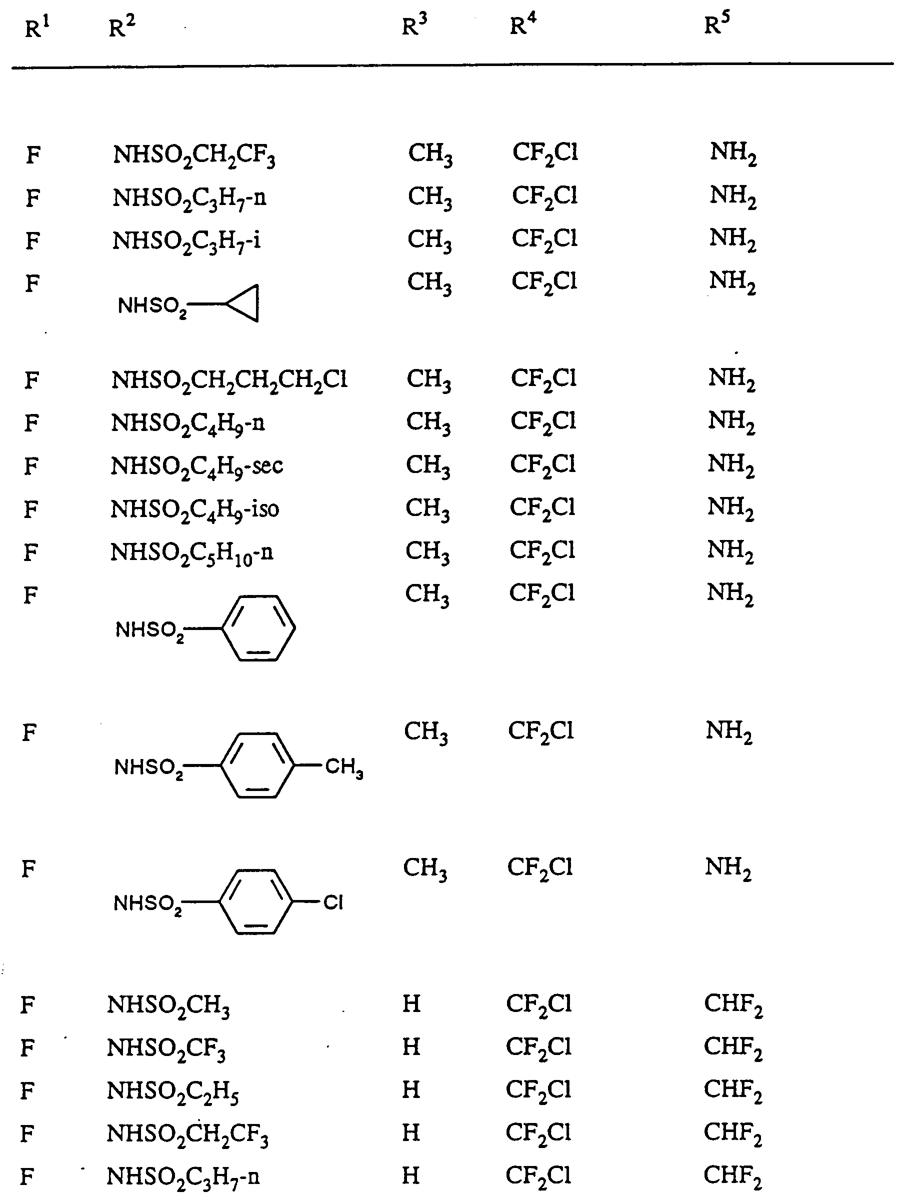

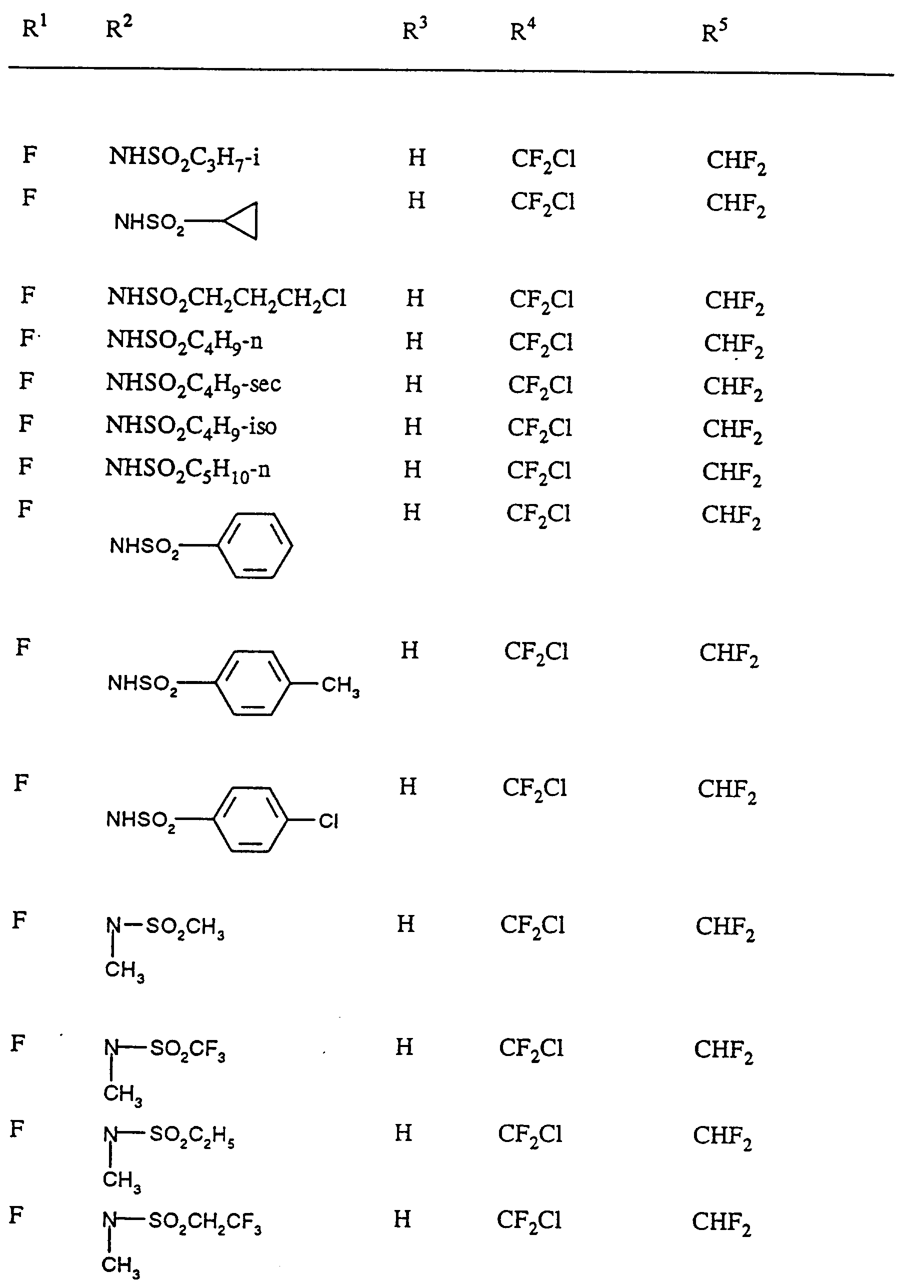

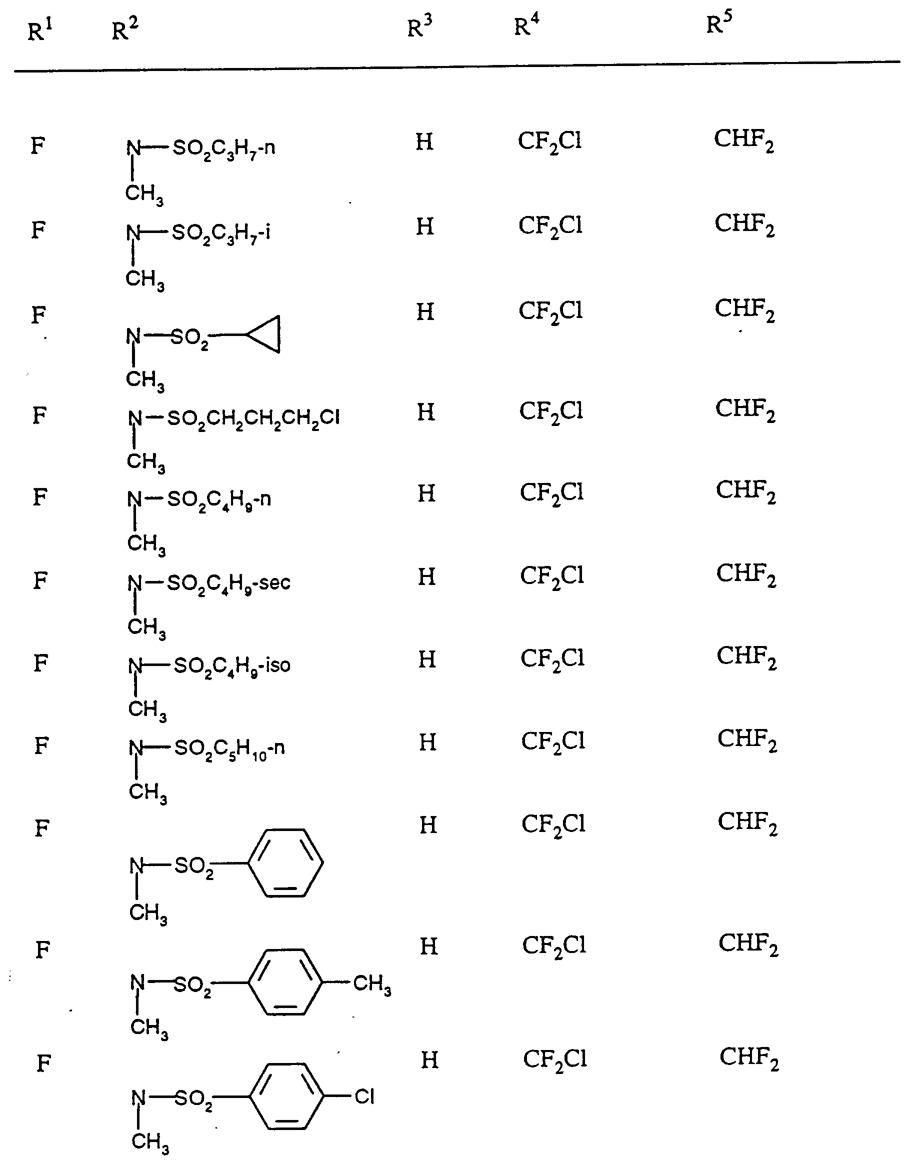

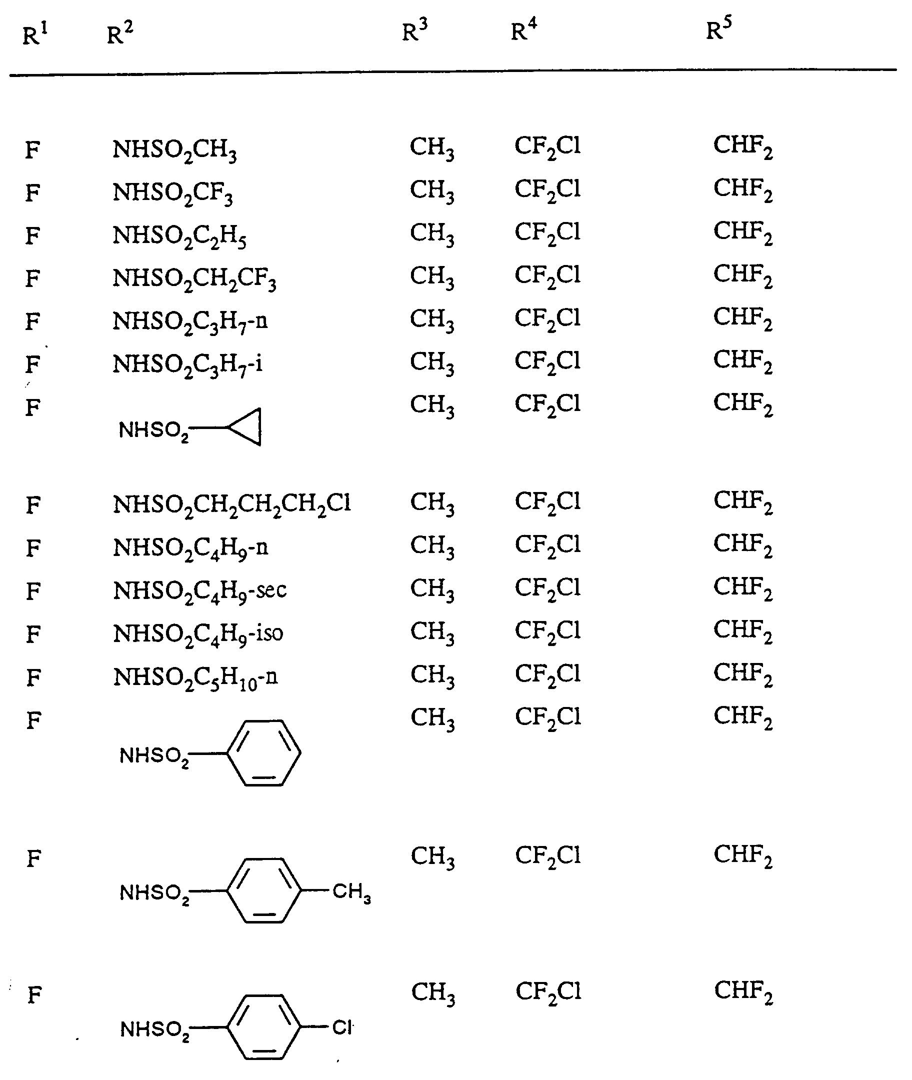

Man erhält N-Cyanoaryl-Stickstoffheterocyclen der Formeln (IA) und (IB), in welchen R⁵ für Wasserstoff steht sowie R¹, R², R³ und R⁴ die oben angegebenen Bedeutungen haben,
indem man
Aminoalkensäureester der allgemeinen Formel (II) 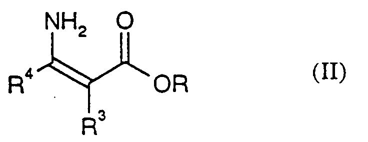 in welcher
- R³ und R⁴: die oben angegebenen Bedeutungen haben und
- R: für Alkyl, Aryl oder Arylalkyl steht,
mit Cyanoarylisocyanaten der allgemeinen Formel (III) 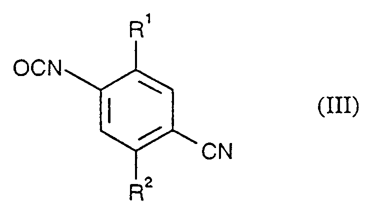 in welcher
- R¹ und R²: die oben angegebenen Bedeutungen haben,
oder mit Cyanoarylurethanen (Cyanoarylcarbamaten) der allgemeinen Formel (IV) 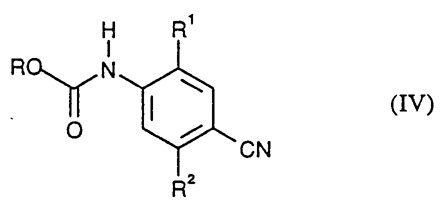 in welcher
- R¹ und R²: die oben angegebenen Bedeutungen haben und
- R: für Alkyl, Aryl oder Arylalkyl steht,
gegebenenfalls in Gegenwart eines Reaktionshilfsmittels und gegebenenfalls in Gegenwart eines Verdünnungsmittels umsetzt.

Verwendet man beispielsweise 3-Aminocrotonsäuremethylester und 4-Cyano-2,5-difluor-phenylisocyanat als Ausgangsstoffe, so kann der Reaktionsablauf beim obigen Verfahren durch das folgende Formelschema skizziert werden:

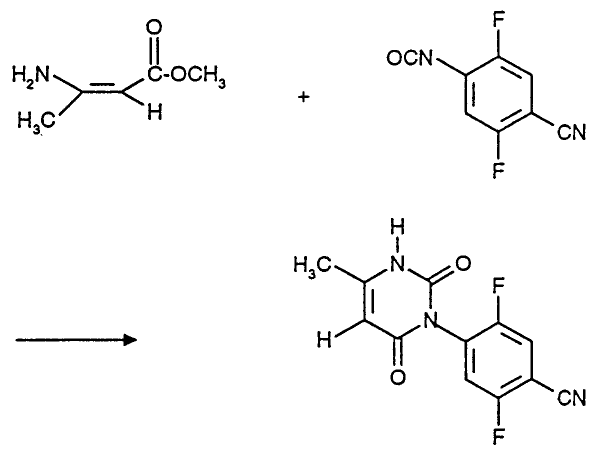

Verwendet man beispielsweise 1-(3-Chlor-4-cyano-phenyl)-3,6-dihydro-2,6-dioxo-4-trifluormethyl-1(2H)-pyrimidin und Methylbromid als Ausgangsstoffe, so kann der Reaktionsablauf beim erfindungsgemäßen Verfahren (a) durch das folgende Formelschema skizziert werden:

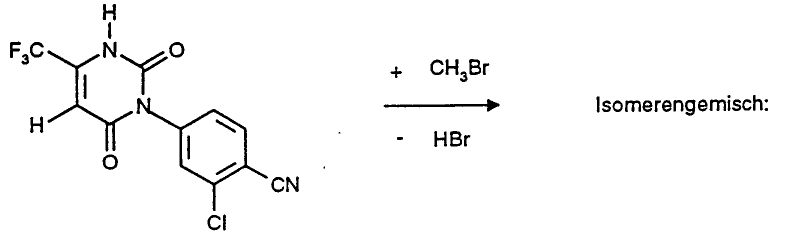

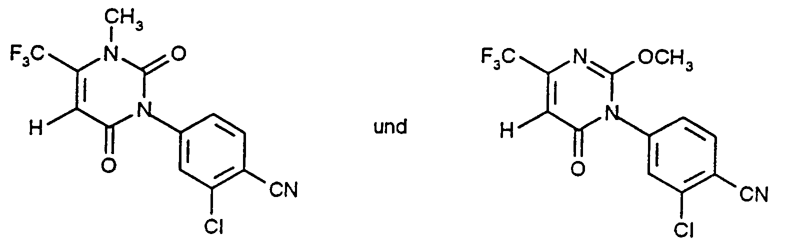

Verwendet man beispielsweise 1-(4-Cyano-2,5-difluor-phenyl)-3,6-dihydro-3,4-dimethyl-2,6-dioxo-1(2H)-pyrimidin und Ethansulfonamid als Ausgangsstoffe, so kann der Reaktionsablauf beim erfindungsgemäßen Verfahren (c) durch das folgende Formelschema skizziert werden:

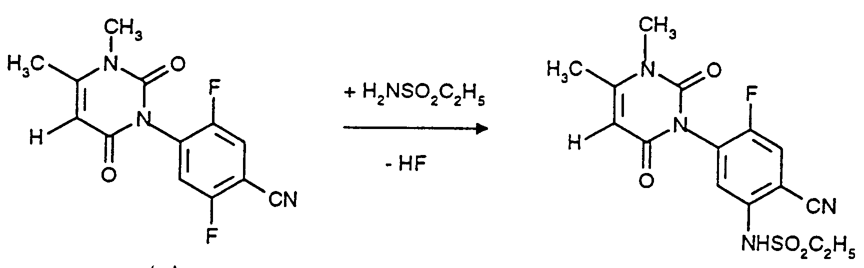

Die beim obigen Verfahren als Ausgangsstoffe zu verwendenden Aminoalkensäureester sind durch die Formel (II) allgemein definiert.

In Formel (II) haben R³ und R⁴ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R³ und R⁴ angegeben wurden.

R steht vorzugsweise für C₁-C₄-Alkyl, Phenyl oder Benzyl, insbesondere für Methyl oder Phenyl.

Die Ausgangsstoffe der Formel (II) sind bekannt und/oder können nach bekannten Verfahren hergestellt werden (vgl. J. Heterocycl. Chem. 9 (1972), 513-522).

Die beim obigen Verfahren weiter als Ausgangsstoffe zu verwendenden Cyanoarylisocyanate sind durch die Formel (III) allgemein definiert.

In Formel (III) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden. Die Cyanoarylisocyanate der allgemeinen Formel (III) sind noch nicht aus der Literatur bekannt und sind als neue Stoffe Gegenstand der vorliegenden Anmeldung.

Man erhält die neuen Cyanoarylisocyanate der Formel (III), wenn man Cyanoarylamine der allgemeinen Formel (VIII) 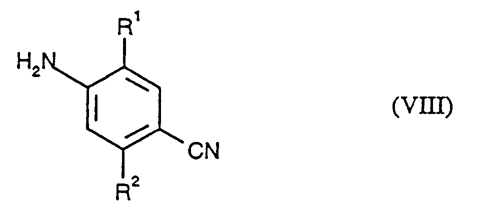 in welcher
- R¹ und R²: die oben angegebenen Bedeutungen haben,
mit Phosgen in Gegenwart eines Verdünnungsmittels, wie z.B. Chlorbenzol, bei Temperaturen zwischen -20°C und +150°C umsetzt (vgl. die Herstellungsbeispiele).

Die beim obigen Verfahren gegebenenfalls als Ausgangsstoffe zu verwendenden Cyanoarylurethane sind durch die Formel (IV) allgemein definiert. In der Formel (IV) haben R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R¹ und R² angegeben wurden; R steht vorzugsweise für C₁-C₄-Alkyl, Phenyl oder Benzyl, insbesondere für Methyl oder Phenyl.

Die Cyanoarylurethane der allgemeinen Formel (IV) sind noch nicht aus der Literatur bekannt; sie sind als neue Stoffe Gegenstand der vorliegenden Anmeldung.

Man erhält die neuen Cyanoarylurethane der Formel (IV), wenn man Cyanoarylamine der allgemeinen Formel (VIII) - oben - mit Chlorcarbonylverbindungen der allgemeinen Formel (IX)

RO-CO-Cl (IX)

in welcher
- R: die oben angegebene Bedeutung hat,
gegebenenfalls in Gegenwart eines Säureakzeptors, wie z.B. Pyridin, und gegebenenfalls in Gegenwart eines Verdünnungsmittels, wie z.B. Methylenchlorid, bei Temperaturen zwischen 0°C und 100°C umsetzt (vgl. die Herstellungsbeispiele).

Das obige Verfahren zur Herstellung von N-Cyanoaryl-Stickstoffheterocyclen der Formeln (IA) und (IB), in welchen R⁵ für Wasserstoff steht, wird vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlormethan, Chlorbenzol und o-Dichlorbenzol, Ether wie Diethyl- und Dibutyl-ether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäure-methylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Das obige Verfahren zur Herstellung von N-Cyanoaryl-Stickstoffheterocyclen der Formeln (IA) und (IB), in welchen R⁵ für Wasserstoff steht, wird vorzugsweise in Gegenwart eines Reaktionshilfsmittels durchgeführt. Als Reaktionshilfsmittel sind hierbei vor allem Säureakzeptoren geeignet. Vorzugsweise infrage kommen Alkalimetall- und Erdalkalimetall-hydride, wie Lithium-, Natrium-, Kalium- und Calcium-hydrid, Alkalimetall- und Erdalkalimetall-hydroxide, wie Lithium-, Natrium-, Kalium- und Calcium-hydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem obigen Verfahren zur Herstellung von N-Cyanoaryl-Stickstoffheterocyclen der Formeln (IA) und (IB), in welchen R⁵ für Wasserstoff steht, in einem größeren Bereich variiert werden. Im allgemeinen arbeitet man bei Temperaturen zwischen -120°C und +100°C, vorzugsweise bei Temperaturen zwischen -70°C und +80°C.

Das obige Verfahren zur Herstellung von N-Cyanoaryl-Stickstoffheterocyclen der Formeln (IA) und (IB), in welchen R⁵ für Wasserstoff steht, wird im allgemeinen unter Normaldruck durchgeführt. Es ist jedoch auch möglich, unter erhöhtem oder vermindertem Druck zu arbeiten.

Zur Durchführung des obigen Verfahrens zur Herstellung von N-Cyanoaryl-Stickstoffheterocyclen der Formeln (IA) und (IB), in welchen R⁵ für Wasserstoff steht, werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Reaktionshilfsmittels durchgeführt, und das Reaktionsgemisch wird mehrere Stunden bei der jeweils erforderlichen Temperatur gerührt. Die Aufarbeitung erfolgt bei diesem Verfahren jeweils nach üblichen Methoden (vgl. die Herstellungsbeispiele).

Die beim erfindungsgemäßen Verfahren (a) zur Herstellung von Verbindungen der Formel (I) als Ausgangsstoffe zu verwendenden N-Cyanoaryl-Stickstoffheterocyclen sind durch die Formeln (IA) und (IB) - mit der Maßgabe, daß darin R⁵ für Wasserstoff steht - allgemein definiert.

In den Formeln (IA) und (IB) haben R¹, R², R³ und R⁴ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R², R³ und R⁴ angegeben wurden.

Die Ausgangsstoffe der Formeln (IA) und (IB) für Verfahren (a) sind erfindungsgemäße neue Verbindungen; sie können nach dem zuvor beschriebenen Verfahren hergestellt werden.

Die beim erfindungsgemäßen Verfahren (a) weiter als Ausgangsstoffe zu verwendenden Alkylierungsmittel sind durch die Formeln (V) und (VI) allgemein definiert.

In den Formeln (V) und (VI) hat R⁵ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für R⁵ angegeben wurde.

Die Ausgangsstoffe der Formeln (V) und (VI) sind bekannte organische Synthesechemikalien.

Das erfindungsgemäße Verfahren (a) wird vorzugsweise unter Verwendung eines Verdünnungsmittels durchgeführt. Es kommen hierbei vor allem diejenigen Verdünnungsmittel in Betracht, die bereits bei der Beschreibung des zuvor erwähnten Verfahrens genannt wurden.

Als Säureakzeptoren können bei dem erfindungsgemäßen Verfahren (a) alle üblicherweise für derartige Umsetzungen verwendbaren Säurebindemittel eingesetzt werden. Vorzugsweise infrage kommen Alkalimetall- und Erdalkalimetallhydride, wie Lithium-, Natrium-, Kalium- und Calcium-hydrid, Alkalimetall- und Erdalkalimetall-hydroxide, wie Lithium-, Natrium-, Kalium- und Calciumhydroxid, Alkalimetall- und Erdalkalimetall-carbonate und -hydrogencarbonate, wie Natrium- und Kalium-carbonat oder -hydrogencarbonat sowie Calciumcarbonat, Alkalimetallacetate, wie Natrium- und Kalium-acetat, Alkalimetallalkoholate, wie Natrium- und Kalium-methylat, -ethylat, -propylat, -isopropylat, -butylat, -isobutylat und tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren (a) in einem größeren Bereich variiert werden. Im allgemeinen arbeitet man bei Temperaturen zwischen 0°C und 120°C, vorzugsweise bei Temperaturen zwischen 10°C und 100°C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (a) jeweils nach üblichen Methoden (vgl. die Herstellungsbeispiele).

Die beim erfindungsgemäßen Verfahren (b) zur Herstellung von Verbindungen der Formel (I) als Ausgangsstoffe zu verwendenden N-Cyanoaryl-Stickstoffheterocyclen sind durch die Formel (I) - mit der Maßgabe, daß darin R² für Halogen steht - allgemein definiert.

In Formel (I) haben dann R¹, R³, R⁴ und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹, R³, R⁴ und Z angegeben wurden.

Die Ausgangsstoffe der Formel (I) für Verfahren (b) sind erfindungsgemäße neue Verbindungen; sie können nach den zuvor genannten Verfahren hergestellt werden.

Die beim erfindungsgemäßen Verfahren (b) weiter als Ausgangsstoffe zu verwendenden Amide sind durch die Formel (VII) allgemein definiert.

In Formel (VII) hat A vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für A angegeben wurde.

Die Ausgangsstoffe der Formel (VII) sind bekannte organische Synthesechemikalien.

Das erfindungsgemäße Verfahren (b) wird vorzugsweise unter Verwendung eines Verdünnungsmittels durchgeführt. Es kommen hierbei vor allem diejenigen Verdünnungsmittel in Betracht, die bereits bei der Beschreibung des zuerst genannten Verfahrens genannt wurden.

Das erfindungsgemäße Verfahren (b) wird gegebenenfalls in Gegenwart eines Säureakzeptors durchgeführt. Es kommen hierbei diejenigen Säureakzeptoren in Betracht, die bereits bei der Beschreibung des erfindungsgemäßen Verfahrens (a) genannt wurden.

Die Reaktionstemperaturen können bei dem erfindungsgemäßen Verfahren (b) in einem größeren Bereich variiert werden. Im allgemeinen arbeitet man bei Temperaturen zwischen 0°C und 200°C, vorzugsweise bei Temperaturen zwischen 20°C und 180°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.
Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen und dikotylen Kulturen, wie z.B. in Weizen, Mais und Soja, sowohl im Vorauflauf- als auch im Nachauflauf-Verfahren, wobei aber die einzelnen Verbindungen auch in all den genannten Kulturen selektiv wirken.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Aryl-sulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuronethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

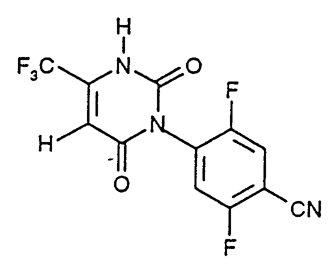

1,8 g (10 mMol) 3-Amino-4,4,4-trifluor-crotonsäureethylester werden in 30 ml Dimethylformamid und 2 ml Toluol vorgelegt und bei 0°C bis 5°C mit 0,3 g (10 mMol) Natriumhydrid (80%ig) versetzt. Das Gemisch wird 30 Minuten bei 0°C bis 5°C gerührt. Nach Abkühlen der Mischung auf -70°C werden 0,9 g (5 mMol) 4-Cyano-2,5-difluor-phenylisocyanat - gelöst in 10 ml Toluol - dazugegeben und das Gemisch wird 150 Minuten bei -60°C bis -70°C gerührt. Nach Entfernen des Kühlbades werden 2 ml Essigsäure dazugegeben. Dann wird mit Wasser auf etwa das doppelte Volumen verdünnt und mit Essigsäureethylester extrahiert. Die organische Phase wird eingeengt und der Rückstand mit Diisopropylether zur Kristallisation gebracht.

Man erhält 1,1 g (69% der Theorie) 1-(4-Cyano-2,5-difluor-phenyl)-3,6-dihydro-2,6-dioxo-4-trifluormethyl-1(2H)-pyrimidin vom Schmelzpunkt 194°C.

### Beispiel 2

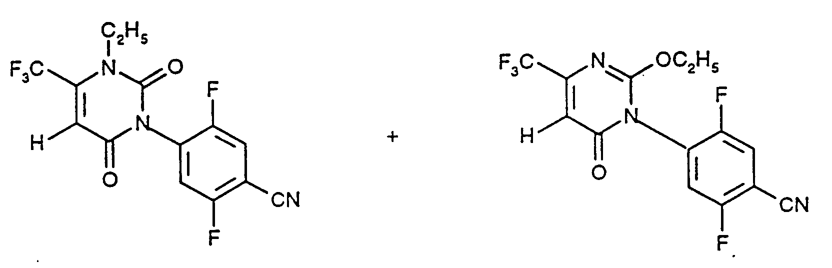

### (Verfahren (a))

Eine Mischung aus 3,2 g (10 mMol) 1-(4-Cyano-2,5-difluor-phenyl)-3,6-dihydro-2,6-dioxo-4-trifluormethyl-1(2H)-pyrimidin, 3,1 g (20 mMol) Diethylsulfat, 2,8 g Kaliumcarbonat und 100 ml Acetonitril wird 3 Stunden unter Rückfluß erhitzt. Dann wird im Vakuum eingeengt, mit Wasser/Chloroform geschüttelt, die organische Phase wiederum im Vakuum eingeengt und der Rückstand durch Säulenchromatographie (Kieselgel, Petrolether/Essigsäureethylester 1:1) aufgetrennt.

Man erhält als erste Fraktion 0,30 g 1-(4-Cyano-2,5-difluor-phenyl)-1,6-dihydro-2-ethoxy-6-oxo-4-trifluormethyl-pyrimidin (amorph) und als zweite Fraktion 0,38 g 1-(4-Cyano-2,5-difluor-phenyl)-3,6-dihydro-2,6-dioxo-3-ethyl-4-trifluormethyl-1(2H)-pyrimidin (amorph, ¹H-NMR: d = 6,36 ppm). Gesamtausbeute: 0,68 g (20% der Theorie).

### Beispiel 3

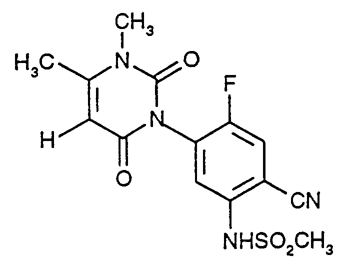

### (Verfahren (b))

Eine Mischung aus 0,83 g (3 mMol) 1-(4-Cyano-2,5-difluor-phenyl)-3,6-dihydro-2,6-dioxo-3,4-dimethyl-1(2H)-pyrimidin, 0,32 g (3 mMol) Methansulfonamid, 0,6 g Kaliumcarbonat und 10 ml Dimethylsulfoxid wird 10 Stunden auf 120°C erhitzt. Nach Abkühlen wird die Mischung auf Eiswasser gegossen und mit 2N-Salzsäure angesäuert. Dann wird mit Essigsäureethylester extrahiert, die organische Phase mit Wasser gewaschen, mit Natriumsulfat getrocknet und filtriert. Vom Filtrat wird das Lösungsmittel im Wasserstrahlvakuum sorgfältig abdestilliert.

Man erhält 0,8 g (76% der Theorie) 1-(4-Cyano-2-fluor-5-methylsulfonylaminophenyl)-3,6-dihydro-2,6-dioxo-3,4-dimethyl-1(2H)-pyrimidin als kristallinen Rückstand (Schmelzpunkt >250°C).

Analog zu den Beispielen 1 bis 3 sowie entsprechend der allgemeinen Beschreibung der erfindungsgemäßen Herstellungsverfahren können beispielsweise die in der nachstehenden Tabelle 2 aufgeführten Verbindungen der Formel (I) - bzw. der Formeln (IA) und (IB) - hergestellt werden:

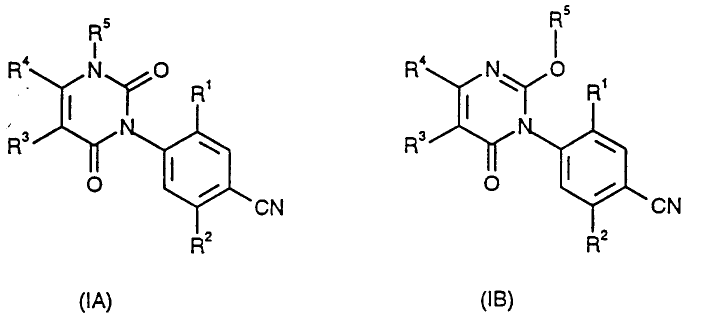

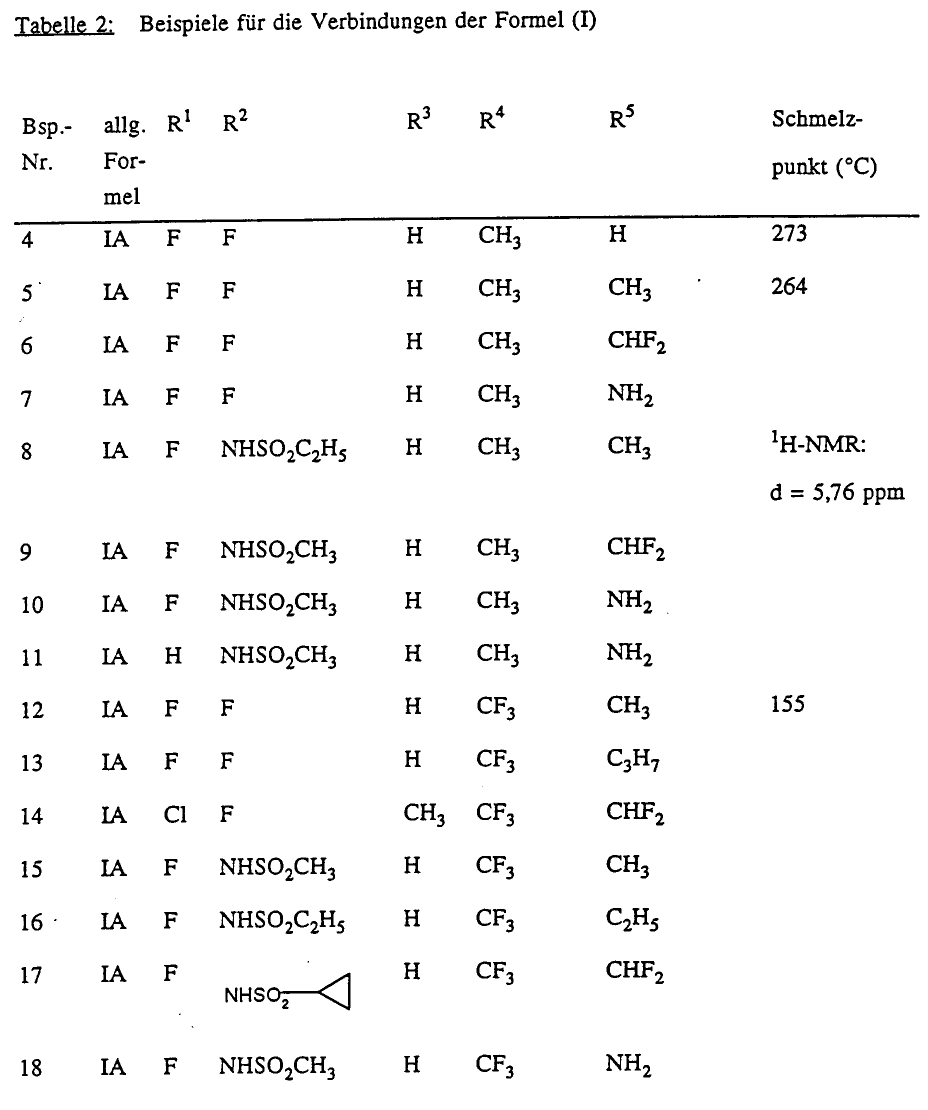

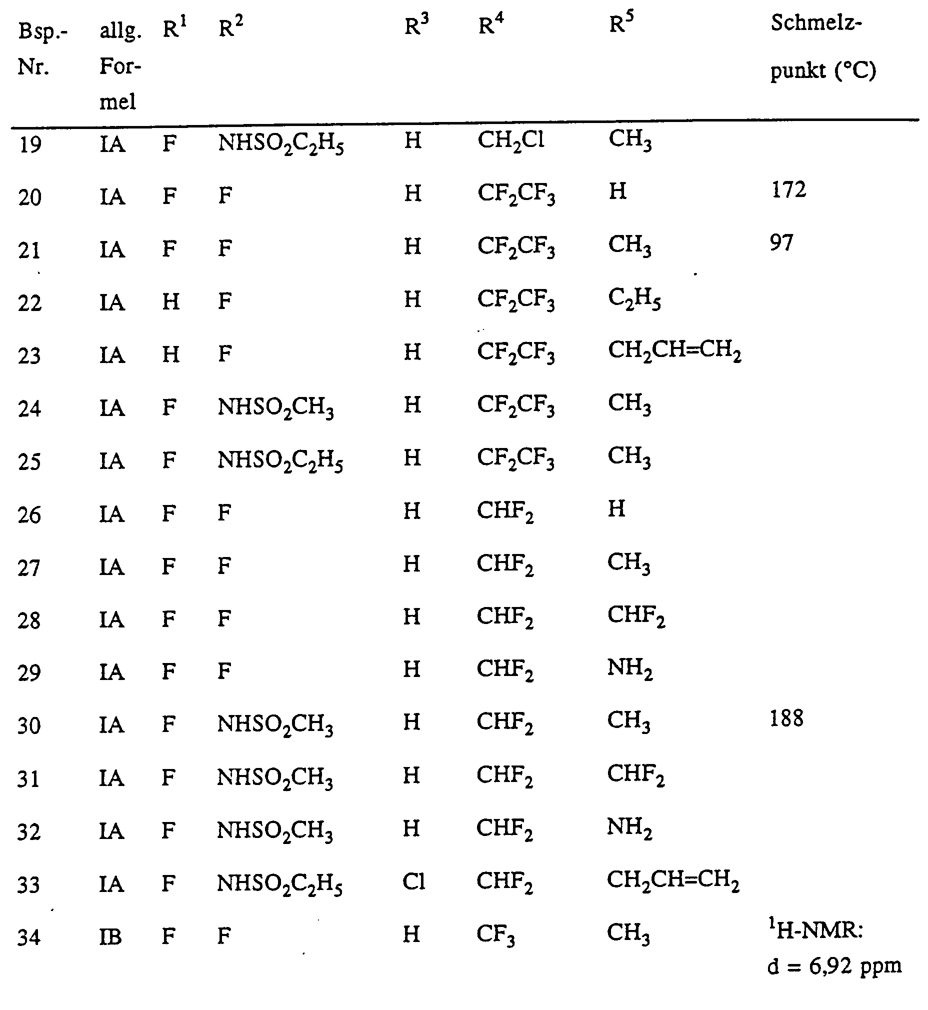

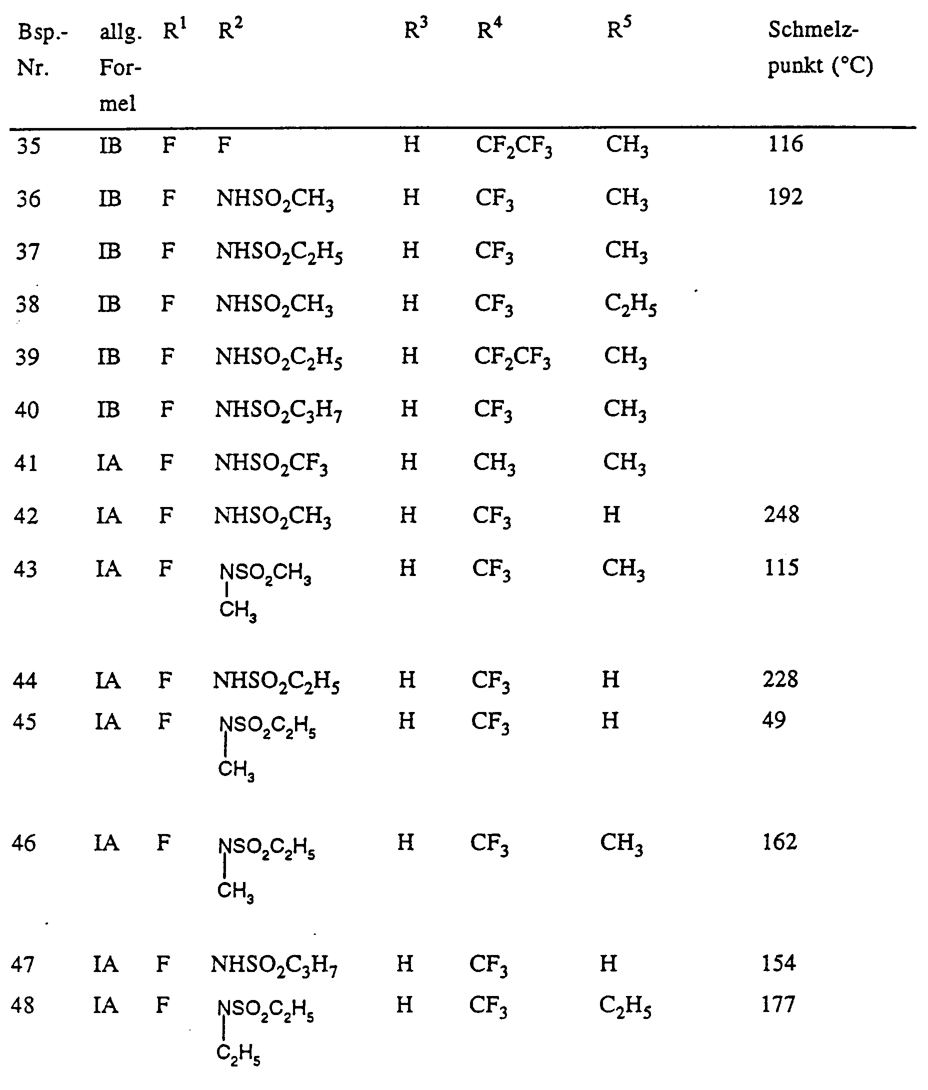

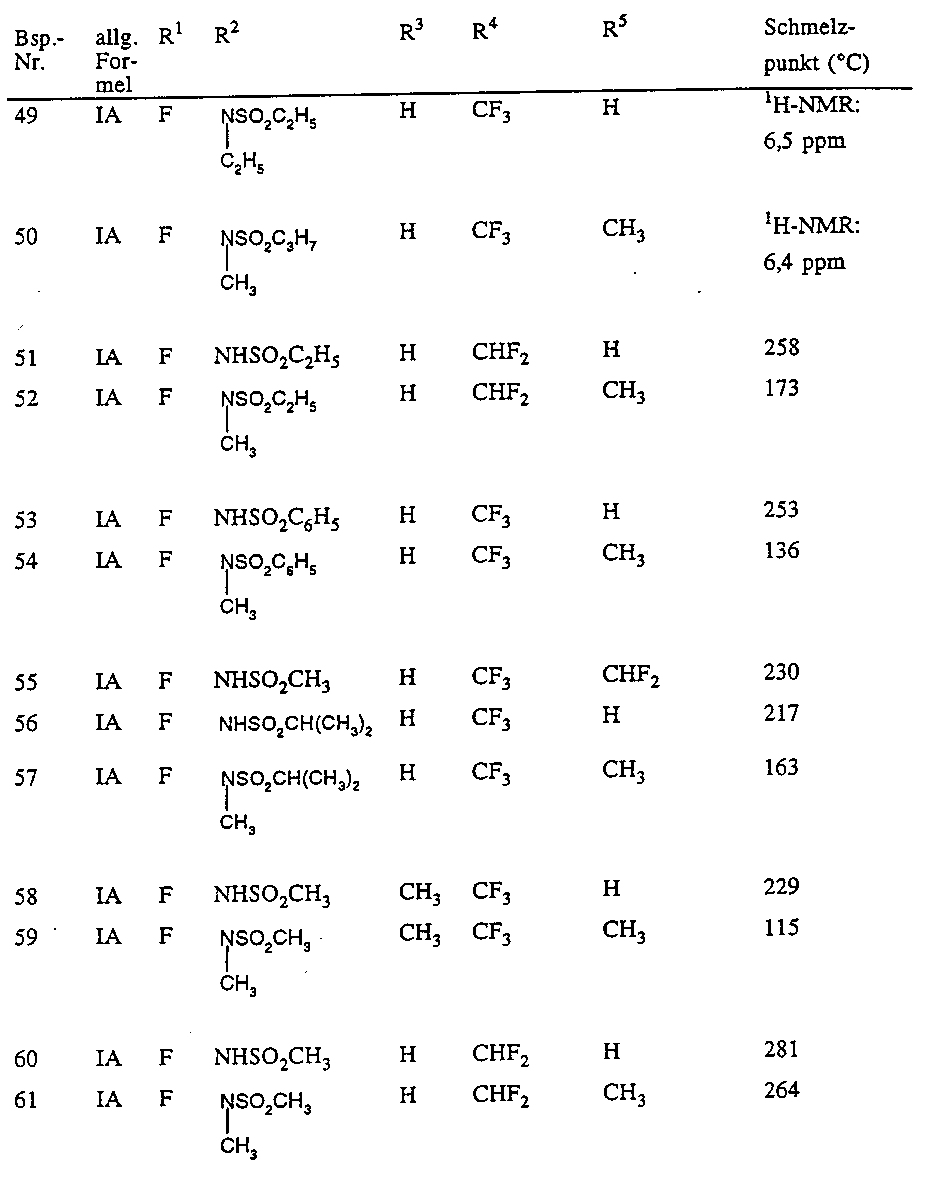

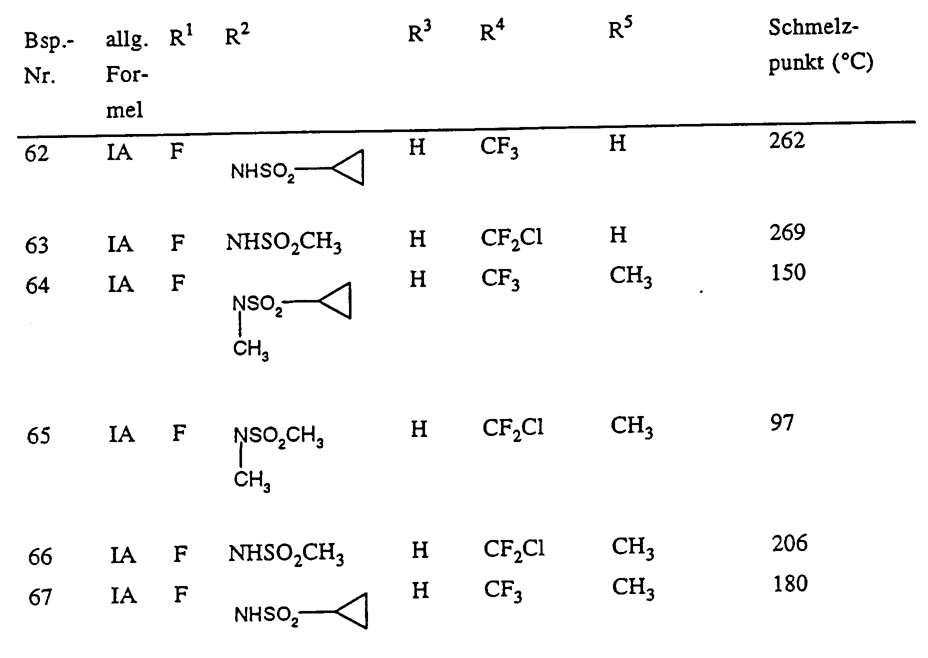

### Ausgangsstoffe der Formel (III):

### Beispiel (III-1)

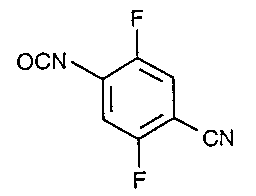

660 g Phosgen werden bei -5°C bis 0°C in 1,6 Liter Chlorbenzol einkondensiert. Dann wird bei der gleichen Temperatur eine Lösung von 400 g 4-Cyano-2,5-difluor-anilin in 800 ml Chlorbenzol innerhalb von 60 Minuten zugetropft. Anschließend wird die Reaktionsmischung langsam (HCl-Entwicklung!) auf 120°C aufgeheizt, wobei nach Erreichen einer Temperatur von 80°C fortwährend Phosgen eingeleitet wird. Nach einstündigem Erhitzen bei 120°C wird überschüssiges Phosgen mit Stickstoff ausgeblasen und dann mittels Destillation aufgearbeitet.

Man erhält 248 g (53% der Theorie) 4-Cyano-2,5-difluor-phenylisocyanat vom Siedepunkt 108-110°C/14 mbar (Schmelzpunkt: 53°C).

### Ausgangsstoffe der Formel (IV):

### Beispiel (IV-1)

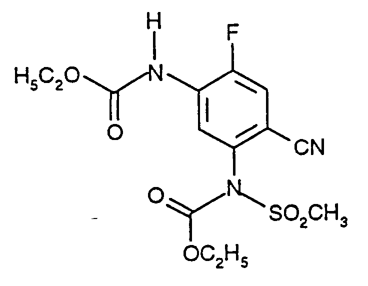

Eine Mischung aus 11,5 g (0,05 Mol) 4-Cyano-2-fluor-5-methylsulfonylaminoanilin, 18 g Pyridin und 400 ml Methylenchlorid wird bei 20°C unter Rühren mit 13,0 g (0,05 Mol) Chlorameisensäure-ethylester tropfenweise versetzt und das Reaktionsgemisch wird noch 30 Minuten bei 20°C bis 30°C nachgerührt. Dann wird mit Wasser, anschließend mit 5%iger Salzsäure und wiederum mit Wasser gewaschen, mit Natriumsulfat getrocknet, filtriert und das Filtrat eingeengt. Der hierbei verbleibende Rückstand wird mit wenig Essigsäureethylester digeriert und das hierbei kristallin angefallene Produkt durch Absaugen isoliert.

Man erhält 15,8 g (85% der Theorie) N-Ethoxycarbonyl-N-(2-cyano-4-fluor-5-ethoxycarbonylamino-phenyl)-methansulfonamid vom Schmelzpunkt 129°C.

Analog Beispiel (IV-1) können beispielsweise auch die in der nachstehenden Tabelle 3 aufgeführten Verbindungen der Formel (IV) hergestellt werden.

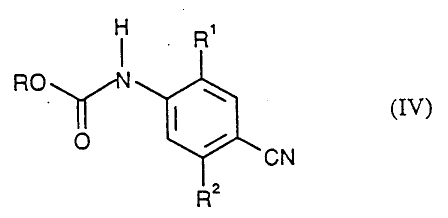

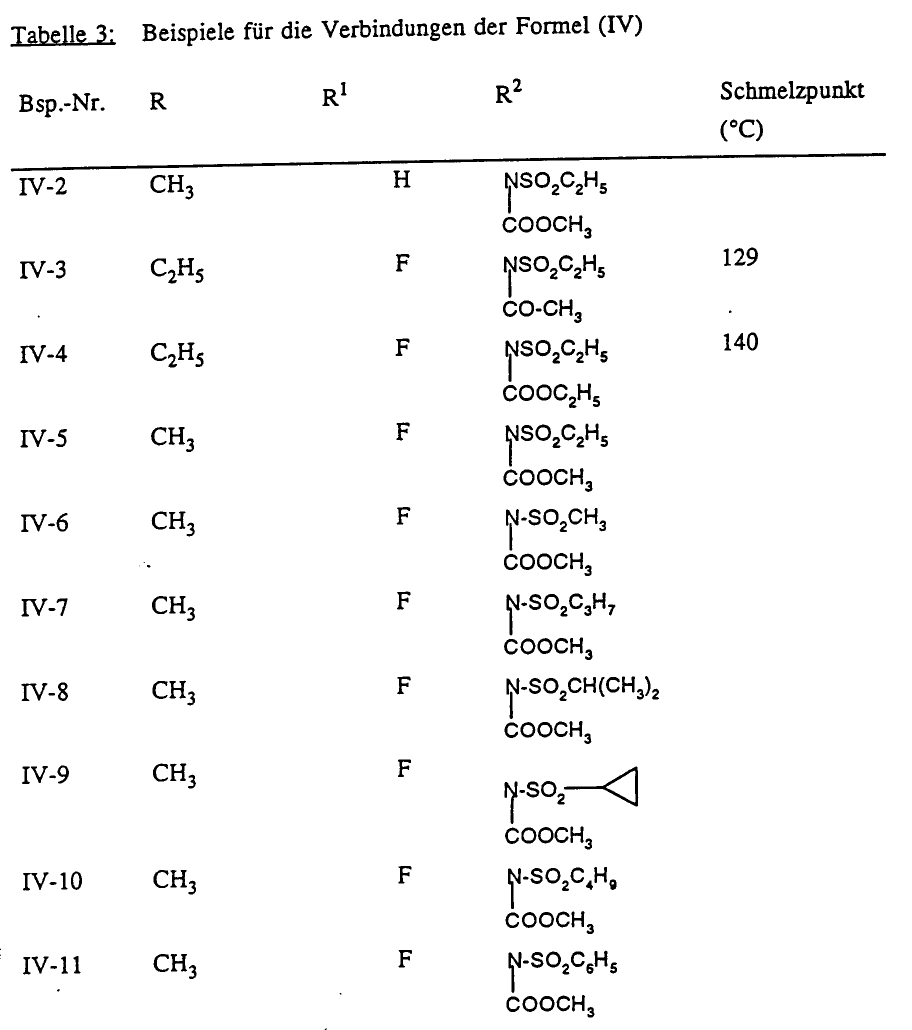

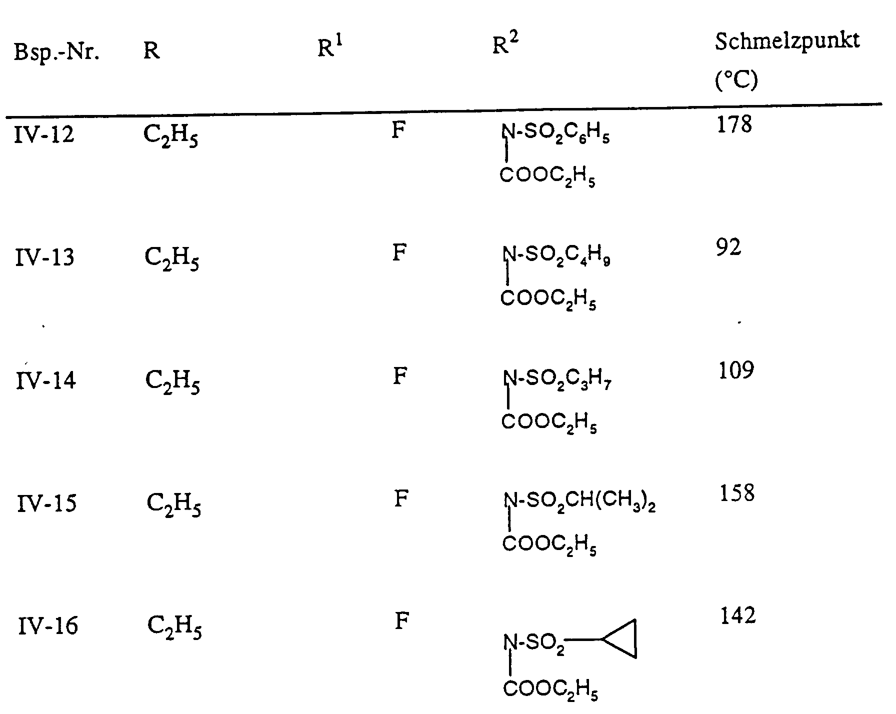

### Anwendungsbeispiele:

### Beispiel A

### Pre-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß Herstellungsbeispiel 2 (2. Fraktion) und 12 bei guter Verträglichkeit gegenüber Kulturpflanzen, wie z.B. Mais, sehr starke Wirkung gegen Unkräuter.

### Beispiel B

### Post-emergence-Test

- Lösungsmittel:: 5 Gewichtsteile Aceton
- Emulgator:: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration. Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 2 (2. Fraktion) und 12 bei guter Verträglichkeit gegenüber Kulturpflanzen, wie z.B. Weizen, starke Wirkung gegen Unkräuter.

## Patentansprüche

1. N-Cyanoaryl-Stickstoffheterocyclen der allgemeinen Formel (I) 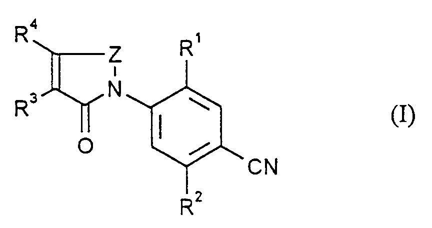 in welcher
R¹ für Fluor steht,
R² für Fluor, Chlor, Brom, Cyano oder die Gruppierung -N(A¹)SO₂A steht, worin
A für einen jeweils gegebenenfalls durch Fluor, Chlor, Brom, Cyano oder C₁-C₄-Alkoxy substituierten Rest der Reihe Alkyl, Alkenyl oder Alkinyl mit jeweils bis zu 10 Kohlenstoffatomen steht,
A weiterhin für jeweils gegebenenfalls durch Fluor, Chlor, Brom, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl, Phenyloxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl, C₁-C₄-Alkylsulfonyl oder C₁-C₄-Alkoxycarbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Heterocyclyl oder Heterocyclylalkyl mit 2 bis 6 Kohlenstoffatomen und 1 bis 4 Stickstoffatomen und/oder 1 bis 2 Sauerstoff- oder Schwefelatomen im gesättigten oder ungesättigten Heterocyclylteil und gegebenenfalls 1 bis 4 Kohlenstoffatomen im Alkylteil steht, und
A¹ für Wasserstoff oder für jeweils gegebenenfalls durch Halogen oder C₁-C₄-Alkoxy substituiertes Alkyl, Alkenyl, Alkinyl, Alkoxy, Alkylcarbonyl oder Alkoxycarbonyl mit jeweils bis zu 6 Kohlenstoffatomen, oder für Phenylcarbonyl, Naphthylcarbonyl, Phenylmethylcarbonyl, Naphthylmethylcarbonyl, Phenoxycarbonyl oder Naphthyloxycarbonyl (welche jeweils gegebenenfalls durch Fluor, Chlor, Brom, Cyano, Methyl, Methoxy, Trifluormethyl und/oder Trifluormethoxy substituiert sind) steht,
R³ für Wasserstoff, Fluor, Chlor, Brom, Cyano oder für gegebenenfalls durch Fluor und/oder Chlor substituiertes Alkyl mit 1 bis 6 Kohlenstoffatomen steht,
R⁴ für gegebenenfalls durch Fluor, Chlor, Brom, Methoxy oder Ethoxy substituiertes Alkyl mit 1 bis 6 Kohlenstoffatomen steht,
oder zusammen mit R³ für Alkandiyl mit 2 bis 8 Kohlenstoffatomen steht, und
Z für eine der nachstehenden Gruppierungen steht 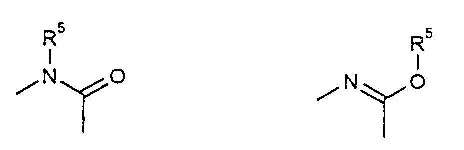 worin
R⁵ für Amino oder durch Cyano, C₁-C₄-Alkoxy, C₁-C₄-Alkylcarbonyl oder C₁-C₄-Alkoxy-carbonyl substituiertes Alkyl mit bis zu 6 Kohlenstoffatomen steht sowie deren Salze mit Basen, wie z.B. Natrium-, Kalium- oder Calcium-hydroxid, -hydrid, -amid oder -carbonat, Natrium- oder Kalium-C₁-C₄-alkanolaten, Ammoniak, C₁-C₄-Alkylamino, Di-(C₁-C₄-alkyl)-aminen, Tri-(C₁-C₄-alkyl)-aminen oder auch mit Tris-(2-hydroxyethyl)amin.

2. N-Cyanoaryl-Stickstoffheterocyclen der allgemeinen Formel (I) gemäß Anspruch 1, dadurch gekennzeichnet, daß
R¹ für Fluor steht,
R² für Fluor, Chlor, Brom, Cyano oder die Gruppierung -N(A¹)SO₂A steht, worin
A für jeweils gegebenenfalls durch Fluor oder Chlor substituiertes Methyl, Ethyl, n- oder i-Propyl, n-, i-, s- oder t-Butyl, n-, i-, s- oder t-Pentyl steht,
A weiterhin für jeweils gegebenenfalls durch Fluor, Chlor, Brom, Methyl und/oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
A weiterhin für jeweils gegebenenfalls durch Fluor, Chlor, Brom, Cyano, Nitro, Carboxy, Methyl, Trifluormethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl, Dimethylaminosulfonyl, Methoxycarbonyl, Ethoxycarbonyl, Propoxycarbonyl oder Phenyl substituiertes Phenyl, Naphthyl, Phenylmethyl oder Phenylethyl steht,
A weiterhin für jeweils gegebenenfalls durch Fluor, Chlor, Brom, Cyano, Methyl, Ethyl, Trifluormethyl, Methoxy, Ethoxy, Methylthio, Ethylthio, Methylsulfinyl, Ethylsulfinyl, Methylsulfonyl, Ethylsulfonyl, Methoxycarbonyl oder Ethoxycarbonyl substituiertes Thienyl, Pyrazolyl oder Pyridyl steht, und
A¹ für Wasserstoff, für jeweils gegebenenfalls durch Fluor, Chlor, Methoxy oder Ethoxy substituiertes Methyl, Ethyl, n- oder i-Propyl, n-, i- oder s-Butyl, Propenyl, Butenyl, Propinyl, Butinyl, Methoxy, Ethoxy, n- oder i-Propoxy, n-, i-, s- oder t-Butoxy, Acetyl, Propionyl, Methoxycarbonyl oder Ethoxycarbonyl, für Phenylcarbonyl oder Phenoxycarbonyl steht,
R³ für Wasserstoff, Fluor, Chlor, Brom, Methyl, Trifluormethyl, Ethyl, n- oder i-Propyl steht,
R⁴ für jeweils gegebenenfalls durch Fluor oder Chlor substituiertes Methyl, Ethyl, n- oder i-Propyl steht,
oder zusammen mit R³ für Trimethylen oder Tetramethylen steht, und
Z für eine der nachstehenden Gruppierungen steht 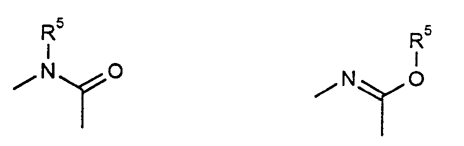 worin
R⁵ für Amino oder für Cyano substituiertes Methyl, Ethyl, n- oder i-Propyl, n-, i- oder s-Butyl, steht sowie deren Salze mit Basen, wie z.B. Natrium-, Kalium- oder Calcium-hydroxid, -hydrid, -amid oder -carbonat, Natrium- oder Kalium-C₁-C₄-alkanolaten, Ammoniak, C₁-C₄-Alkylaminen, Di-(C₁-C₄-alkyl)-aminen, Tri-(C₁-C₄-alkyl)-aminen oder auch mit Tris-(2-hydroxyethyl)amin.

3. N-Cyanoaryl-Stickstoffheterocyclen der allgemeinen Formel (I) 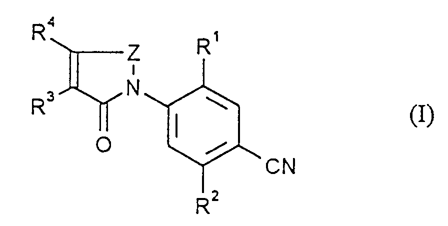 in welcher
R¹ für Fluor steht,
R² für Fluor, Chlor, Brom, Cyano oder die Gruppierung -N(A¹)SO₂A steht, worin
A für einen jeweils gegebenenfalls durch Fluor, Chlor, Brom, Cyano oder C₁-C₄-Alkoxy substituierten Rest der Reihe Alkyl, Alkenyl oder Alkinyl mit jeweils bis zu 10 Kohlenstoffatomen steht,
A weiterhin für jeweils gegebenenfalls durch Fluor, Chlor, Brom, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl, Phenyloxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl, C₁-C₄-Alkylsulfonyl oder C₁-C₄-Alkoxycarbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Heterocyclyl oder Heterocyclylalkyl mit 2 bis 6 Kohlenstoffatomen und 1 bis 4 Stickstoffatomen und/oder 1 bis 2 Sauerstoff- oder Schwefelatomen im gesättigten oder ungesättigten Heterocyclylteil und gegebenenfalls 1 bis 4 Kohlenstoffatomen im Alkylteil steht, und
A¹ für Wasserstoff oder für jeweils gegebenenfalls durch Halogen oder C₁-C₄-Alkoxy substituiertes Alkyl, Alkenyl, Alkinyl, Alkoxy, Alkylcarbonyl oder Alkoxycarbonyl mit jeweils bis zu 6 Kohlenstoffatomen, oder für Phenylcarbonyl, Naphthylcarbonyl, Phenylmethylcarbonyl, Naphthylmethylcarbonyl, Phenoxycarbonyl oder Naphthyloxycarbonyl (welche jeweils gegebenenfalls durch Fluor, Chlor, Brom, Cyano, Methyl, Methoxy, Trifluormethyl und/oder Trifluormethoxy substituiert sind) steht,
R³ für Wasserstoff, Fluor, Chlor, Brom, Cyano oder für gegebenenfalls durch Fluor und/oder Chlor substituiertes Alkyl mit 1 bis 6 Kohlenstoffatomen steht,
R⁴ für gegebenenfalls durch Fluor, Chlor, Brom, Methoxy oder Ethoxy substituiertes Alkyl mit 1 bis 6 Kohlenstoffatomen steht, oder zusammen mit R³ für Alkandiyl mit 2 bis 8 Kohlenstoffatomen steht, und
Z für eine der nachstehenden Gruppierungen steht 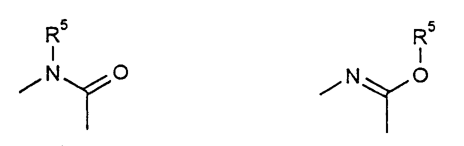 worin
R⁵ für jeweils gegebenenfalls durch Fluor, Chlor, Brom, Cyano, C₁-C₄-Alkoxy, C₁-C₄-Alkyl-carbonyl oder C₁-C₄-Alkoxycarbonyl substituiertes Alkenyl, Alkinyl, Alkylcarbonyl oder Alkoxycarbonyl mit jeweils bis zu 6 Kohlenstoffatomen steht sowie deren Salze mit Basen, wie z.B. Natrium-, Kalium- oder Calcium-hydroxid, -hydrid, -amid oder -carbonat, Natrium- oder Kalium-C₁-C₄-alkanolaten, Ammoniak, C₁-C₄-Alkylamino, Di-(C₁-C₄-alkyl)-aminen, Tri-(C₁-C₄-alkyl)-aminen oder auch mit Tris-(2-hydroxyethyl)amin.

4. N-Cyanoaryl-Stickstoffheterocyclen der allgemeinen Formel (I) gemäß Anspruch 3 dadurch gekennzeichnet, daß
R¹ für Fluor steht,
R² für Fluor, Chlor, Brom, Cyano oder die Gruppierung -N(A¹)SO₂A steht, worin
A für jeweils gegebenenfalls durch Fluor oder Chlor substituiertes Methyl, Ethyl, n- oder i-Propyl, n-, i-, s- oder t-Butyl, n-, i-, s- oder t-Pentyl steht,
A weiterhin für jeweils gegebenenfalls durch Fluor, Chlor, Brom, Methyl und/oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
A weiterhin für jeweils gegebenenfalls durch Fluor, Chlor, Brom, Cyano, Nitro, Carboxy, Methyl, Trifluormethyl, Methoxy, Ethoxy, Difluormethoxy, Trifluormethoxy, Methylthio, Ethylthio, Methylsulfinyl, Ethylsulfinyl, Methylsulfonyl, Ethylsulfonyl, Dimethylaminosulfonyl, Methoxycarbonyl, Ethoxycarbonyl, Propoxycarbonyl oder Phenyl substituiertes Phenyl, Naphthyl, Phenylmethyl oder Phenylethyl steht,
A weiterhin für jeweils gegebenenfalls durch Fluor, Chlor, Brom, Cyano, Methyl, Ethyl, Trifluormethyl, Methoxy, Ethoxy, Methylthio, Ethylthio, Methylsulfinyl, Ethylsulfinyl, Methylsulfonyl, Ethylsulfonyl, Methoxycarbonyl oder Ethoxycarbonyl substituiertes Thienyl, Pyrazolyl oder Pyridyl steht, und
A¹ für Wasserstoff, für jeweils gegebenenfalls durch Fluor, Chlor, Methoxy oder Ethoxy substituiertes Methyl, Ethyl, n- oder i-Propyl, n-, i- oder s-Butyl, Propenyl, Butenyl, Propinyl, Butinyl, Methoxy, Ethoxy, n- oder i-Propoxy, n-, i-, s- oder t-Butoxy, Acetyl, Propionyl, Methoxycarbonyl oder Ethoxycarbonyl, für Phenylcarbonyl oder Phenoxycarbonyl steht,
R³ für Wasserstoff, Fluor, Chlor, Brom, Methyl, Trifluormethyl, Ethyl, n- oder i-Propyl steht,
R⁴ für jeweils gegebenenfalls durch Fluor oder Chlor substituiertes Methyl, Ethyl, n- oder i-Propyl steht,
oder zusammen mit R³ für Trimethylen oder Tetramethylen steht, und
Z für eine der nachstehenden Gruppierungen steht 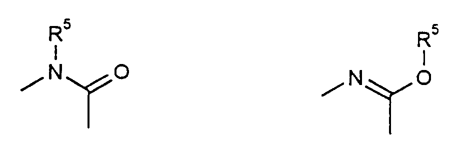 worin
R⁵ für jeweils gegebenenfalls durch Fluor, Chlor oder Cyano substituiertes Propenyl, Butenyl, Propinyl, Butinyl, Acetyl, Propionyl, Methoxycarbonyl oder Ethoxycarbonyl steht sowie deren Salze mit Basen, wie z.B. Natrium-, Kalium- oder Calcium-hydroxid, -hydrid, -amid oder -carbonat, Natrium- oder Kalium-C₁-C₄-alkanolaten, Ammoniak, C₁-C₄-Alkylaminen, Di-(C₁-C₄-alkyl)-aminen, Tri-(C₁-C₄-alkyl)-aminen oder auch mit Tris-(2-hydroxyethyl)amin.

5. N-Cyanoaryl-Stickstoffheterocyclen der allgemeinen Formeln (IA) und (IB) 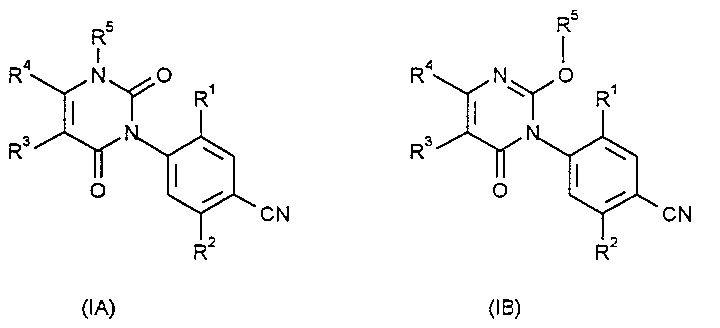 dadurch gekennzeichnet, daß die Reste R¹, R², R³, R⁴ und R⁵ die folgenden Bedeutungen haben:

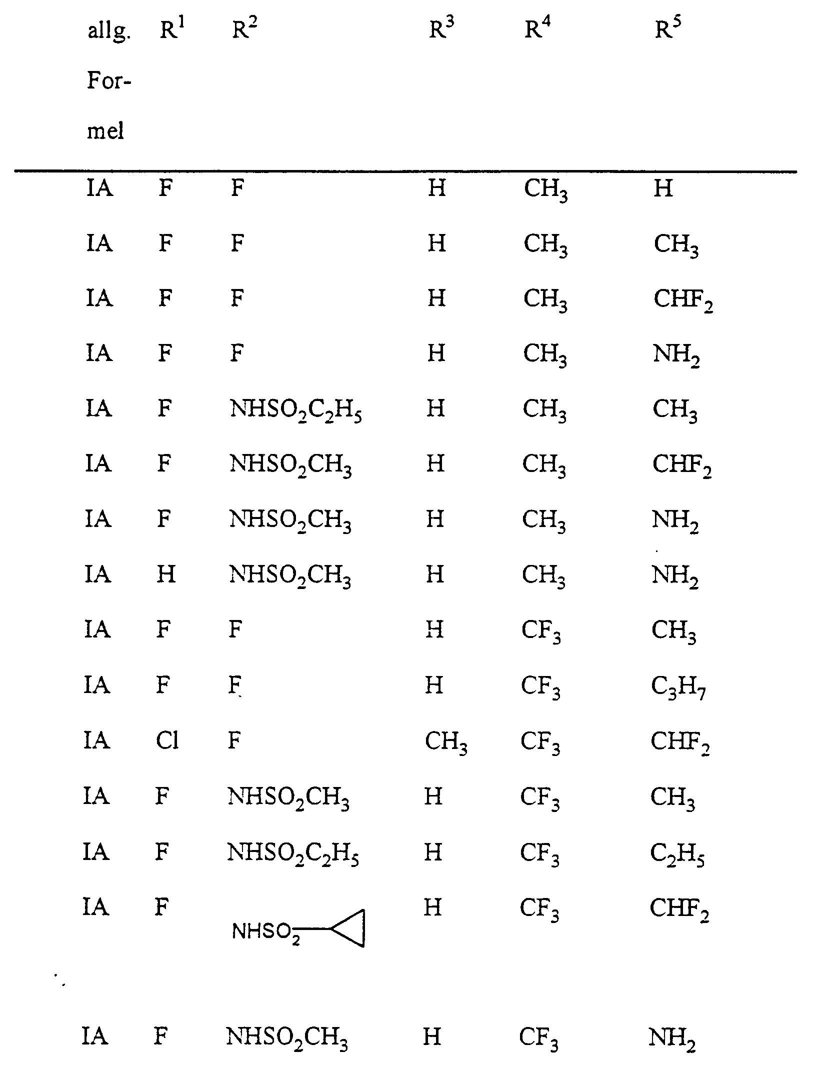

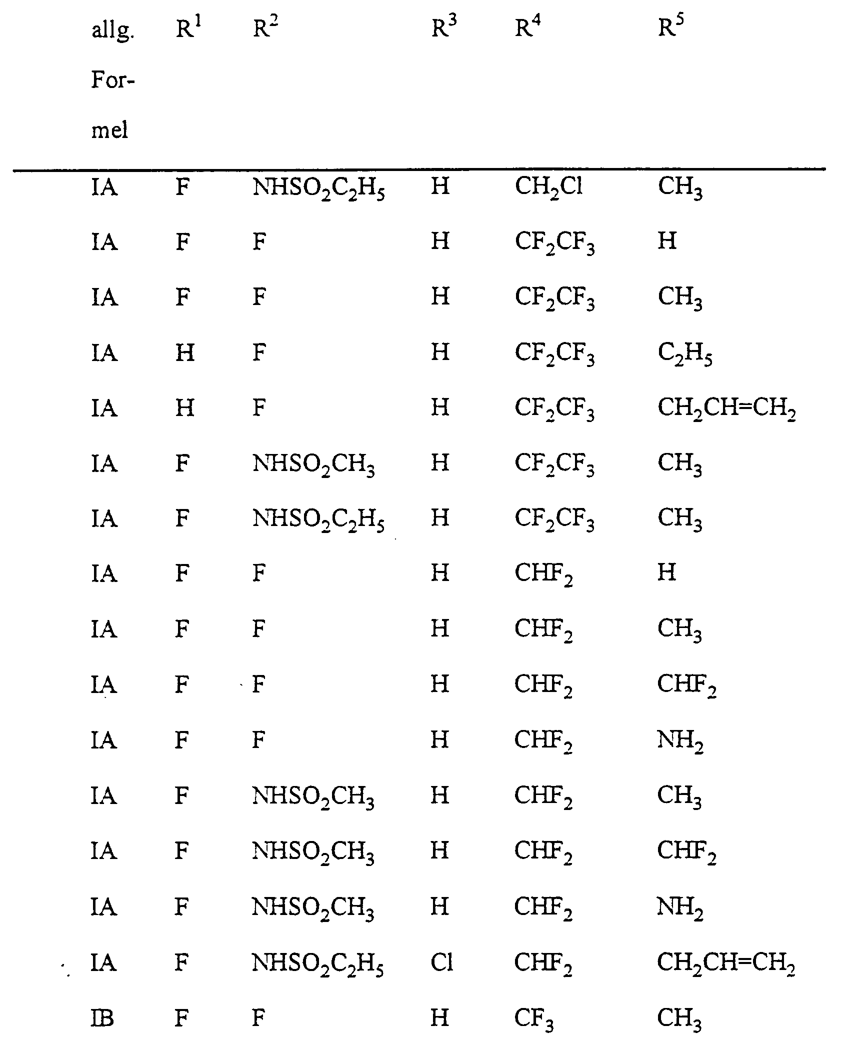

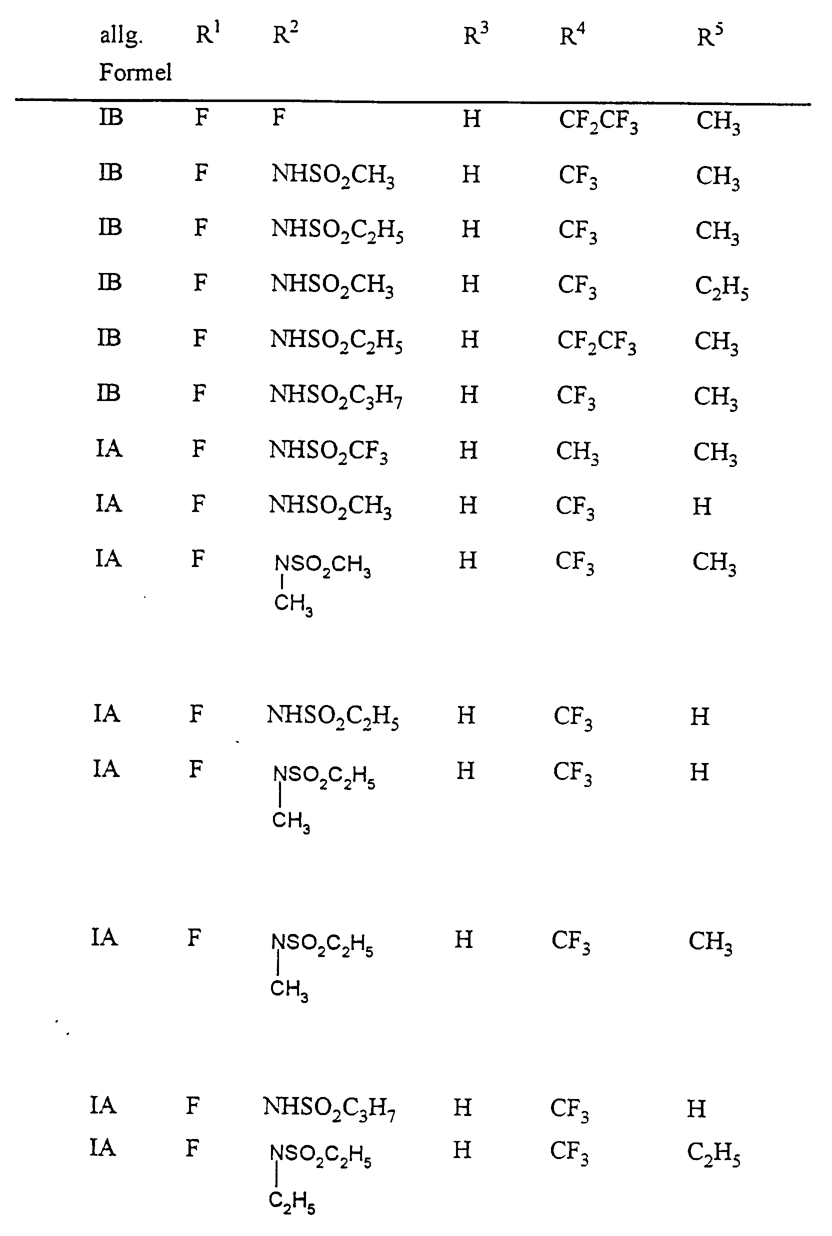

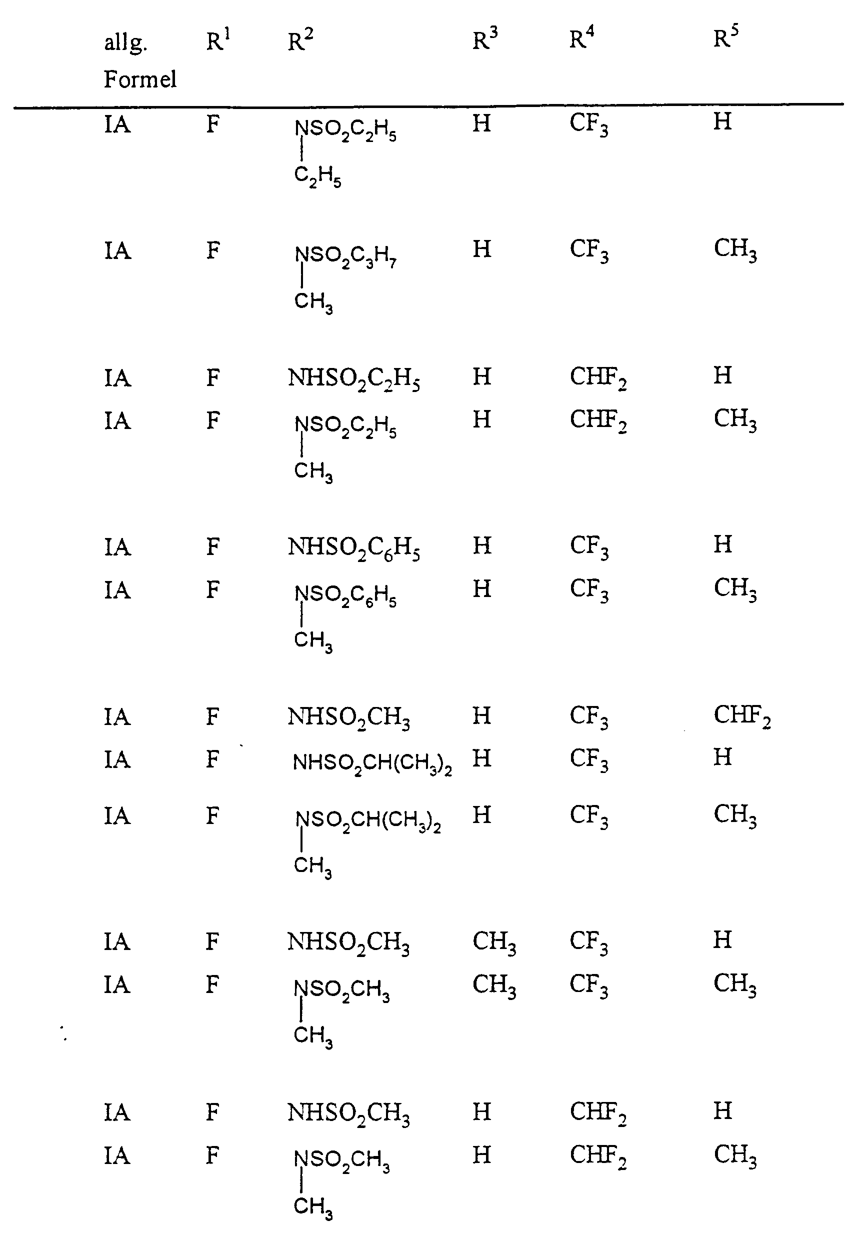

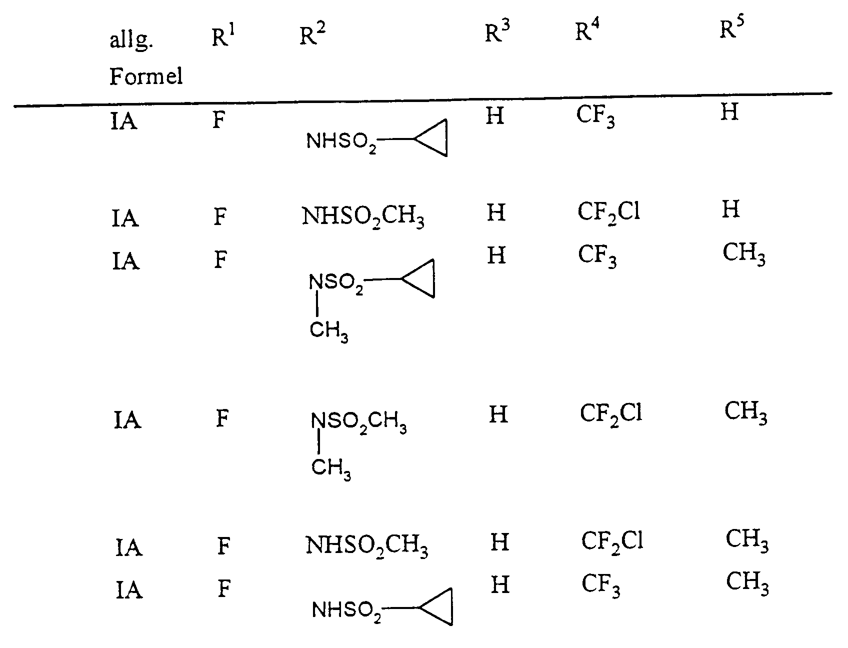

6. N-Cyanoaryl-Stickstoffheterocyclen der Formeln

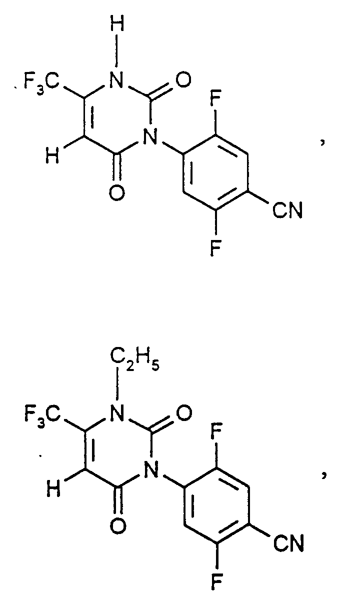

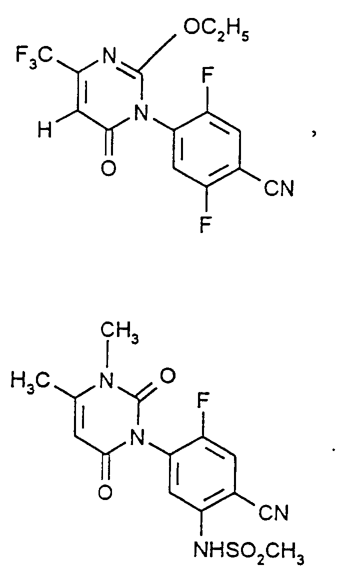

7. Verfahren zur Herstellung von N-Cyanoaryl-Stickstoffheterocyclen der allgemeinen Formel (I) 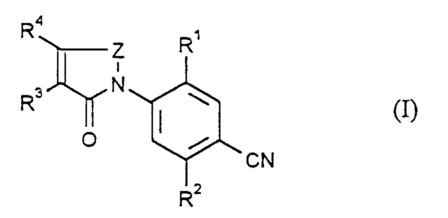 in welcher
R¹, R², R³, R⁴ und Z die in den Ansprüchen 1 bis 5 angegebenen Bedeutungen haben
sowie deren Salze, dadurch gekennzeichnet, daß man
(a) zur Herstellung von Verbindungen der Formeln (IA) und/oder (IB), in welchen R⁵ für Cyano, C₁-C₄-Alkoxy, C₁-C₄-Alkylcarbonyl oder C₁-C₄-Alkoxycarbonyl substituiertes Alkyl mit bis zu 6 Kohlenstoffatomen oder für jeweils gegebenenfalls durch Fluor, Chlor, Brom, Cyano, C₁-C₄-Alkoxy, C₁-C₄-Alkylcarbonyl oder C₁-C₄-Alkoxycarbonyl substituiertes Alkenyl, Alkinyl, Alkylcarbonyl oder Alkoxycarbonyl mit jeweils bis zu 6 Kohlenstoffatomen steht sowie R¹, R², R³ und R⁴ die oben angegebenen Bedeutungen haben,
N-Cyanoaryl-Stickstoffheterocyclen der allgemeinen Formeln (IA) oder (IB)
in welchen R⁵ für Wasserstoff steht sowie R¹, R², R³ und R⁴ die oben angegebenen Bedeutungen haben,
mit Alkylierungsmitteln bzw. Acylierungsmitteln der allgemeinen Formeln (V) oder (VI)
X-R⁵ (V)
R⁵-O-SO₂-O-R⁵ (VI)
in welchen R⁵ die oben angegebene Bedeutung hat und X in der Formel (V) für Halogen steht,
gegebenenfalls in Gegenwart eines Säureakzeptors und gegebenenfalls in Gegenwart eines Verdünnungsmittels umsetzt,
oder daß man
(b) zur Herstellung von Verbindungen der Formel (I), in welcher R² für die Gruppierung -N(A¹)SO₂A steht sowie A, A¹, R¹, R³ R⁴ und Z die oben angegebenen Bedeutungen haben,
N-Cyanoaryl-Stickstoffheterocyclen der allgemeinen Formel (I), in welcher R² für Fluor, Chlor, Brom steht sowie R¹, R³, R⁴ und Z die oben angegebenen Bedeutungen haben,
mit Ammoniak oder mit Amiden der allgemeinen Formel (VII)
HN(A¹)SO₂A (VII)
in welcher
A und A¹ die oben angegebenen Bedeutungen haben,
gegebenenfalls in Gegenwart eines Säureakzeptors und gegebenenfalls in Gegenwart eines Verdünnungsmittels umsetzt.

8. Herbizide Mittel gekennzeichnet durch einen Gehalt an mindestens einem N-Cyanoaryl-Stickstoffheterocyclus der Formel (I) gemäß den Ansprüchen 1 bis 4, der Formeln (IA) und (IB) gemäß Anspruch 5 bzw. der Formeln gemäß Anspruch 6.

9. Verfahren zur Bekämpfung von unerwünschten Pflanzen, dadurch gekennzeichnet, daß man N-Cyanoaryl-Stickstoffheterocyclen der allgemeinen Formel (I) gemäß den Ansprüchen 1 bis 4, der Formeln (IA) und (IB) gemäß Anspruch 5 bzw. der Formeln gemäß Anspruch 6 auf die Pflanzen und/oder ihren Lebensraum einwirken läßt.

10. Verwendung von N-Cyanoaryl-Stickstoffheterocyclen der allgemeinen Formel (I) gemäß der Ansprüche 1 bis 4, der Formeln (IA) und (IB) gemäß Anspruch 5 bzw. der Formeln gemäß Anspruch 6 zur Bekämpfung von unerwünschten Pflanzen.

11. Verfahren zur Herstellung von herbiziden Mitteln, dadurch gekennzeichnet, daß man N-Cyanoaryl-Stickstoffheterocyclen der allgemeinen Formel (I) gemäß der Ansprüche 1 bis 4, der Formeln (IA) und (IB) gemäß Anspruch 5 bzw. der Formeln gemäß Anspruch 6 mit Streckmitteln und/oder oberflächenaktiven Substanzen vermischt.

12. Cyanoarylisocyanate der allgemeinen Formel (III) 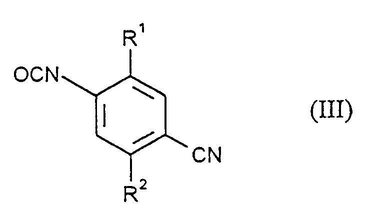 in welcher
R¹ für Fluor steht,
R² für Fluor, Chlor, Brom, Cyano oder die Gruppierung -N(A¹)SO₂A steht, worin
A für einen jeweils gegebenenfalls durch Fluor, Chlor, Brom, Cyano oder C₁-C₄-Alkoxy substituierten Rest der Reihe Alkyl, Alkenyl oder Alkinyl mit jeweils bis zu 10 Kohlenstoffatomen steht,
A weiterhin für jeweils gegebenenfalls durch Fluor, Chlor, Brom, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl, Phenyloxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl, C₁-C₄-Alkylsulfonyl oder C₁-C₄-Alkoxycarbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Heterocyclyl oder Heterocyclylalkyl mit 2 bis 6 Kohlenstoffatomen und 1 bis 4 Stickstoffatomen und/oder 1 bis 2 Sauerstoff- oder Schwefelatomen im gesättigten oder ungesättigten Heterocyclylteil und gegebenenfalls 1 bis 4 Kohlenstoffatomen im Alkylteil steht, und
A¹ für Wasserstoff oder für jeweils gegebenenfalls durch Halogen oder C₁-C₄-Alkoxy substituiertes Alkyl, Alkenyl, Alkinyl, Alkoxy, Alkylcarbonyl oder Alkoxycarbonyl mit jeweils bis zu 6 Kohlenstoffatomen, oder für Phenylcarbonyl, Naphthylcarbonyl, Phenylmethylcarbonyl, Naphthylmethylcarbonyl, Phenoxycarbonyl oder Naphthyloxycarbonyl (welche jeweils gegebenenfalls durch Fluor, Chlor, Brom, Cyano, Methyl, Methoxy, Trifluormethyl und/oder Trifluormethoxy substituiert sind) steht.

13. Cyanoarylurethane der allgemeinen Formel (IV) 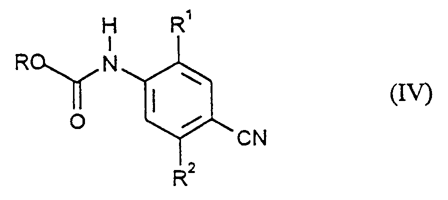 in welcher
R¹ für Fluor steht,
R² für Fluor, Chlor, Brom, Cyano oder die Gruppierung -N(A¹)SO₂A steht, worin
A für einen jeweils gegebenenfalls durch Fluor, Chlor, Brom, Cyano oder C₁-C₄-Alkoxy substituierten Rest der Reihe Alkyl, Alkenyl oder Alkinyl mit jeweils bis zu 10 Kohlenstoffatomen steht,
A weiterhin für jeweils gegebenenfalls durch Fluor, Chlor, Brom, Cyano oder C₁-C₄-Alkyl substituiertes Cycloalkyl oder Cycloalkylalkyl mit 3 bis 8 Kohlenstoffatomen im Cycloalkylteil und gegebenenfalls 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Fluor und/oder Chlor substituiert sind), durch Dimethylaminosulfonyl oder Diethylaminosulfonyl, durch C₁-C₄-Alkoxy-carbonyl (welches gegebenenfalls durch Fluor, Chlor, Brom, Methoxy oder Ethoxy substituiert ist), durch Phenyl, Phenyloxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Aryl oder Arylalkyl mit 6 oder 10 Kohlenstoffatomen im Arylteil und 1 bis 4 Kohlenstoffatomen im Alkylteil steht,
A weiterhin für jeweils gegebenenfalls durch Fluor, Chlor, Brom, Cyano, Nitro, Carboxy, Carbamoyl, durch C₁-C₄-Alkyl, C₁-C₄-Alkoxy, C₁-C₄-Alkylthio, C₁-C₄-Alkylsulfinyl, C₁-C₄-Alkylsulfonyl oder C₁-C₄-Alkoxycarbonyl (welche jeweils gegebenenfalls durch Fluor und/oder Chlor substituiert sind), durch Phenyl, Phenoxy oder Phenylthio (welche jeweils gegebenenfalls durch Fluor, Chlor, Brom, Cyano, Methyl, Methoxy, Trifluormethyl und/oder Trifluormethoxy substituiert sind) substituiertes Heterocyclyl oder Heterocyclylalkyl mit 2 bis 6 Kohlenstoffatomen und 1 bis 4 Stickstoffatomen und/oder 1 bis 2 Sauerstoff- oder Schwefelatomen im gesättigten oder ungesättigten Heterocyclylteil und gegebenenfalls 1 bis 4 Kohlenstoffatomen im Alkylteil steht, und
A¹ für Wasserstoff oder für jeweils gegebenenfalls durch Halogen oder C₁-C₄-Alkoxy substituiertes Alkyl, Alkenyl, Alkinyl, Alkoxy, Alkylcarbonyl oder Alkoxycarbonyl mit jeweils bis zu 6 Kohlenstoffatomen, oder für Phenylcarbonyl, Naphthylcarbonyl, Phenylmethylcarbonyl, Naphthylmethylcarbonyl, Phenoxycarbonyl oder Naphthyloxycarbonyl (welche jeweils gegebenenfalls durch Fluor, Chlor, Brom, Cyano, Methyl, Methoxy, Trifluormethyl und/oder Trifluormethoxy substituiert sind) steht und
R für C₁-C₄-Alkyl, Phenyl oder Benzyl steht.

## Claims

1. N-Cyanoaryl nitrogen heterocycles of the general formula (I) 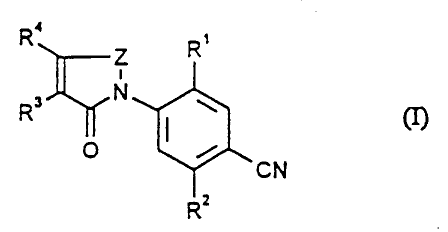 in which
R¹ represents fluorine,
R² represents fluorine, chlorine, bromine, cyano or the group -N(A¹)SO₂A, where
A represents a radical from the series consisting of alkyl, alkenyl or alkinyl, in each case having up to 10 carbon atoms, in each case optionally substituted by fluorine, chlorine, bromine, cyano or C₁-C₄-alkoxy,
A furthermore represents cycloalkyl or cycloalkylalkyl having 3 to 8 carbon atoms in the cycloalkyl moiety and, if appropriate, 1 to 4 carbon atoms in the alkyl moiety, in each case optionally substituted by fluorine, chlorine, bromine, cyano or C₁-C₄-alkyl,
A furthermore represents aryl or arylalkyl having 6 or 10 carbon atoms in the aryl moiety and 1 to 4 carbon atoms in the alkyl moiety, in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl or C₁-C₄-alkylsulphonyl (which are in each case optionally substituted by fluorine and/or chlorine), by dimethylaminosulphonyl or diethylaminosulphonyl, by C₁-C₄-alkoxycarbonyl (which is optionally substituted by fluorine, chlorine, bromine, methoxy or ethoxy), by phenyl, phenyloxy or phenylthio (which are in each case optionally substituted by fluorine, chlorine, bromine, cyano, methyl, methoxy, trifluoromethyl and/or trifluoromethoxy),
A furthermore represents heterocyclyl or heterocyclylalkyl having 2 to 6 carbon atoms and 1 to 4 nitrogen atoms and/or 1 to 2 oxygen or sulphur atoms in the saturated or unsaturated heterocyclyl moiety and, if appropriate, 1 to 4 carbon atoms in the alkyl moiety, in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl, C₁-C₄-alkylsulphonyl or C₁-C₄-alkoxycarbonyl (which are in each case optionally substituted by fluorine and/or chlorine), by phenyl, phenoxy or phenylthio (which are in each case optionally substituted by fluorine, chlorine, bromine, cyano, methyl, methoxy, trifluoromethyl and/or trifluoromethoxy), and
A¹ represents hydrogen, or represents alkyl, alkenyl, alkinyl, alkoxy, alkylcarbonyl or alkoxycarbonyl having in each case up to 6 carbon atoms, in each case optionally substituted by halogen or C₁-C₄-alkoxy, or represents phenylcarbonyl, naphthylcarbonyl, phenylmethylcarbonyl, naphthylmethylcarbonyl, phenoxycarbonyl or naphthyloxycarbonyl (which are in each case optionally substituted by fluorine, chlorine, bromine, cyano, methyl, methoxy, trifluoromethyl and/or trifluoromethoxy),
R³ represents hydrogen, fluorine, chlorine, bromine, cyano, or represents alkyl having 1 to 6 carbon atoms which is optionally substituted by fluorine and/or chlorine,
R⁴ represents alkyl having 1 to 6 carbon atoms which is optionally substituted by fluorine, chlorine, bromine, methoxy or ethoxy,
or together with R³ represents alkanediyl having 2 to 8 carbon atoms, and
Z represents one of the following groups 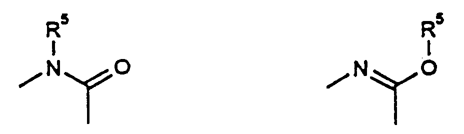 where
R⁵ represents amino, or represents alkyl having up to 6 carbon atoms which is substituted by cyano, C₁-C₄-alkoxy, C₁-C₄-alkylcarbonyl or C₁-C₄-alkoxycarbonyl, and their salts with bases such as, for example, sodium hydroxide, potassium hydroxide, calcium hydroxide, sodium hydride, potassium hydride, calcium hydride, sodium amide, potassium amide, calcium amide, sodium carbonate, potassium carbonate, calcium carbonate, sodium C₁-C₄-alkoxides, potassium C₁-C₄-alkoxides, ammonia, C₁-C₄-alkylamines, di-(C₁-C₄-alkyl)amines, tri-(C₁-C₄-alkyl)amines, or else with tris-(2-hydroxyethyl)amine.

2. N-Cyanoaryl nitrogen heterocycles of the general formula (I) according to Claim 1, characterized in that
R¹ represents fluorine,
R² represents fluorine, chlorine, bromine, cyano or the group -N(A¹)SO₂A, where
A represents methyl, ethyl, n- or i-propyl, n-, i-, s- or t-butyl or n-, i-, s- or t-pentyl, in each case optionally substituted by fluorine or chlorine,
A furthermore represents cyclopropyl, cyclobutyl, cyclopentyl, cyclohexyl, cyclopropylmethyl, cyclobutylmethyl, cyclopentylmethyl or cyclohexylmethyl, in each case optionally substituted by fluorine, chlorine, bromine, methyl and/or ethyl,
A furthermore represents phenyl, naphthyl, phenylmethyl or phenylethyl, in each case optionally substituted by fluorine, chlorine, bromine, cyano, nitro, carboxyl, methyl, trifluoromethyl, methoxy, ethoxy, difluoromethoxy, trifluoromethoxy, methylthio, ethylthio, methylsulphinyl, ethylsulphinyl, methylsulphonyl, ethylsulphonyl, dimethylaminosulphonyl, methoxycarbonyl, ethoxycarbonyl, propoxycarbonyl or phenyl,
A furthermore represents thienyl, pyrazolyl or pyridyl, in each case optionally substituted by fluorine, chlorine, bromine, cyano, methyl, ethyl, trifluoromethyl, methoxy, ethoxy, methylthio, ethylthio, methylsulphinyl, ethylsulphinyl, methylsulphonyl, ethylsulphonyl, methoxycarbonyl or ethoxycarbonyl, and
A¹ represents hydrogen, or represents methyl, ethyl, n- or i-propyl, n-, i- or s-butyl, propenyl, butenyl, propinyl, butinyl, methoxy, ethoxy, n- or i-propoxy, n-, i-, s- or t-butoxy, acetyl, propionyl, methoxycarbonyl or ethoxycarbonyl, in each case optionally substituted by fluorine, chlorine, methoxy or ethoxy, or represents phenylcarbonyl or phenoxycarbonyl,
R³ represents hydrogen, fluorine, chlorine, bromine, methyl, trifluoromethyl, ethyl, n- or i-propyl,
R⁴ represents methyl, ethyl or n- or i-propyl, in each case optionally substituted by fluorine or chlorine,
or together with R³ represents trimethylene or tetramethylene, and
Z represents one of the following groups 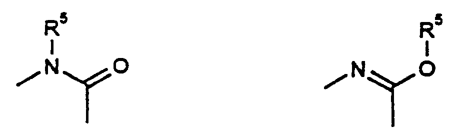 where
R⁵ represents amino or represents cyano-substituted methyl, ethyl, n- or i-propyl or n-, i- or s-butyl, and their salts with bases such as, for example, sodium hydroxide, potassium hydroxide, calcium hydroxide, sodium hydride, potassium hydride, calcium hydride, sodium amide, potassium amide, calcium amide, sodium carbonate, potassium carbonate, calcium carbonate, sodium C₁-C₄-alkoxides, potassium C₁-C₄-alkoxides, ammonia, C₁-C₄-alkylamines, di-(C₁-C₄-alkyl)amines, tri-(C₁-C₄-alkyl)-amines, or else with tris-(2-hydroxyethyl)amine.

3. N-Cyanoaryl nitrogen heterocycles of the general formula (I) 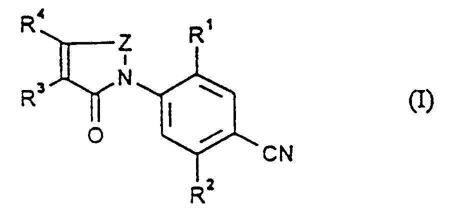 in which
R¹ represents fluorine,
R² represents fluorine, chlorine, bromine, cyano or the group -N(A¹)SO₂A, where
A represents a radical from the series consisting of alkyl, alkenyl or alkinyl, in each case having up to 10 carbon atoms, in each case optionally substituted by fluorine, chlorine, bromine, cyano or C₁-C₄-alkoxy,
A furthermore represents cycloalkyl or cycloalkylalkyl having 3 to 8 carbon atoms in the cycloalkyl moiety and, if appropriate, 1 to 4 carbon atoms in the alkyl moiety, in each case optionally substituted by fluorine, chlorine, bromine, cyano or C₁-C₄-alkyl,
A furthermore represents aryl or arylalkyl having 6 or 10 carbon atoms in the aryl moiety and 1 to 4 carbon atoms in the alkyl moiety, in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl or C₁-C₄-alkylsulphonyl (which are in each case optionally substituted by fluorine and/or chlorine), by dimethylaminosulphonyl or diethylaminosulphonyl, by C₁-C₄-alkoxycarbonyl (which is optionally substituted by fluorine, chlorine, bromine, methoxy or ethoxy), by phenyl, phenyloxy or phenylthio (which are in each case optionally substituted by fluorine, chlorine, bromine, cyano, methyl, methoxy, trifluoromethyl and/or trifluoromethoxy),
A furthermore represents heterocyclyl or heterocyclylalkyl having 2 to 6 carbon atoms and 1 to 4 nitrogen atoms and/or 1 to 2 oxygen or sulphur atoms in the saturated or unsaturated heterocyclyl moiety and, if appropriate, 1 to 4 carbon atoms in the alkyl moiety, in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl, C₁-C₄-alkylsulphonyl or C₁-C₄-alkoxycarbonyl (which are in each case optionally substituted by fluorine and/or chlorine), by phenyl, phenoxy or phenylthio (which are in each case optionally substituted by fluorine, chlorine, bromine, cyano, methyl, methoxy, trifluoromethyl and/or trifluoromethoxy), and
A¹ represents hydrogen, or represents alkyl, alkenyl, alkinyl, alkoxy, alkylcarbonyl or alkoxycarbonyl having in each case up to 6 carbon atoms, in each case optionally substituted by halogen or C₁-C₄-alkoxy, or represents phenylcarbonyl, naphthylcarbonyl, phenylmethylcarbonyl, naphthylmethylcarbonyl, phenoxycarbonyl or naphthyloxycarbonyl (which are in each case optionally substituted by fluorine, chlorine, bromine, cyano, methyl, methoxy, trifluoromethyl and/or trifluoromethoxy),
R³ represents hydrogen, fluorine, chlorine, bromine, cyano, or represents alkyl having 1 to 6 carbon atoms which is optionally substituted by fluorine and/or chlorine,
R⁴ represents alkyl having 1 to 6 carbon atoms which is optionally substituted by fluorine, chlorine, bromine, methoxy or ethoxy,
or together with R³ represents alkanediyl having 2 to 8 carbon atoms, and
Z represents one of the following groups 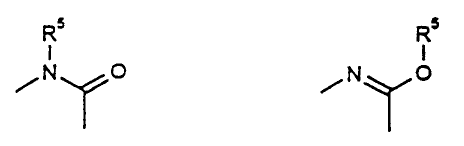 where
R⁵ represents alkenyl, alkinyl, alkylcarbonyl or alkoxycarbonyl having in each case up to 6 carbon atoms, in each case optionally substituted by fluorine, chlorine, bromine, cyano, C₁-C₄-alkoxy, C₁-C₄-alkylcarbonyl or C₁-C₄-alkoxycarbonyl, and their salts with bases such as, for example, sodium hydroxide, potassium hydroxide, calcium hydroxide, sodium hydride, potassium hydride, calcium hydride, sodium amide, potassium amide, calcium amide, sodium carbonate, potassium carbonate, calcium carbonate, sodium C₁-C₄-alkoxides, potassium C₁-C₄-alkoxides, ammonia, C₁-C₄-alkylamines, di-(C₁-C₄-alkyl)amines, tri-(C₁-C₄-alkyl)-amines, or else with tris-(2-hydroxyethyl)amine.

4. N-Cyanoaryl nitrogen heterocycles of the general formula (I) according to Claim 3, characterized in that
R¹ represents fluorine,
R² represents fluorine, chlorine, bromine, cyano or the group -N(A¹)SO₂A, where
A represents methyl, ethyl, n- or i-propyl, n-, i-, s- or t-butyl or n-, i-, s- or t-pentyl, in each case optionally substituted by fluorine or chlorine,
A furthermore represents cyclopropyl, cyclobutyl, cyclopentyl, cyclohexyl, cyclopropylmethyl, cyclobutylmethyl, cyclopentylmethyl or cyclohexylmethyl, in each case optionally substituted by fluorine, chlorine, bromine, methyl and/or ethyl,
A furthermore represents phenyl, naphthyl, phenylmethyl or phenylethyl, in each case optionally substituted by fluorine, chlorine, bromine, cyano, nitro, carboxyl, methyl, trifluoromethyl, methoxy, ethoxy, difluoromethoxy, trifluoromethoxy, methylthio, ethylthio, methylsulphinyl, ethylsulphinyl, methylsulphonyl, ethylsulphonyl, dimethylaminosulphonyl, methoxycarbonyl, ethoxycarbonyl, propoxycarbonyl or phenyl,
A furthermore represents thienyl, pyrazolyl or pyridyl, in each case optionally substituted by fluorine, chlorine, bromine, cyano, methyl, ethyl, trifluoromethyl, methoxy, ethoxy, methylthio, ethylthio, methylsulphinyl, ethylsulphinyl, methylsulphonyl, ethylsulphonyl, methoxycarbonyl or ethoxycarbonyl, and
A¹ represents hydrogen, or represents methyl, ethyl, n- or i-propyl, n-, i- or s-butyl, propenyl, butenyl, propinyl, butinyl, methoxy, ethoxy, n- or i-propoxy, n-, i-, s- or t-butoxy, acetyl, propionyl, methoxycarbonyl or ethoxycarbonyl, in each case optionally substituted by fluorine, chlorine, methoxy or ethoxy, or represents phenylcarbonyl or phenoxycarbonyl,
R³ represents hydrogen, fluorine, chlorine, bromine, methyl, trifluoromethyl, ethyl, n- or i-propyl,
R⁴ represents methyl, ethyl, n- or i-propyl, in each case optionally substituted by fluorine or chlorine,
or together with R³ represents trimethylene or tetramethylene, and
Z represents one of the following groups 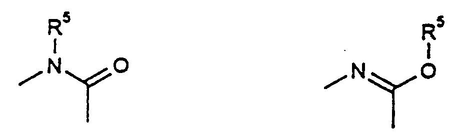 where
R⁵ represents propenyl, butenyl, propinyl, butinyl, acetyl, propionyl, methoxycarbonyl or ethoxycarbonyl, in each case optionally substituted by fluorine, chlorine or cyano, and their salts with bases such as, for example, sodium hydroxide, potassium hydroxide, calcium hydroxide, sodium hydride, potassium hydride, calcium hydride, sodium amide, potassium amide, calcium amide, sodium carbonate, potassium carbonate, calcium carbonate, sodium C₁-C₄-alkoxides, potassium C₁-C₄-alkoxides, ammonia, C₁-C₄-alkylamines, di-(C₁-C₄-alkyl)amines, tri-(C₁-C₄-alkyl)-amines, or else with tris-(2-hydroxyethyl)amine.

5. N-Cyanoaryl nitrogen heterocycles of the general formulae (IA) and (IB) 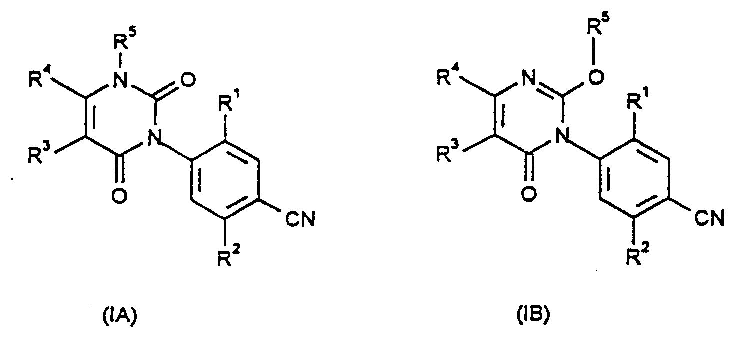 characterized in that the radicals R¹, R², R³, R⁴ and R⁵ have the following meanings:

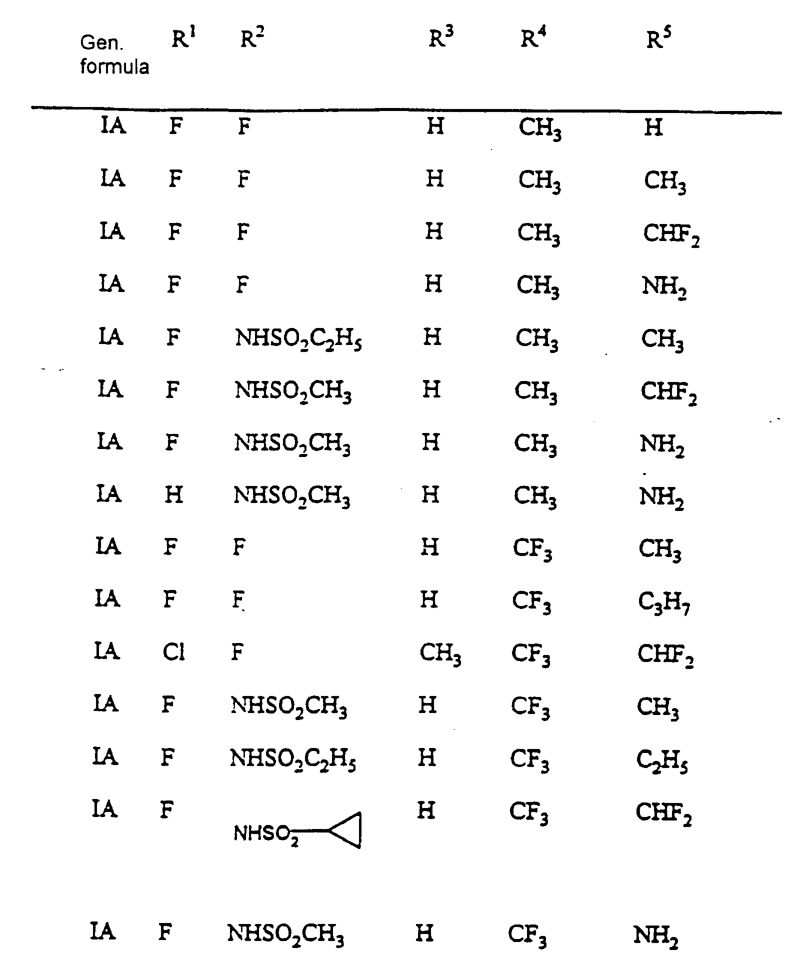

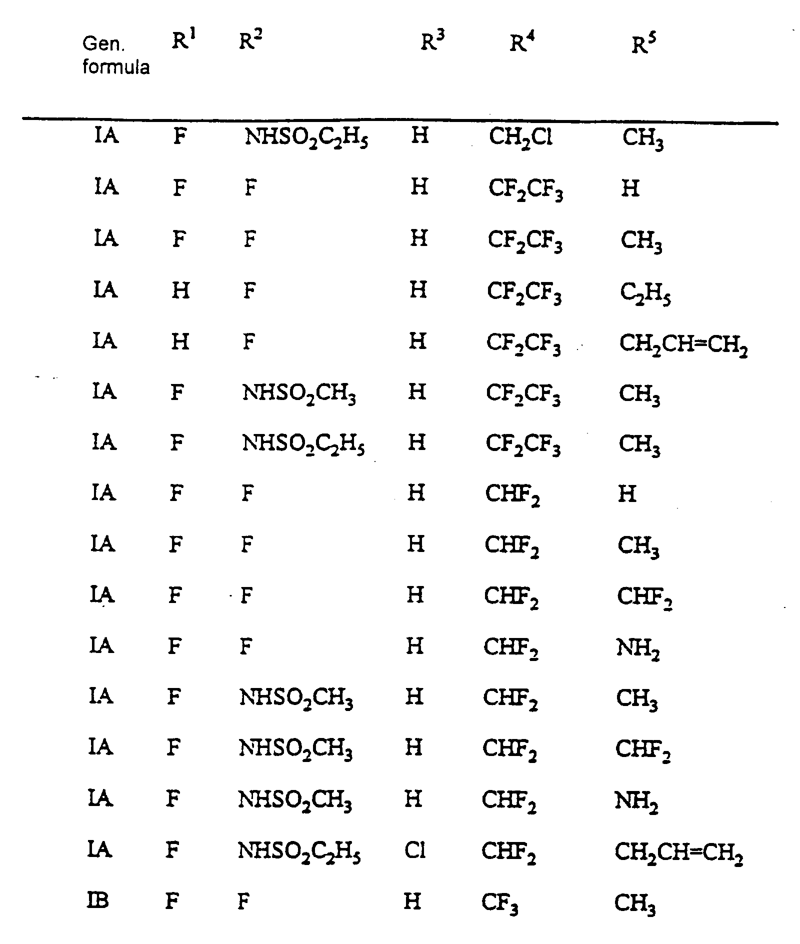

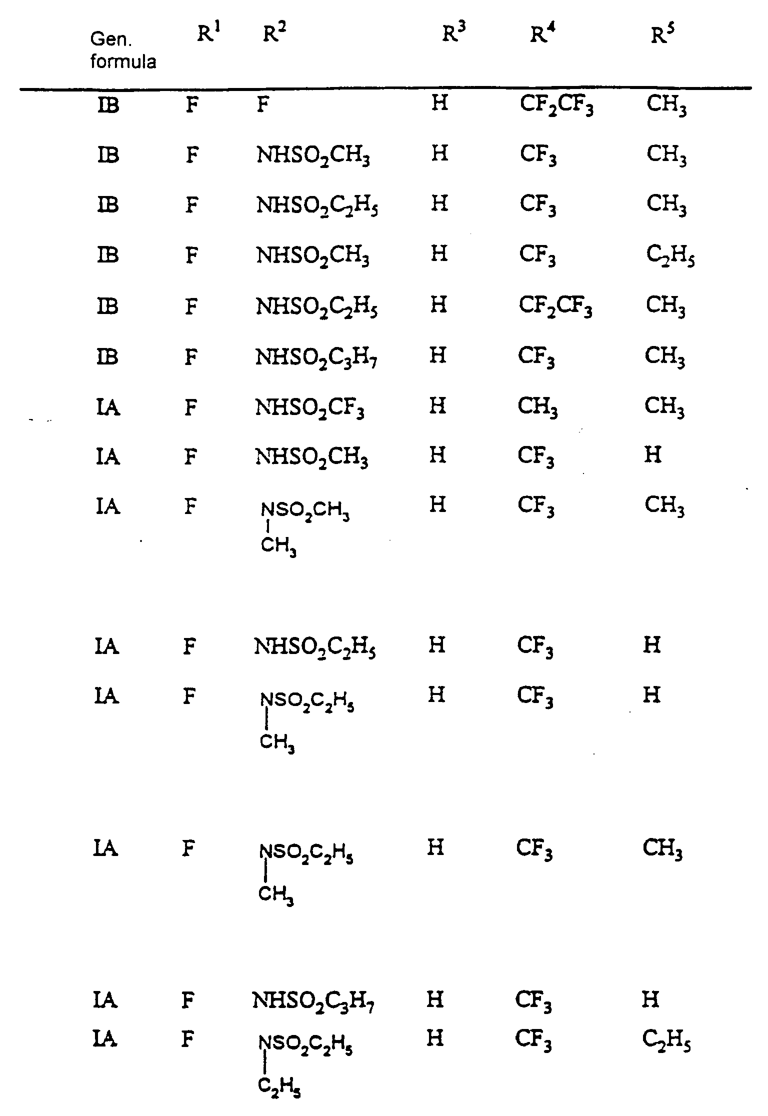

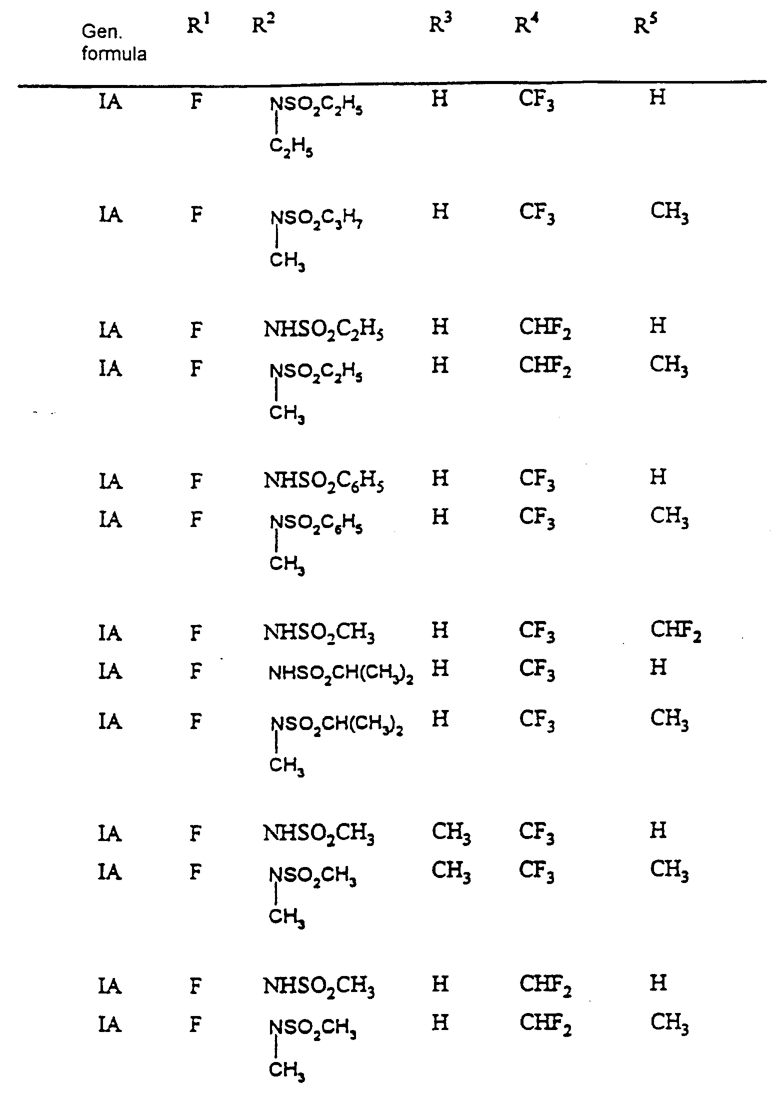

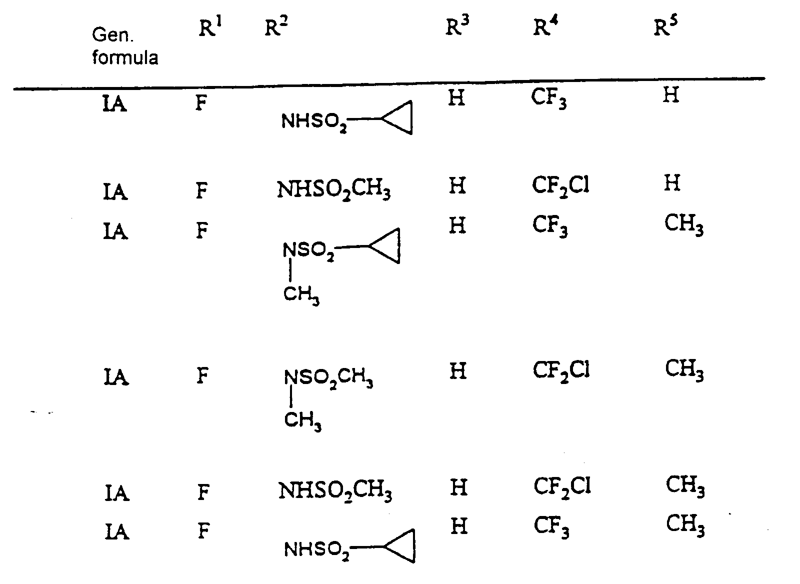

6. N-Cyanoaryl nitrogen heterocycles of the formulae

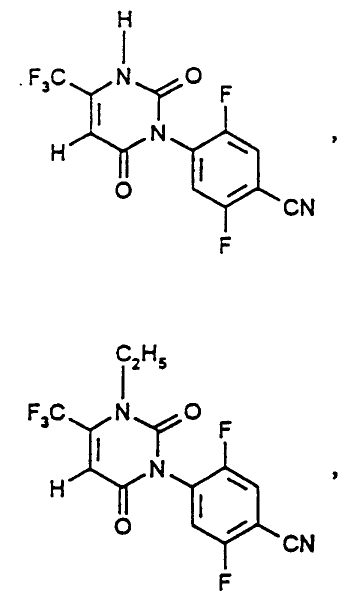

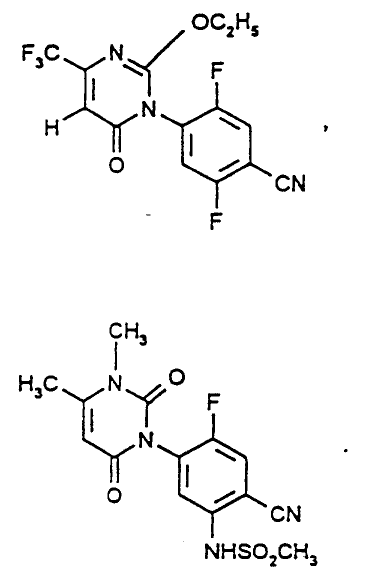

7. Process for the preparation of N-cyanoaryl nitrogen heterocycles of the general formula (I) 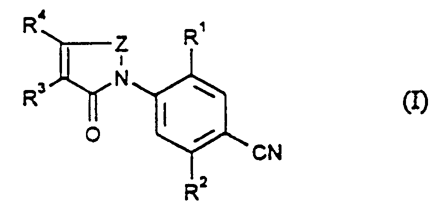 in which
R¹, R², R³, R⁴ and Z have the meanings given in Claims 1 to 5
and of their salts, characterized in that
(a) to prepare compounds of the formulae (IA) and/or (IB), in which R⁵ represents alkyl having up to 6 carbon atoms which is substituted by cyano, C₁-C₄-alkoxy, C₁-C₄-alkylcarbonyl or C₁-C₄-alkoxycarbonyl, or represents alkenyl, alkinyl, alkylcarbonyl or alkoxycarbonyl, in each case having up to 6 carbon atoms, in each case optionally substituted by fluorine, chlorine, bromine, cyano, C₁-C₄-alkoxy, C₁-C₄-alkylcarbonyl or C₁-C₄-alkoxycarbonyl, and R¹, R², R³ and R⁴ have the abovementioned meanings, or
N-cyanoaryl nitrogen heterocycles of the general formulae (IA) or (IB)
in which R⁵ represents hydrogen and R¹, R², R³ and R⁴ have the abovementioned meanings
are reacted with alkylating agents or acylating agents of the general formulae (V) or (VI)
X-R⁵ (V)
R⁵-O-SO₂-O-R⁵ (VI)
in which R⁵ has the abovementioned meaning and X in formula (V) represents halogen,
if appropriate in the presence of an acid acceptor and if appropriate in the presence of a diluent,
or in that
(b) to prepare compounds of the formula (I), in which R² represents the group -N(A¹)SO₂A and A, A¹, R¹, R³, R⁴ and Z have the abovementioned meanings,
N-cyanoaryl nitrogen heterocycles of the general formula (I), in which R² represents fluorine, chlorine or bromine and R¹, R³, R⁴ and Z have the abovementioned meanings,
are reacted with ammonia or with amides of the general formula (VII)
HN(A¹)SO₂A (VII)
in which
A and A¹ have the abovementioned meanings,
if appropriate in the presence of an acid acceptor and if appropriate in the presence of a diluent.

8. Herbicidal compositions, characterized in that they comprise at least one N-cyanoaryl nitrogen heterocycle of the formula (I) according to Claims 1 to 4, of the formulae (IA) and (IB) according to Claim 5 or of the formula according to Claim 6.

9. Method of controlling undesired plants, characterized in that N-cyanoaryl nitrogen heterocycles of the general formula (I) according to Claims 1 to 4, of the formulae (IA) and (IB) according to Claim 5 or of the formulae according to Claim 6 are allowed to act on the plants and/or their environment.

10. Use of N-cyanoaryl nitrogen heterocycles of the general formula (I) according to Claims 1 to 4, of the formulae (IA) and (IB) according to Claim 5 or of the formulae according to Claim 6 for controlling undesired plants.

11. Process for the preparation of herbicidal compositions, characterized in that N-cyanoaryl nitrogen heterocycles of the general formula (I) according to Claims 1 to 4, of the formulae (IA) and (IB) according to Claim 5 or of the formulae according to Claim 6 are mixed with extenders and/or surfactants.

12. Cyanoaryl isocyanates of the general formula (III) 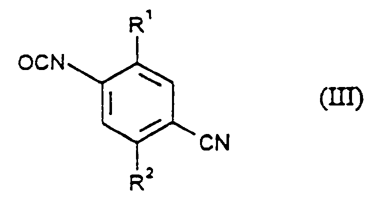 in which
R¹ represents fluorine,
R² represents fluorine, chlorine, bromine, cyano or the group -N(A¹)SO₂A, where
A represents a radical from the series consisting of alkyl, alkenyl or alkinyl, in each case having up to 10 carbon atoms, in each case optionally substituted by fluorine, chlorine, bromine, cyano or C₁-C₄-alkoxy,
A furthermore represents cycloalkyl or cycloalkylalkyl having 3 to 8 carbon atoms in the cycloalkyl moiety and, if appropriate, 1 to 4 carbon atoms in the alkyl moiety, in each case optionally substituted by fluorine, chlorine, bromine, cyano or C₁-C₄-alkyl,
A furthermore represents aryl or arylalkyl having 6 or 10 carbon atoms in the aryl moiety and 1 to 4 carbon atoms in the alkyl moiety, in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl or C₁-C₄-alkylsulphonyl (which are in each case optionally substituted by fluorine and/or chlorine), by dimethylaminosulphonyl or diethylaminosulphonyl, by C₁-C₄-alkoxycarbonyl (which is optionally substituted by fluorine, chlorine, bromine, methoxy or ethoxy), by phenyl, phenyloxy or phenylthio (which are in each case optionally substituted by fluorine, chlorine, bromine, cyano, methyl, methoxy, trifluoromethyl and/or trifluoromethoxy),
A furthermore represents heterocyclyl or heterocyclylalkyl having 2 to 6 carbon atoms and 1 to 4 nitrogen atoms and/or 1 to 2 oxygen or sulphur atoms in the saturated or unsaturated heterocyclyl moiety and, if appropriate, 1 to 4 carbon atoms in the alkyl moiety, in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl, C₁-C₄-alkylsulphonyl or C₁-C₄-alkoxycarbonyl (which are in each case optionally substituted by fluorine and/or chlorine), by phenyl, phenoxy or phenylthio (which are in each case optionally substituted by fluorine, chlorine, bromine, cyano, methyl, methoxy, trifluoromethyl and/or trifluoromethoxy), and
A¹ represents hydrogen, or represents alkyl, alkenyl, alkinyl, alkoxy, alkylcarbonyl or alkoxycarbonyl having in each case up to 6 carbon atoms, in each case optionally substituted by halogen or C₁-C₄-alkoxy, or represents phenylcarbonyl, naphthylcarbonyl, phenylmethylcarbonyl, naphthylmethylcarbonyl, phenoxycarbonyl or naphthyloxycarbonyl (which are in each case optionally substituted by fluorine, chlorine, bromine, cyano, methyl, methoxy, trifluoromethyl and/or trifluoromethoxy),

13. Cyanoarylurethanes of the general formula (IV) 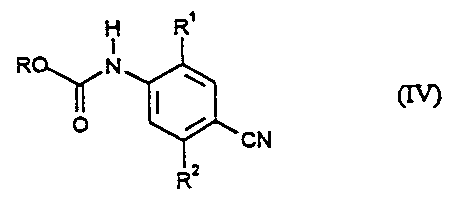 in which
R¹ represents fluorine,
R² represents fluorine, chlorine, bromine, cyano or the group -N(A¹)SO₂A, where
A represents a radical from the series consisting of alkyl, alkenyl or alkinyl, in each case having up to 10 carbon atoms, in each case optionally substituted by fluorine, chlorine, bromine, cyano or C₁-C₄-alkoxy,
A furthermore represents cycloalkyl or cycloalkylalkyl having 3 to 8 carbon atoms in the cycloalkyl moiety and, if appropriate, 1 to 4 carbon atoms in the alkyl moiety, in each case optionally substituted by fluorine, chlorine, bromine, cyano or C₁-C₄-alkyl,
A furthermore represents aryl or arylalkyl having 6 or 10 carbon atoms in the aryl moiety and 1 to 4 carbon atoms in the alkyl moiety, in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl or C₁-C₄-alkylsulphonyl (which are in each case optionally substituted by fluorine and/or chlorine), by dimethylaminosulphonyl or diethylaminosulphonyl, by C₁-C₄-alkoxycarbonyl (which is optionally substituted by fluorine, chlorine, bromine, methoxy or ethoxy), by phenyl, phenyloxy or phenylthio (which are in each case optionally substituted by fluorine, chlorine, bromine, cyano, methyl, methoxy, trifluoromethyl and/or trifluoromethoxy),
A furthermore represents heterocyclyl or heterocyclylalkyl having 2 to 6 carbon atoms and 1 to 4 nitrogen atoms and/or 1 to 2 oxygen or sulphur atoms in the saturated or unsaturated heterocyclyl moiety and, if appropriate, 1 to 4 carbon atoms in the alkyl moiety, in each case optionally substituted by fluorine, chlorine, bromine, cyano, nitro, carboxyl, carbamoyl, by C₁-C₄-alkyl, C₁-C₄-alkoxy, C₁-C₄-alkylthio, C₁-C₄-alkylsulphinyl, C₁-C₄-alkylsulphonyl or C₁-C₄-alkoxycarbonyl (which are in each case optionally substituted by fluorine and/or chlorine), by phenyl, phenoxy or phenylthio (which are in each case optionally substituted by fluorine, chlorine, bromine, cyano, methyl, methoxy, trifluoromethyl and/or trifluoromethoxy), and
A¹ represents hydrogen, or represents alkyl, alkenyl, alkinyl, alkoxy, alkylcarbonyl or alkoxycarbonyl having in each case up to 6 carbon atoms, in each case optionally substituted by halogen or C₁-C₄-alkoxy, or represents phenylcarbonyl, naphthylcarbonyl, phenylmethylcarbonyl, naphthylmethylcarbonyl, phenoxycarbonyl or naphthyloxycarbonyl (which are in each case optionally substituted by fluorine, chlorine, bromine, cyano, methyl, methoxy, trifluoromethyl and/or trifluoromethoxy), and
R represents C₁-C₄-alkyl, phenyl or benzyl.

## Revendications

1. Hétérocycles azotés N-cyanarylés de formule générale (I) 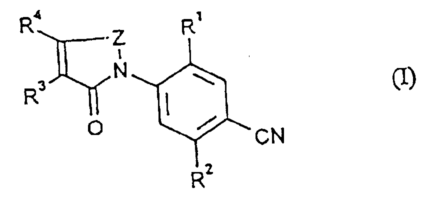 dans laquelle
R¹ représente du fluor,
R² est du fluor, du chlore, du brome, un groupe cyano ou le groupement -N(A¹)SO₂A, dans lequel
A représente un reste de la série des restes alkyle, alcényle ou alcynyle ayant chacun jusqu'à 10 atomes de carbone, éventuellement substitué dans chaque cas par du fluor, du chlore, du brome, un radical cyano ou alkoxy en C₁ à C₄,
A représente en outre un reste cycloalkyle ou cycloalkylalkyle ayant 3 à 8 atomes de carbone dans la partie cycloalkyle et le cas échéant 1 à 4 atomes de carbone dans la partie alkyle, chacun étant éventuellement substitué par du fluor, du chlore, du brome, un radical cyano ou alkyle en C₁ à C₄,
A représente en outre un reste aryle ou arylalkyle ayant 6 ou 10 atomes de carbone dans la partie aryle et 1 à 4 atomes de carbone dans la partie alkyle, chacun étant substitué le cas échéant par du fluor, du chlore, du brome, des radicaux cyano, nitro, carboxy, carbamoyle, par des radicaux alkyle en C₁ à C₄, alkoxy en C₁ à C₄, alkylthio en C₁ à C₄, alkylsulfinyle en C₁ à C₄ ou alkylsulfonyle en C₁ à C₄ (qui sont substitués chacun le cas échéant par du fluor et/ou du chlore), par des radicaux diméthylaminosulfonyle ou diéthylaminosulfonyle, par un radical (alkoxy en C₁ à C₄)carbonyle (qui est substitué le cas échéant par du fluor, du chlore, du brome, un radical méthoxy ou éthoxy), par des radicaux phényle, phényloxy ou phénylthio (qui sont substitués le cas échéant par du fluor, du chlore, du brome, des radicaux cyano, méthyle, méthoxy, trifluorométhyle et/ou trifluorométhoxy),
A représente en outre un reste hétérocyclyle ou hétérocyclyalkyle ayant 2 à 6 atomes de carbone et 1 à 4 atomes d'azote et/ou 1 ou 2 atomes d'oxygène ou de soufre dans la partie hétérocyclyle saturée ou non saturée et le cas échéant 1 à 4 atomes de carbone dans la partie alkyle, chacun étant substitué le cas échéant par du fluor, du chlore, du brome, des radicaux cyano, nitro, carboxy, carbamoyle, par des radicaux alkyle en C₁ à C₄, alkoxy en C₁ à C₄, alkylthio en C₁ à C₄, alkylsulfinyle en C₁ à C₄, alkylsulfonyle en C₁ à C₄ ou (alkoxy en C₁ à C₄)-carbonyle (qui sont substitués chacun le cas échéant par du fluor et/ou du chlore), par des radicaux phényle, phénoxy ou phénylthio (qui sont sutstitués chacun le cas échéant par du fluor, du chlore, du brome, des radicaux cyano, méthyle, méthoxy, trifluorométhyle et/ou trifluorométhoxy), et
A¹ représente de l'hydrogène ou des restes alkyle, alcényle, alcynyle, alkoxy, alkylcarbonyle ou alkoxycarbonyle ayant chacun jusqu'à 6 atomes de carbone et dont chacun est substitué le cas échéant par un halogène ou un radical alkoxy en C₁ à C₄, ou des restes phénylcarbonyle, naphtylcarbonyle, phénylméthylcarbonyle, naphtylméthylcarbonyle, phénoxycarbonyle ou naphtyloxycarbonyle (qui sont substitués chacun le cas échéant par du fluor, du chlore, du brome, des radicaux cyano, méthyle, méthoxy, trifluorométhyle et/ou trifluorométhoxy),
R³ représente de l'hydrogène, du fluor, du chlore, du brome, un groupe cyano, un radical alkyle ayant 1 à 6 atomes de carbone éventuellement substitué par du fluor, et/ou du chlore,
R⁴ représente un reste alkyle ayant 1 à 6 atomes de carbone éventuellement substitué par du fluor, du chlore, du brome, un radical méthoxy ou éthoxy,
ou forme conjointement avec R³ un groupe alcanediyle ayant 2 à 8 atomes de carbone, et
Z représente l'un des groupements suivants : 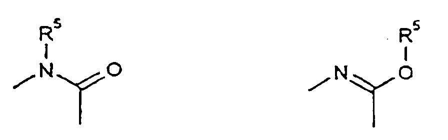 dans lesquels
R⁵ est un groupe amino ou un reste alkyle ayant jusqu'à 6 atomes de carbone substitué par un radical cyano, alkoxy en C₁ à C₄ (alkyle en C₁ à C₄)-carbonyle, ou (alkoxy en C₁ à C₄)-carbonyle, ainsi que leurs sels avec des bases, par exemple avec l'hydroxyde, l'hydrure, l'amidure ou le carbonate de sodium, de potassium ou de calcium ou des alcanolates en C₁ à C₄ de sodium ou de potassium, l'ammoniac, des alkylamines en C₁ à C₄, des di(alkyle en C₁ à C₄)-amines, des tri(alkyle en C₁ à C₄)-amines ou également avec la tris-(2-hydroxyéthyl)amine.

2. Hétérocycles azotés N-cyanarylés de formule générale (I) suivant la revendication 1, caractérisés en ce que
R¹ est du fluor,
R² est du fluor, du chlore, du brome, un groupe cyano, ou le groupement -N(A¹)SO₂A, dans lequel
A représente un reste méthyle, éthyle, n-propyle, isopropyle, n-butyle, isobutyle, sec.-butyle, tertio-butyle, n-pentyle, isopentyle, sec.-pentyle ou tertio-butyle, chacun étant substitué le cas échéant par du fluor ou du chlore,
A représente en outre un reste cyclopropyle, cyclobutyle, cyclopentyle, cyclohexyle, cyclopropylméthyle, cyclobutylméthyle, cyclopentylméthyle ou cyclohexylméthyle dont chacun est substitué le cas échéant par du fluor, du chlore, du brome, des radicaux méthyle et/ou éthyle,
A représente en outre un reste phényle, naphtyle, phénylméthyle, ou phényléthyle dont chacun est substitué le cas échéant par du fluor, du chlore, du brome, un radical cyano, nitro, carboxy, méthyle, trifluorométhyle, méthoxy, éthoxy, difluorométhoxy, trifluorométhoxy, méthylthio, éthylthio, méthylsulfinyle, éthylsulfinyle, méthylsulfonyle, éthylsulfonyle, diméthylaminosulfonyle, méthoxycarbonyle, éthoxycarbonyle, propoxycarbonyle ou phényle,
A représente en outre un reste thiényle, pyrazolyle ou pyridyle, chacun étant substitué le cas échéant par du fluor, du chlore, du brome, un radical cyano, méthyle, éthyle, trifluorométhyle, méthoxy, éthoxy, méthylthio, éthylthio, méthylsulfinyle, éthylsulfinyle, méthylsulfonyle, éthylsulfonyle, méthoxycarbonyle ou éthoxycarbonyle, et
A¹ représente de l'hydrogène, un reste méthyle, éthyle, n-propyle, isopropyle, n-butyle, isobutyle, sec.-butyle, propényle, butényle, propynyle, butynyle, méthoxy, éthoxy, n-propoxy, isopropoxy, n-butoxy, isobutoxy, sec.-butoxy, tertio-butoxy, acétyle, propionyle, méthoxycarbonyle ou éthoxycarbonyle dont chacun est substitué le cas échéant par du fluor, du chlore, un radical méthoxy ou éthoxy, un reste phénylcarbonyle ou phénoxycarbonyle,
R³ représente de l'hydrogène, du fluor, du chlore, du brome, un reste méthyle, trifluorométhyle, éthyle, n-propyle ou isopropyle,
R⁴ représente un reste méthyle, éthyle, n-propyle, ou isopropyle dont chacun est substitué le cas échéant par du fluor ou du chlore, ou forme conjointement avec R³ un groupe triméthylène ou tétraméthylène, et
Z représente l'un des groupements suivants : 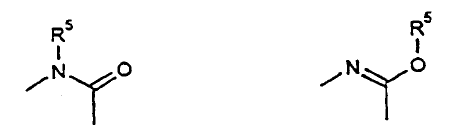 où
R⁵ représente un groupe amino ou un reste méthyle, éthyle, n-propyle, isopropyle, n-butyle, isobutyle ou sec.-butyle substitué par un radical cyano, ainsi que leurs sels avec des bases telles que, par exemple, l'hydroxyde, l'hydrure, l'amidure, ou le carbonate de sodium, de potassium ou de calcium, des alcanolates en C₁ à C₄ de sodium ou de potassium, l'ammoniac, des alkylamines en C₁ à C₄, des di(alkyle en C₁ à C₄)-amines, des tri(alkyle en C₁ à C₄)-amines, ou aussi avec la tris(2-hydroxyéthyl)-amine.

3. Hétérocycles azotés N-cyanarylés de formule générale (I) 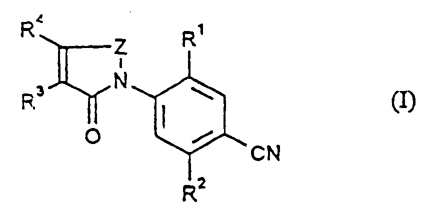 dans laquelle
R¹ représente le fluor,
R² représente du fluor, du chlore, du brome, un reste cyano ou le groupement -N(A¹)SO₂A, dans lequel
A représente un reste de la série des restes alkyle, alcényle ou alcynyle ayant chacun jusqu'à 10 atomes de carbone, chaque reste étant éventuellement substitué par du fluor, du chlore, du brome, un radical cyano ou alkoxy en C₁ à C₄,
A représente en outre un reste cycloalkyle ou un reste cycloalkylalkyle ayant 3 à 8 atomes de carbone dans la partie cycloalkyle et le cas échéant 1 à 4 atomes de carbone dans la partie alkyle, chacun de ces restes étant éventuellement substitué par du fluor, du chlore, du brome, un radical cyano ou alkyle en C₁ à C₄,
A représente en outre un reste aryle ou un reste arylalkyle ayant 6 ou 10 atomes de carbone dans la partie aryle et 1 à 4 atomes de carbone dans la partie alkyle, chacun étant substitué le cas échéant par du fluor, du chlore, du brome, des radicaux cyano, nitro, carboxy, carbamoyle, des radicaux alkyle en C₁ à C₄, alkoxy en C₁ à C₄, alkylthio en C₁ à C₄, alkylsulfinyle en C₁ à C₄, ou alkylsulfonyle en C₁ à C₄, (dont chacun est substitué le cas échéant par du fluor et/ou du chlore), par des radicaux diméthylaminosulfonyle ou diéthylaminosulfonyle, par un radical (alkoxy en C₁ à C₄)-carbonyle, (qui est substitué le cas échéant par du fluor, du chlore, du brome, un radical méthoxy ou éthoxy), par des radicaux phényle, phényloxy ou phénylthio (qui sont substitués chacun le cas échéant par du fluor, du chlore, du brome, des radicaux cyano, méthyle, méthoxy, trifluorométhyle, et/ou trifluorométhoxy),
A représente en outre un reste hétérocyclyle ou hétérocyclyalkyle ayant 2 à 6 atomes de carbone et 1 à 4 atomes d'azote et/ou 1 ou 2 atomes d'oxygène ou de soufre dans la partie hétérocyclyle saturée ou non saturée et le cas échéant 1 à 4 atomes de carbone dans la partie alkyle, chacun étant substitué le cas échéant par du fluor, du chlore, du brome, un radical cyano, nitro, carboxy, carbamoyle, par des radicaux alkyle en C₁ à C₄, alkoxy en C₁ à C₄, alkylthio en C₁ à C₄, alkylsulfinyle en C₁ à C₄, alkylsulfonyle en C₁ à C₄ ou (alkoxy en C₁ à C₄)-carbonyle, (dont chacun est substitué le cas échéant par du fluor, et/ou du chlore), par des radicaux phényle, phénoxy ou phénylthio (dont chacun est substitué le cas échéant par du fluor, du chlore, du brome, des radicaux cyano, méthyle, méthoxy, trifluorométhyle et/ou trifluorométhoxy), et
A¹ represente de l'hydrogène ou des restes alkyle, alcényle, alcynyle, alkoxy, alkylcarbonyle ou alkoxycarbonyle ayant chacun jusqu'à 6 atomes de carbone, dont chacun est éventuellement substitué par un halogène ou un radical alkoxy en C₁ à C₄, ou des restes phénylcarbonyle, napthylcarbonyle, phénylméthylcarbonyle, napthylméthylcarbonyle, phénoxycarbonyle ou naphtyloxycarbonyle (qui sont substitués chacun le cas échéant par du fluor, du chlore, du brome, des radicaux cyano, méthyle, méthoxy, trifluorométhyle et/ou trifluorométhoxy),
R³ représente de l'hydrogène, du fluor, du chlore, du brome, un reste cyano ou un reste alkyle ayant 1 à 6 atomes de carbone éventuellement substitué par du fluor et/ou du chlore,
R⁴ représente un reste alkyle ayant 1 à 6 atomes de carbone éventuellement substitué par du fluor, du chlore, du brome, un radical méthoxy ou éthoxy,
ou forme conjointement avec R³ un groupe alcanediyle ayant 2 à 8 atomes de carbone, et
Z représente l'un des groupements suivants : 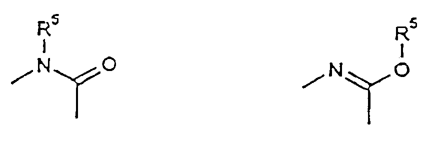 dans lesquels
R⁵ représente un reste alcényle, alcynyle, alkylcarbonyle ou alkoxycarbonyle ayant chacun jusqu'à 6 atomes de carbone et dont chacun est éventuellement substitué par du fluor, du chlore, du brome, un radical cyano, alkoxy en C₁ à C₄ (alkyle en C₁ à C₄)-carbonyle ou (alkoxy en C₁ à C₄)-carbonyle, ainsi que leurs sels avec des bases telles que, par exemple, l'hydroxyde, l'hydrure, l'amidure ou le carbonate de sodium, de potassium ou de calcium ou des alcanolates en C₁ à C₄ de sodium ou de potassium, l'ammoniac, des alkylamines en C₁ à C₄, des di-(alkyle en C₁ à C₄)-amines, des tri-(alkyle en C₁ à C₄)-amines ou aussi avec la tris-(2-hydroxyéthyl)amine.

4. Hétérocycles azotés N-cyanarylés de formule générale (I) suivant la revendication 3, caractérisés en ce que
R¹ représente du fluor,
R² représente du fluor, du chlore, du brome, un groupe cyano ou le groupement -N(A¹)SO₂A, dans lequel
A représente un reste méthyle, éthyle, n-propyle, isopropyle, n-butyle, isobutyle, sec.-butyle, tertio-butyle, n-pentyle, isopentyle, sec.-pentyle ou tertio-pentyle dont chacun est éventuellement substitué par du fluor ou du chlore,
A représente en outre un reste cyclopropyle, cyclobutyle, cylopentyle, cyclohexyle, cyclopropylméthyle, cyclobutylméthyle, cyclopentylméthyle, ou cyclohexylméthyle, dont chacun est substitué le cas échéant par du fluor, du chlore, du brome, des radicaux méthyle et/ou éthyle,
A représente en outre un reste phényle, napthyle, phénylméthyle ou phényléthyle, dont chacun est substitué le cas échéant par du fluor, du chlore, du brome, un radical cyano, nitro, carboxy, méthyle, trifluorométhyle, méthoxy, éthoxy, difluorométhoxy, trifluorométhoxy, méthylthio, éthylthio, méthylsulfinyle, éthylsulfinyle, méthylsulfonyle, éthylsulfonyle, diméthylaminosulfonyle, méthoxycarbonyle, éthoxycarbonyle, propoxycarbonyle ou phényle,
A représente en outre un reste thiényle, pyrazolyle ou pyridyle, dont chacun est substitué le cas échéant par du fluor, du chlore, du brome, un radical cyano, méthyle, éthyle, trifluorométhyle, méthoxy, éthoxy, méthylthio, éthylthio, méthylsulfinyle, éthylsulfinyle, méthylsulfonyle, éthylsulfonyle, méthoxycarbonyle ou éthoxycarbonyle, et
A¹ représente de l'hydrogène, un reste méthyle, éthyle, n-propyle, isopropyle, n-butyle, isobutyle, sec.-butyle, propényle, butényle, propynyle, butynyle, méthoxy, éthoxy, n-propoxy, isopropoxy, n-méthoxy, isobutoxy, sec.-butoxy, tertio-butoxy, acétyle, propionyle, méthoxycarbonyle ou éthoxycarbonyle dont chacun est substitué le cas échéant par du fluor, du chlore, un radical méthoxy ou éthoxy, un reste phénylcarbonyle ou un reste phénoxycarbonyle,
R³ représente de l'hydrogène, du fluor, du chlore, du brome, un reste méthyle, trifluorométhyle, éthyle, n-propyle ou isopropyle,
R⁴ représente un reste méthyle, éthyle, n-propyle ou isopropyle éventuellement substitué par du fluor ou du chlore,
ou forme conjointement avec R³ un groupe triméthylène ou tétraméthylène,
et
Z représente l'un des groupes suivants : 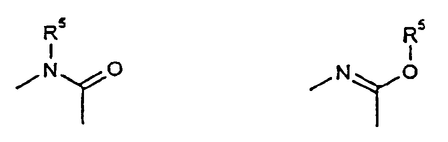 dans lesquels
R⁵ représente un reste propényle, butényle, propynyle, butynyle, acétyle, propionyle, méthoxycarbonyle ou éthoxycarbonyle dont chacun est substitué le cas échéant par du fluor, du chlore ou un radical cyano, ainsi que leurs sels avec des bases telles que l'hydroxyde, l'hydrure, l'amidure ou le carbonate de sodium, de potassium ou de calcium, des alcanolates en C₁ à C₄ de sodium ou de potassium, l'ammoniac, des alkylamines en C₁ à C₄, des di-(alkyle en C₁ à C₄)-amines, des tri-(alkyle en C₁ à C₄)-amines ou aussi avec la tris-(2-hydroxyéthyl)amine.

5. Hétérocycles azotés N-cyanarylés de formules générales (IA) et (IB) 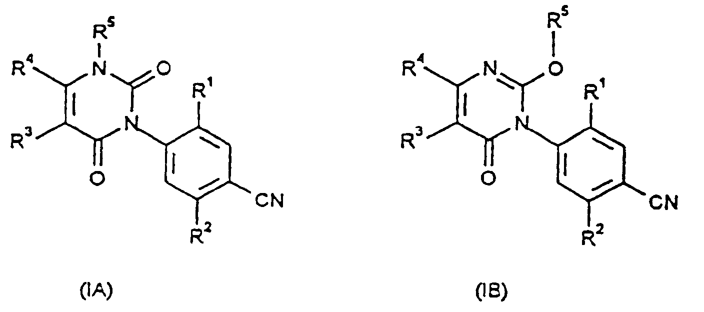 caractérisés en ce que les restes R¹, R², R³, R⁴ et R⁵ ont les définitions suivantes :

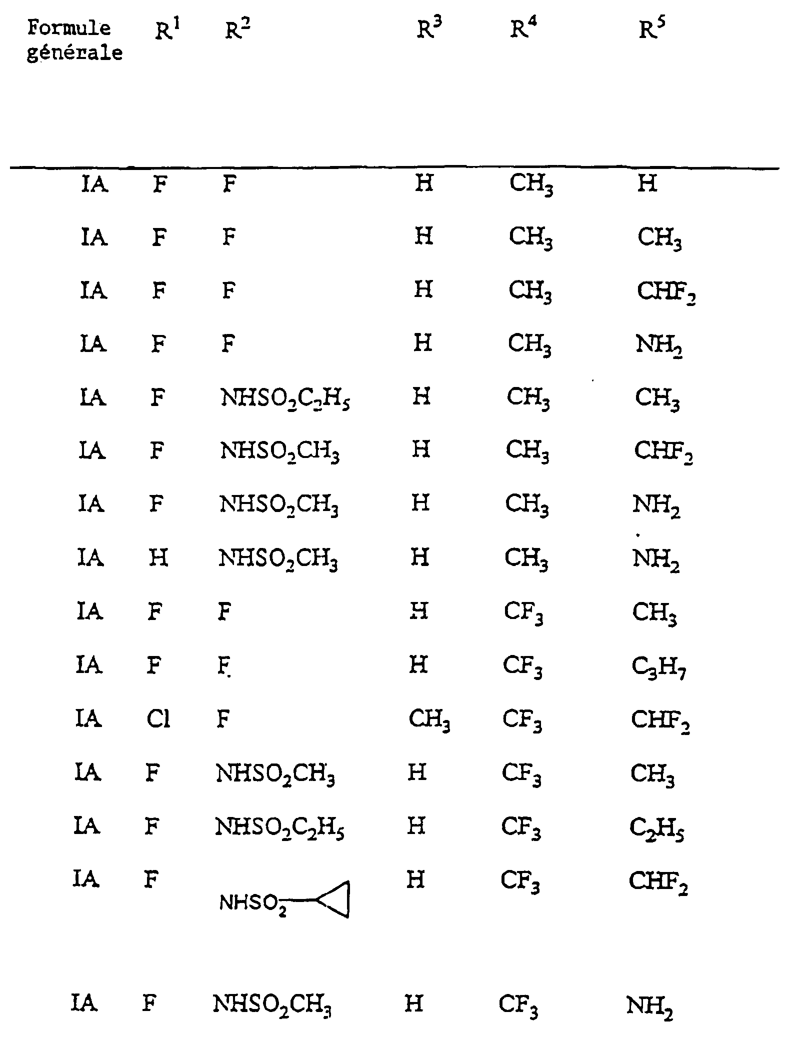

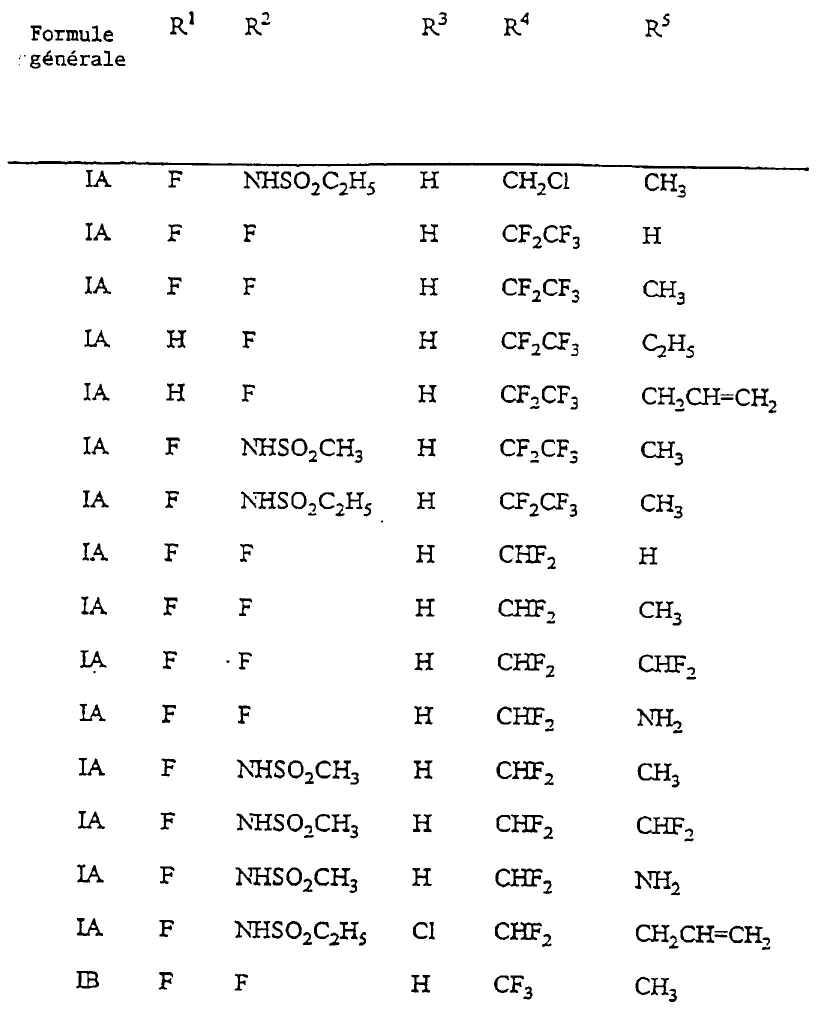

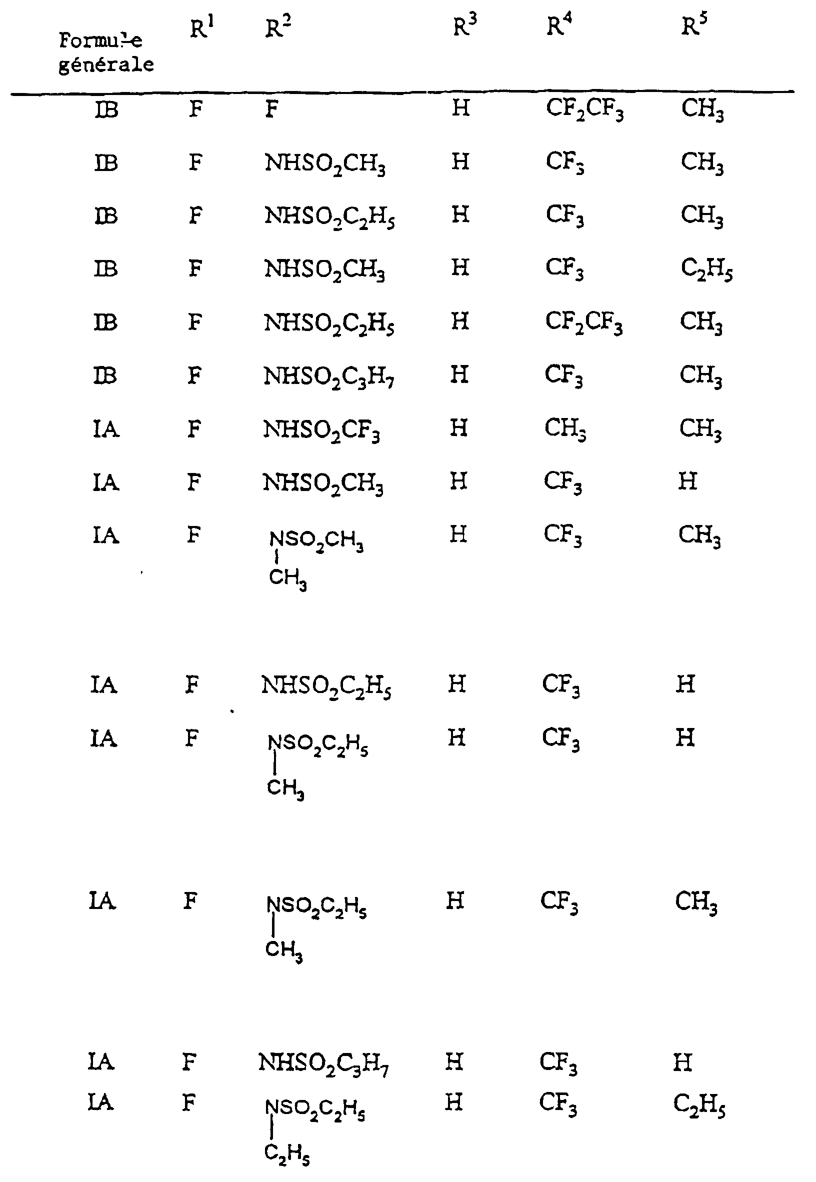

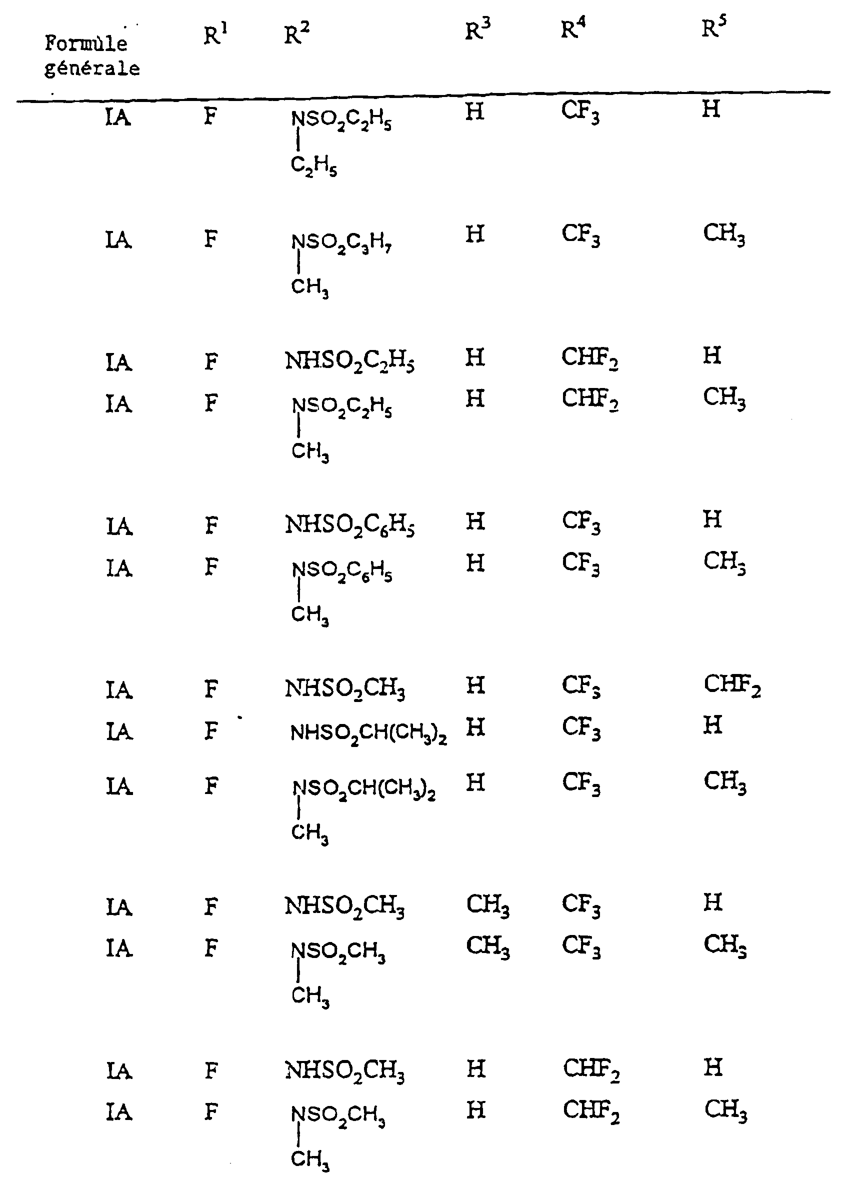

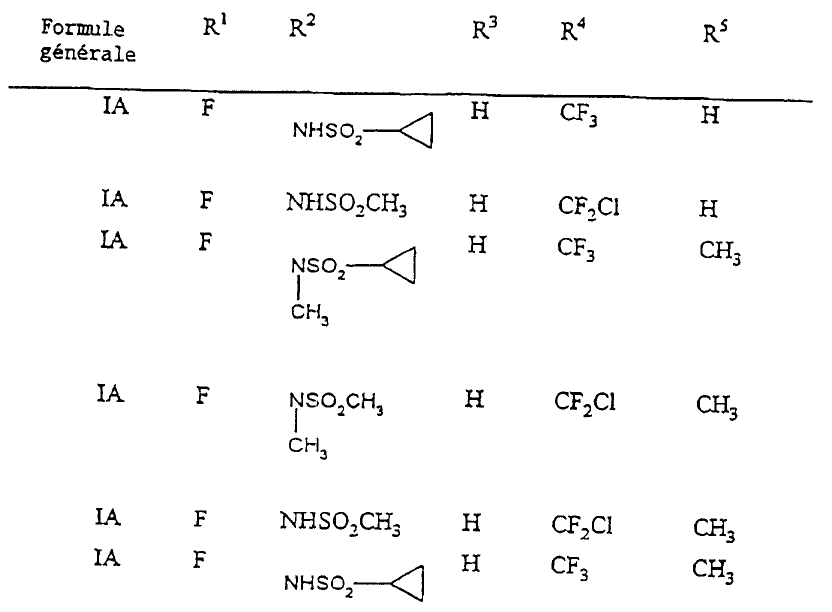

6. Hétérocycles azotés N-cyanarylés de formules

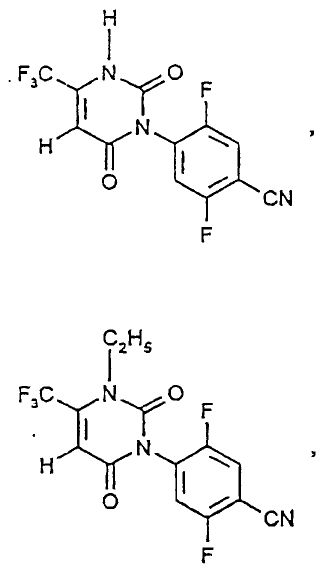

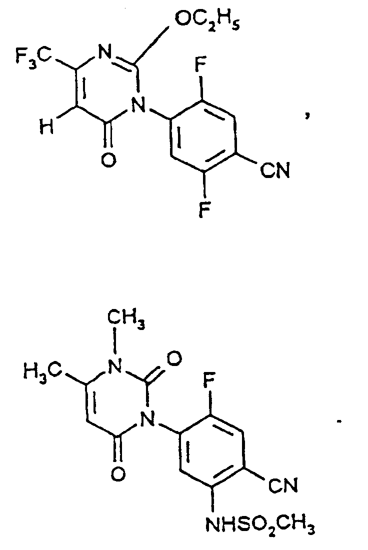

7. Procédé de production d'hétérocycles azotés N-cyanarylés de formule générale (I) 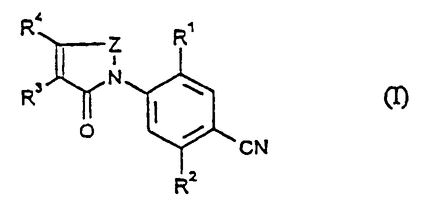 dans laquelle
R¹, R², R³, R⁴ et Z ont les définitions indiquées dans les revendications 1 à 5
ainsi que de leurs sels, caractérisé en ce que :
(a) pour l'obtention de composés de formules (IA) et/ou (IB) dans lesquelles R⁵ est un reste alkyle ayant jusqu'à 6 atomes de carbone, substitué par un radical cyano, alkoxy en C₁ à C₄, (alkyle en C₁ à C₄)-carbonyle ou (alkoxy en C₁ à C₄)-carbonyle ou un reste alcényle, alcynyle, alkylcarbonyle ou alkoxycarbonyle ayant dans chaque cas jusqu'à 6 atomes de carbone et dont chacun est substitué le cas échéant par du fluor, du chlore, du brome, un radical cyano, alkoxy en C₁ à C₄, (alkyle en C₁ à C₄)-carbonyle ou (alkoxy en C₁ à C₄)-carbonyle, de même que R¹, R², R³ et R⁴ ont les définitions indiquées ci-dessus, on fait réagir des hétérocycles azotés N-cyanarylés de formules générales (IA) ou (IB)
dans lesquelles R⁵ représente de l'hydrogène, de même que R¹, R², R³ et R⁴ ont les définitions indiquées ci-dessus, avec des agents alkylants ou respectivement des agents acylants de formules générales (V) ou (VI)
X-R⁵ (V)
R⁵-O-SO₂-O-R⁵ (VI)
dans lesquels R⁵ a la définition indiquée ci-dessus et X dans la formule (V) représente un halogène,
le cas échéant en présence d'un accepteur d'acide et en la présence éventuelle d'un diluant,
ou bien
(b) pour l'obtention de composés de formule (I) dans laquelle R² représente le groupe -N(A¹)SO₂A, de même que A, A¹, R¹, R³, R⁴ et Z ont les définitions indiquées ci-dessus,
on fait réagir des hétérocycles azotés N-cyanarylés de formule générale (I) dans laquelle R² représente du fluor, du chlore, du brome, de même que R¹, R³, R⁴ et Z ont les définitions indiquées ci-dessus,
avec l'ammoniac ou avec des amides de formule générale (VII)
HN(A¹)SO₂A (VII)
dans laquelle
A et A¹ ont les définitions indiquées ci-dessus,
le cas échéant en présence d'un accepteur d'acide et en la présence éventuelle d'un diluant.

8. Compositions herbicides, caractérisées par une teneur en au moins un hétérocycle azoté N-cyanarylé de formule (I) suivant les revendications 1 à 4, des formules (IA) et (IB) suivant la revendication 5 et respectivement des formules suivant la revendication 6.

9. Procédé pour combattre des plantes indésirables, caractérisé en ce qu'on fait réagir sur les plantes et/ou sur leurs milieux des hétérocycles azotés N-cyanarylés de formule générale (I) suivant les revendications 1 à 4, de formules (IA) et (IB) suivant la revendication 5 et respectivement des formules suivant la revendication 6.

10. Utilisation d'hétérocycles azotés N-cyanarylés de formule générale (I) suivant les revendications 1 à 4, des formules (IA) et (IB) suivant la revendication 5 et respectivement des formules suivant la revendication 6 pour combattre des plantes indésirables.

11. Procédé de préparation de compositions herbicides, caractérisé en ce qu'on mélange des hétérocycles azotés N-cyanarylés de formule générale (I) suivant les revendications 1 à 4, des formules (IA) et (IB) suivant la revendication 5 et respectivement des formules suivant la revendication 6 avec des diluants et/ou des agents tensio-actifs.

12. Cyanarylisocyanates de formule générale (III) 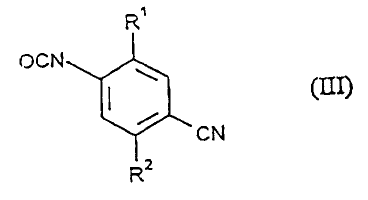 dans laquelle
R¹ représente du fluor,
R² représente du fluor, du chlore, du brome, un groupe cyano ou le groupement -N(A¹)SO₂A, dans lequel
A représente un reste de la série des restes alkyle, alcényle ou alcynyle ayant chacun jusqu'à 10 atomes de carbone, chacun de ces restes étant éventuellement substitué par du fluor, du chlore, du brome, un radical cyano ou alkoxy en C₁ à C₄,
A représente en outre un reste cycloalkyle ou cycloalkylalkyle ayant 3 à 8 atomes de carbone dans la partie cycloalkyle et le cas échéant 1 à 4 atomes de carbone dans la partie aryle, chacun étant substitué le cas échéant par du fluor, du chlore, du brome, un radical cyano ou alkyle en C₁ à C₄,
A représente en outre un reste aryle ou un reste arylalkyle ayant 6 ou 10 atomes de carbone dans la partie aryle et 1 à 4 atomes de carbone dans la partie alkyle, chacun étant substitué le cas échéant par du fluor, du chlore, du brome, un radical cyano, nitro, carboxy, carbamoyle, par des radicaux alkyle en C₁ à C₄, alkoxy en C₁ à C₄, alkylthio en C₁ à C₄, alkylsulfinyle en C₁ à C₄ ou alkylsulfonyle en C₁ à C₄, (qui sont substitués chacun le cas échéant par du fluor et/ou du chlore), par un radical diméthylaminosulfonyle ou diéthylaminosulfonyle, par un radical (alkoxy en C₁ à C₄)-carbonyle (qui est substitué le cas échéant par du fluor, du chlore, du brome, un radical méthoxy ou éthoxy), par des radicaux phényle, phényloxy ou phénylthio (qui sont substitués chacun le cas échéant par du fluor, du chlore, du brome, des radicaux cyano, méthyle, méthoxy, trifluorométhyle et/ou trifluorométhoxy),
A représente en outre un radical hétérocyclyle ou hétérocyclylalkyle ayant 2 à 6 atomes de carbone et 1 à 4 atomes d'azote et/ou 1 ou 2 atomes d'oxygène ou de soufre dans la partie hétérocyclyle saturée ou non saturée et le cas échéant 1 à 4 atomes de carbone dans la partie alkyle, chacun étant substitué le cas échéant par du fluor, du chlore, du brome, un radical cyano, nitro, carboxy, carbamoyle, par des radicaux alkyle en C₁ à C₄, alkoxy en C₁ à C₄, alkylthio en C₁ à C₄, alkylsulfinyle en C₁ à C₄, alkylsulfonyle en C₁ à C₄ ou (alkoxy en C₁ à C₄)-carbonyle (qui sont substitués chacun le cas échéant par du fluor et/ou du chlore), par des radicaux phényle, phénoxy ou phénylthio (qui sont substitués chacun le cas échéant par du fluor, du chlore, du brome, des radicaux cyano, méthyle méthoxy, trifluorométhyle et/ou trifluorométhoxy), et
A¹ représente de l'hydrogène ou un reste alkyle, alcényle, alcynyle, alkoxy, alkylcarbonyle ou alkoxycarbonyle ayant chacun jusqu'à 6 atomes de carbone et dont chacun est substitué le cas échéant par un halogène ou un radical alkoxy en C₁ à C₄, ou des restes phénylcarbonyle, napthylcarbonyle, phénylméthylcarbonyle, naphtylméthylcarbonyle, phénoxycarbonyle ou naphtyloxycarbonyle (dont chacun est substitué le cas échéant par du fluor, du chlore, du brome, des radicaux cyano, méthyle, méthoxy, trifluorométhyle et/ou trifluorométhoxy).

13. Cyanaryluréthannes de formule générale (IV) 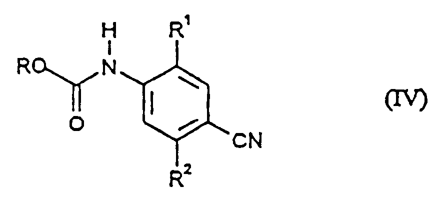 dans laquelle
R¹ représente du fluor,
R² représente du fluor, du chlore, du brome, un groupe cyano ou le groupement -N(A¹)S0₂A, dans lequel
A représente un reste de la série des restes alkyle, alcényle ou alcynyle ayant chacun jusqu'à 10 atomes de carbone, chaque reste étant éventuellement substitué par du fluor, du chlore, du brome, un radical cyano ou alkoxy en C₁ à C₄,
A représente en outre un reste cyclaolkyle ou cycloalkylalkyle ayant 3 à 8 atomes de carbone dans la partie cycloalkyle ou le cas échéant 1 à 4 atomes de carbone dans la partie alkyle, chacun étant substitué le cas échéant par du fluor, du chlore, du brome, un radical cyano ou alkyle en C₁ à C₄,
A représente en outre un reste aryle ou un reste arylalkyle de 6 à 10 atomes de carbone dans la partie aryle et 1 à 4 atomes de carbone dans la partie alkyle, chacun étant substitué le cas échéant par du fluor, du chlore, du brome, un radical cyano, nitro, carboxy, carbamoyle, par des radicaux alkyle en C₁ à C₄, alkoxy en C₁ à C₄, alkylthio en C₁ à C₄, alkylsulfinyle en C₁ à C₄ ou alkylsulfonyle en C₁ à C₄ (dont chacun est substitué le cas échéant par du fluor et/ou du chlore), par un radical diméthylaminosulfonyle ou diéthylaminosulfonyle, par un radical (alkoxy en C₁ à C₄)-carbonyle, (qui est substitué le cas échéant par du fluor, du chlore, du brome, un radical méthoxy ou éthoxy), par des radicaux phényle, phényloxy ou phénylthio (qui sont substitués chacun le cas échéant par du fluor, du chlore, du brome, des radicaux cyano, méthyle, méthoxy, trifluorométhyle et/ou trifluorométhoxy),
A représente en outre un reste hétérocyclyle ou hétérocyclylalkyle ayant 2 à 6 atomes de carbone et 1 à 4 atomes d'azote et/ou 1 ou 2 atomes d'oxygène ou de soufre dans la partie hétérocyclyle saturé ou non saturé et le cas échéant 1 à 4 atomes de carbone dans la partie alkyle, chacun étant substitué le cas échéant par du fluor, du chlore, du brome, un radical cyano, nitro, carboxy, carbamoyle, par des radicaux alkyle en C₁ à C₄, alkoxy en C₁ à C₄, alkylthio en C₁ à C₄, alkylsulfinyle en C₁ à C₄, alkylsulfonyle en C₁ à C₄ ou (alkoxy en C₁ à C₄)-carbonyle (dont chacun est substitué le cas échéant par du fluor et/ou du chlore), par des radicaux phényle, phénoxy ou phénylthio (qui sont substitués chacun le cas échéant par du fluor, du chlore, du brome, des radicaux cyano, méthyle méthoxy, trifluorométhyle et/ou trifluorométhoxy), et
A¹ représente de l'hydrogène ou des restes alkyle, alcényle, alcynyle, alkoxy, alkylcarbonyle ou alkoxycarbonyle ayant chacun jusqu'à 6 atomes de carbone, chacun étant substitué le cas échéant par un halogène ou un radical alkoxy en C₁ à C₄, ou des restes phénylcarbonyle, naphtylcarbonyle, phénylméthylcarbonyle, naphtylméthylcarbonyle, phénoxycarbonyle ou naphtyloxycarbonyle (qui sont substitués chacun le cas échéant par du fluor, du chlore, du brome, des radicaux cyano, méthyle, méthoxy, trifluorométhyle et/ou trifluorométhoxy) et
R représente un reste alkyle en C₁ à C₄, phényle ou benzyle.
